# EUROPEAN PATENT APPLICATION

(11) **EP 4 190 771 A1**
(43) Date of publication of application: **07.06.2023**
(21) Application number: 21850661.6
(22) Date of filing: 20.07.2021
(51) Int. Cl.: C07D 307/77, C07D 493/04, C07D 405/04, H01L 51/00, H01L 51/50, C09K 11/06

(54) **NOVEL HETEROCYCLIC COMPOUND AND ORGANIC LIGHT-EMITTING DEVICE COMPRISING SAME**

(30) Priority: 29.07.2020 KR 20200094575; 27.08.2020 KR 20200108859; 19.07.2021 KR 20210094345
(71) Applicant: SFC Co., Ltd., Cheongju-si, Chungcheongbuk-do 28122 (KR)
(72) Inventor: CHA, Soon-wook, Cheongju-si Chungcheongbuk-do 28122 (KR); KIM, Sung-woo, Cheongju-si Chungcheongbuk-do 28122 (KR); LEE, Ji-won, Cheongju-si Chungcheongbuk-do 28122 (KR); LEE, Se-jin, Cheongju-si Chungcheongbuk-do 28122 (KR); KIM, Si-in, Cheongju-si Chungcheongbuk-do 28122 (KR); PARK, Seok-bae, Cheongju-si Chungcheongbuk-do 28122 (KR); KIM, Hee-dae, Cheongju-si Chungcheongbuk-do 28122 (KR); CHOI, Yeong-tae, Cheongju-si Chungcheongbuk-do 28122 (KR); LEE, Seung-soo, Cheongju-si Chungcheongbuk-do 28122 (KR); KIM, Ji-yung, Cheongju-si Chungcheongbuk-do 28122 (KR); KIM, Kyeong-hyeon, Cheongju-si Chungcheongbuk-do 28122 (KR); KIM, Kyung-tae, Cheongju-si Chungcheongbuk-do 28122 (KR); KIM, Myeong-jun, Cheongju-si Chungcheongbuk-do 28122 (KR); LEE, Tae-gyun, Cheongju-si Chungcheongbuk-do 28122 (KR); KIM, Joon-ho, Cheongju-si Chungcheongbuk-do 28122 (KR)
(74) Representative: Epping - Hermann - Fischer
(86) International application number: PCT/KR2021/009354
(87) International publication number: WO 2022/025511

(57) **Abstract**

Disclosed herein are pyrene heterocyclic compounds useful for an organic light-emitting diode and an organic light-emitting diode including same. More specifically, compound represented by [Chemical Formula A] or [Chemical Formula B] and an organic light-emitting diode are provided. [Chemical Formula A] and [Chemical Formula B] are as defined in the Description.

## Description

### Technical Field

The present disclosure relates to a novel heterocyclic compound for an organic light-emitting diode and, more particularly, to a novel heterocyclic compound available as a host material of a light-emitting layer in an organic light-emitting diode, which shows excellent diode characteristics including high luminous efficiency and longevity, and an organic light-emitting diode comprising the same.

### Background Art

Organic light-emitting diodes (OLEDs), based on self-luminescence, enjoy the advantage of having a wide viewing angle and being able to be made thinner and lighter than liquid crystal displays (LCDs). In addition, an OLED display exhibits a very fast response time. Accordingly, OLEDs find applications in the full-color display field or the illumination field.

In general, the term "organic light-emitting phenomenon" refers to a phenomenon in which electrical energy is converted to light energy using an organic material. An OLED using the organic light phenomenon has a structure usually comprising an anode, a cathode, and an organic material layer interposed therebetween. In this regard, the organic material layer may be, for the most part, of a multilayer structure consisting of different materials, for example, a hole injection layer, a hole transport layer, a light-emitting layer, an electron transport layer, and an electron injection layer, in order to improve the efficiency and stability of the organic light-emitting diode. In the organic light-emitting diode having such a structure, when a voltage is applied between the two electrodes, a hole injected from the anode migrates to the organic layer while an electron is released from the cathode and moves toward the organic layer. In the luminescent zone, the hole and the electron recombine to produce an exciton. When the exciton returns to the ground state from the excited state, the molecule of the organic layer emits light. Such an organic light-emitting diode is known to have characteristics such as self-luminescence, high luminance, high efficiency, low driving voltage, a wide viewing angle, high contrast, and high-speed response.

Materials used as organic layers in OLEDs may be divided into luminescent materials and charge transport materials, for example, a hole injection material, a hole transport material, an electron injection material, and an electron transport material. As for the luminescent materials, there are two main families of OLED: those based on small molecules and those employing polymers. The light-emitting mechanism forms the basis for classification of the luminescent materials as fluorescent or phosphorescent materials, which use excitons in singlet and triplet states, respectively.

Meanwhile, when a single material is employed as the luminescent material, intermolecular actions cause the wavelength of maximum luminescence to shift toward a longer wavelength, decreasing color purity or attenuating light with the consequent reduction in the efficiency of the diode. In this regard, a host-dopant system may be used as a luminescent material so as to increase the color purity and the light emission efficiency through energy transfer.

This is based on the principle whereby, when a dopant is smaller in energy band gap than a host accounting for the light-emitting layer, the addition of a small amount of the dopant to the host generates excitons from the light-emitting layer so that the excitons are transported to the dopant, emitting light at high efficiency. Here, the light of desired wavelengths can be obtained depending on the kinds of dopants because the wavelength of the host moves to the wavelength range of the dopant.

With respect to related arts pertaining to heterocyclic compounds as host compounds available in light-emitting layers, reference may be made to Korean Patent No. 10-2017-0116843 A (October 20, 2017), which discloses a compound structured to have a benzofluorene ring fused with a nitrogenbearing heteroring and an organic light-emitting diode comprising the same and Korean Patent No. 10-2017-0055743 A (May 22, 2017), which describes a compound to an aryl substituent or a heteroaryl substituent is coupled to a fused fluorene ring bearing heteroatoms such as oxygen, nitrogen, and sulfur and an organic light-emitting diode comprising the same.

In spite of various kinds of compounds prepared for use in luminescent layers of organic light-emitting diodes inclusive of the related art, there is still the continued need to develop novel materials that can be applied to organic light-emitting diodes and are characterized by high efficiency and longevity, and organic light-emitting diodes comprising same.

### Disclosure

### Technical Problem

Accordingly, a first aspect of the present disclosure is to provide a novel heterocyclic compound useful as a host material for a light-emitting layer in an organic light-emitting diode.

In addition, a second aspect of the present disclosure is to provide an organic light-emitting diode (OLED) having the heterocyclic compound applied as a host material therein and thus exhibiting high emission efficiency and longevity.

### Technical Solution

According to an aspect thereof, the present disclosure provides a heterocyclic compound represented by the following [Chemical Formula A] or [Chemical Formula B]: 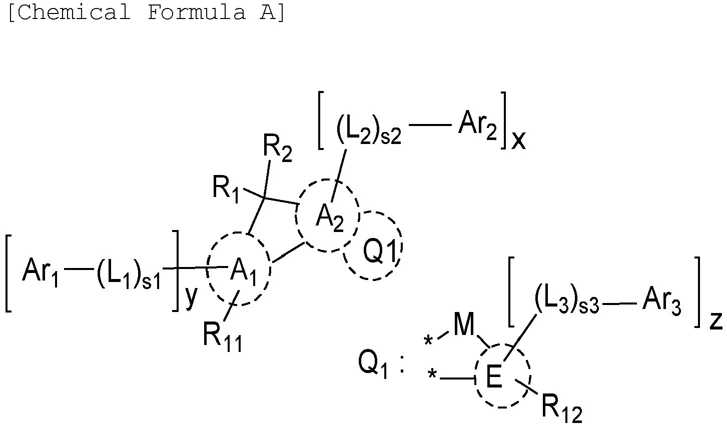 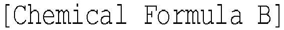 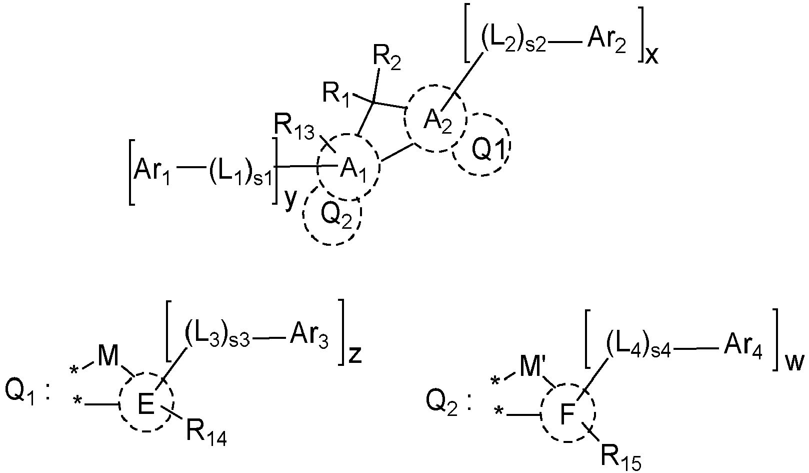
wherein, A₁, A₂, E, and F, which may be same or different, are each independently a substituted or unsubstituted aromatic hydrocarbon ring of 6 to 50 carbon atoms;
wherein two adjacent carbon atoms of the aromatic ring A₁ and two adjacent carbon atoms of the aromatic ring A₂ form a 5-membered fused ring together with the carbon atom having substituents R₁ and R₂ linked thereto;
linkers L₁ to L₄, which may be same or different, are each independently a single bond or a substituted or unsubstituted arylene of 6 to 20 carbon atoms;
M is one selected from N-R₃, CR₄R₅, O, and S,
M' is one selected from N-R₆, CR₇R₈, O, and S;
R1 to R8 and R11 to R15, which may be same or different, are each independently selected from a hydrogen atom, a deuterium atom, a substituted or unsubstituted alkyl of 1 to 30 carbon atoms, a substituted or unsubstituted aryl of 6 to 50 carbon atoms, a substituted or unsubstituted cycloalkyl of 3 to 30 carbon atoms, a substituted or unsubstituted heterocycloalkyl of 2 to 30 carbon atoms, a substituted or unsubstituted heteroaryl of 2 to 50 carbon atoms, a substituted or unsubstituted alkylsilyl of 1 to 30 carbon atoms, a substituted or unsubstituted arylsilyl of 6 to 30 carbon atoms, a cyano, a nitro, and a halogen,
with the proviso that R₁ and R₂ may be bonded to each other to form a mono- or polycyclic aliphatic or aromatic ring;
s1 to s4, which may be same or different, are each independently an integer of 1 to 3, with the proviso that when any of them is 2 or greater, the corresponding linkers L₁ to L₄ may be same or different,
x, y, z, and w, which may be same or different, are each independently an integer of 0 or 1, satisfying the following relationship:

   x + y + z = 1 or x + y + z = 2 in Chemical Formula A,

   x + y + z + w = 1 or x + y + z + w = 2 in Chemical Formula B,
two adjacent carbon atoms of the A₂ ring moiety of Chemical Formula A occupy respective positions * of Structural Formula Q₁ to form a fused ring,
two adjacent carbon atoms of the A₁ ring moiety of Chemical Formula B occupy respective positions * of structural Formula Q₂ to form a fused ring, and two adjacent carbon atoms of the A₂ ring moiety of Chemical Formula B occupy respective positions * of Structural Formula Q₁ to form a fused ring,
Ar₁ to Ar₄, which may be same or different, are each independently represented by the following Structural Formula C; 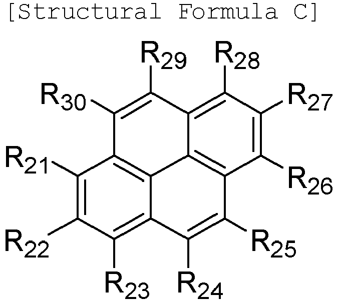
wherein R₂₁ to R₃₀, which may be same or different, are each independently any one selected from a hydrogen atom, a deuterium atom, a substituted or unsubstituted alkyl of 1 to 30 carbon atoms, a substituted or unsubstituted aryl of 6 to 50 carbon atoms, a substituted or unsubstituted cycloalkyl of 3 to 30 carbon atoms, a substituted or unsubstituted heteroaryl of 2 to 50 carbon atoms, a substituted or unsubstituted heterocycloalkyl of 2 to 30 carbon atoms, a substituted or unsubstituted alkylsilyl of 1 to 30 carbon atoms, a substituted or unsubstituted arylsilyl of 5 to 30 carbon atoms, a cyano, a nitro, and a halogen,
any one of R₂₁ to R₃₀ being a single bond to any one of the linkers L₁ to L₄;
wherein, the term "substituted" in the expression "a substituted or unsubstituted" used for the compound of Chemical Formulas A and B and Structural Formula C means having at least one substituent selected from the group consisting of a deuterium atom, a cyano, a halogen, a hydroxy, a nitro, an alkyl of 1 to 24 carbon atoms, a halogenated alkyl of 1 to 24 carbon atoms, an alkenyl of 1 to 24 carbon atoms, an alkynyl of 1 to 24 carbon atoms, a cycloalkyl of 3 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 2 to 24 carbon atoms, an alkoxy of 1 to 24 carbon atoms, an alkylamino of 1 to 24 carbon atoms, a diarylamino of 12 to 24 carbon atoms, a diheteroarylamino of 2 to 24 carbon atoms, an aryl(heteroaryl)amino of 7 to 24 carbon atoms, an alkylsilyl of 1 to 24 carbon atoms, an arylsilyl of 6 to 24 carbon atoms, an aryloxy of 6 to 24 carbon atoms, and an arylthionyl of 6 to 24 carbon atoms.

### Advantageous Effects

When used as a host material in an organic light-emitting diode, the novel heterocyclic compound according to the present disclosure allows for the provision of an organic light-emitting diode that has higher luminous efficiency and longevity, compared to conventional organic light-emitting diodes.

### Brief Description of the Drawings

Figure is a diagram of the structure of an organic light-emitting diode according to some embodiments of the present disclosure.

### Best Mode for Carrying out the Invention

Below, a detailed description will be given of the present disclosure. In each drawing of the present disclosure, sizes or scales of components may be enlarged or reduced from their actual sizes or scales for better illustration, and known components may not be depicted therein to clearly show features of the present disclosure. Therefore, the present disclosure is not limited to the drawings. When describing the principle of the embodiments of the present disclosure in detail, details of well-known functions and features may be omitted to avoid unnecessarily obscuring the presented embodiments.

In the drawing, for convenience of description, sizes of components may be exaggerated for clarity. For example, since sizes and thicknesses of components in drawings are arbitrarily shown for convenience of description, the sizes and thicknesses are not limited thereto. Furthermore, throughout the description, the terms "on" and "over" are used to refer to the relative positioning, and mean not only that one component or layer is directly disposed on another component or layer but also that one component or layer is indirectly disposed on another component or layer with a further component or layer being interposed therebetween. Also, spatially relative terms, such as "below", "beneath", "lower", and "between" may be used herein for ease of description to refer to the relative positioning.

Throughout the specification, when a portion may "include" a certain constituent element, unless explicitly described to the contrary, it may not be construed to exclude another constituent element but may be construed to further include other constituent elements. Further, throughout the specification, the word "on" means positioning on or below the object portion, but does not essentially mean positioning on the lower side of the object portion based on a gravity direction.

The present disclosure provides a heterocyclic compound represented by the following Chemical Formula A or Chemical Formula B: 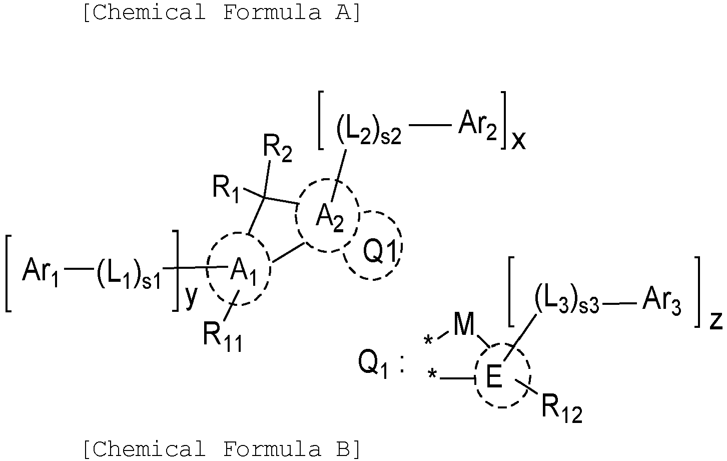 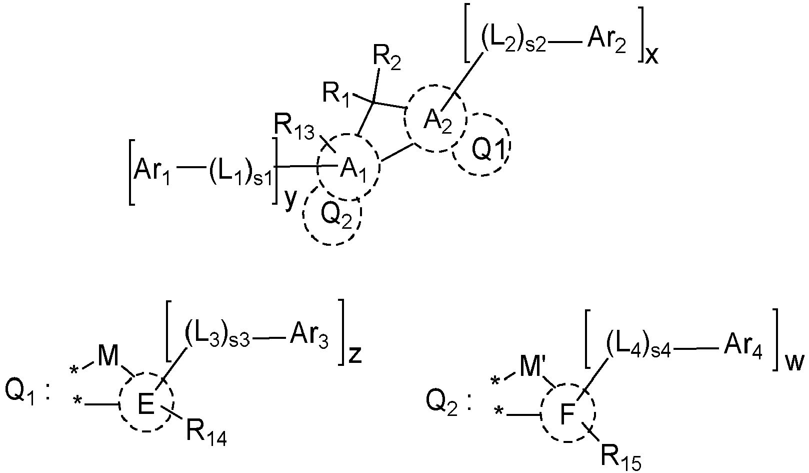 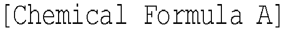 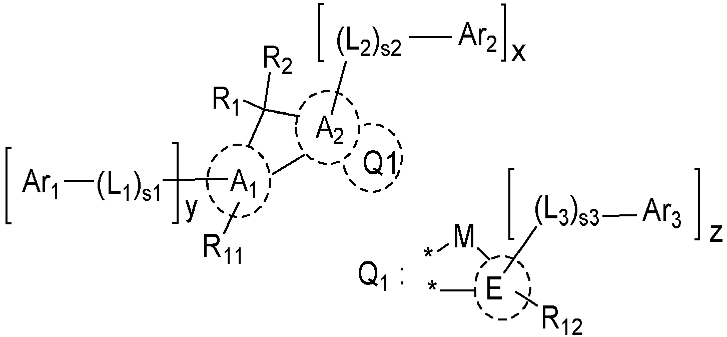 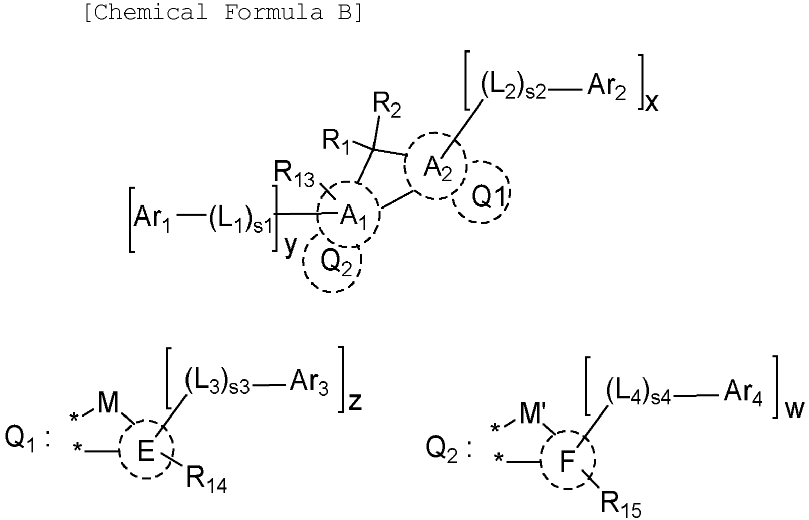
wherein, A₁, A₂, E, and F, which may be same or different, are each independently a substituted or unsubstituted aromatic hydrocarbon ring of 6 to 50 carbon atoms;
wherein two adjacent carbon atoms of the aromatic ring A₁ and two adjacent carbon atoms of the aromatic ring A₂ form a 5-membered fused ring together with the carbon atom having substituents R₁ and R₂ linked thereto;
linkers L₁ to L₄, which may be same or different, are each independently a single bond or a substituted or unsubstituted arylene of 6 to 20 carbon atoms;
M is one selected from N-R₃, CR₄R₅, O, and S,
M' is one selected from N-R₆, CR₇R₈, O, and S;
R1 to R8 and R11 to R15, which may be same or different, are each independently selected from a hydrogen atom, a deuterium atom, a substituted or unsubstituted alkyl of 1 to 30 carbon atoms, a substituted or unsubstituted aryl of 6 to 50 carbon atoms, a substituted or unsubstituted cycloalkyl of 3 to 30 carbon atoms, a substituted or unsubstituted heterocycloalkyl of 2 to 30 carbon atoms, a substituted or unsubstituted heteroaryl of 2 to 50 carbon atoms, a substituted or unsubstituted alkylsilyl of 1 to 30 carbon atoms, a substituted or unsubstituted arylsilyl of 6 to 30 carbon atoms, a cyano, a nitro, and a halogen,
with the proviso that R₁ and R₂ may be bonded to each other to form a mono- or polycyclic aliphatic or aromatic ring;
s1 to s4, which may be same or different, are each independently an integer of 1 to 3, with the proviso that when any of them is 2 or greater, the corresponding linkers L₁ to L₄ may be same or different,
x, y, z, and w, which may be same or different, are each independently an integer of 0 or 1, satisfying the following relationship:

   x + y + z = 1 or x + y + z = 2 in Chemical Formula A,

   x + y + z + w = 1 or x + y + z + w = 2 in Chemical Formula B,
two adjacent carbon atoms of the A₂ ring moiety of Chemical Formula A occupy respective positions * of Structural Formula Q₁ to form a fused ring,
two adjacent carbon atoms of the A₁ ring moiety of Chemical Formula B occupy respective positions * of structural Formula Q₂ to form a fused ring, and two adjacent carbon atoms of the A₂ ring moiety of Chemical Formula B occupy respective positions * of Structural Formula Q₁ to form a fused ring,
Ar₂ to Ar₄, which may be same or different, are each independently represented by the following Structural Formula C; 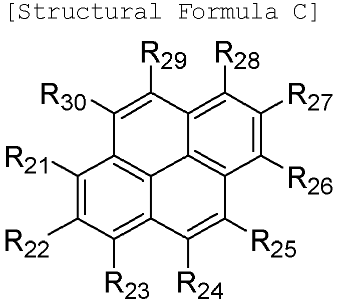
wherein R₂₁ to R₃₀, which may be same or different, are each independently any one selected from a hydrogen atom, a deuterium atom, a substituted or unsubstituted alkyl of 1 to 30 carbon atoms, a substituted or unsubstituted aryl of 6 to 50 carbon atoms, a substituted or unsubstituted cycloalkyl of 3 to 30 carbon atoms, a substituted or unsubstituted heteroaryl of 2 to 50 carbon atoms, a substituted or unsubstituted heterocycloalkyl of 2 to 30 carbon atoms, a substituted or unsubstituted alkylsilyl of 1 to 30 carbon atoms, a substituted or unsubstituted arylsilyl of 5 to 30 carbon atoms, a cyano, a nitro, and a halogen,
any one of R₂₁ to R₃₀ being a single bond to any one of the linkers L₁ to L₄;
wherein, the term "substituted" in the expression "a substituted or unsubstituted" used for the compound of Chemical Formulas A and B and Structural Formula C means having at least one substituent selected from the group consisting of a deuterium atom, a cyano, a halogen, a hydroxy, a nitro, an alkyl of 1 to 24 carbon atoms, a halogenated alkyl of 1 to 24 carbon atoms, an alkenyl of 1 to 24 carbon atoms, an alkynyl of 1 to 24 carbon atoms, a cycloalkyl of 3 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 2 to 24 carbon atoms, an alkoxy of 1 to 24 carbon atoms, an alkylamino of 1 to 24 carbon atoms, a diarylamino of 12 to 24 carbon atoms, a diheteroarylamino of 2 to 24 carbon atoms, an aryl(heteroaryl)amino of 7 to 24 carbon atoms, an alkylsilyl of 1 to 24 carbon atoms, an arylsilyl of 6 to 24 carbon atoms, an aryloxy of 6 to 24 carbon atoms, and an arylthionyl of 6 to 24 carbon atoms.

The expression indicating the number of carbon atoms, such as "a substituted or unsubstituted alkyl of 1 to 30 carbon atoms", "a substituted or unsubstituted aryl of 5 to 50 carbon atoms", etc. means the total number of carbon atoms of, for example, the alkyl or aryl radical or moiety alone, exclusive of the number of carbon atoms of substituents attached thereto. For instance, a phenyl group with a butyl at the para position falls within the scope of an aryl of 6 carbon atoms, even though it is substituted with a butyl radical of 4 carbon atoms.

As used herein, the term "aryl" means an organic radical derived from an aromatic hydrocarbon by removing one hydrogen that is bonded to the aromatic hydrocarbon. The aromatic system may include a fused ring that is formed by adjacent substituents on the aryl radical.

Concrete examples of the aryl include phenyl, o-biphenyl, m-biphenyl, p-biphenyl, o-terphenyl, m-terphenyl, p-terphenyl, naphthyl, anthryl, phenanthryl, pyrenyl, indenyl, fluorenyl, tetrahydronaphthyl, perylenyl, chrysenyl, naphthacenyl, and fluoranthenyl. At least one hydrogen atom of the aryl may be substituted by a deuterium atom, a halogen atom, a hydroxy, a nitro, a cyano, a silyl, an amino (-NH₂, -NH(R), -N(R') (R") wherein R' and R" are each independently an alkyl of 1 to 10 carbon atoms, in this case, called "alkylamino"), an amidino, a hydrazine, a hydrazone, a carboxyl, a sulfonic acid, a phosphoric acid, an alkyl of 1 to 24 carbon atoms, a halogenated alkyl of 1 to 24 carbon atoms, an alkenyl of 2 to 24 carbon atoms, an alkynyl of 2 to 24 carbon atoms, a heteroalkyl of 1 to 24 carbon atoms, an aryl of 6 to 24 carbon atoms, an arylalkyl of 6 to 24 carbon atoms, a heteroaryl of 2 to 24 carbon atoms, or a heteroarylalkyl of 2 to 24 carbon atoms.

The substituent "heteroaryl" used in the compound of the present disclosure means a hetero aromatic radical of 2 to 24 carbon atoms, bearing one to three heteroatoms selected from among N, O, P, Si, S, Ge, Se, and Te. In the aromatic radical, two or more rings may be fused. One or more hydrogen atoms on the heteroaryl may be substituted by the same substituents as on the aryl.

In addition, the term "heteroaromatic ring", as used herein, refers to an aromatic hydrocarbon ring bearing at least one heteroatom as an aromatic ring member. In the heteroaromatic ring, one to three carbon atoms of the aromatic hydrocarbon may be substituted by at least one selected particularly from N, O, P, Si, S, Ge, Se, and Te.

As used herein, the term "alkyl" refers to an alkane missing one hydrogen atom and includes linear or branched structures. Examples of the alkyl substituent useful in the present disclosure include methyl, ethyl, propyl, isopropyl, isobutyl, sec-butyl, tert-butyl, pentyl, iso-amyl, hexyl, and the like. At least one hydrogen atom of the alkyl may be substituted by the same substituent as in the aryl.

The term "cyclo" as used in substituents of the compounds of the present disclosure, such as cycloalkyl, etc., refers to a structure responsible for a mono- or polycyclic ring of saturated hydrocarbons. Concrete examples of cycloalkyl radicals include cyclopropyl, cyclobutyl, cyclopentyl, cyclohexyl, methylcyclopentyl, methylcyclohexyl, ethylcyclopentyl, ethylcyclohexyl, adamantyl, dicyclopentadienyl, decahydronaphthyl, norbornyl, bornyl, isobornyl, and so on. One or more hydrogen atoms on the cycloalkyl may be substituted by the same substituents as on the aryl.

The term "alkoxy" as used in the compounds of the present disclosure refers to an alkyl or cycloalkyl singularly bonded to oxygen. Concrete examples of the alkoxy include methoxy, ethoxy, propoxy, isobutoxy, sec-butoxy, pentoxy, iso-amyloxy, hexyloxy, cyclobutyloxy, cyclopentyloxy, adamantyloxy, dicyclopentyloxy, bornyloxy, isobornyloxy, and the like. One or more hydrogen atoms on the alkoxy may be substituted by the same substituents as on the aryl.

Concrete examples of the arylalkyl used in the compounds of the present disclosure include phenylmethyl (benzyl), phenylethyl, phenylpropyl, naphthylmethyl, naphthylethyl, and the like. One or more hydrogen atoms on the arylalkyl may be substituted by the same substituents as on the aryl.

Concrete examples of the silyl radicals used in the compounds of the present disclosure include trimethylsilyl, triethylsilyl, triphenylsilyl, trimethoxysilyl, dimethoxyphenylsilyl, diphenylmethylsilyl, diphenylvinlysilyl, methylcyclobutylsilyl, and dimethyl furylsilyl. One or more hydrogen atoms on the silyl may be substituted by the same substituents as on the aryl.

As used herein, the term "alkenyl" refers to a hydrocarbon group containing a carbon-carbon double bond and the term "alkynyl" refers to a hydrocarbon group containing a carbon-carbon triple bond.

As used herein, the term "alkylene" refers to an organic radical regarded as derived from an alkane by removing two hydrogen atoms from one carbon atom for methylene or different carbon atoms for ethylene or higher, such as propylene, isopropylene, isobutylene, sec-butylene, tert-butylene, pentylene, iso-amylene, hexylene, and the like. One or more hydrogen atoms on the alkylene may be substituted by the same substituents as on the aryl.

As used herein, the term "diarylamino" refers to an amine group having aforementioned, two identical or different aryl radicals bonded to the nitrogen atom thereof, the term "diheteroarylamino" to an amine group having two identical or different heteroaryl radicals bonded to the nitrogen atom thereof, and the term "aryl(heteroaryl)amino" to an amine group having the aryl radical and the heteroaryl radical each bonded to the nitrogen atom thereof.

In a specific embodiment of the present disclosure, the term "substituted" in the expression "a substituted or unsubstituted" used for the compounds of Chemical Formulas A and B and Structural Formula C means having at least one substituent selected from the group consisting of a deuterium atom, a cyano, a halogen, a hydroxy, a nitro, an alkyl of 1 to 12 carbon atoms, a halogenated alkyl of 1 to 12 carbon atoms, an alkenyl of 2 to 12 carbon atoms, an alkynyl of 2 to 12 carbon atoms, a cycloalkyl of 3 to 12 carbon atoms, a heteroalkyl of 1 to 12 carbon atoms, an aryl of 6 to 18 carbon atoms, an arylakyl of 7 to 20 carbon atoms, an alkylaryl of 7 to 20 carbon atoms, a heteroaryl of 2 to 18 carbon atoms, a heteroarylalkyl of 2 to 18 carbon atoms, an alkoxy of 1 to 12 carbon atoms, an alkylamino of 1 to 12 carbon atoms, a diarylamino of 12 to 18 carbon atoms, a diheteroarylamino 2 to 18 carbon atoms, an aryl (heteroaryl) amino of 7 to 18 carbon atoms, an alkylsilyl of 1 to 12 carbon atoms, an arylsilyl of 6 to 18 carbon atoms, an aryloxy of 6 to 18 carbon atoms, and an arylthionyl of 6 to 18 carbon atoms.

In addition, the wording "R₁ and R₂ may be bonded to each other to form a mono- or polycyclic aliphatic or aromatic ring", as used herein, means that after removal of a hydrogen atom each thereof, R₁ and R₂ are linked to each other to form an additional ring.

In the present disclosure, the heterocyclic compound represented by Chemical Formula A or B is technically characterized by the linkage of a substituent including the pyrene structure represented by Structural Formula C to one (x + y + z = 1) or two (x + y + z = 2) of the ring moieties A₁, A₂, and E, which are each independently a substituted or unsubstituted aromatic hydrocarbon ring of 6 to 50 carbon atoms in Chemical Formula A and to one (x + y + z + w = 1) or two (x + y + z + w = 2) of the ring moieties A₁, A₂, E, and F, which are each independently a substituted or unsubstituted aromatic hydrocarbon ring of 6 to 50 carbon atoms in Chemical Formula B.

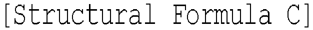

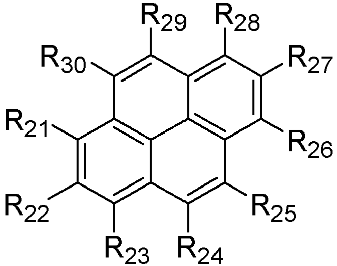

In Chemical Formulas A and B according to the present disclosure, A₁, A₂, E, and F, which may be same or different, are each independently a substituted or unsubstituted aromatic hydrocarbon ring of 6 to 50 carbon atoms, preferably a substituted or unsubstituted aromatic hydrocarbon ring of 6 to 18 carbon atoms, and more preferably a substituted or unsubstituted aromatic hydrocarbon ring of 6 to 14 carbons.

In Chemical Formula A or B, as stated above, when A₁, A₂, E, and F are same or different and are each a substituted or unsubstituted aromatic hydrocarbon ring of 6 to 18 carbon atoms, the aromatic hydrocarbon rings may be same or different and are each independently selected from among [Structural Formula 10] to [Structural Formula 21]: 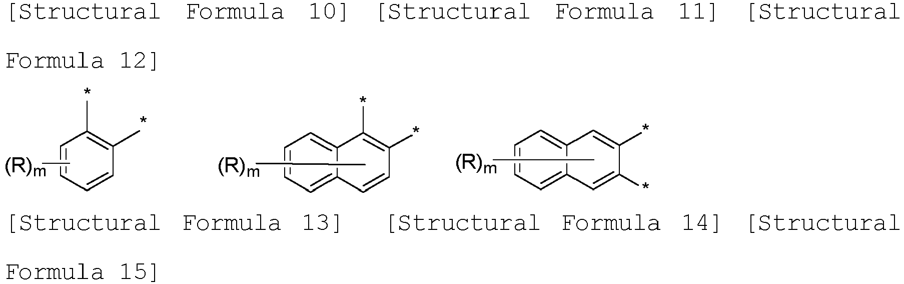 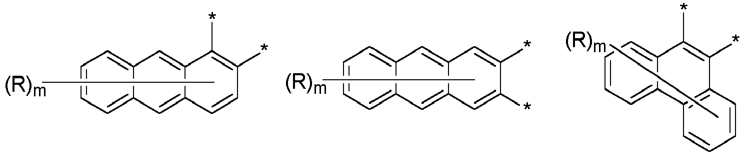 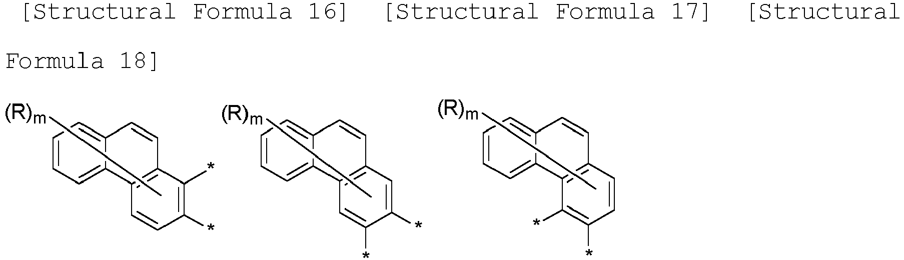 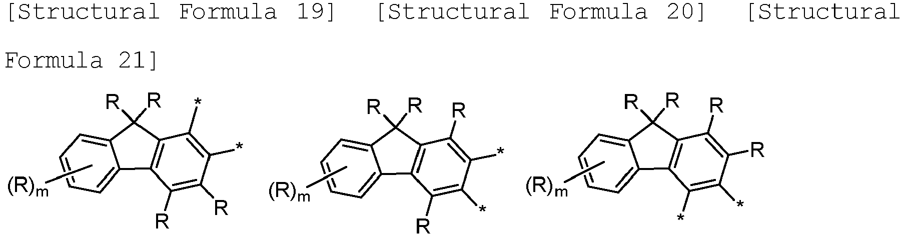
wherein, "-*" denotes a bonding site for forming a 5-membered ring bearing the carbon atom connected to the substituents R₁ and R₂ or a bonding site for forming a 5-membered ring bearing M of Structural Formula Q₁ and Q₂;
when one of the aromatic hydrocarbon rings of [Structural Formula 10] to [Structural Formula 21] for A₁ or A₂ is bonded to Structural Formula Q₁ or Structural Formula Q₂, two adjacent carbon atoms of the aromatic hydrocarbon ring occupy respective positions * of Structural Formula Q₁ or Q₂ to form a fused ring;
R is any one selected from among a hydrogen atom, a deuterium atom, a substituted or unsubstituted alkyl of 1 to 30 carbon atoms, a substituted or unsubstituted aryl of 6 to 20 carbon atoms, a substituted or unsubstituted cycloalkyl of 3 to 30 carbon atoms, a substituted or unsubstituted heteroaryl of 2 to 20 carbon atoms, a substituted or unsubstituted alkylsilyl of 1 to 30 carbon atoms, a substituted or unsubstituted arylsilyl of 6 to 30 carbon atoms, a cyano, a nitro, and a halogen, and
m is an integer of 1 to 8, with the proviso that when m is 2 or greater or two or more R's exist, the corresponding R's may be same or different.

According to one embodiment of the present disclosure, the linkers L₁ to L₄ in Chemical Formula A or B may be a single bond or one selected from among compounds represented by the following Structural Formulas 1 to 5, and s1 to s4 may each be 1 or 2:

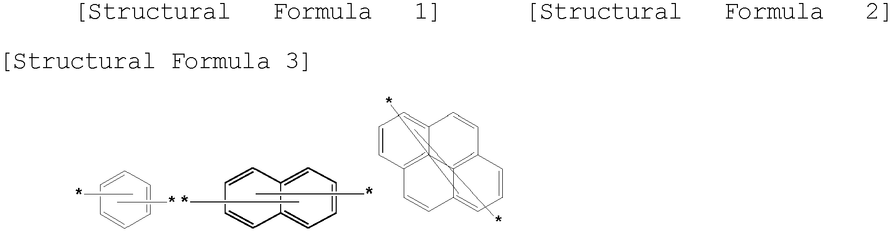

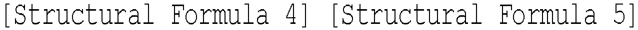

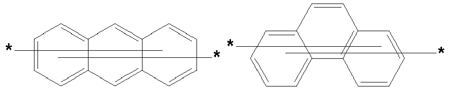

In the linkers, each of the unsubstituted carbon atoms of the aromatic ring moiety is bound with a hydrogen atom or a deuterium atom.

In an embodiment according to the present disclosure, the compound represented by Chemical Formula A is characterized by the structure in which a pyrene substituent represented by Structural Formula C is linked to any one of the ring moieties of A₁, A₂, and E, with a proviso that x is 1 and y and z are each 0; y is 1 and x and z are each 0; or z is 1 and x and y are each 0 or in which a pyrene substituent represented by Structural Formula C is linked to two of the ring moieties A₁, A₂, and E, with a proviso that x and y are each 1 and z 0; x and z are each 1 and y is 0; or y and z are each 1 and x is 0, and the compound represented by Chemical Formula B is characterized by the structure in which a pyrene substituent represented by Structural Formula C is linked to any one of the ring moieties of A₁, A₂, E, and F, with a proviso that x is 1 and y, z, and w are each 0, or in which a pyrene substituent represented by Structural Formula C is linked to two of the ring moieties A₁, A₂, E, and F, with a proviso that x and y are each 1 and z and w are each 0.

According to an embodiment of the present disclosure, the substituents R₁ and R₂ in Chemical Formula A or B, which are same or different, are each independently a substituted or unsubstituted alkyl of 1 to 10 carbon atoms and may be connected to each other to form a ring or may not be connected to each other.

According to an embodiment of the present disclosure, any one of R₂₁ to R₂₃ in the pyrene structure of Structural Formula C may be a single bond to any one of the linkers L₁ to L₄.

In addition, according to an embodiment of the present disclosure, R₂₁ to R₃₀ in Structural Formula C, which are same or different, are each independently any one selected from among a hydrogen atom, a deuterium atom, a substituted or unsubstituted alkyl of 1 to 6 carbon atoms, a substituted or unsubstituted aryl of 6 to 12 carbon atoms, and a substituted or unsubstituted heteroaryl of 2 to 12 carbon atoms, wherein at least one of R₂₁ to R₃₀ of Structural Formula C which are not connected to any of L₁ to L₄ may be any one selected from among a substituted or unsubstituted alkyl of 1 to 6 carbon atoms, a substituted or unsubstituted aryl of 6 to 12 carbon atoms, and a substituted or unsubstituted heteroaryl of 2 to 12 carbon atoms.

According to an embodiment of the present disclosure, R₁₁ to R₁₅, which are same or different, are each independently a substituent selected from among a hydrogen atom, a deuterium atom, a substituted or unsubstituted alkyl of 1 to 10 carbon atoms, a substituted or unsubstituted aryl of 6 to 18 carbon atoms, a substituted or unsubstituted alkylsilyl of 1 to 15 carbon atoms, a substituted or unsubstituted arylsilyl of 6 to 20 carbon atoms, a cyano, and a halogen.

In addition, the compound represented by Chemical Formula A or B may be any one selected from among [H 1] to [H 180]:

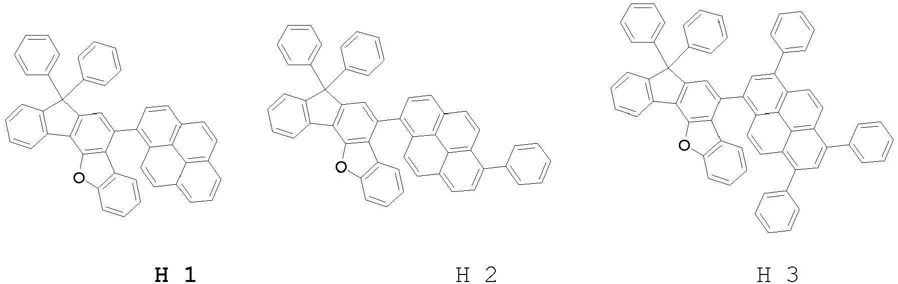

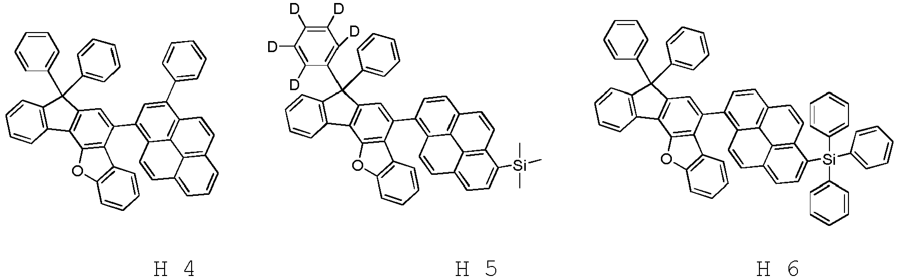

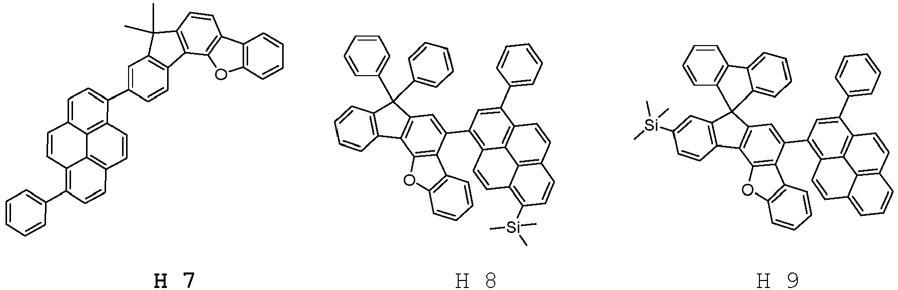

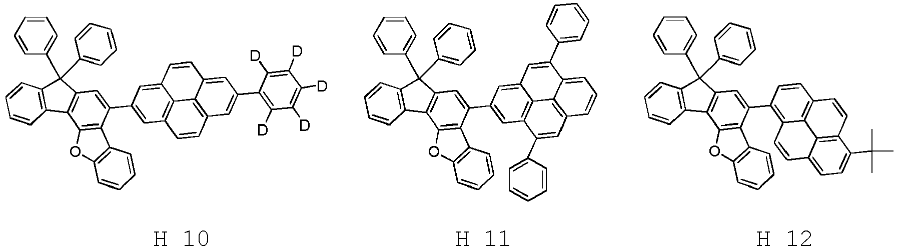

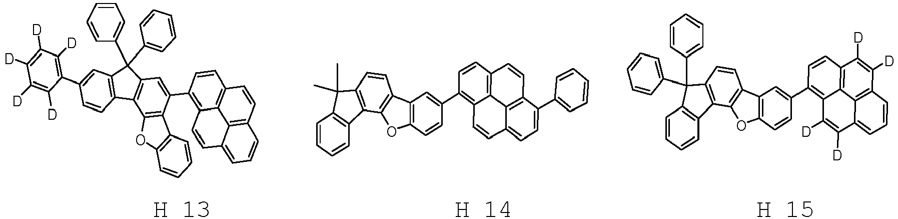

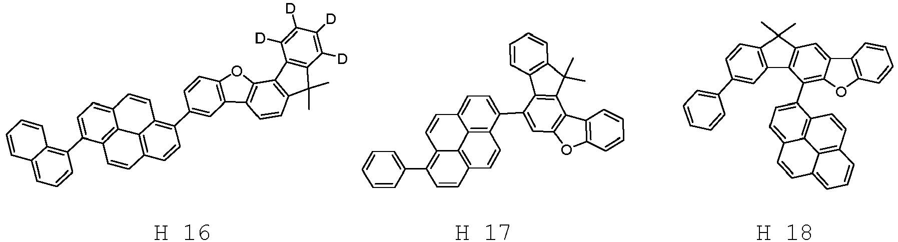

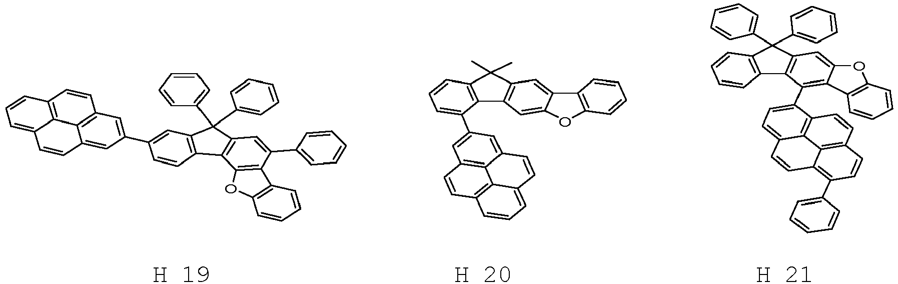

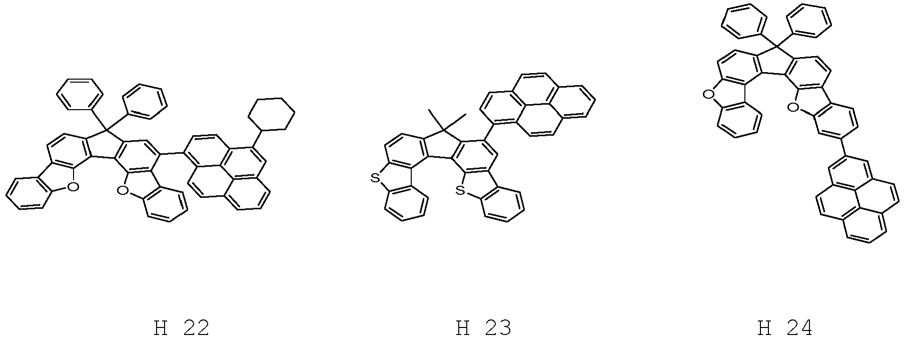

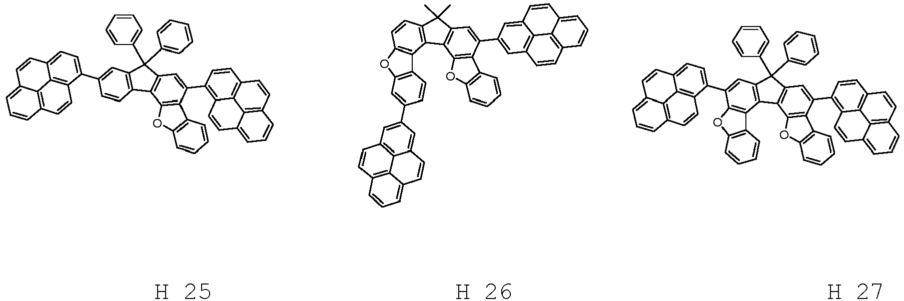

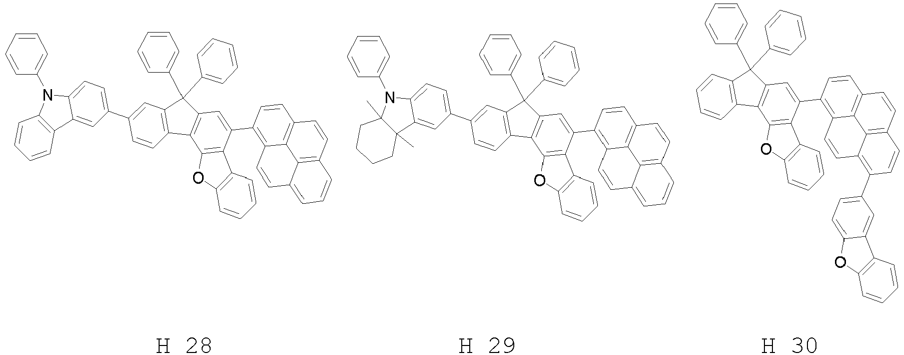

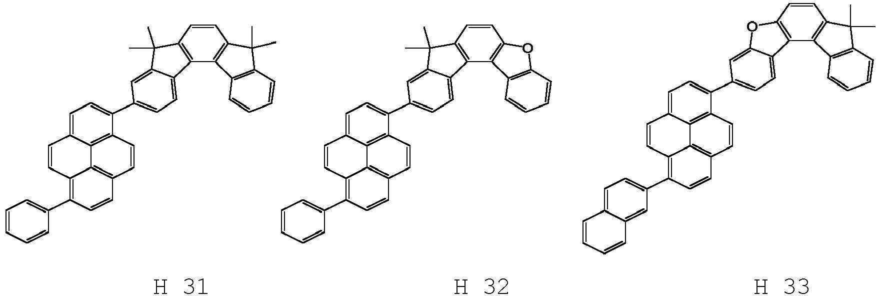

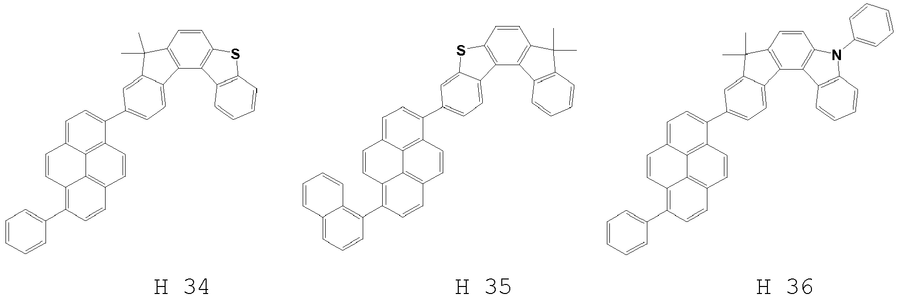

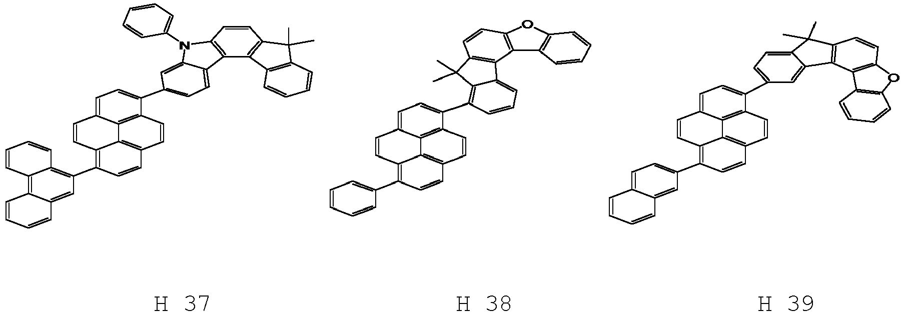

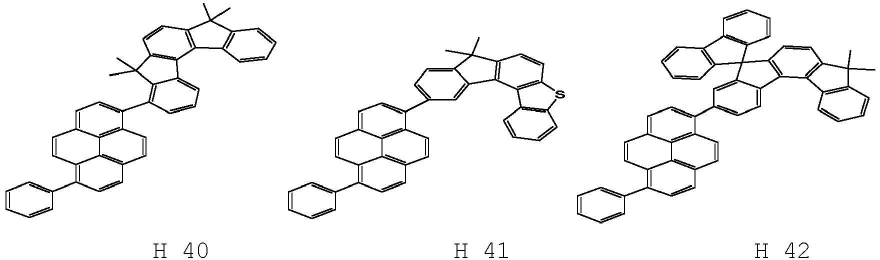

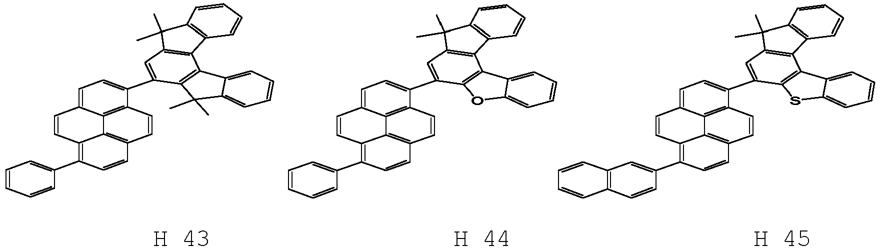

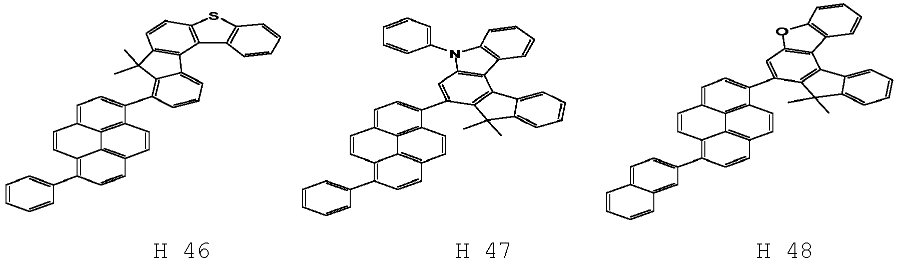

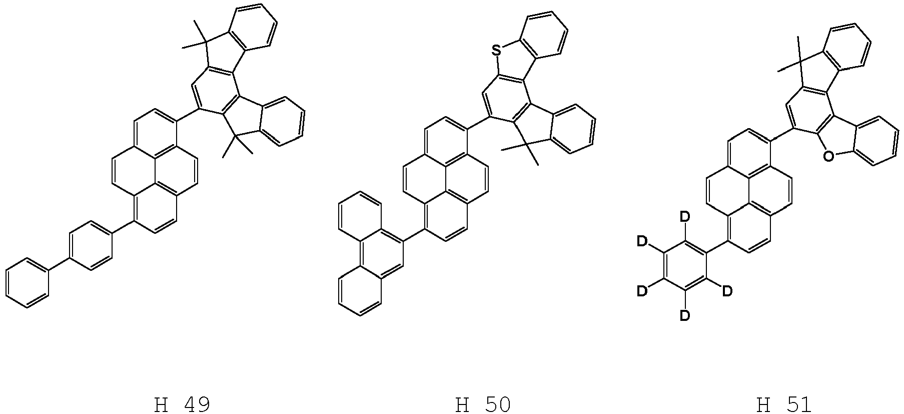

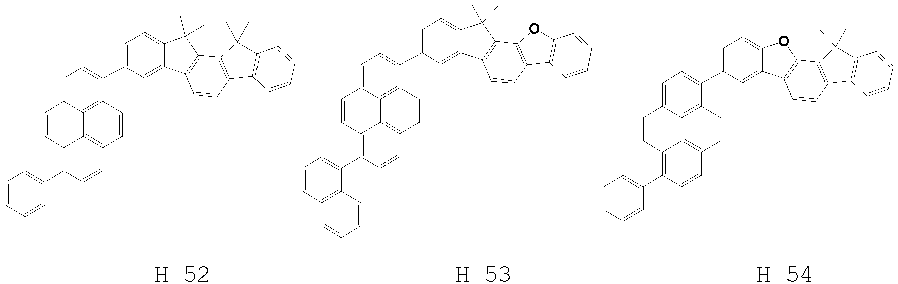

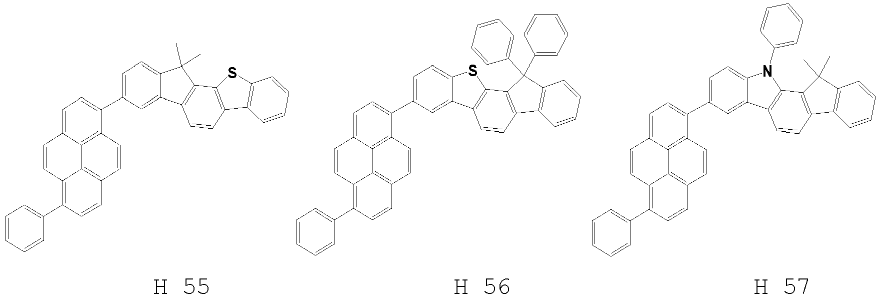

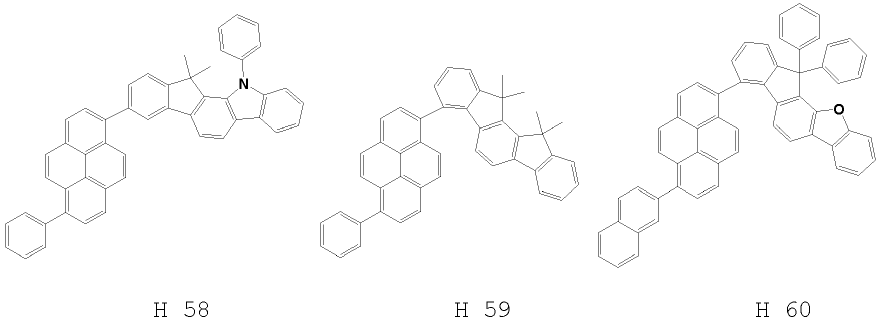

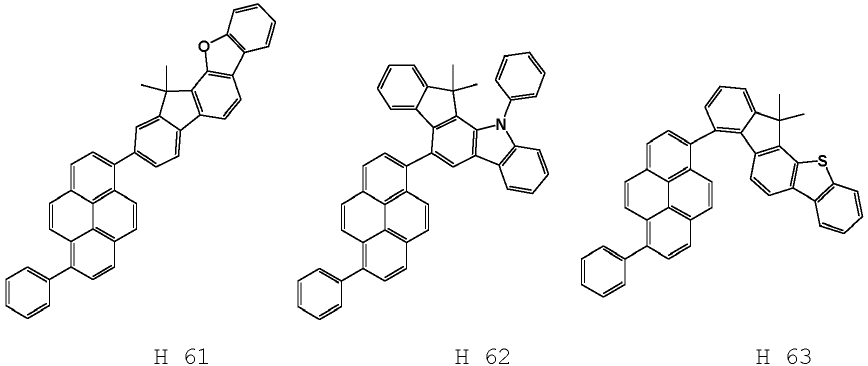

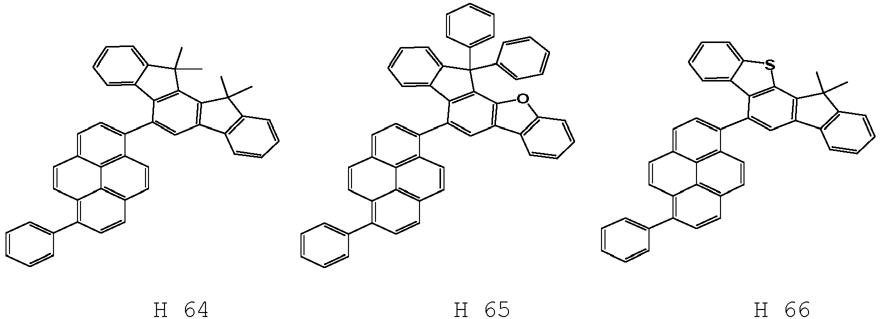

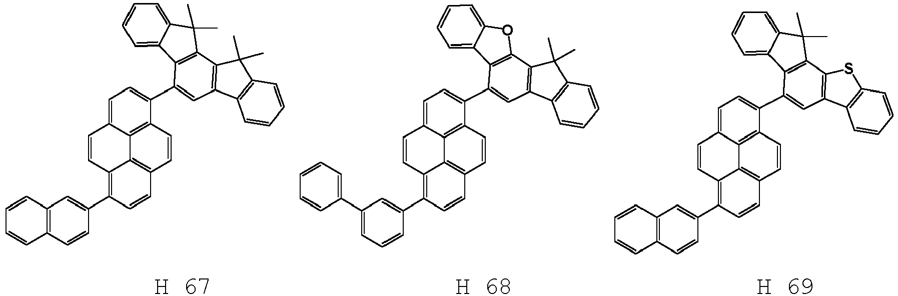

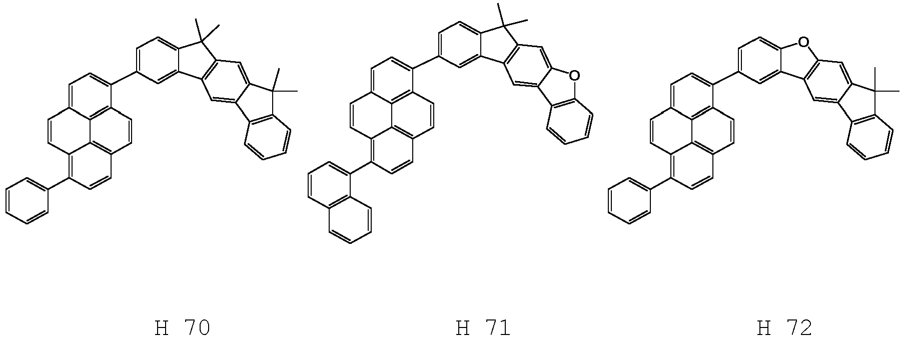

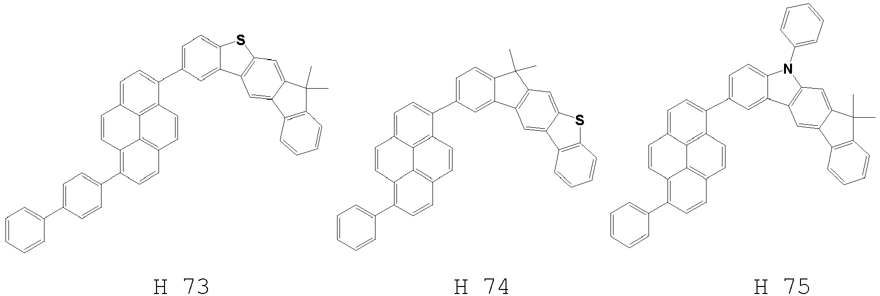

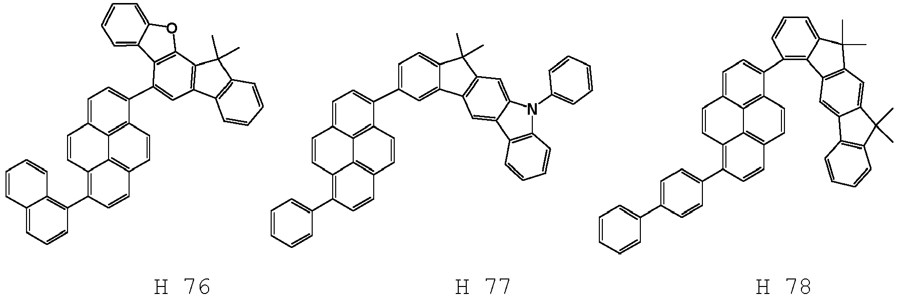

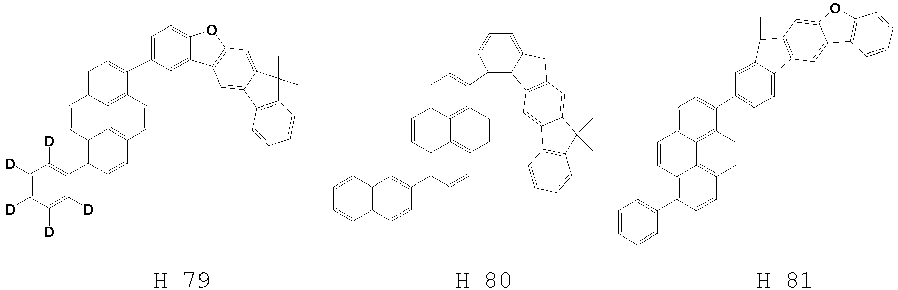

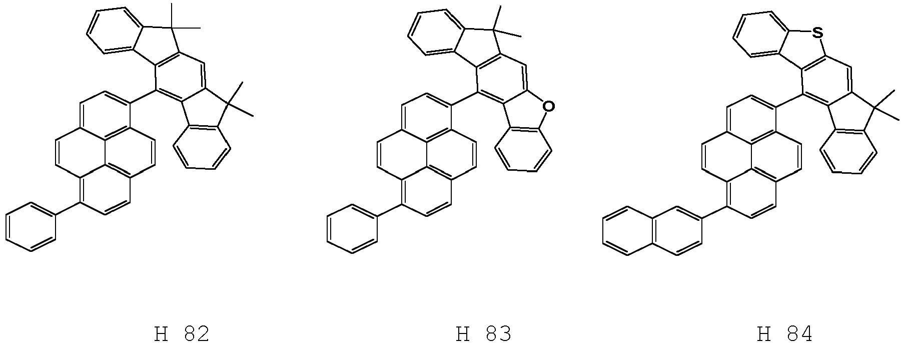

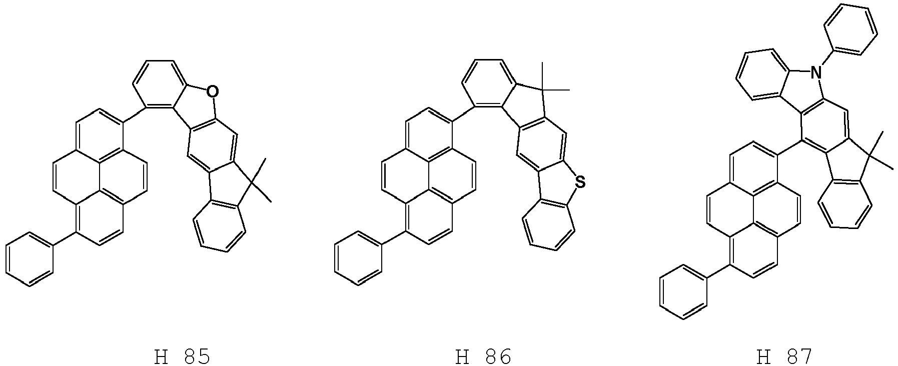

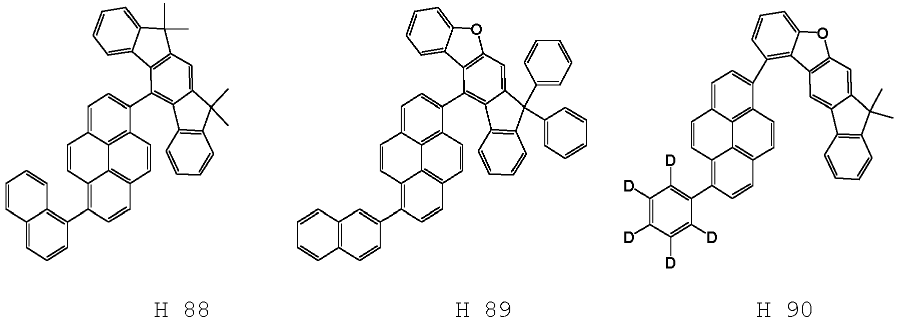

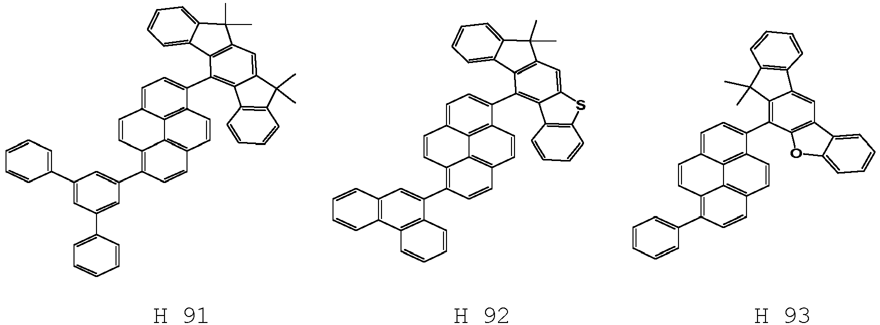

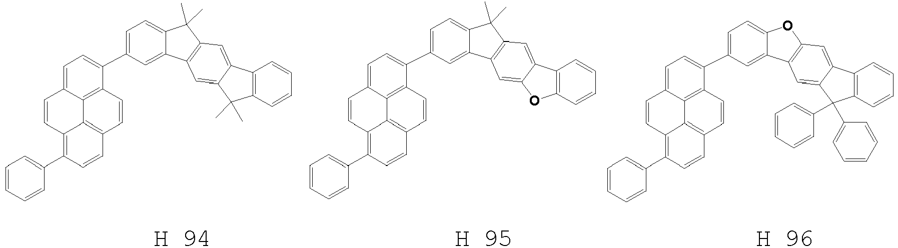

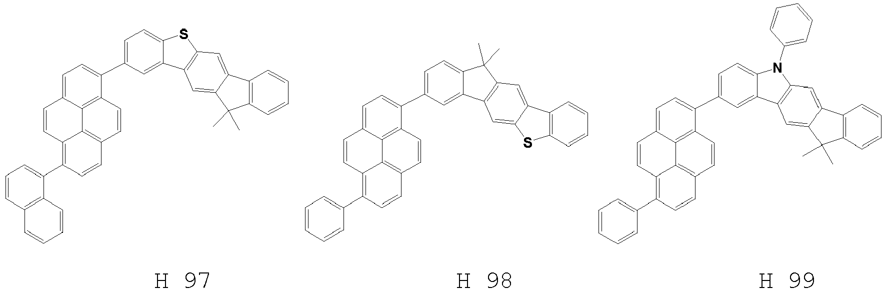

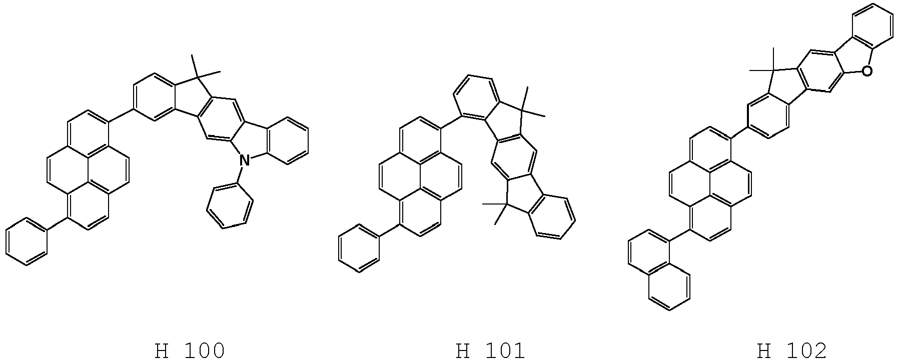

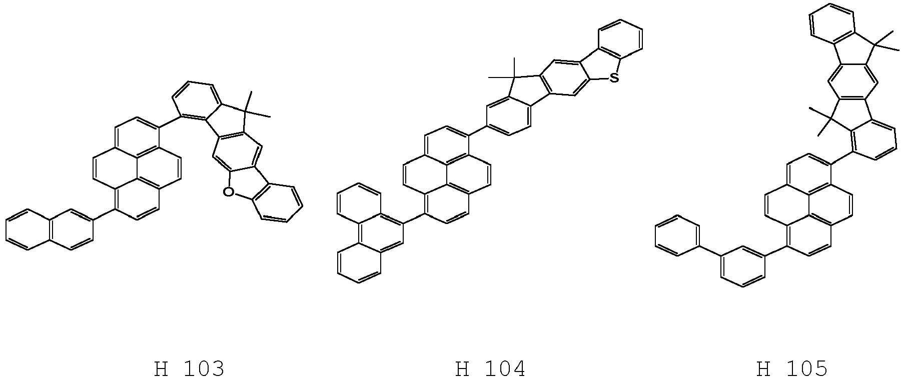

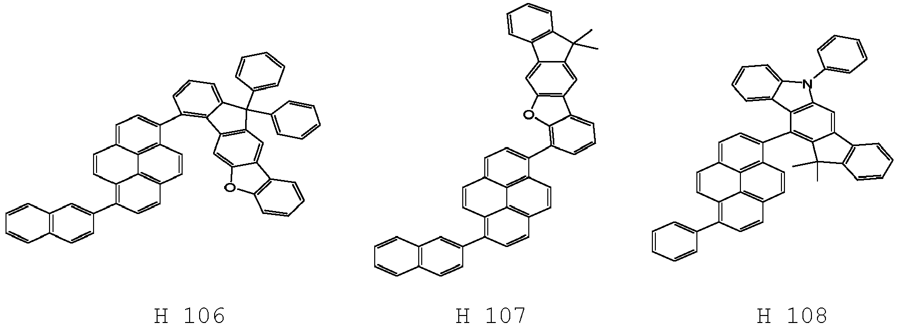

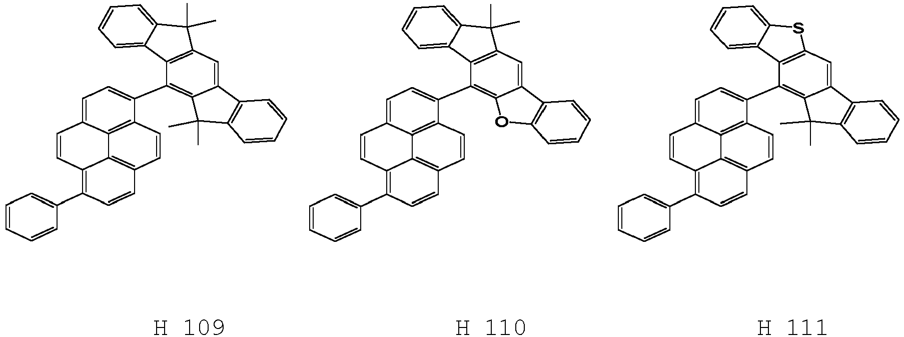

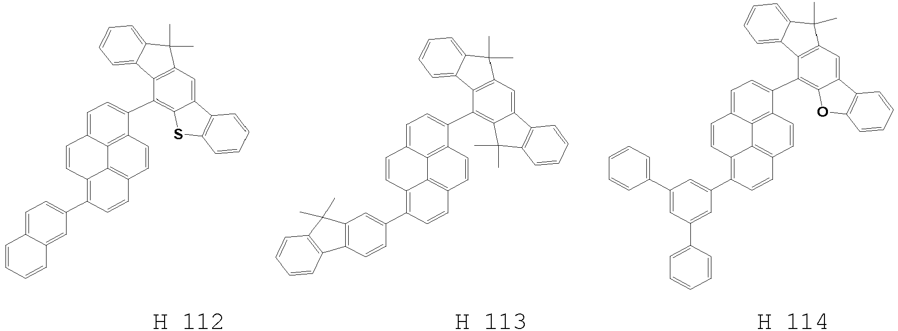

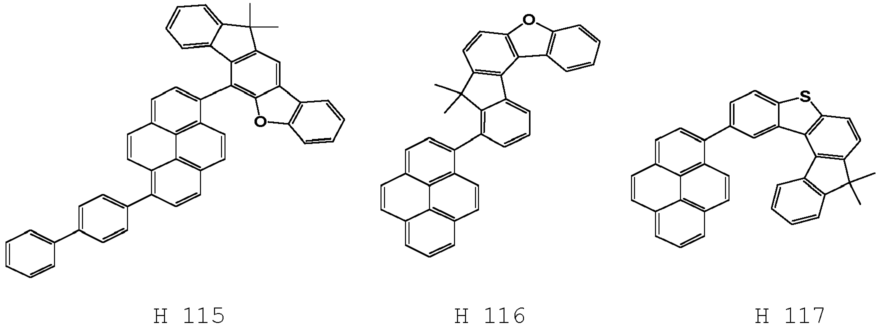

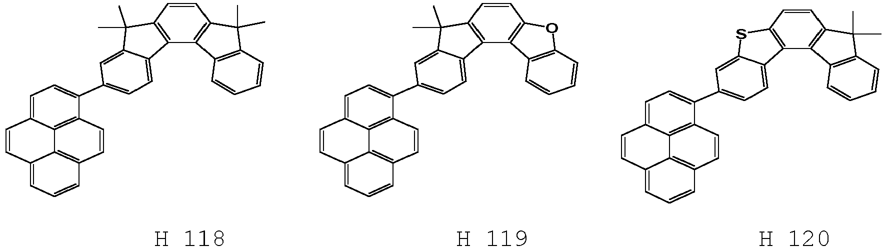

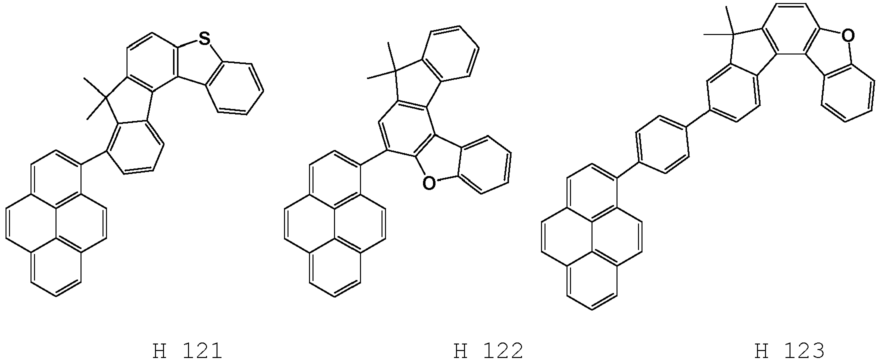

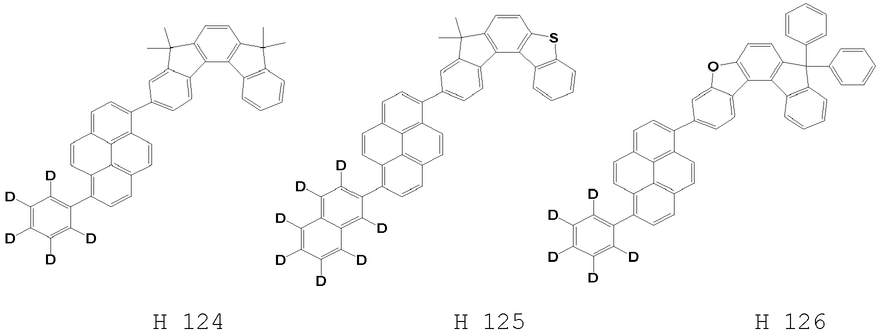

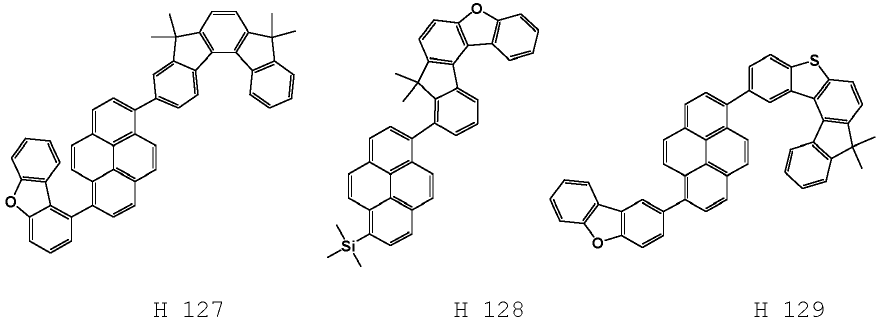

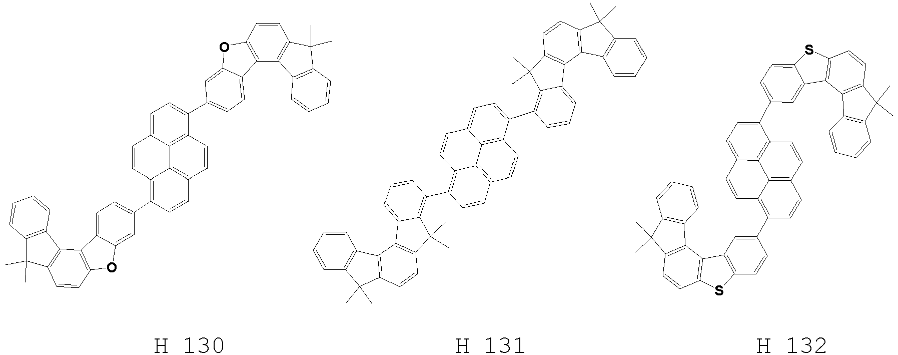

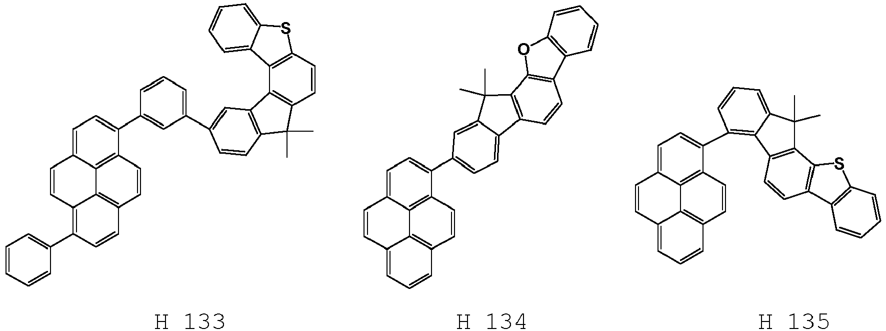

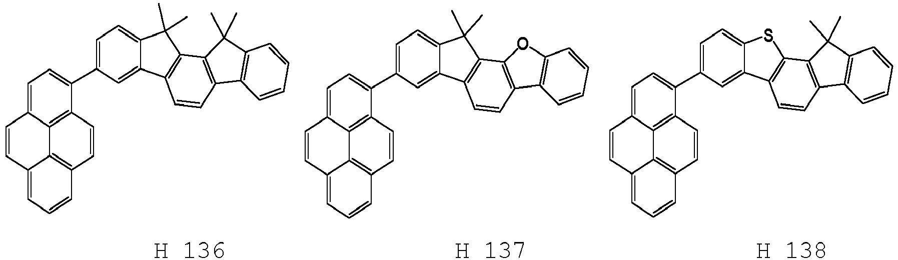

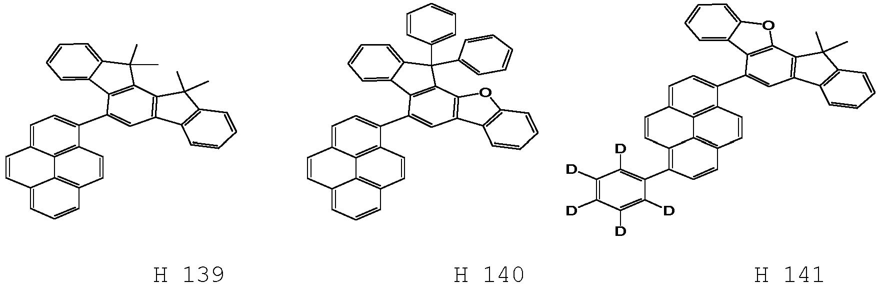

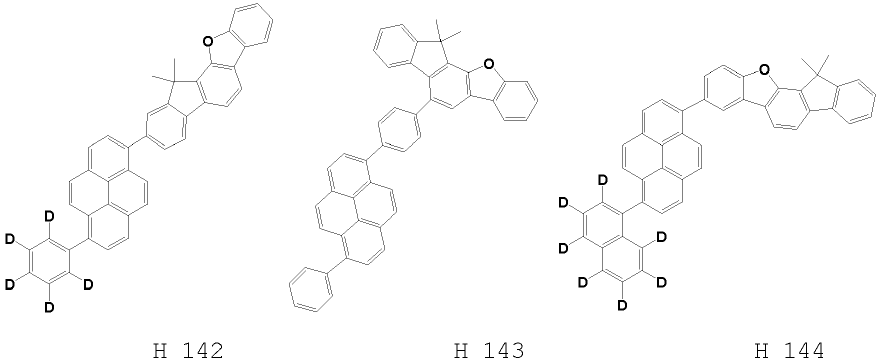

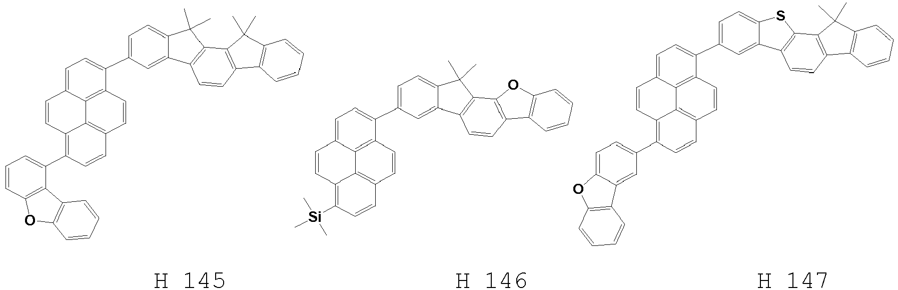

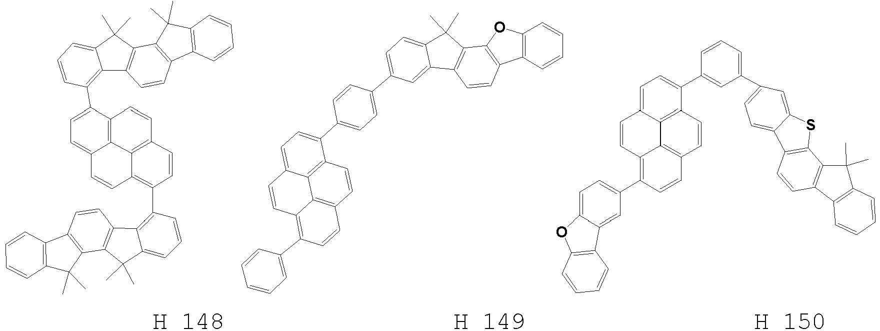

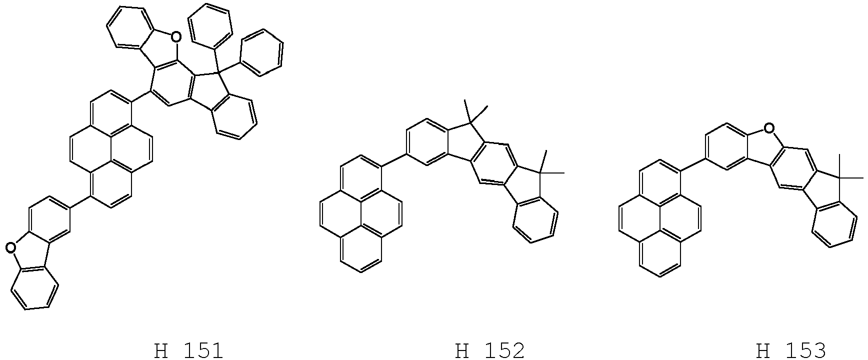

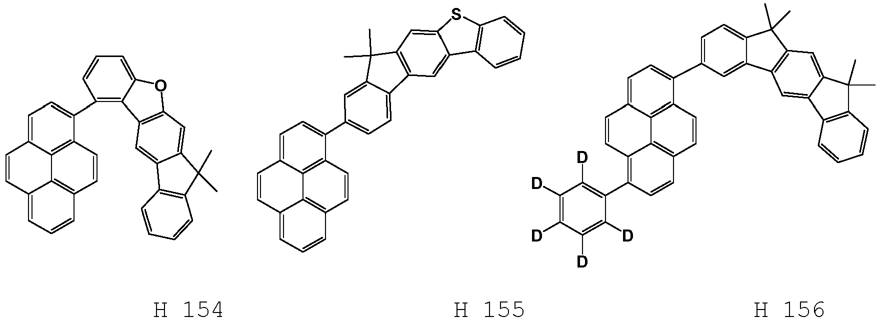

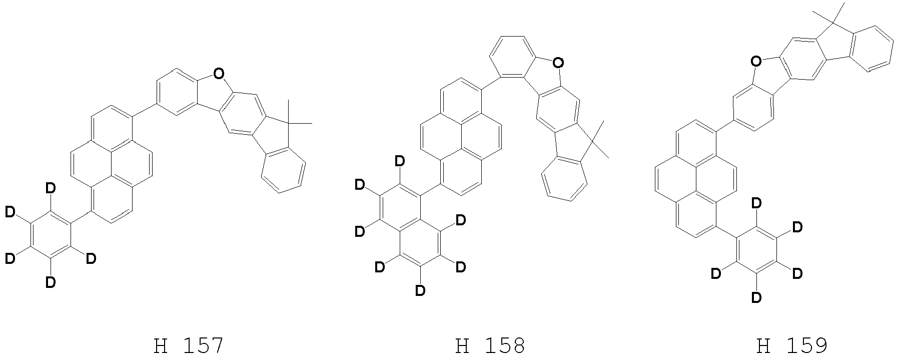

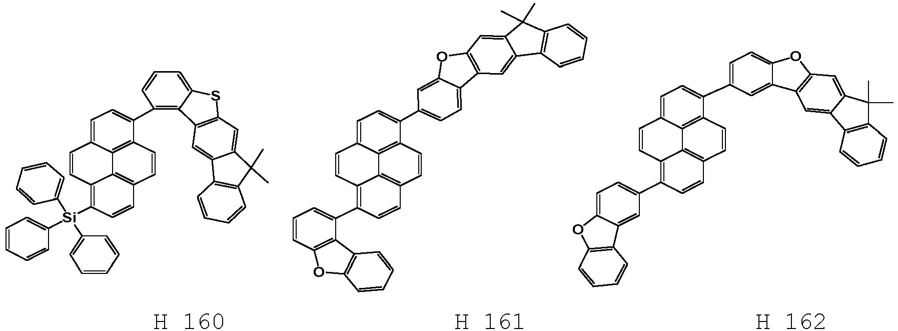

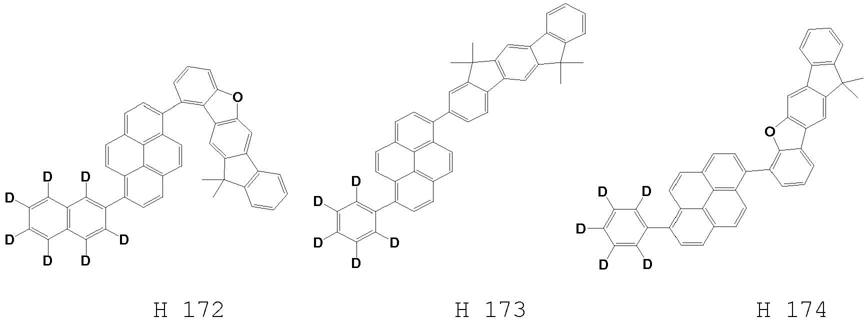

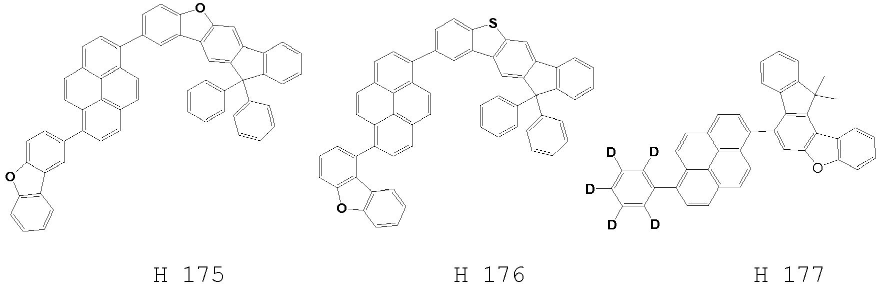

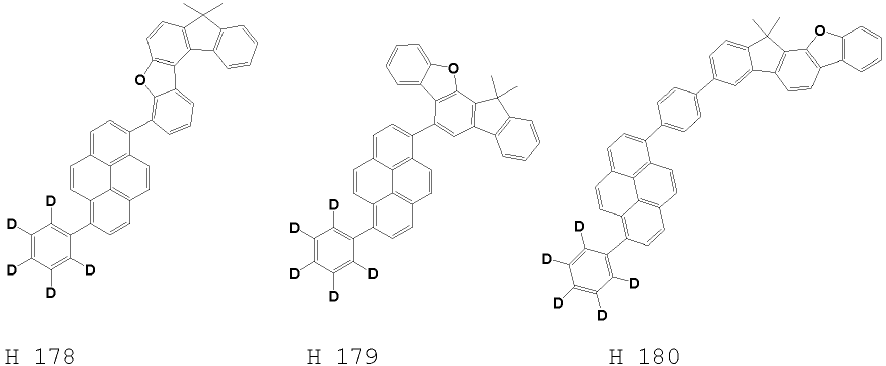

Also, the present disclosure provides an organic light-emitting diode comprising: a first electrode; a second electrode facing the second electrode; and an organic layer interposed between the first electrode and the second electrode, wherein the organic layer comprises at least one of the compounds represented by Chemical Formula A or B.

Throughout the description of the present disclosure, the phrase "(an organic layer) includes at least one organic compound" may be construed to mean that "(an organic layer) may include a single organic compound species or two or more difference species of organic compounds falling within the scope of the present disclosure".

In this regard, the organic layer according to the present disclosure may include at least one of a hole injection layer, a hole transport layer, a functional layer capable of both hole injection and hole transport, a light emitting layer, an electron transport layer, and an electron injection layer.

In more particular embodiments of the present disclosure, the organic layer disposed between the first electrode and the second electrode includes a light-emitting layer composed of a host and a dopant, wherein the compound represented by Chemical Formula A or B serves as the host.

In addition, the light-emitting layer of the present disclosure may contain as a dopant compound at least one of the compounds represented by the following Chemical Formula D1 to Chemical Formula D10: 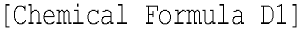 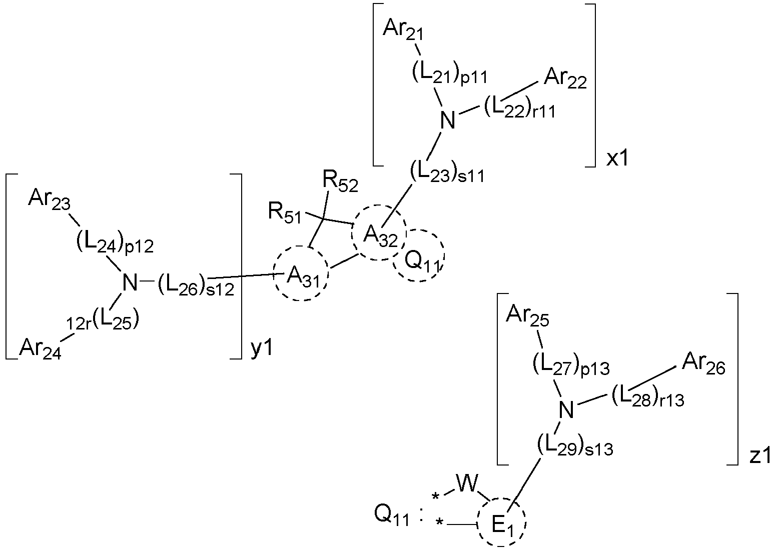 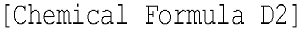 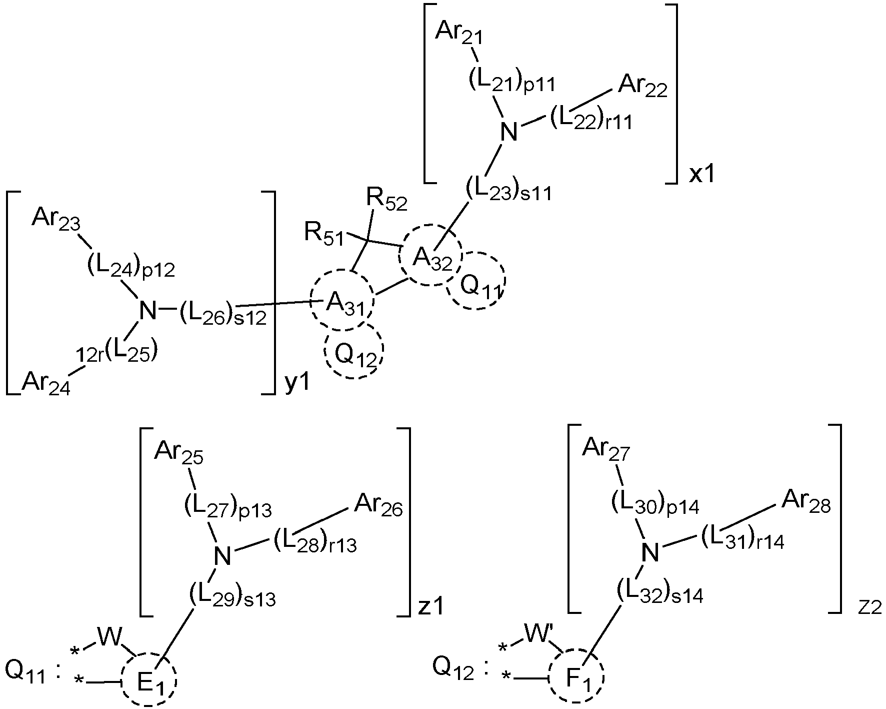 wherein,
A₃₁, A₃₂, E₁ and F₁, which may be same or different, are each independently a substituted or unsubstituted aromatic hydrocarbon ring of 6 to 50 carbon atoms, or a substituted or unsubstituted aromatic heteroring of 2 to 40 carbon atoms,
wherein two adjacent carbon atoms of the aromatic ring A₃₁ and two adjacent carbon atoms of the aromatic ring A₃₂ form a 5-membered fused ring together with a carbon atom to which substituents R₅₁ and R₅₂ are bonded;
linkers L₂₁ to L₃₂, which may be same or different, are each independently selected from a single bond, a substituted or unsubstituted alkylene of 1 to 60 carbon atoms, a substituted or unsubstituted alkenylene of 2 to 60 carbon atoms, a substituted or unsubstituted alkynylene of 2 to 60 carbon atoms, a substituted or unsubstituted cycloalkylene of 3 to 60 carbon atoms, a substituted or unsubstituted heterocycloalkylene of 2 to 60 carbon atoms, a substituted or unsubstituted arylene of 6 to 60 carbon atoms, and a substituted or unsubstituted heteroarylene of 2 to 60 carbon atoms;
W and W', which may be same or different, are each independently any one selected from among N-R₅₃, CR₅₄R₅₅, SiR₅₆R₅₇, GeR₅₈R₅₉, O, S, and Se;
R₅₁ to R₅₉, and Ar₂₁ to Ar₂₈, which may be the same or different, are each independently any one selected from among a hydrogen atom, a deuterium atom, a substituted or unsubstituted alkyl of 1 to 30 carbon atoms, a substituted or unsubstituted aryl of 6 to 50 carbon atoms, a substituted or unsubstituted alkenyl of 2 to 30 carbon atoms, a substituted or unsubstituted alkynyl of 2 to 30 carbon atoms, a substituted or unsubstituted cycloalkyl of 3 to 30 carbon atoms, a substituted or unsubstituted cycloalkenyl of 5 to 30 carbon atoms, a substituted or unsubstituted heteroaryl of 2 to 50 carbon atoms, a substituted or unsubstituted heterocycloalkyl of 2 to 30 carbon atoms, a substituted or unsubstituted alkoxy of 1 to 30 carbon atoms, a substituted or unsubstituted aryloxy of 6 to 30 carbon atoms, a substituted or unsubstituted alkylthioxy of 1 to 30 carbon atoms, a substituted or unsubstituted arylthioxy of 5 to 30 carbon atoms, a substituted or unsubstituted alkylamine of 1 to 30 carbon atoms, a substituted or unsubstituted arylamine of 5 to 30 carbon atoms, a substituted or unsubstituted alkylsilyl of 1 to 30 carbon atoms, a substituted or unsubstituted arylsilyl of 5 to 30 carbon atoms, a substituted or unsubstituted alkyl geraminum of 1 to 30 carbon atoms, a substituted or unsubstituted aryl geraminum of 1 to 30 carbon atoms a cyano, a nitro, and a halogen,
wherein R₅₁ and R₅₂ together may form a mono- or polycyclic aliphatic or aromatic ring that may be a heterocyclic ring bearing a heteroatom selected from N, O, P, Si, S, Ge, Se, and Te as a ring member;
p11 to p14, r11 to r14, and s11 to s14 are each independently an integer of 1 to 3, wherein when any of them is 2 or greater, the corresponding L₂₁ to L₃₂ may be same or different,
x1 is an integer of 1 or 2, and y1 and z1, which may be same or different, are each independently an integer of 0 to 3;
Ar₂₁ may form a ring with Ar₂₂, Ar₂₃ may form a ring with Ar₂₄, Ar₂₅ may form a ring with Ar₂₆, and Ar₂₇ may form a ring with Ar₂₈;
two adjacent carbon atoms of the A₃₂ ring moiety of Chemical Formula D1 may occupy respective positions * of Structural Formula Q₁₁ to form a fused ring; and
two adjacent carbon atoms of the A₃₁ ring moiety of Chemical Formula D2 may occupy respective positions * of structural Formula Q₁₂ to form a fused ring, and two adjacent carbon atoms of the A₃₂ ring moiety of Chemical Formula D2 may occupy respective positions * of Structural Formula Q₁₁ to form a fuse ring; 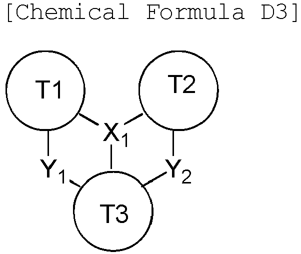
wherein,
X₁ is any one selected from among B, P, and P=O,
T₁ to T₃, which are same or different, are each independently a substituted or unsubstituted aromatic hydrocarbon ring of 6 to 50 carbon atoms, or a substituted or unsubstituted heteroaromatic ring of 2 to 40 carbon atoms;
Y₂ is any one selected from among N-R₆₁, CR₆₂R₆₃, O, S, and SiR₆₄R₆₅;
Y₂ is any one selected from among N-R₆₆, CR₆₆₆₈, O, S, SiR₆₉R₇₀,
wherein R₆₁ to R₇₀, which may be same or different, are each independently any one selected from among a hydrogen atom, a deuterium atom, a substituted or unsubstituted alkyl of 1 to 30 carbon atoms, a substituted or unsubstituted aryl of 6 to 50 carbon atoms, a substituted or unsubstituted cycloalkyl of 3 to 30 carbon atoms, a substituted or unsubstituted heteroaryl of 2 to 50 carbon atoms, a substituted or unsubstituted alkoxy of 1 to 30 carbon atoms, a substituted or unsubstituted aryloxy of 6 to 30 carbon atoms, a substituted or unsubstituted alkylthioxy of 1 to 30 carbon atoms, a substituted or unsubstituted arylthioxy of 5 to 30 carbon atoms, a substituted or unsubstituted alkylamine of 1 to 30 carbon atoms, a substituted or unsubstituted arylamine of 5 to 30 carbon atoms, a substituted or unsubstituted alkylsilyl of 1 to 30 carbon atoms, a substituted or unsubstituted arylsilyl of 5 to 30 carbon atoms, a cyano, and a halogen, and wherein at least one of R₆₁ to R₇₀ may be connected to at least one of T₁ to T₃ to form an additional mono- or polycyclic aliphatic or aromatic ring; 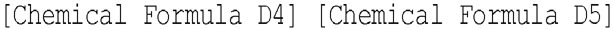 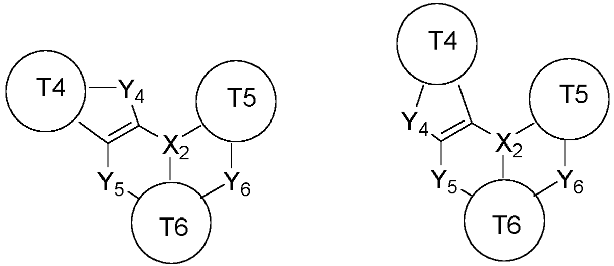
wherein,
X₂ is any one selected from among B, P, and P=O,
T₄ to T₆ are as defined for T₁ to T₃ in Chemical Formula D3,
Y₄ is any one selected from among N-R₆₁, CR₆₂R₆₃, O, S, and SiR₆₄R₆₅;
Y₅ is any one selected from among N-R₆₆, CR₆₆R₆₈, O, S, and SiR₆₉R₇₀, and
Y₆ is any one selected from among N-R₇₁, CR₇₃R₇₃, O, S, and SiR₇₄R₇₃,
R₆₁ to R₇₅ being as defined for R₆₁ to R₇₀ in [Chemical Formula D3]; 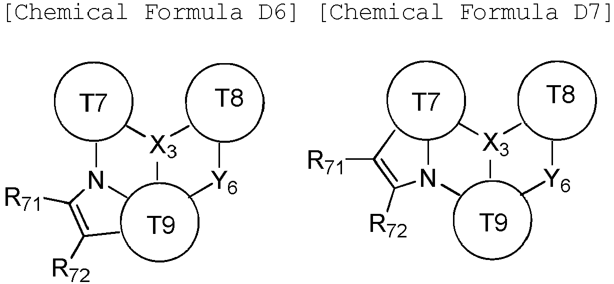
wherein, X₃ is any one selected from among B, P, and P=O,
T₇ to T₉ are as defined for T₁ to T₃ in Chemical Formula D3,
Y₆ is any one selected from among N-R₆₁, CR₆₂R₆₃, O, S, and SiR₆₄R₆₅,
R₆₁ to R₆₅, R₇₁, and R₇₂ are as defined for R₆₁ to R₇₀ in [Chemical Formula D3], respectively,
wherein R₇₁ and R₇₂ may be connected to each other to form an additional mono- or polycyclic aliphatic or aromatic ring or connected to the T7 or T9 ring moiety to form an additional mono- or polycyclic aliphatic or aromatic ring; and 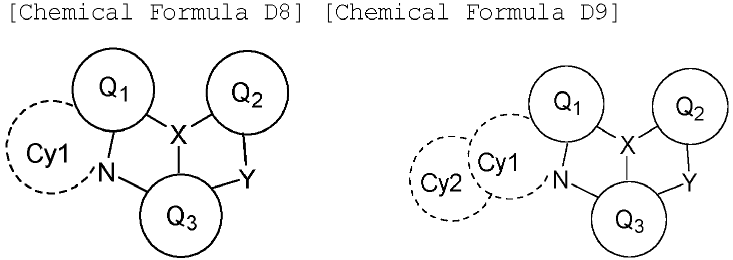 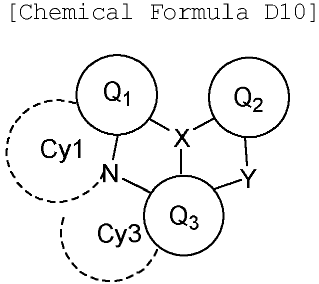
wherein,
X is any one selected from among B, P, and P=O,
Q₁ to Q₃ are as defined for T1 to T3 in Chemical Formula D3,
the linker Y is any one selected from among N-R₃, CR₄R₅, O, S, and Se,
wherein R₃ to R₅ are as defined for R₆₁ to R₇₀ in Chemical Formula D3, respectively,
R₃ to R₅ may each be connected to the Q₂ or Q₃ ring moiety to form an additional mono- or polycyclic aliphatic or aromatic ring,
R₄ and R₅ may be connected to each other to form an additional mono- or polycyclic aliphatic or aromatic ring,
the ring formed by Cy1 is a substituted or unsubstituted alkylene of 1 to 10 carbon atoms, except for the nitrogen (N) atom, the aromatic carbon atom of Q₁ to which the nitrogen (N) atom is connected, and the aromatic carbon atom of Q₁ to which Cy1 is to bond,
Cy2 in Chemical Formula D9 forms a saturated hydrocarbon ring added to Cy1 wherein the ring formed by Cy2 is a substituted or unsubstituted alkylene of 1 to 10 carbon atoms, except for the carbon atoms included in Cy1, and
the ring formed by Cy3 in Chemical Formula D10 is a substituted or unsubstituted alkylene of 1 to 10 carbon atoms, except for the aromatic carbon atom of Q₃ to which Cy3 is to bond, the aromatic carbon atom of Q₃ to which the nitrogen (N) atom is connected, the nitrogen (N) atom, and the carbon atom of Cy1 to which the nitrogen (N) atom is connected,
wherein the term "substituted" in the expression "substituted or unsubstituted" used for compounds of Chemical Formulas D1 to D10 means having at least one substituent selected from the group consisting of a deuterium atom, a cyano, a halogen, a hydroxy, a nitro, an alkyl of 1 to 24 carbon atoms, a halogenated alkyl of 1 to 24 carbon atoms, an alkenyl of 2 to 24 carbon atoms, an alkynyl of 2 to 24 carbon atoms, a heteroalkyl of 1 to 24 carbon atoms, an aryl of 6 to 24 carbon atoms, an arylalkyl of 7 to 24 carbon atoms, a heteroaryl of 2 to 24 carbon atoms or a heteroarylalkyl of 2 to 24 carbon atoms, an alkoxy of 1 to 24 carbon atoms, an alkylamino of 1 to 24 carbon atoms, an arylamino of 6 to 24 carbon atoms, a heteroarylamino of 1 to 24 carbon atoms, an alkylsilyl of 1 to 24 carbon atoms, an arylsilyl of 6 to 24 carbon atoms, and an aryloxy of 6 to 24 carbon atoms.

Among the dopant compounds according to the present disclosure, the boron compounds represented by Chemical Formulas D3 to D10 may have, on the aromatic hydrocarbon rings or heteroaromatic rings of T1 to T9 or on the aromatic hydrocarbon rings or heteroaromatic rings of Q₁ to Q₃, a substituent selected from a deuterium atom, an alkyl of 1 to 24 carbon atoms, an aryl of 6 to 24 carbon atoms, an alkylamino of 1 to 24 carbon atoms, and an arylamino of 6 to 24 carbon atoms, wherein the alkyl radicals or the aryl radicals in the alkylamino of 1 to 24 carbon atoms and the arylamino of 6 to 24 carbon atoms on the rings may be linked to each other, and particularly a substituent selected from an alkyl of 1 to 12 carbon atoms, an aryl of 6 to 18 carbon atoms, an alkylamino of 1 to 12 carbon atoms, and an arylamino of 6 to 18 carbon atoms wherein the alkyl radicals or aryl radicals in the alkylamino of 1 to 12 carbon atoms and the arylamino of 6 to 18 carbon atoms on the rings may be linked to each other.

Concrete examples of the dopant compounds of Chemical Formulas D1 and D2 used in the light-emitting layer of the organic light-emitting diode include the compounds of the following d 1 to d 239:

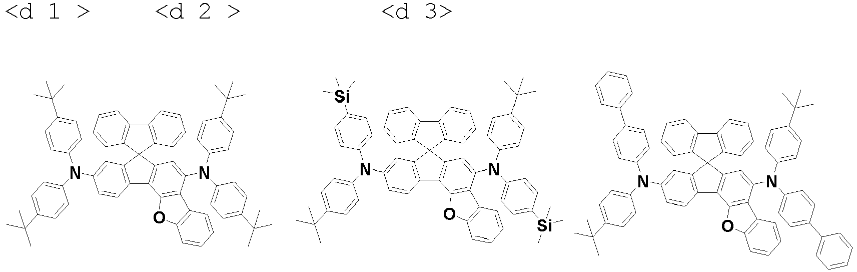

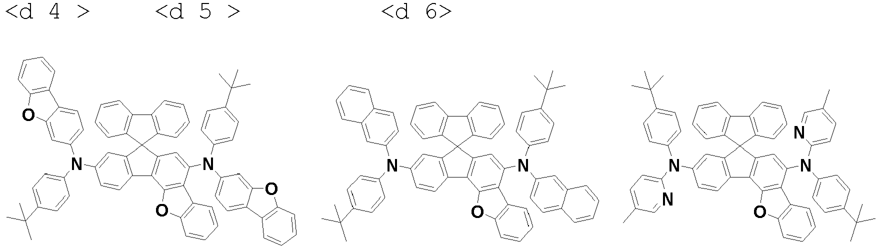

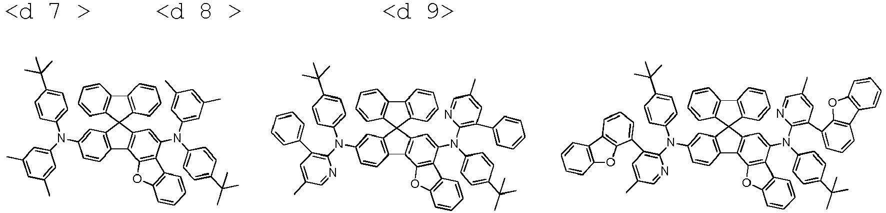

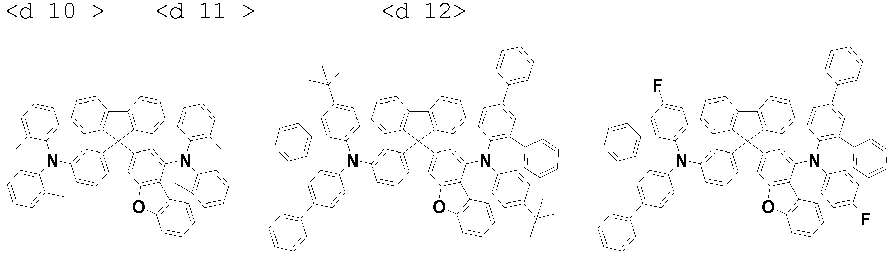

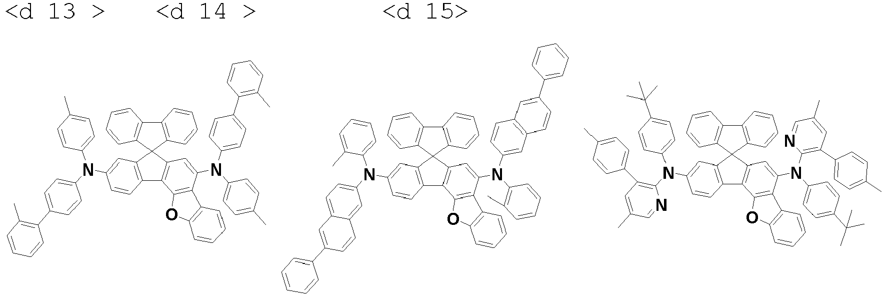

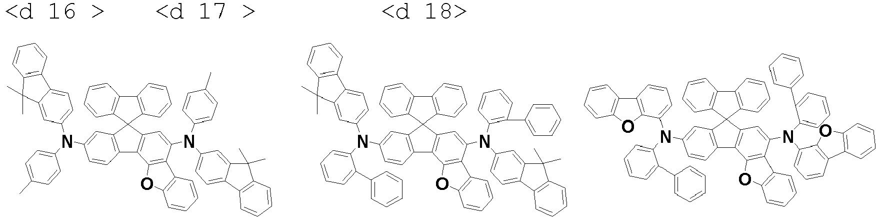

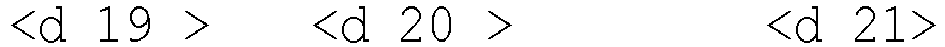

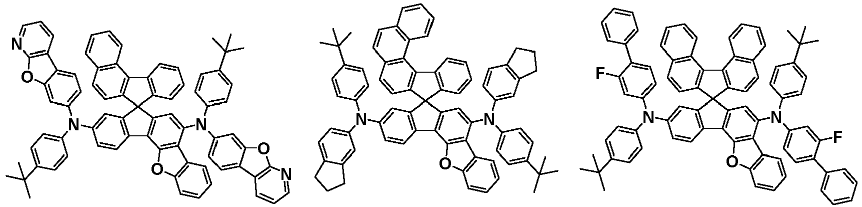

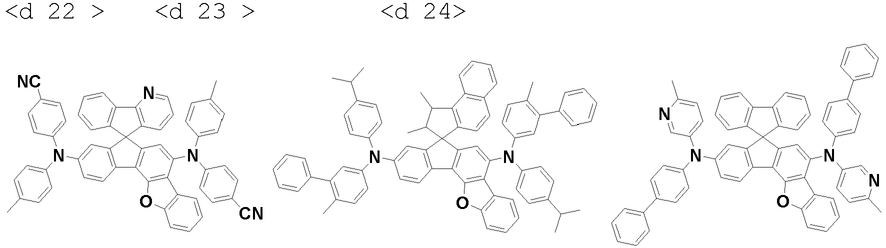

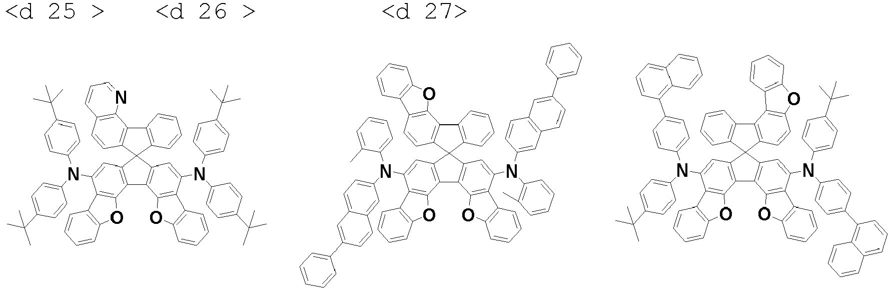

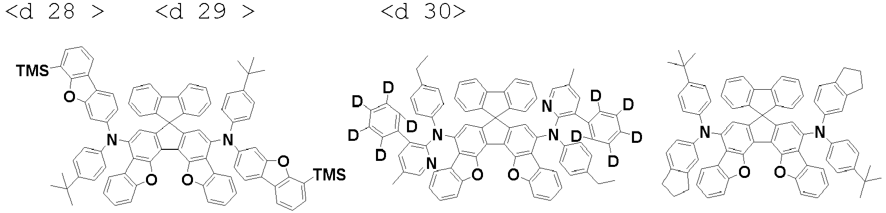

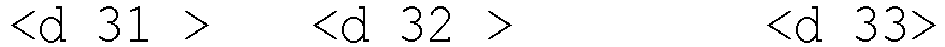

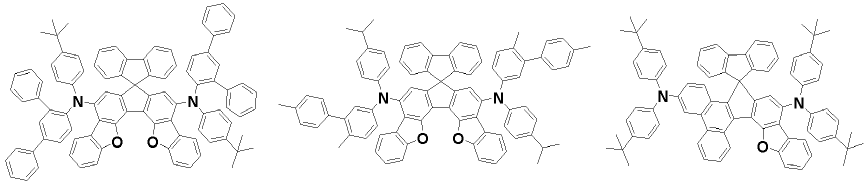

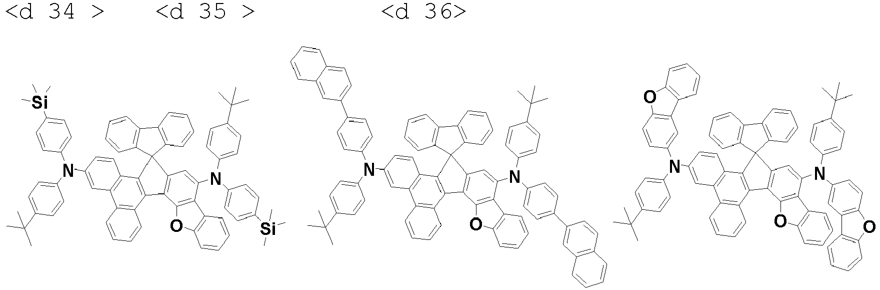

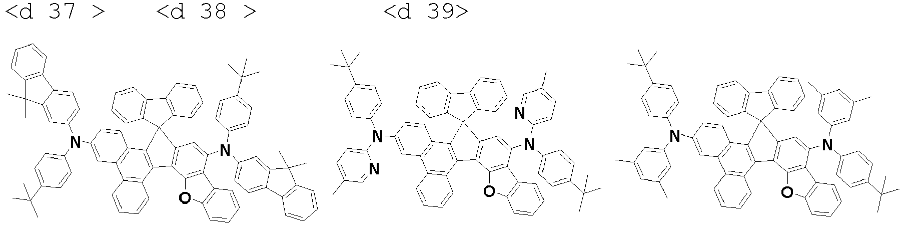

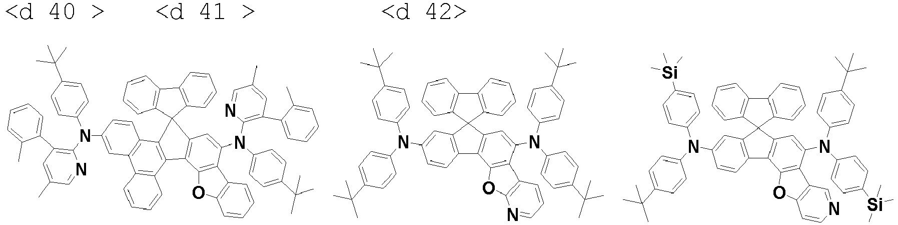

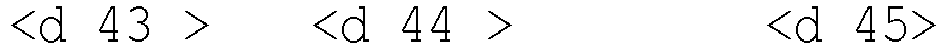

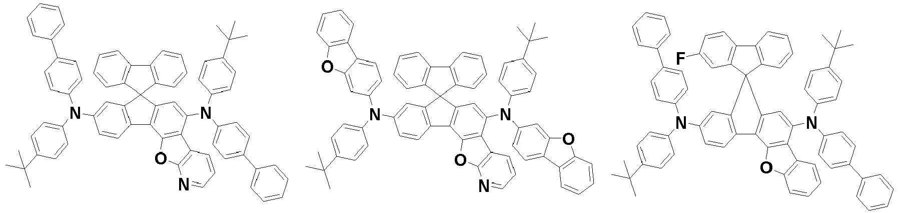

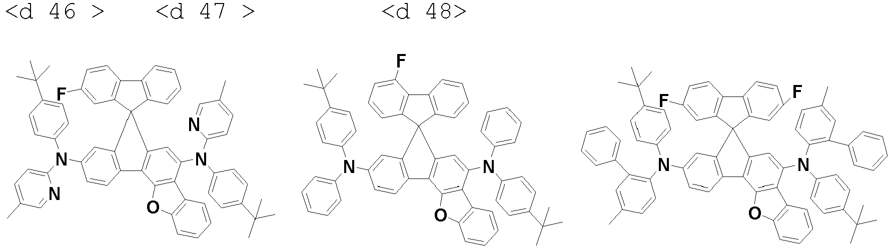

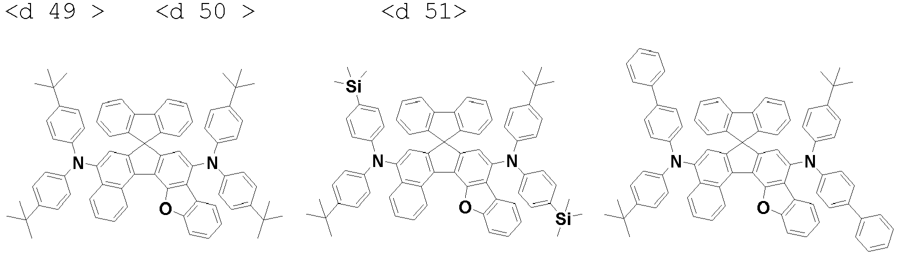

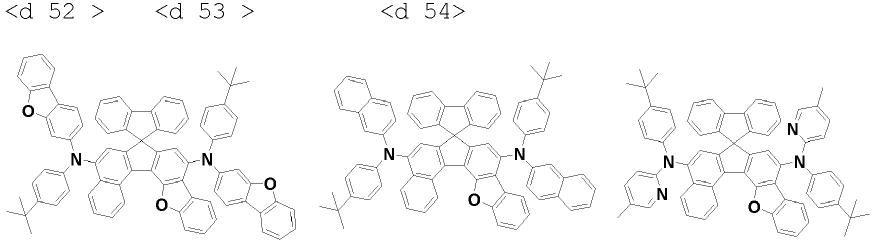

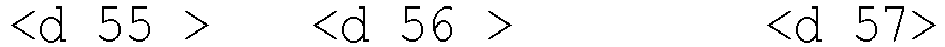

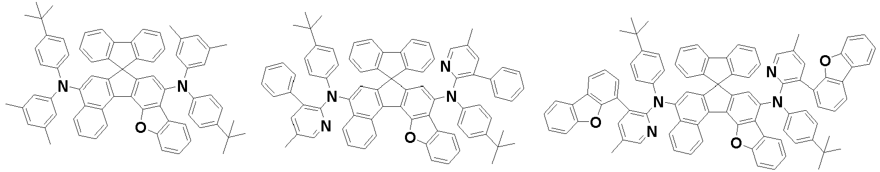

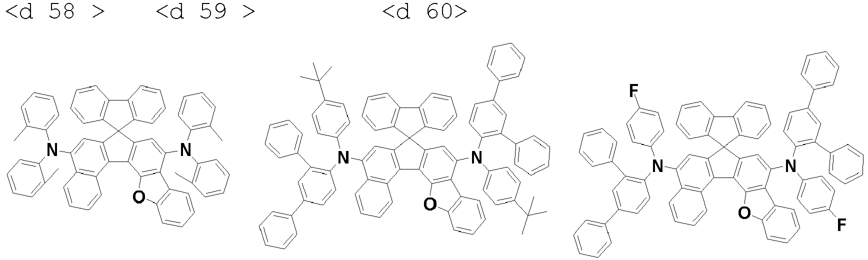

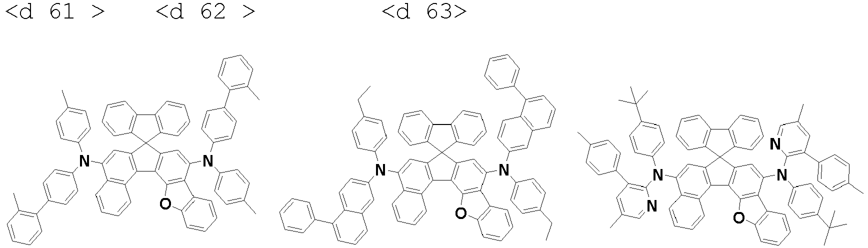

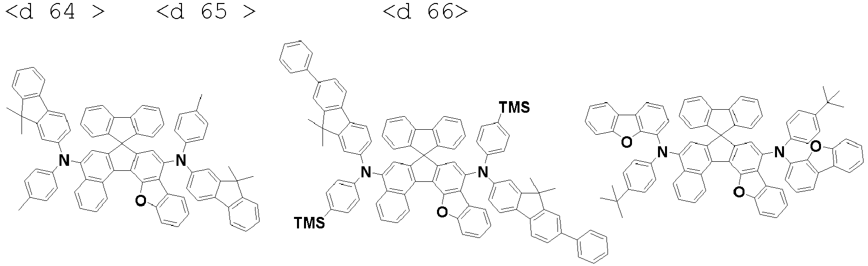

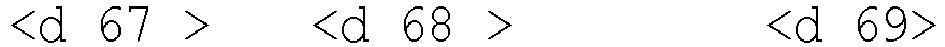

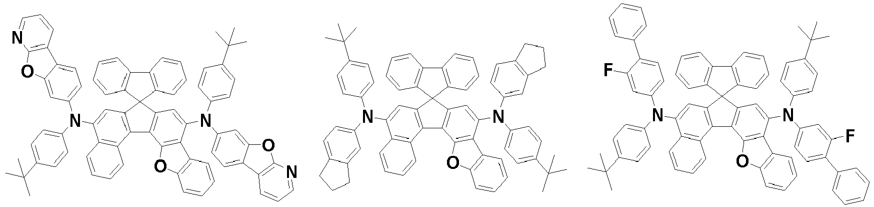

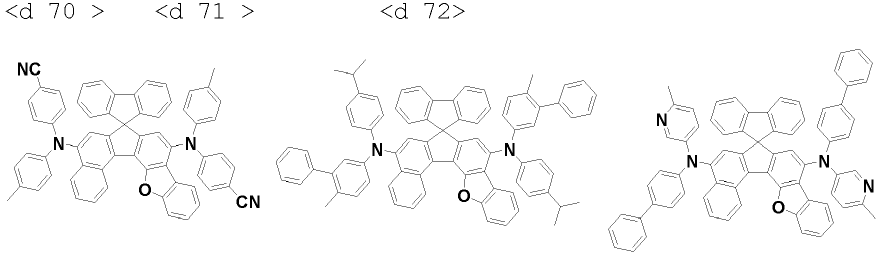

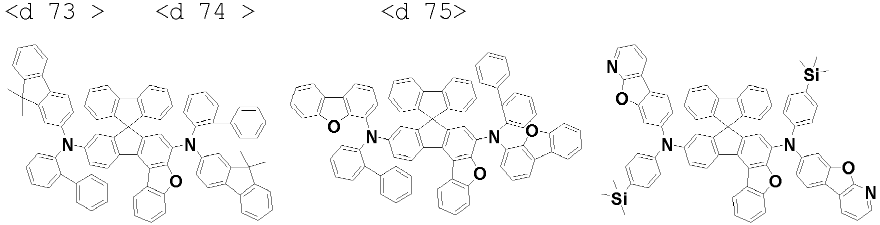

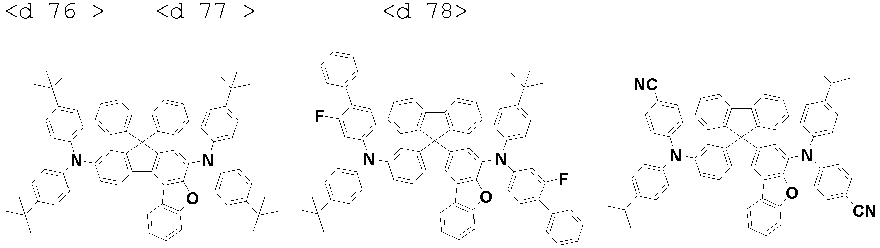

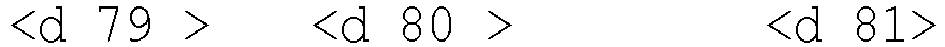

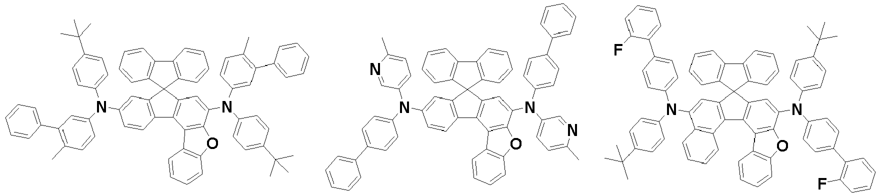

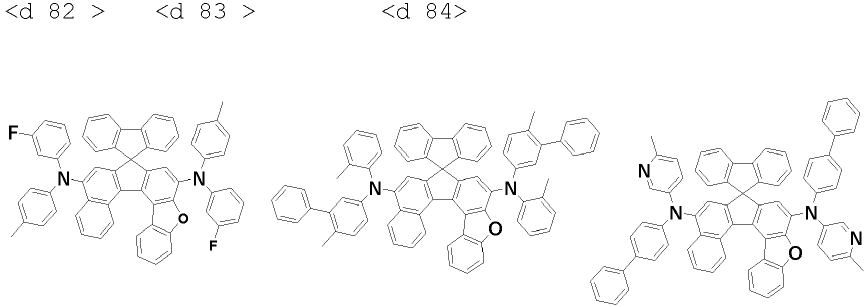

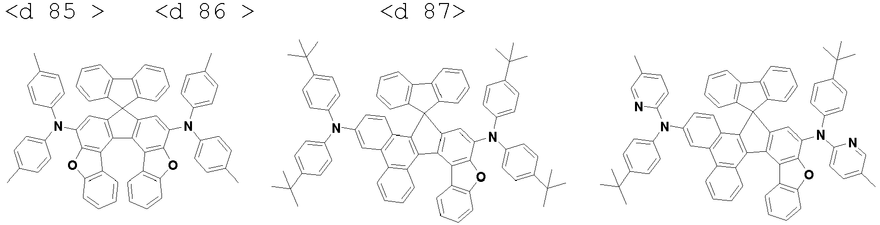

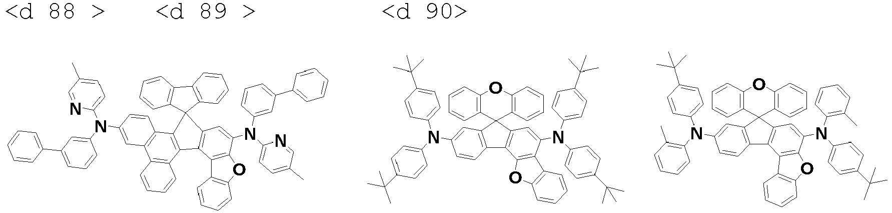

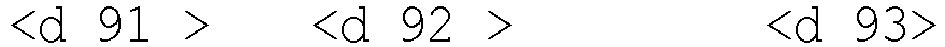

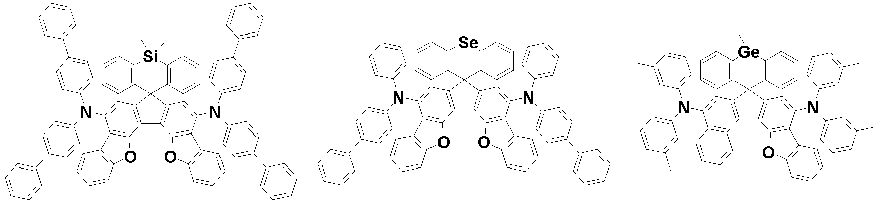

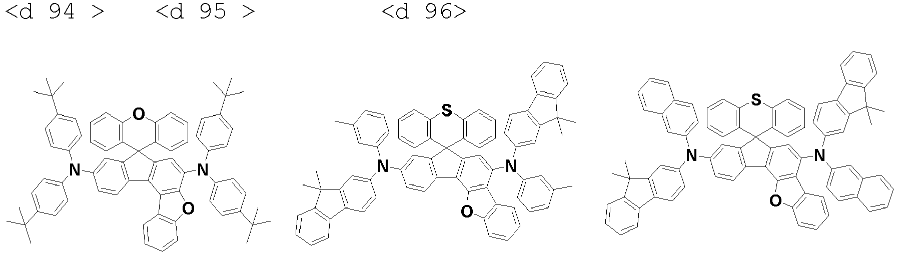

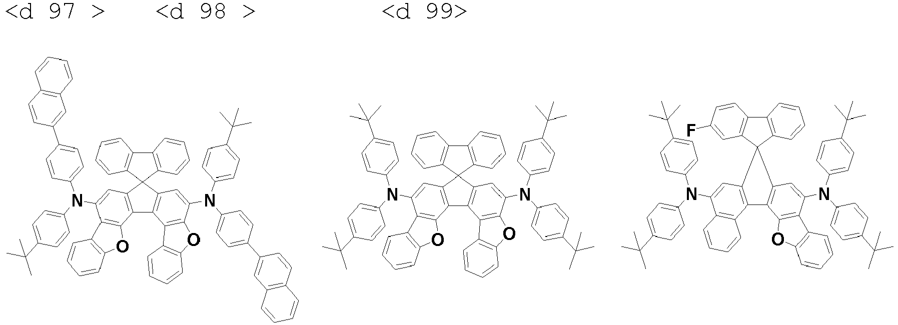

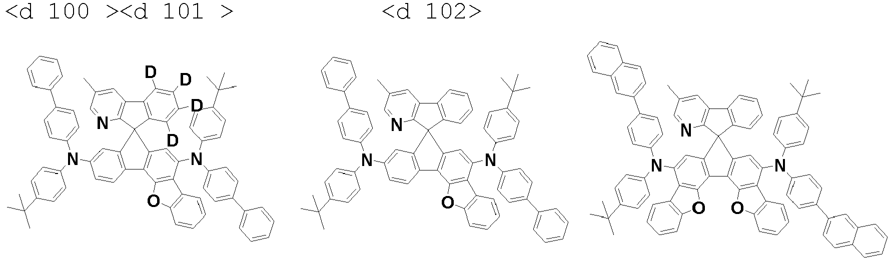

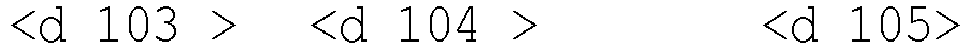

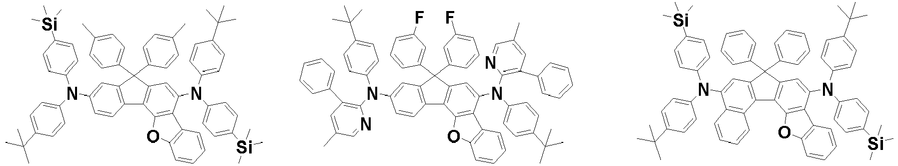

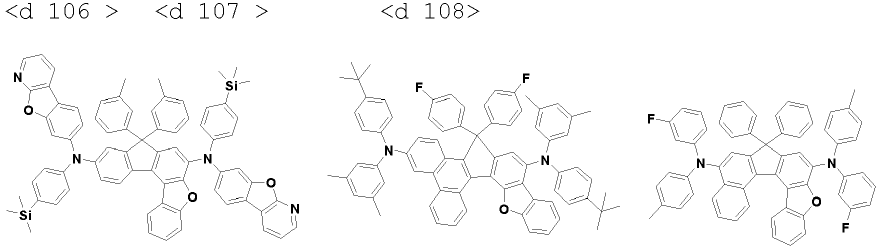

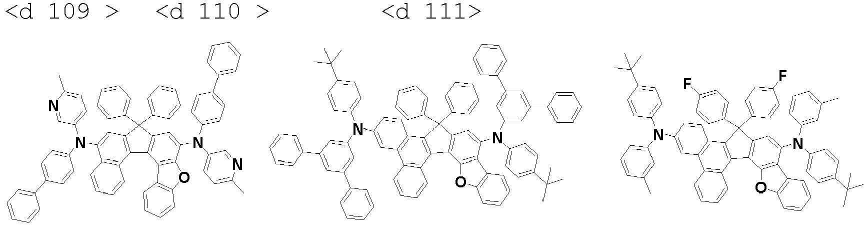

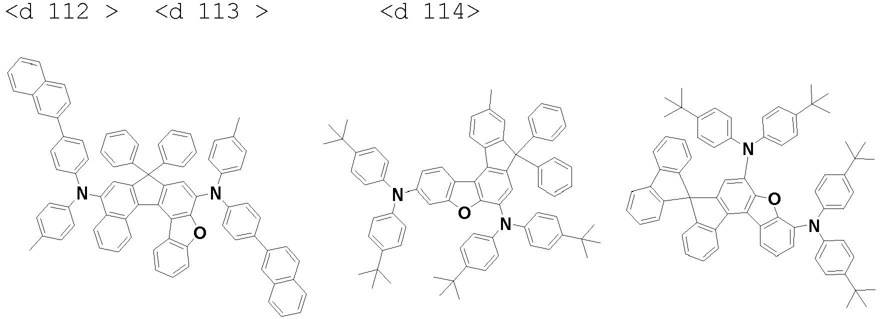

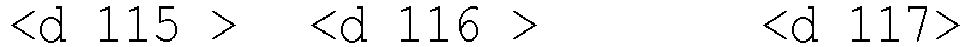

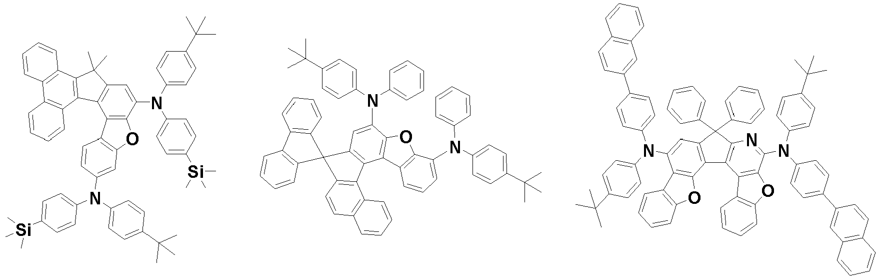

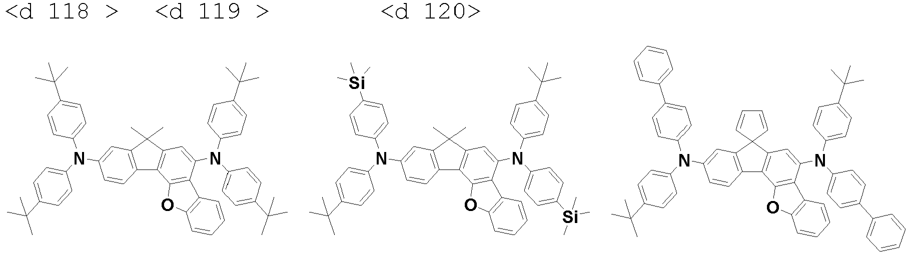

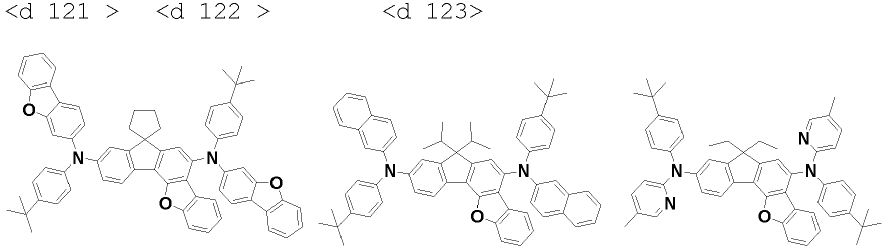

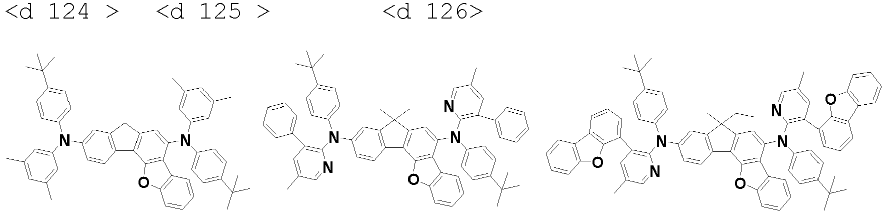

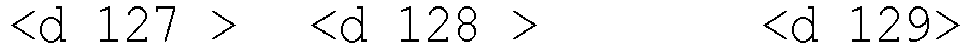

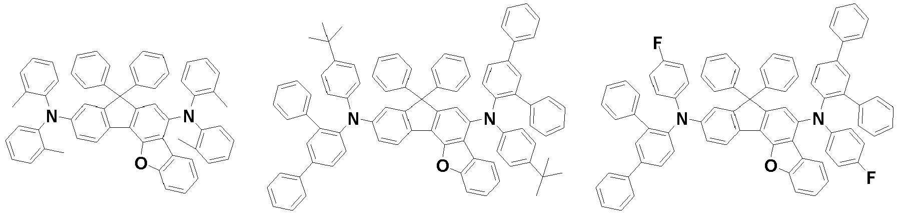

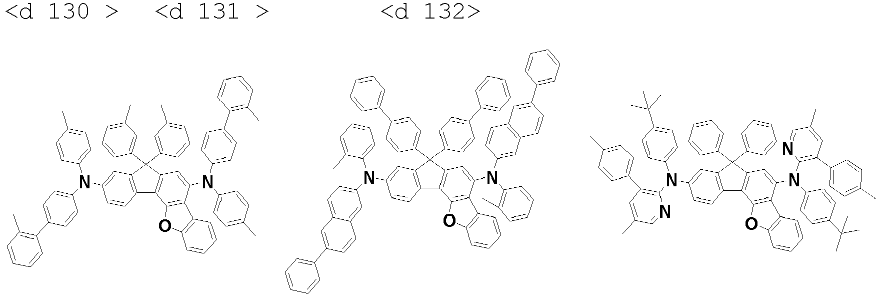

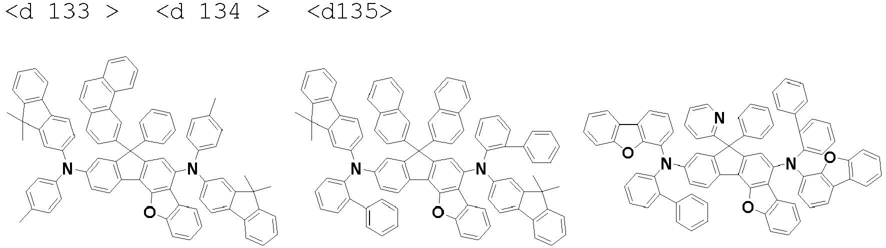

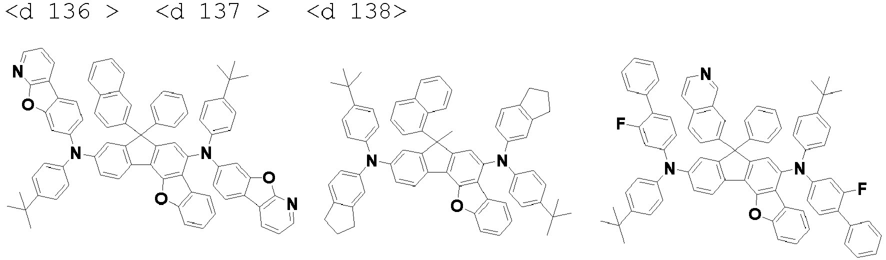

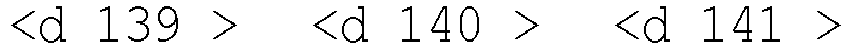

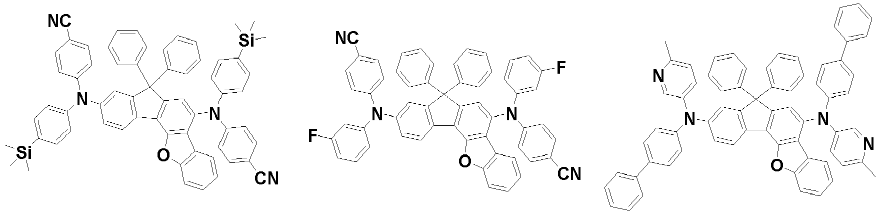

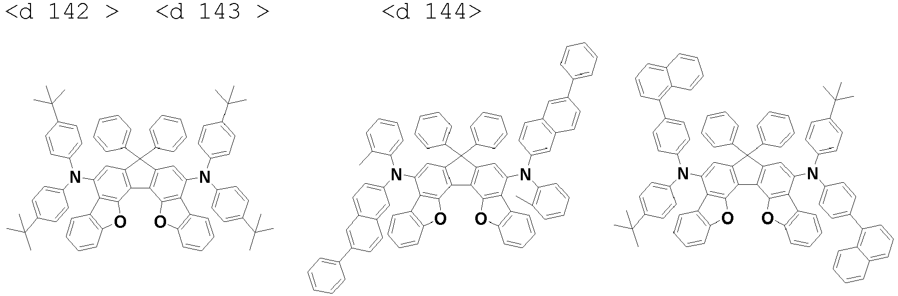

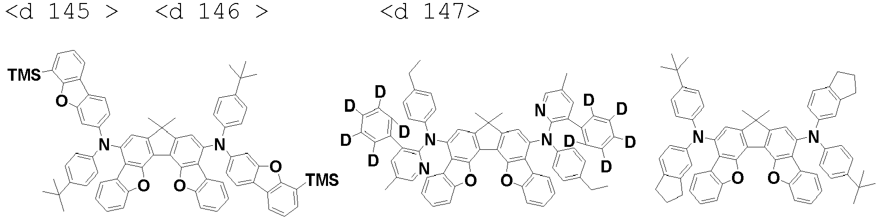

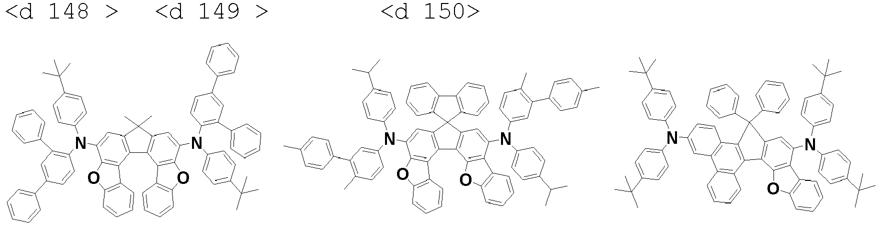

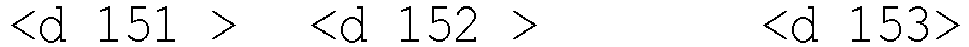

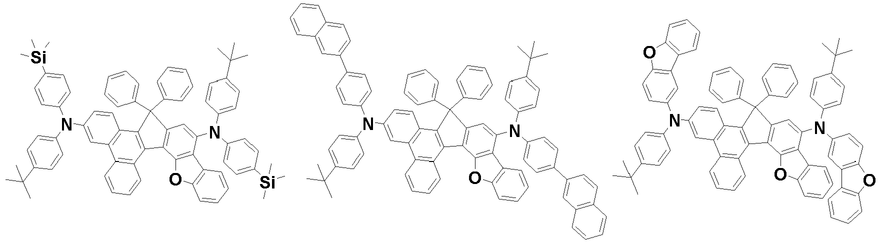

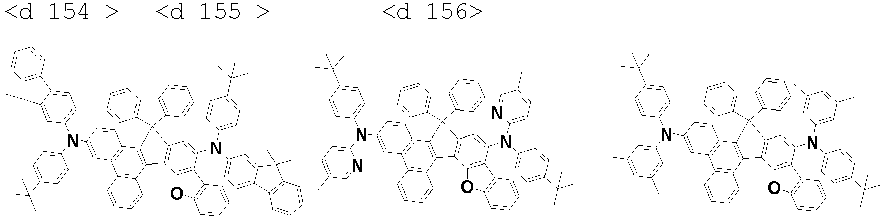

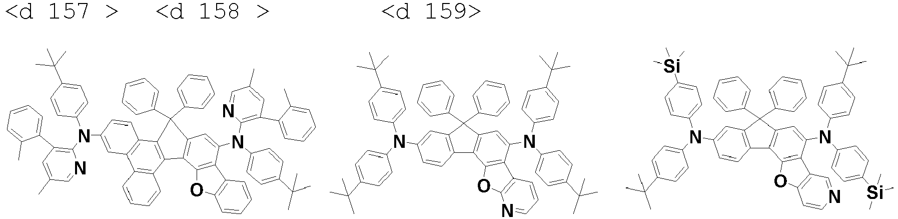

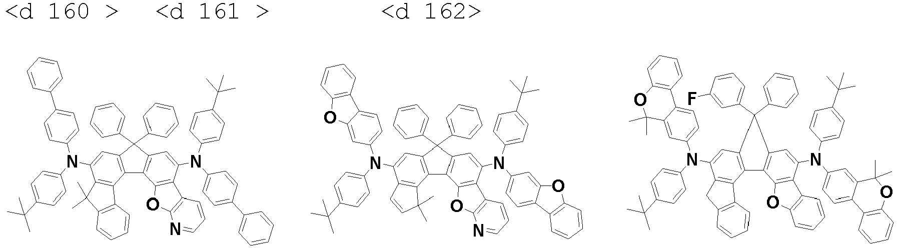

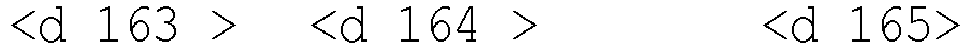

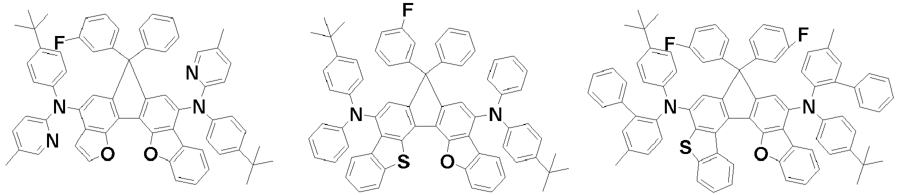

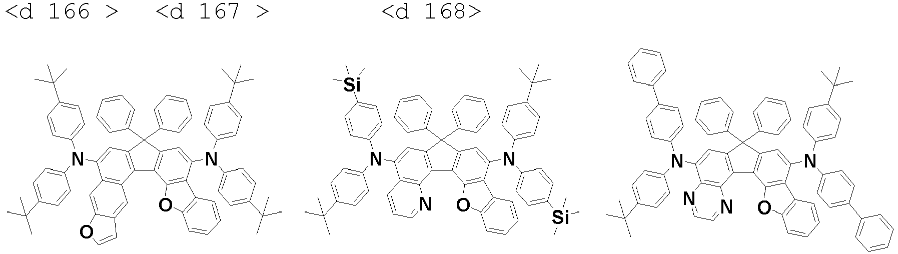

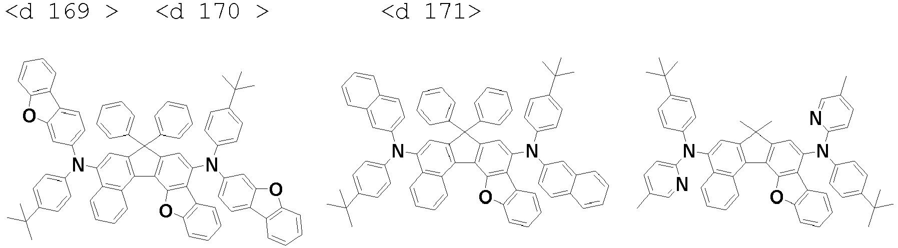

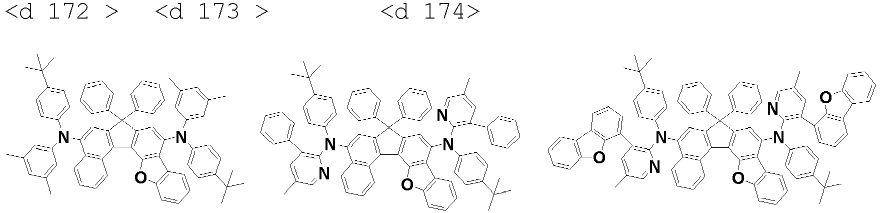

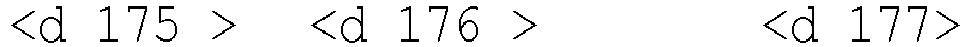

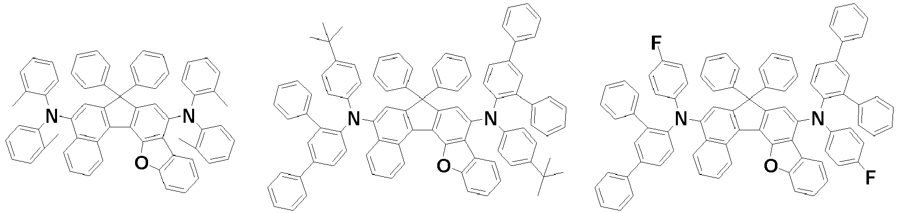

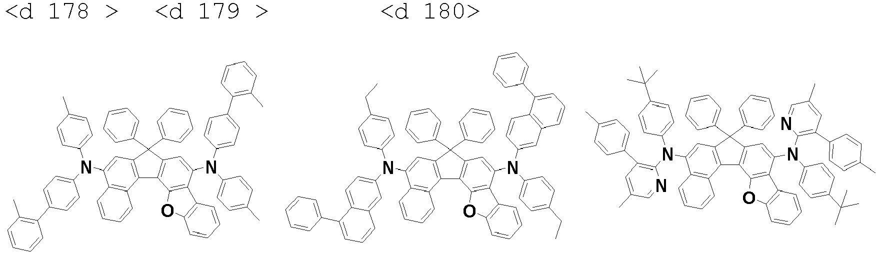

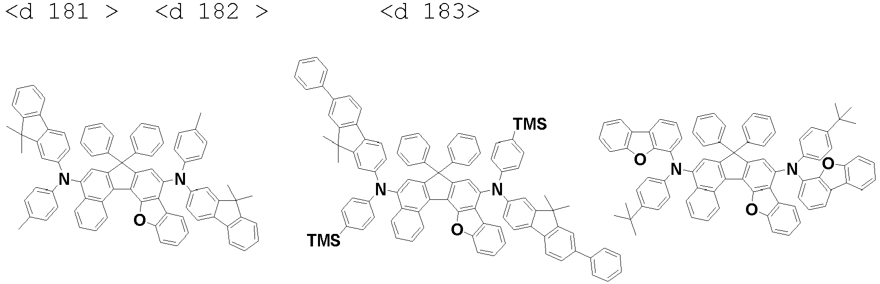

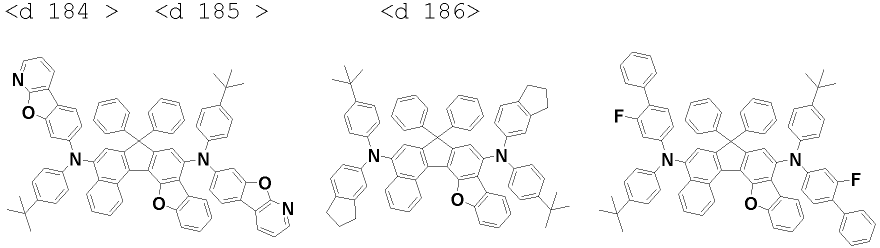

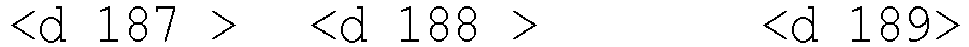

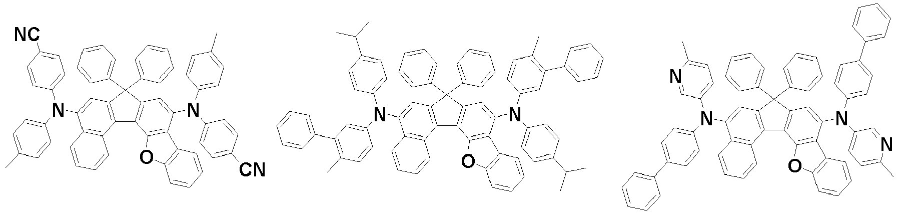

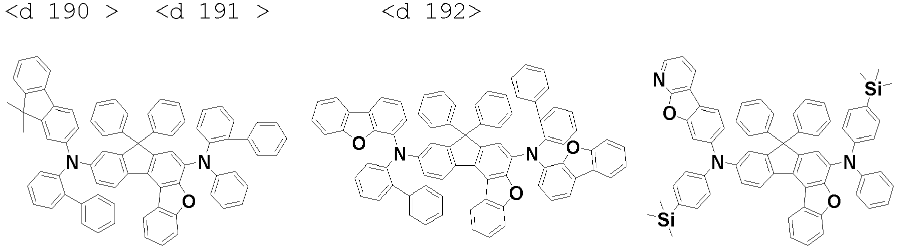

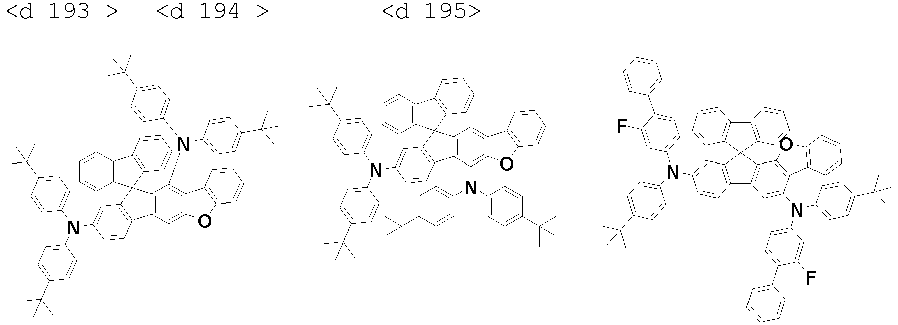

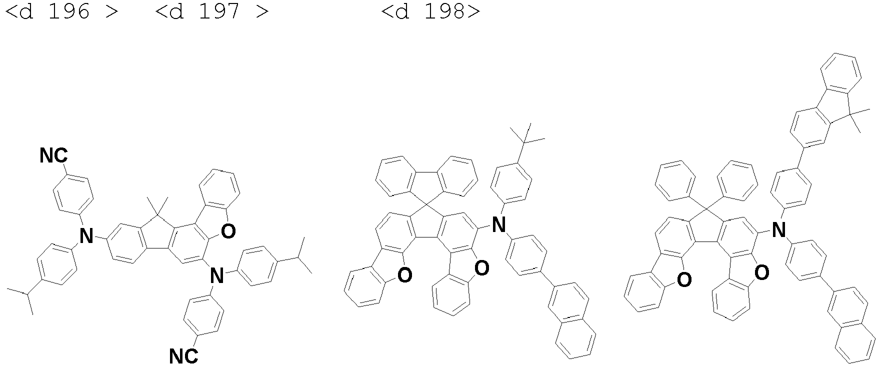

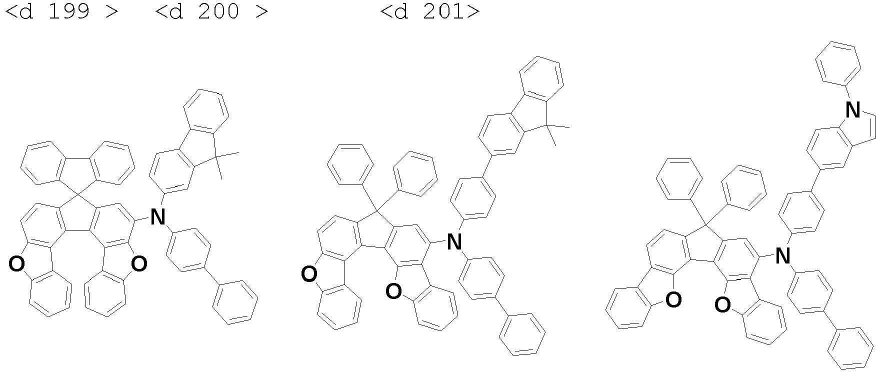

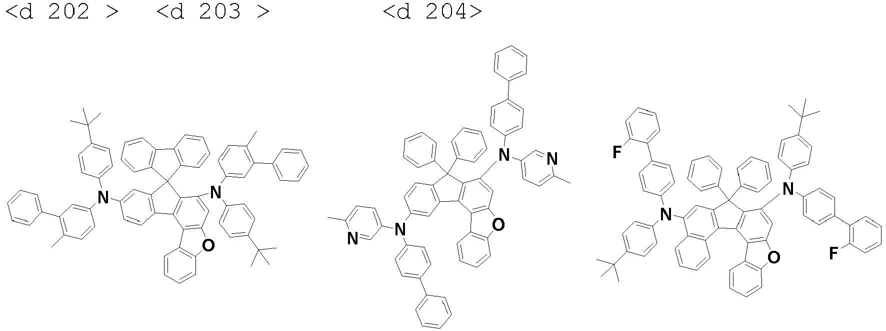

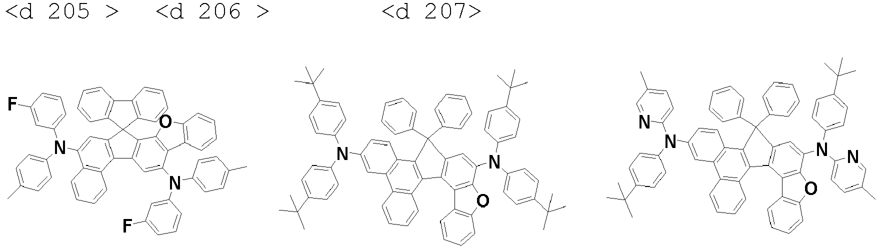

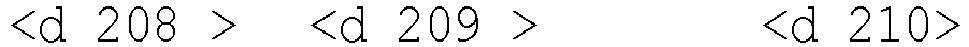

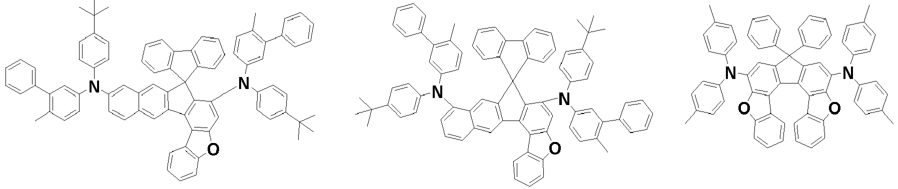

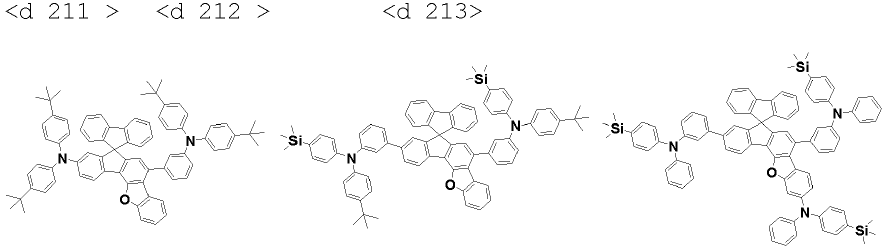

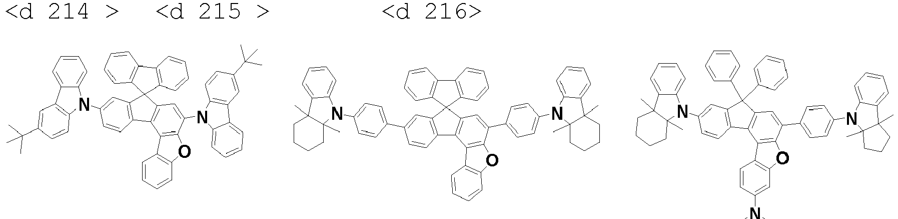

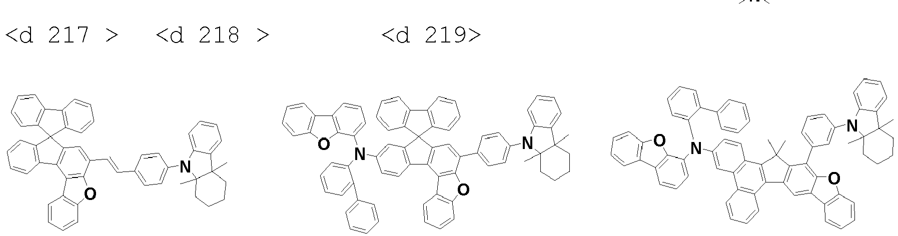

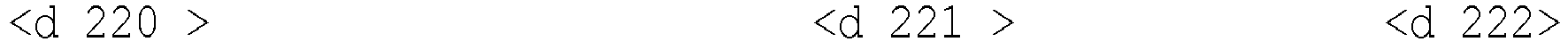

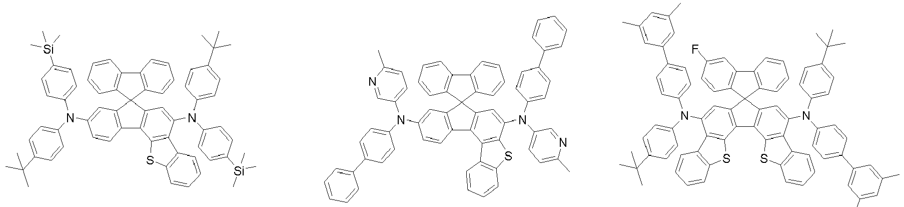

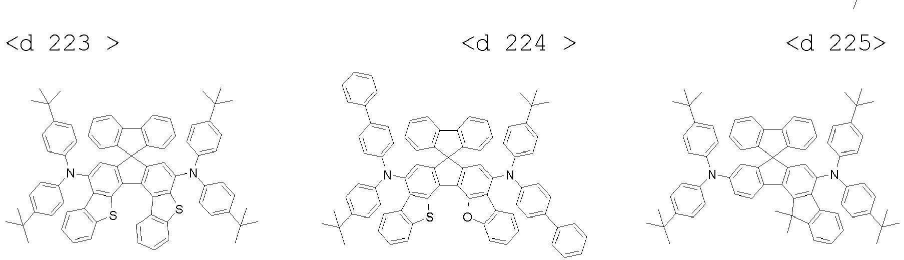

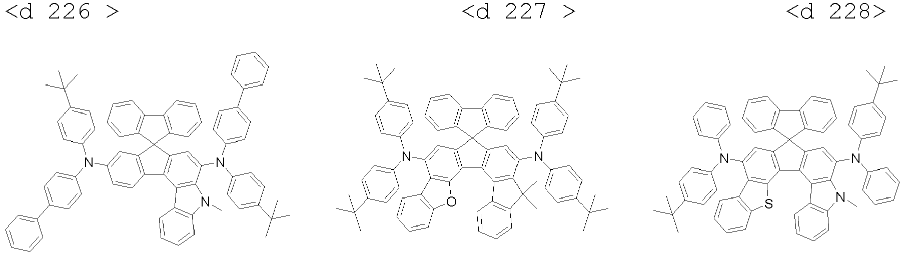

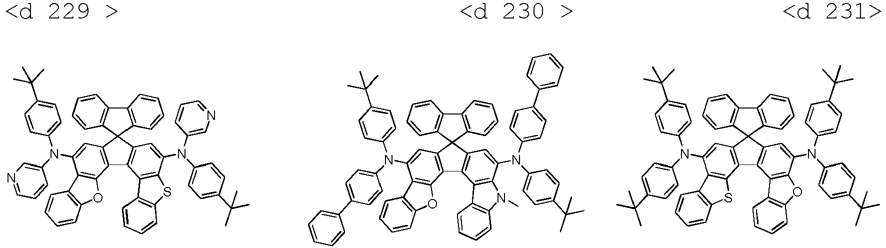

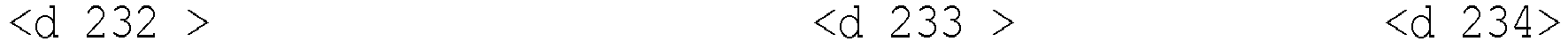

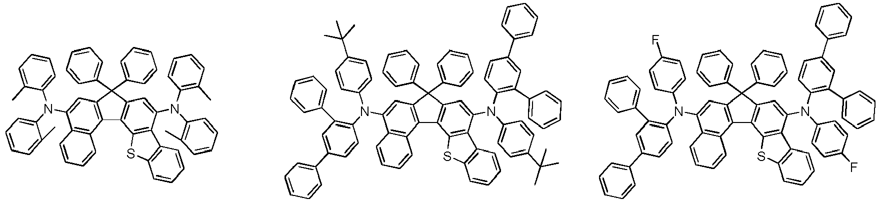

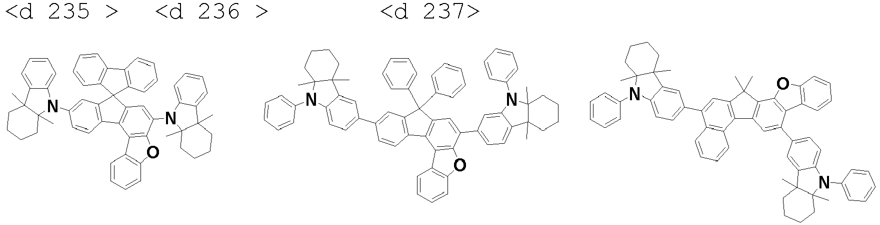

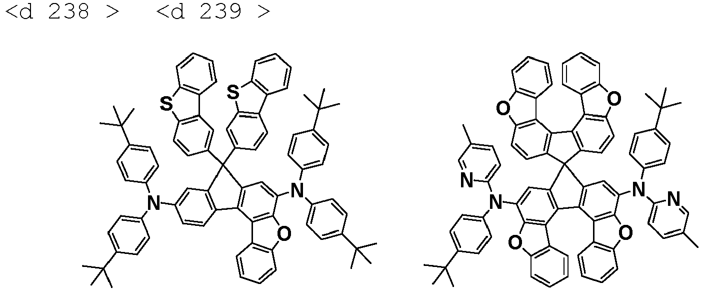

In the present disclosure, examples of the compound represented by Chemical Formula D3 as the dopant include the compounds of the following <D101> to < D130>:

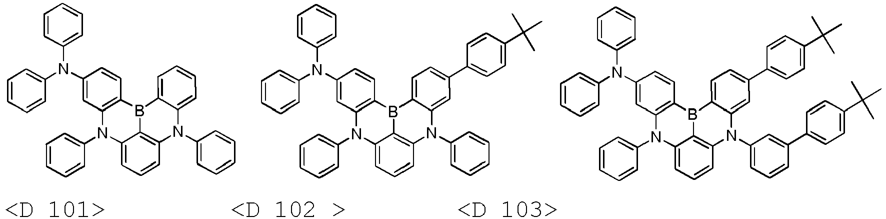

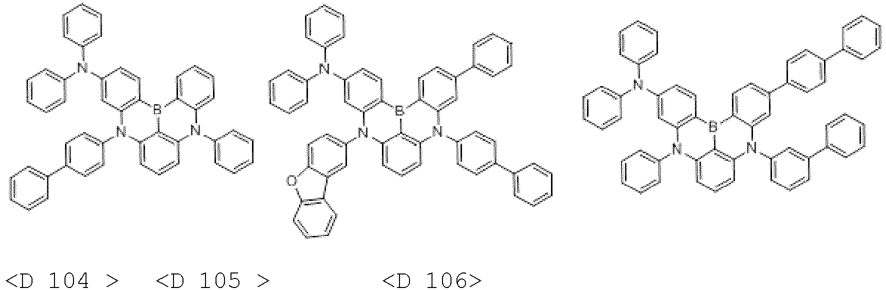

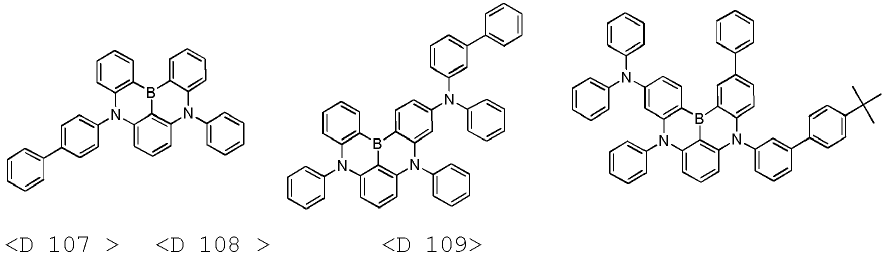

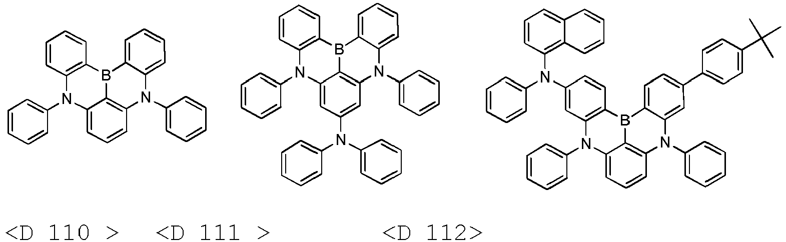

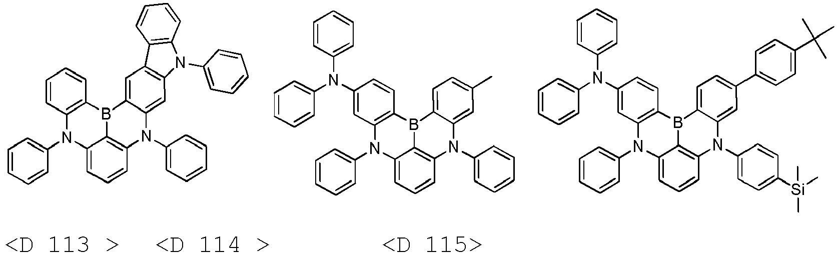

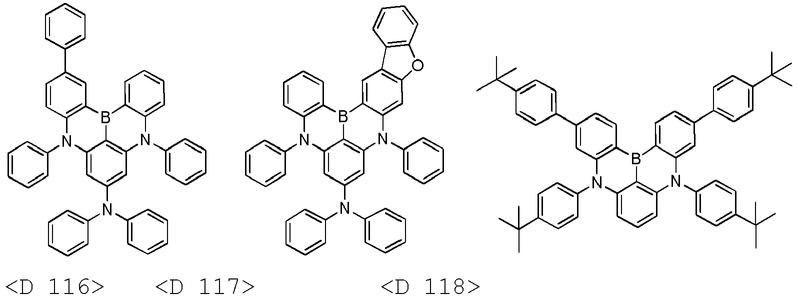

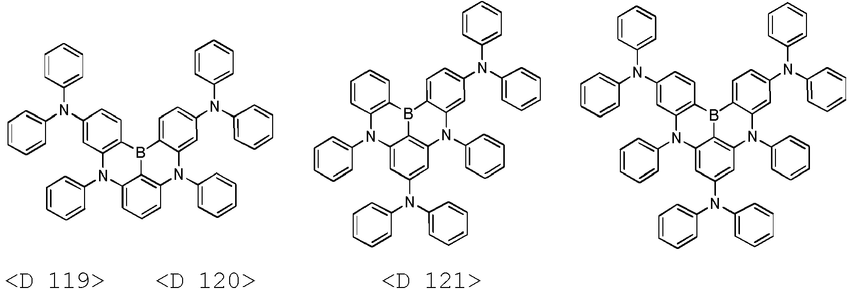

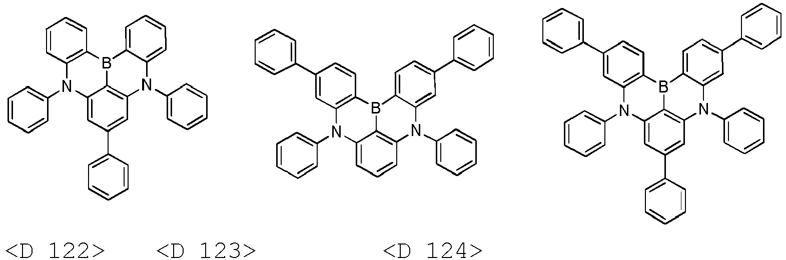

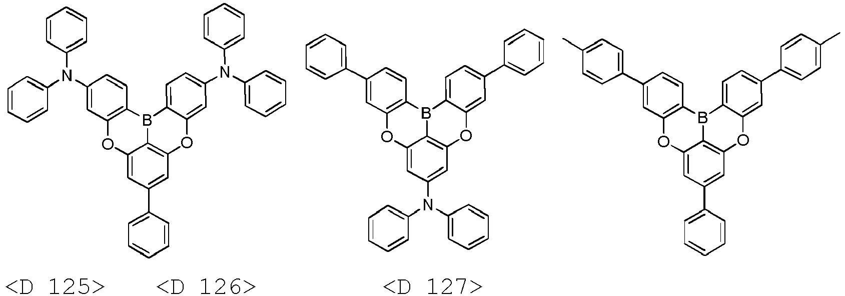

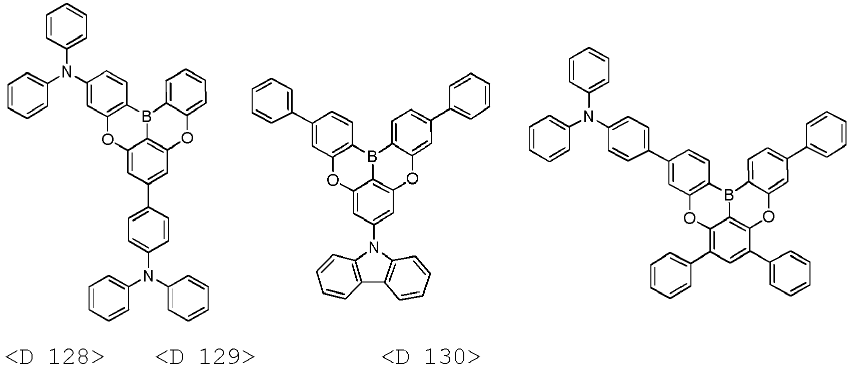

In addition, concrete examples of the compound represented by Chemical Formula D4 or Chemical Formula D5 include [D201] to [D280]:

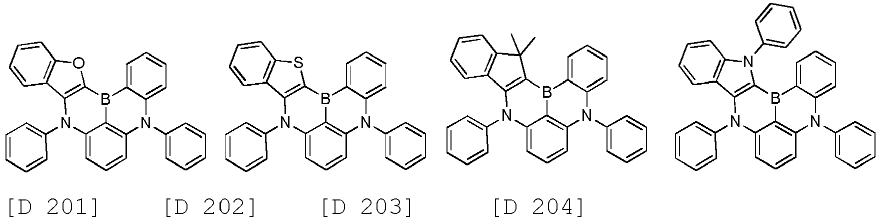

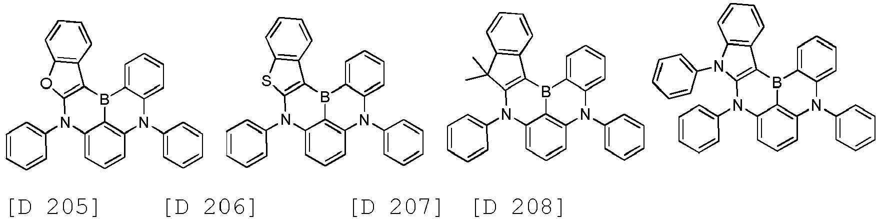

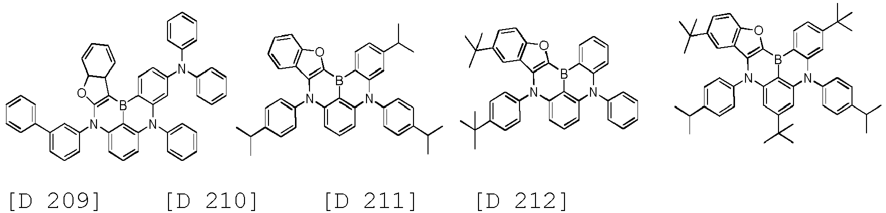

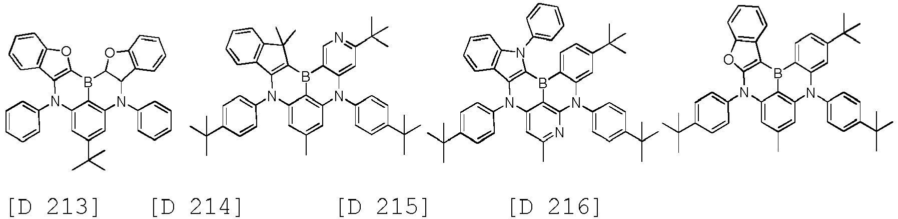

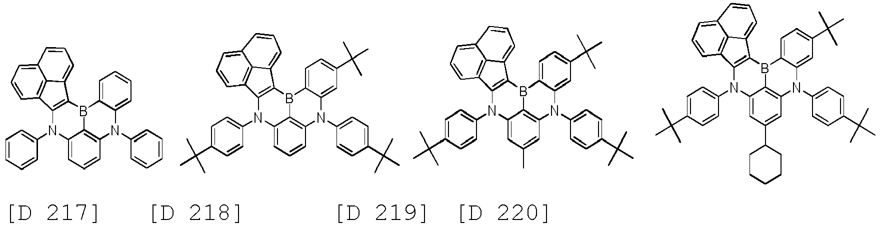

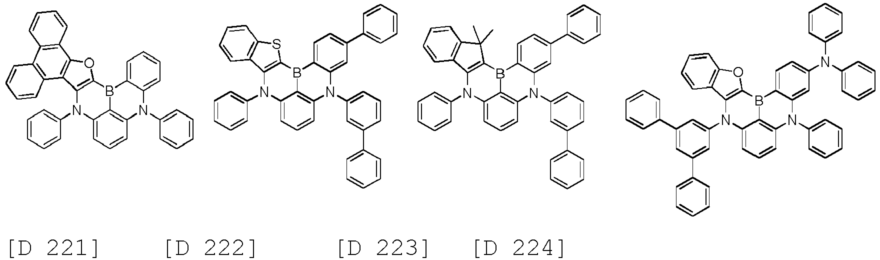

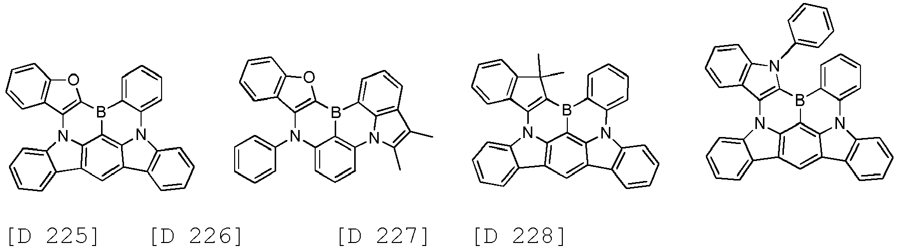

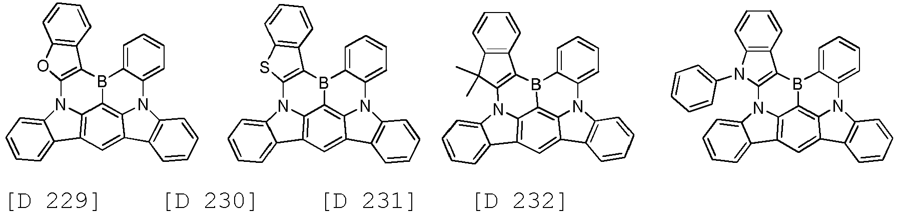

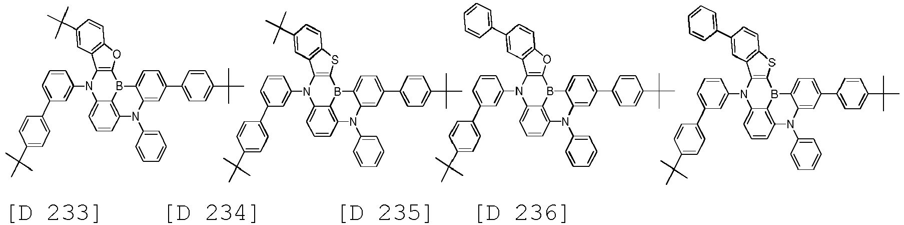

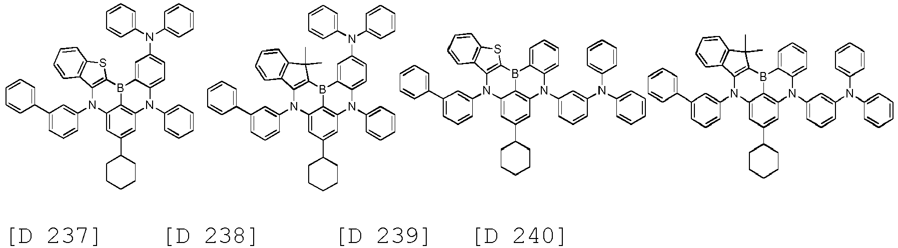

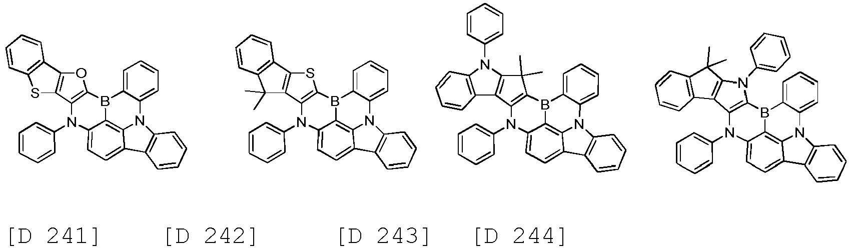

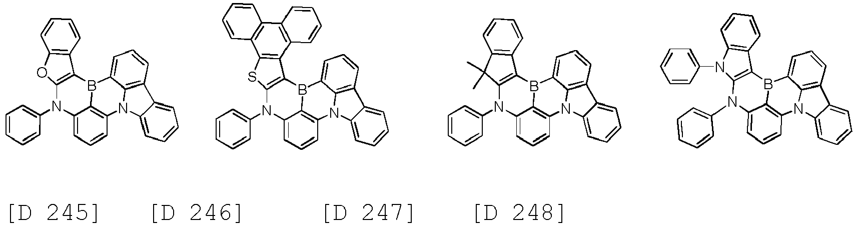

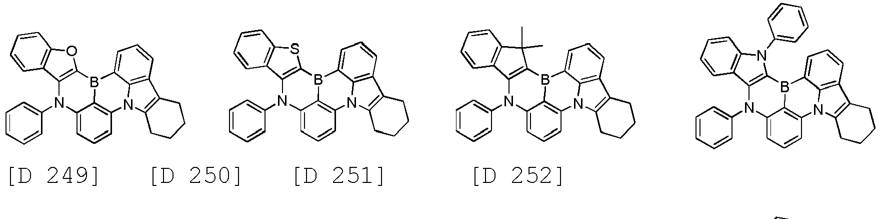

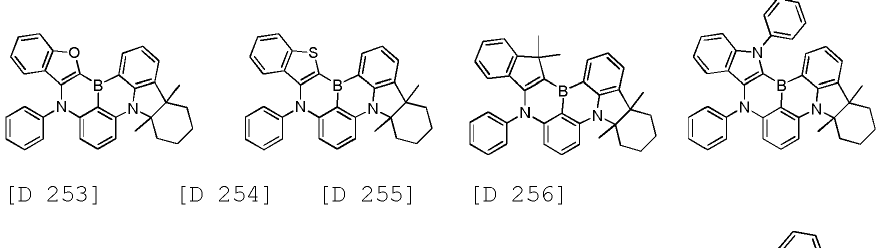

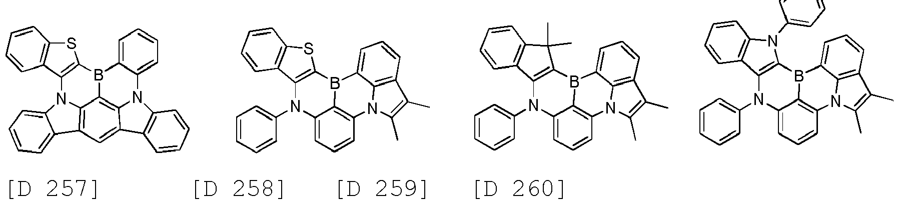

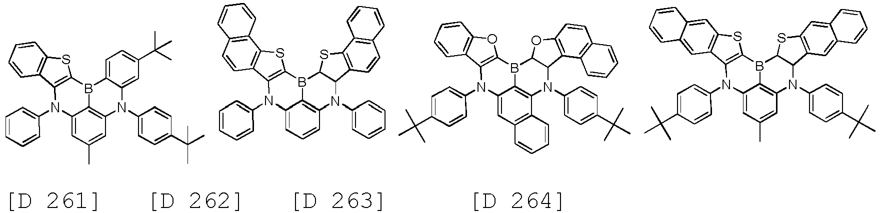

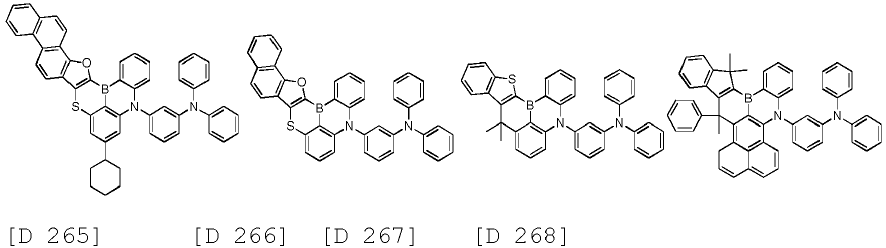

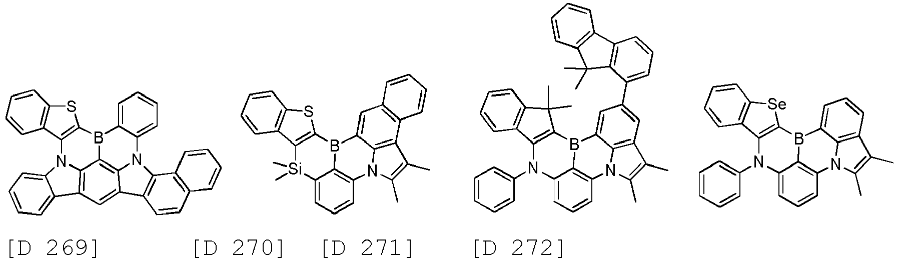

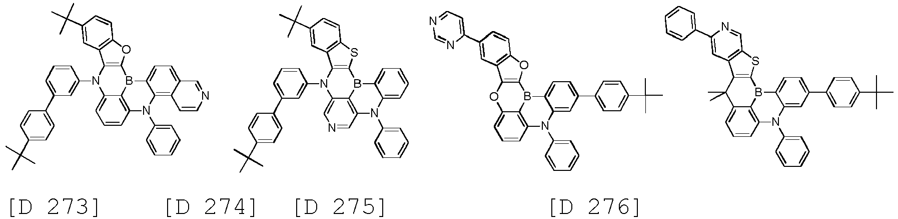

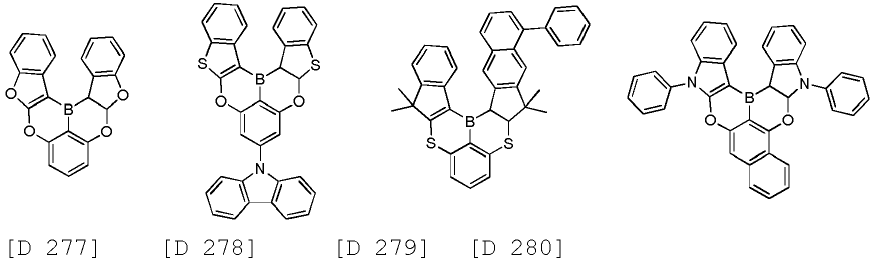

In addition, concrete examples of the compounds represented by [Chemical Formula D6] or [Chemical Formula D7] include compounds of the following <D301> to <D387>:

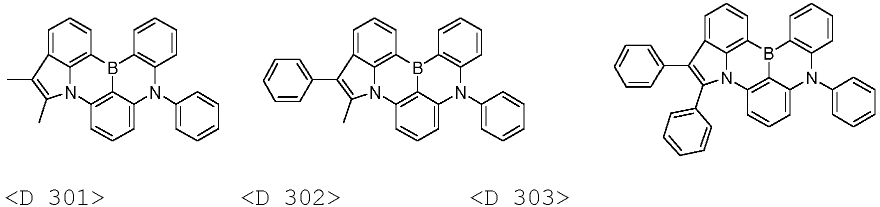

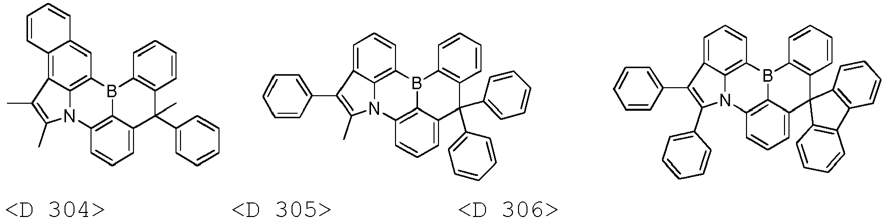

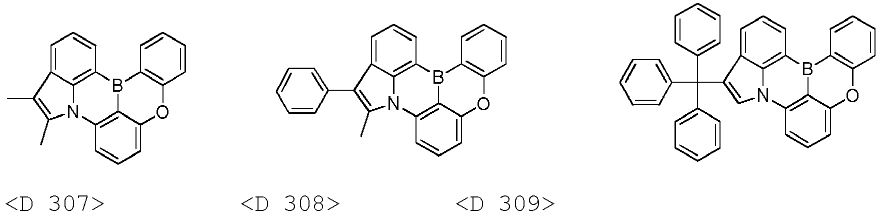

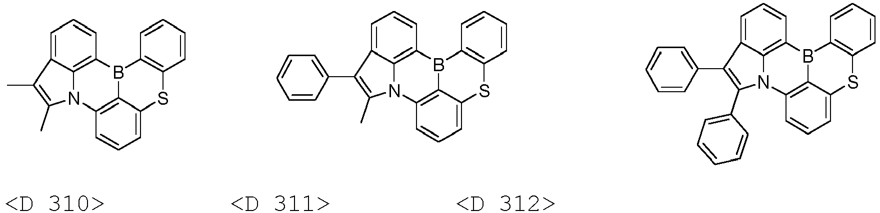

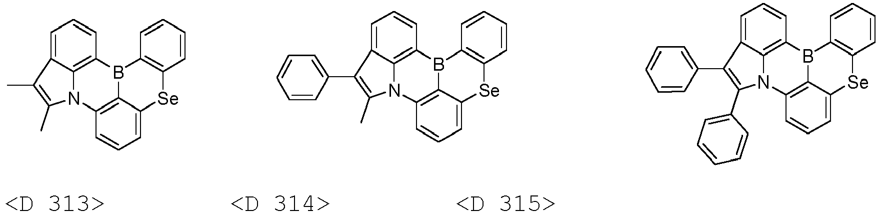

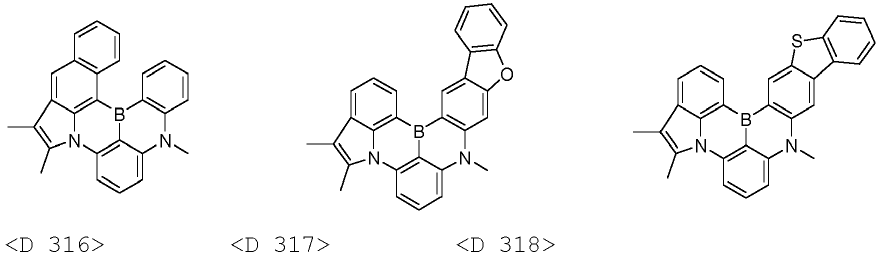

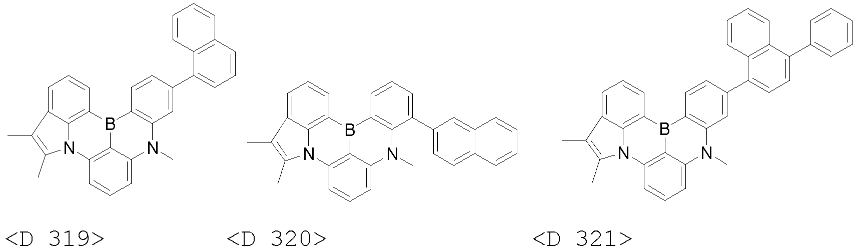

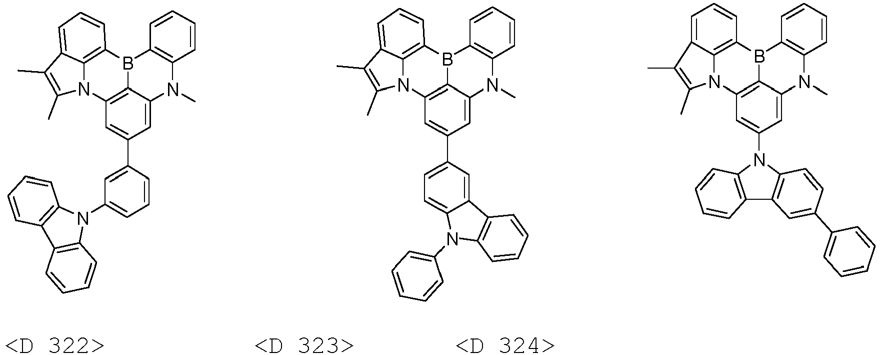

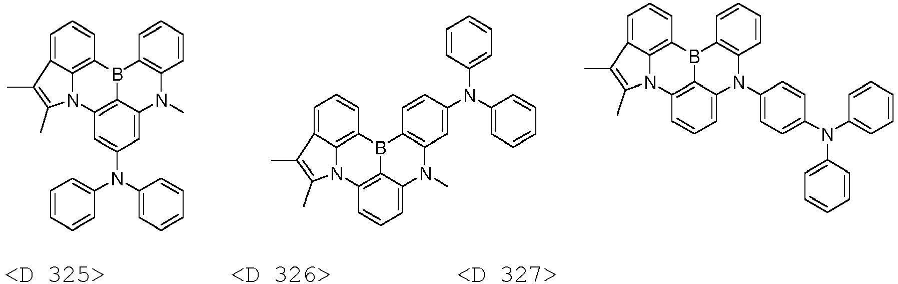

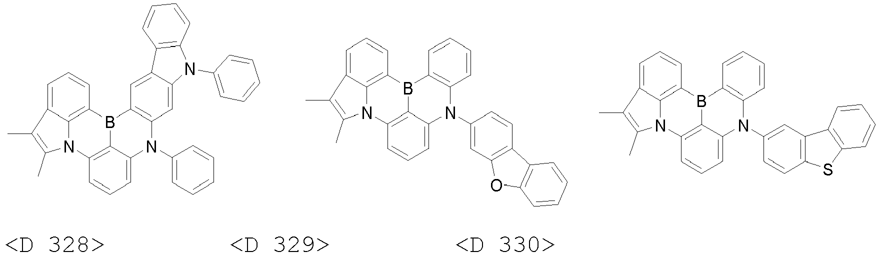

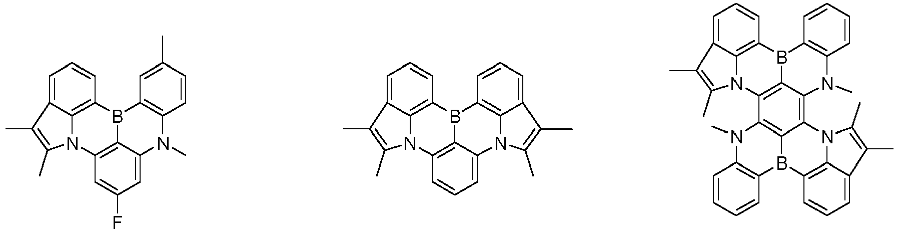

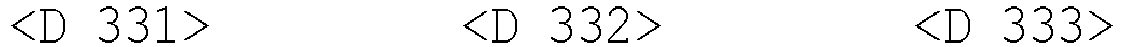

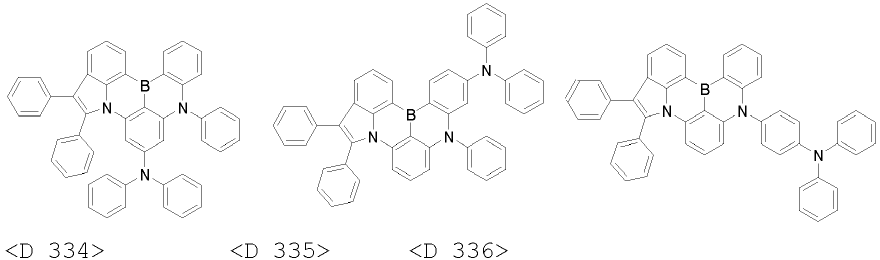

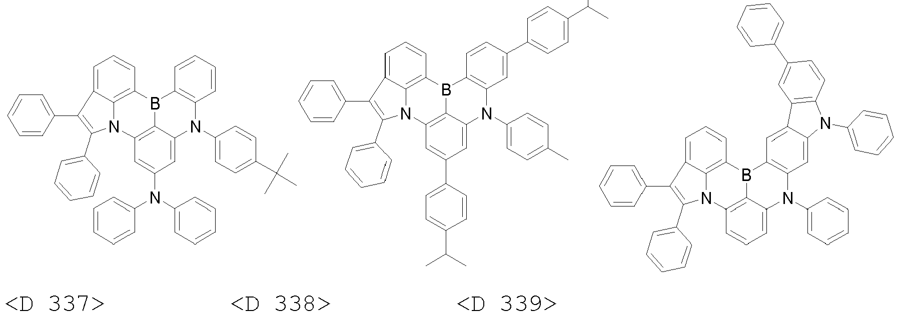

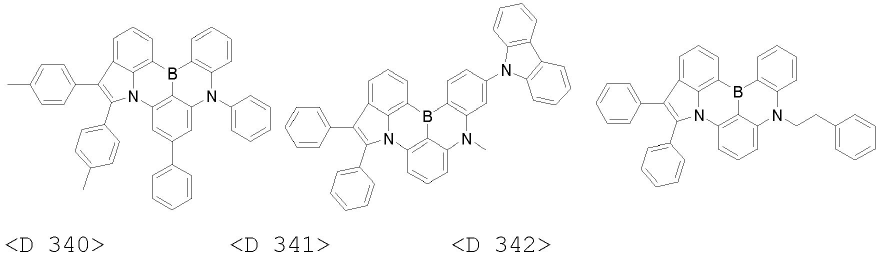

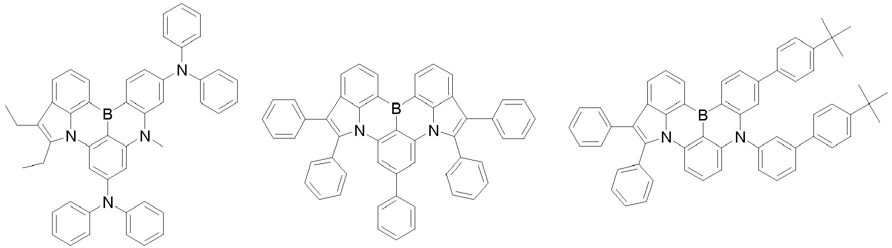

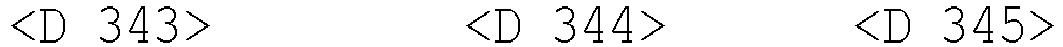

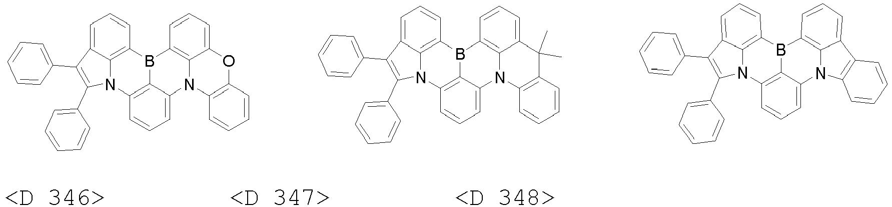

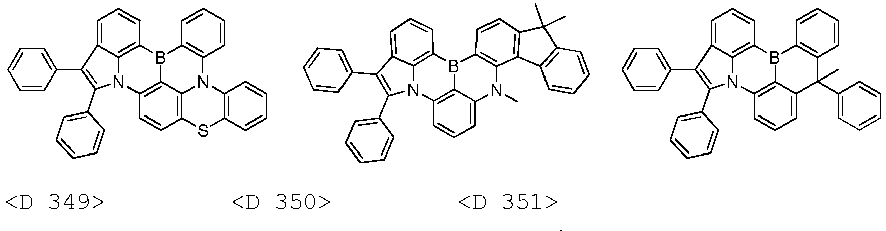

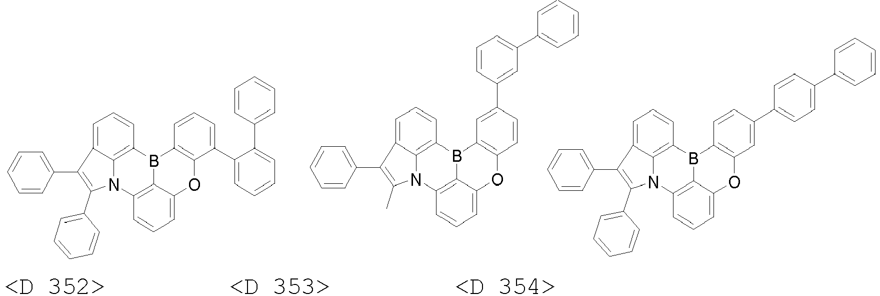

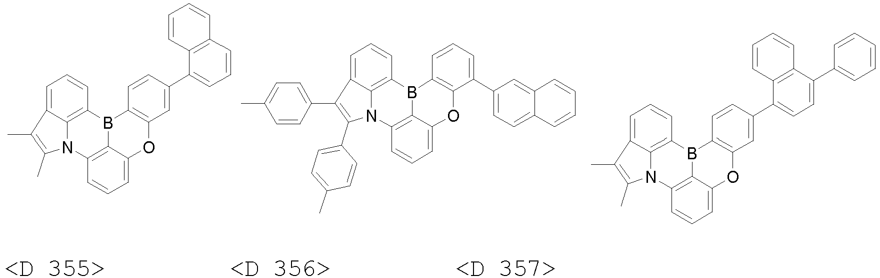

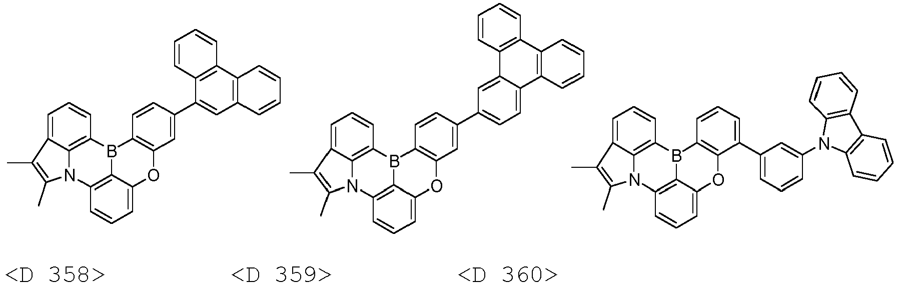

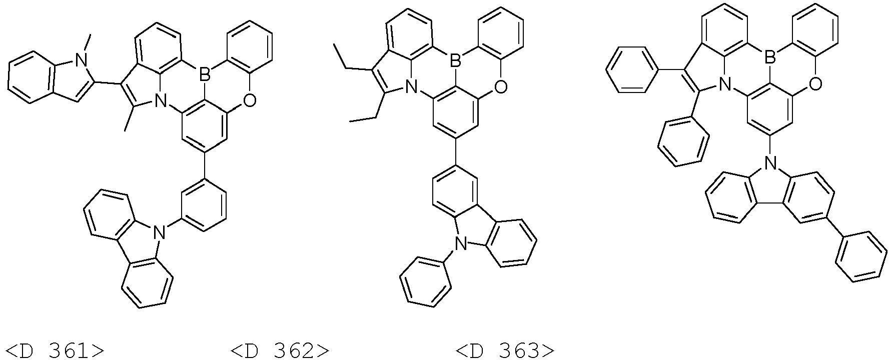

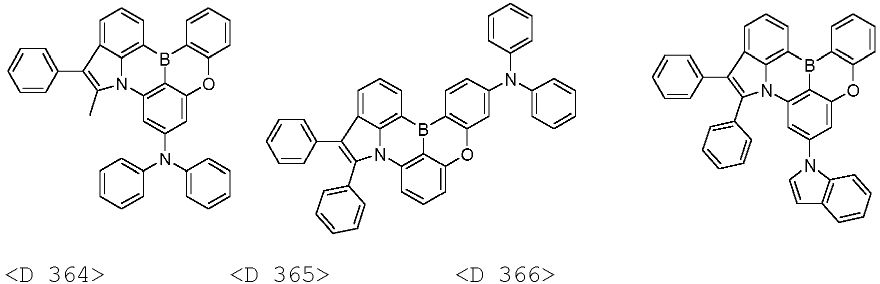

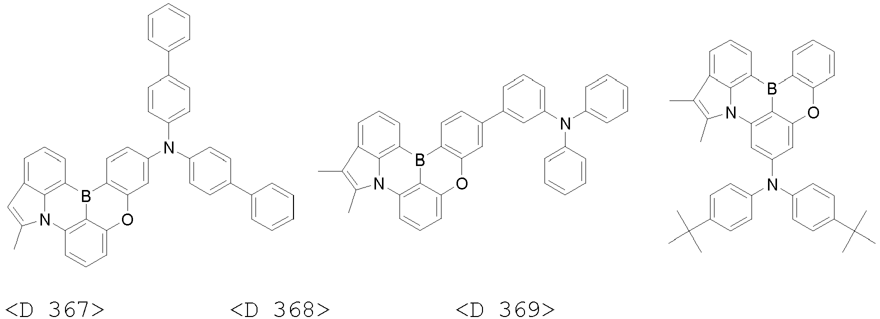

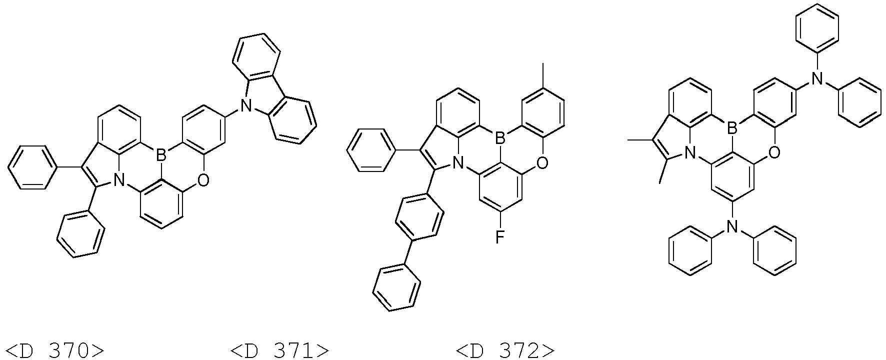

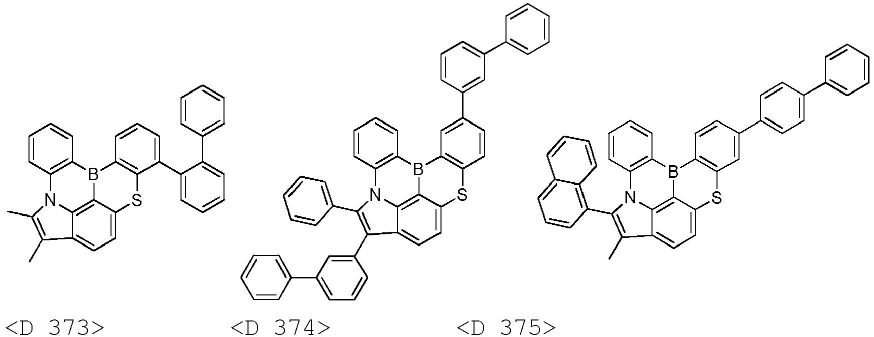

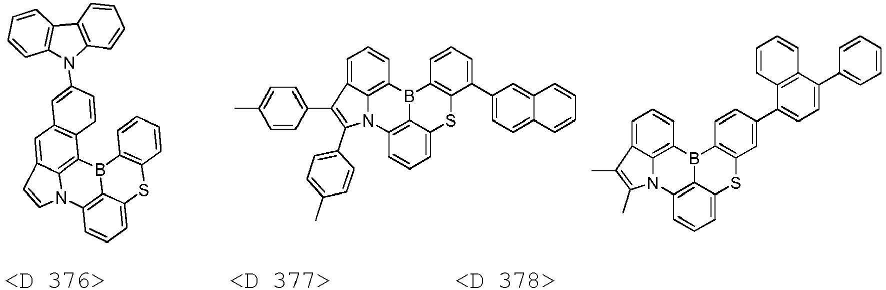

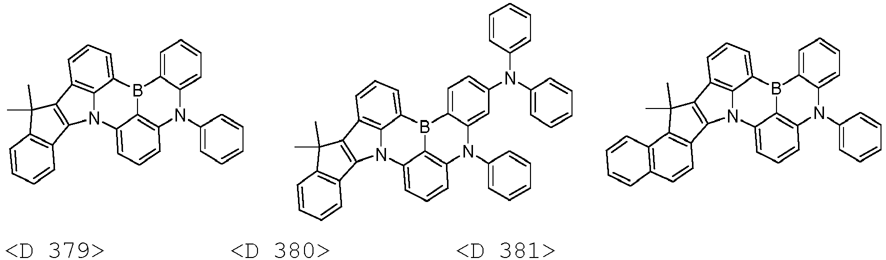

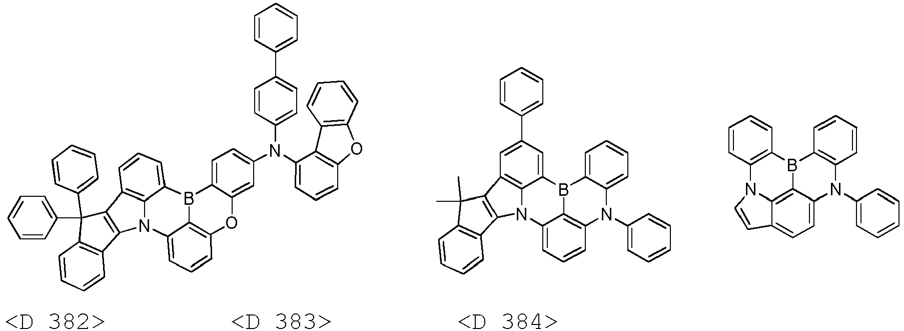

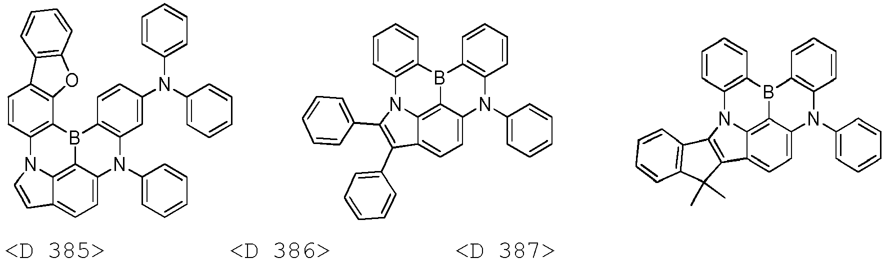

Furthermore, concrete examples of the compound represented by [Chemical Formula D8] to [Chemical Formula D10] include the compounds represented by the following [D401] to [D532] :

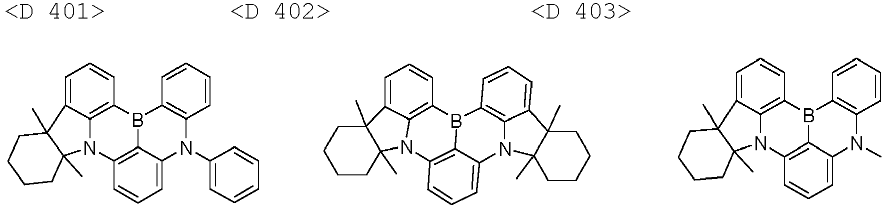

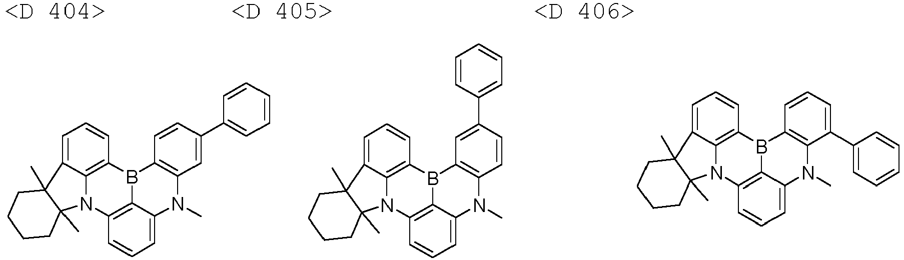

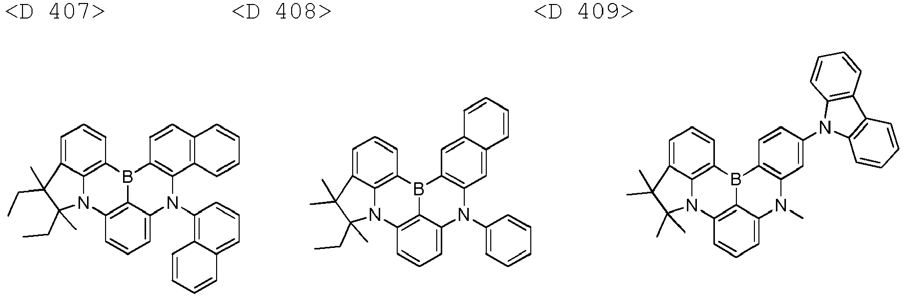

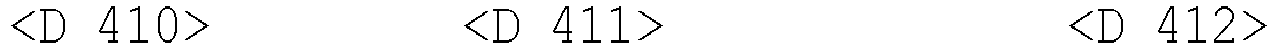

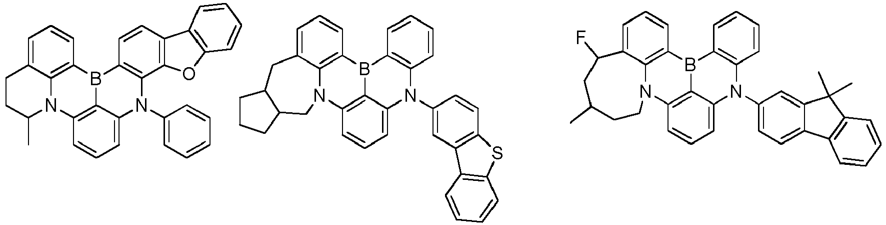

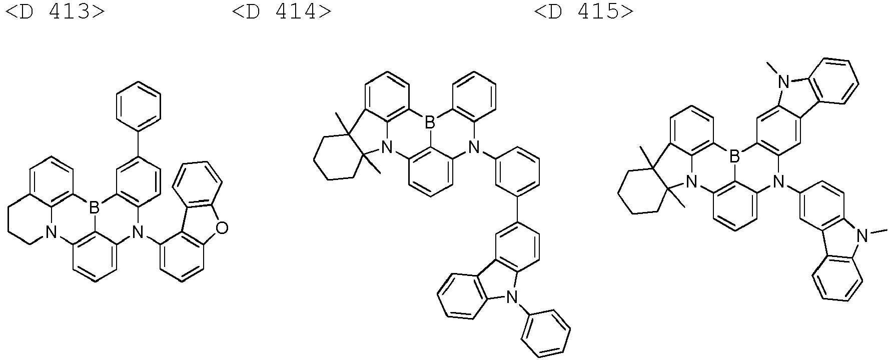

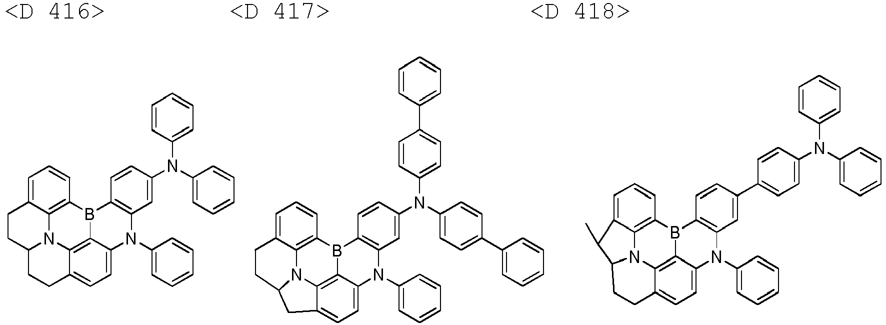

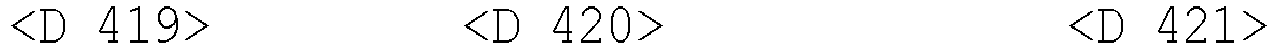

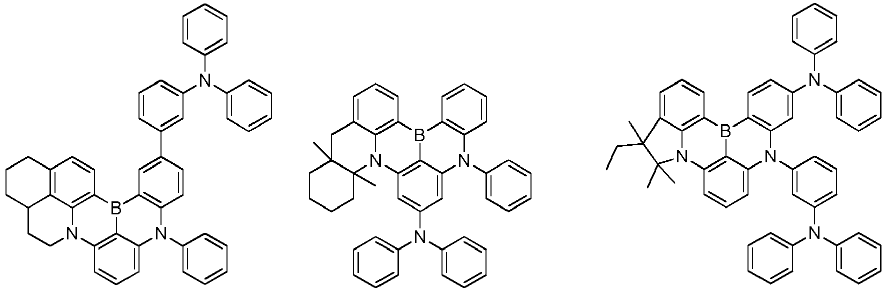

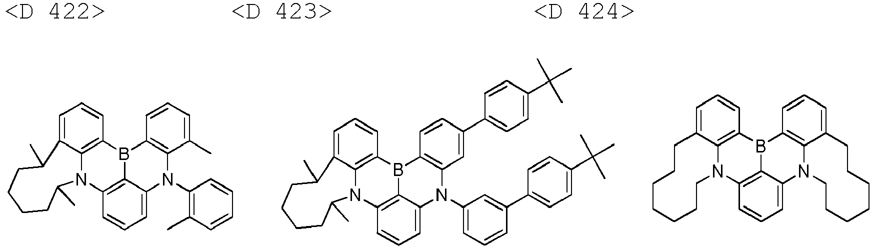

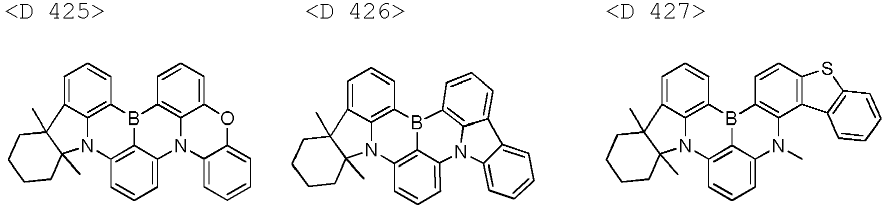

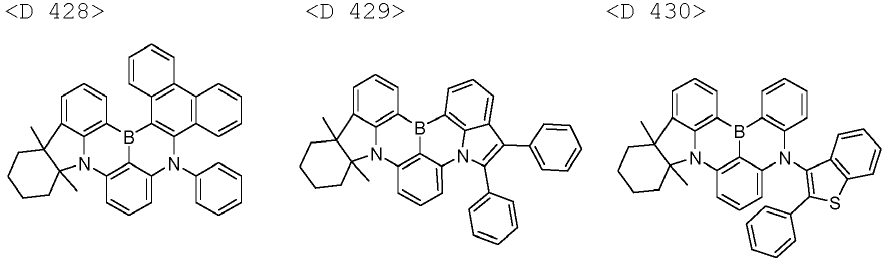

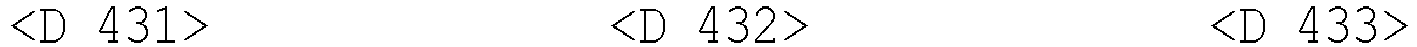

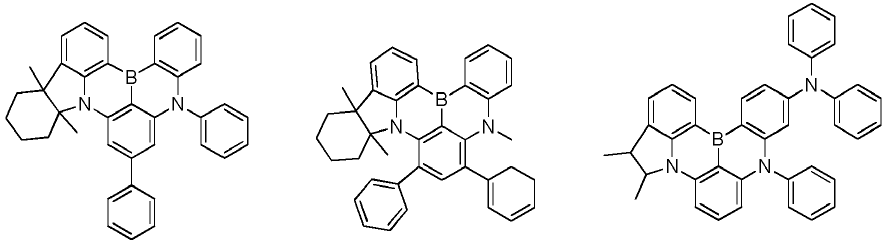

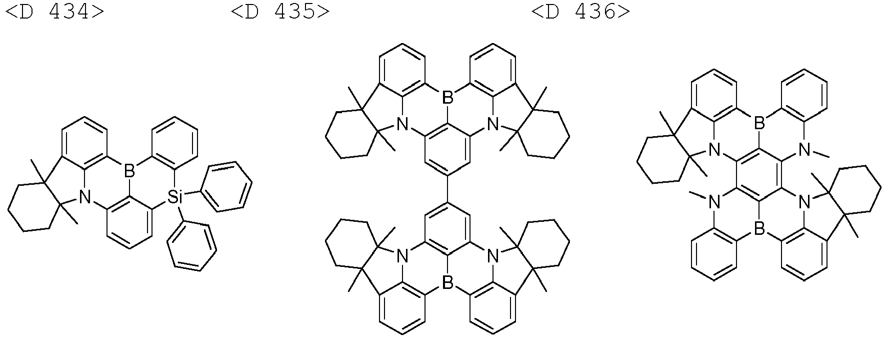

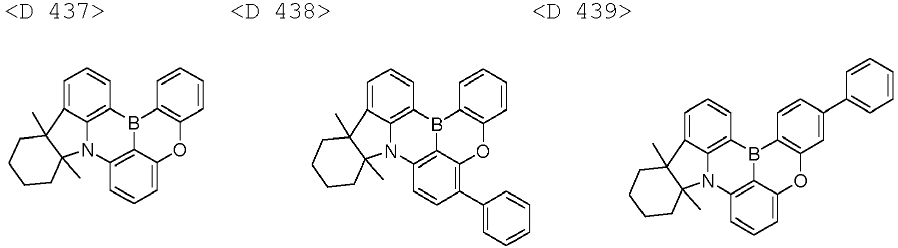

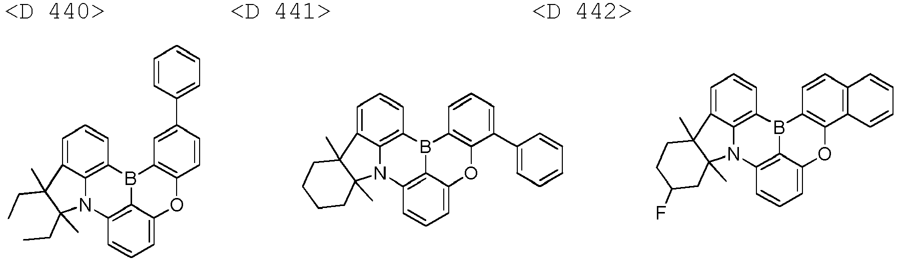

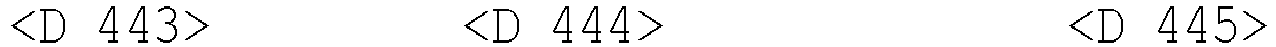

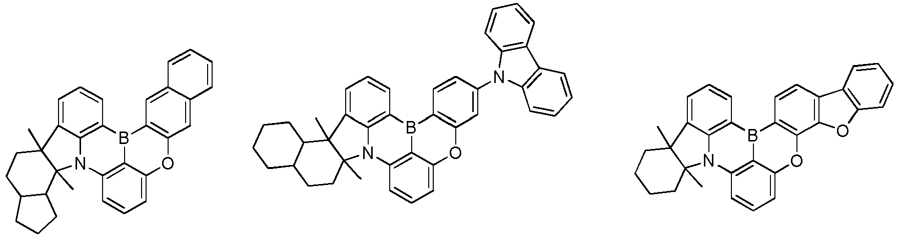

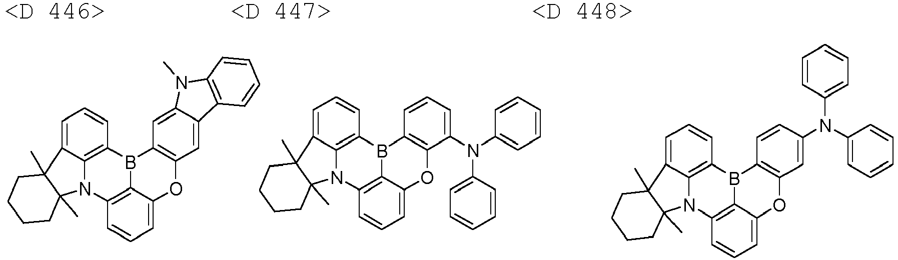

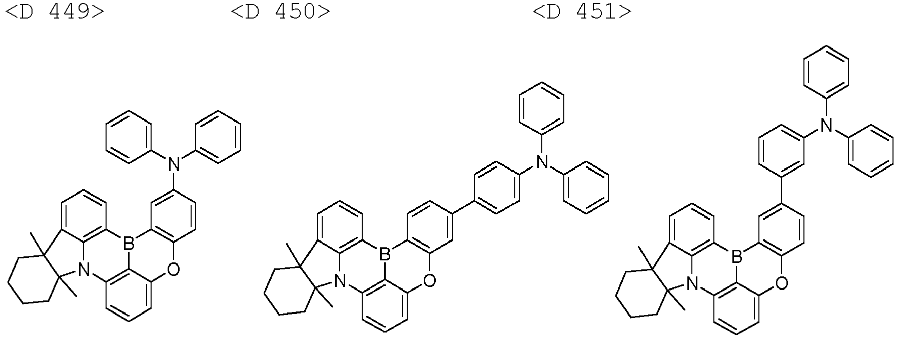

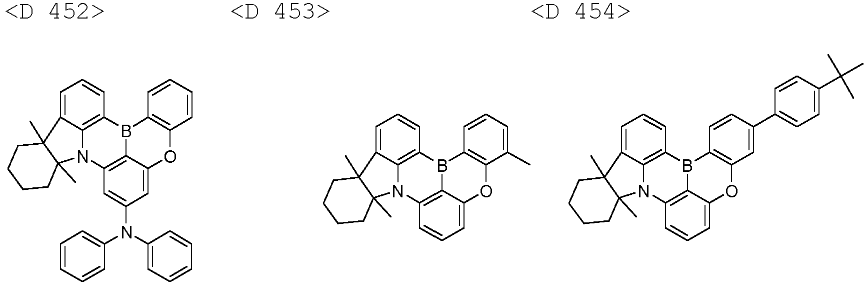

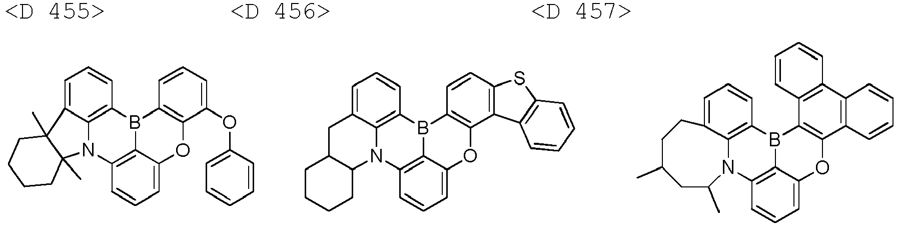

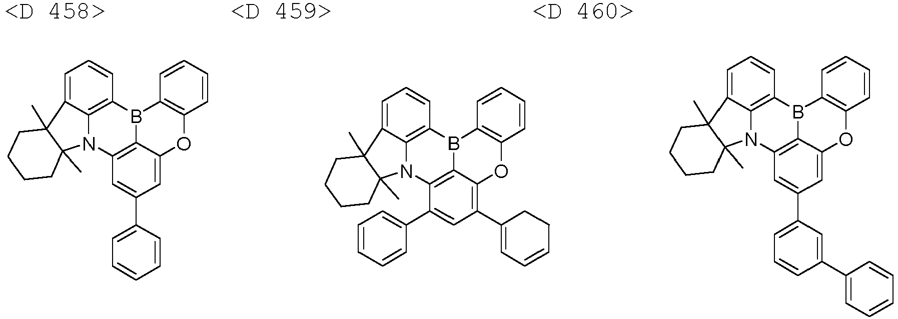

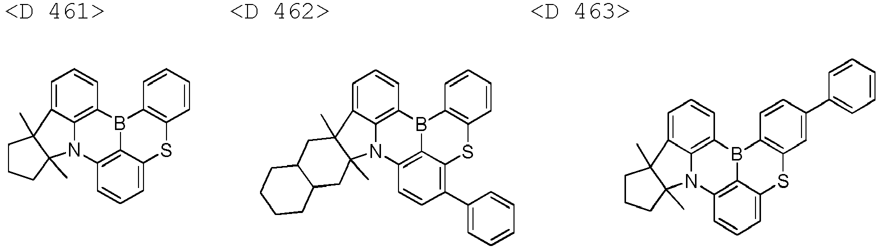

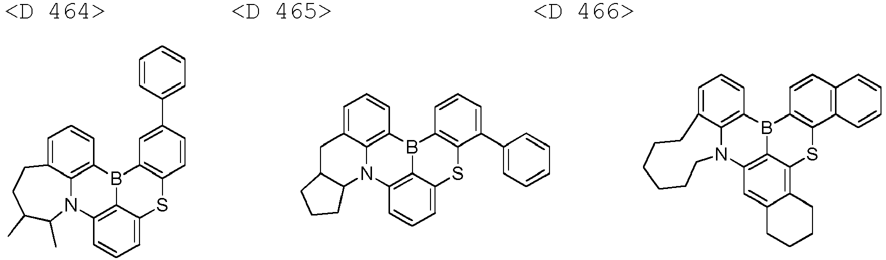

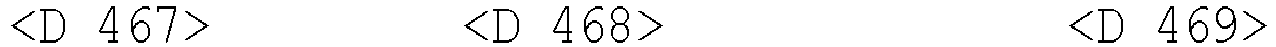

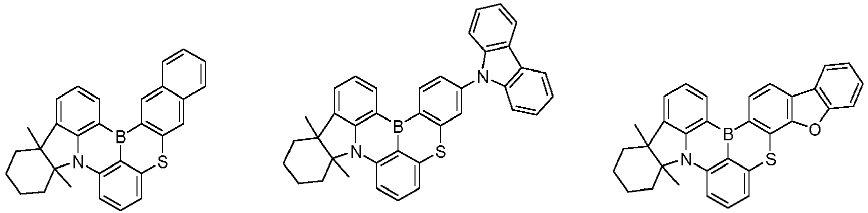

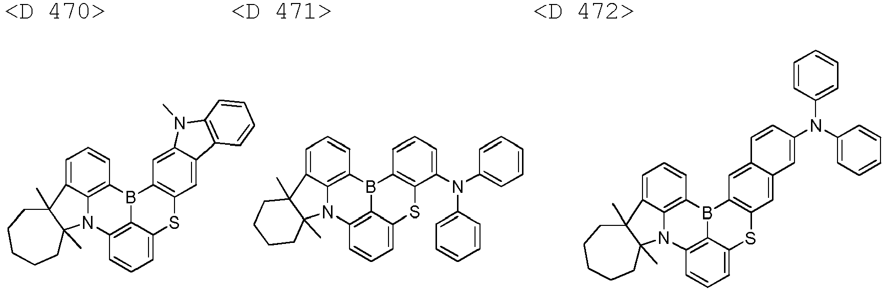

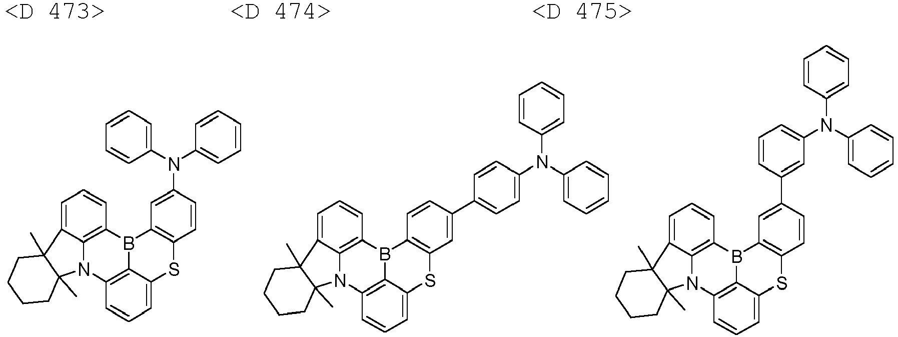

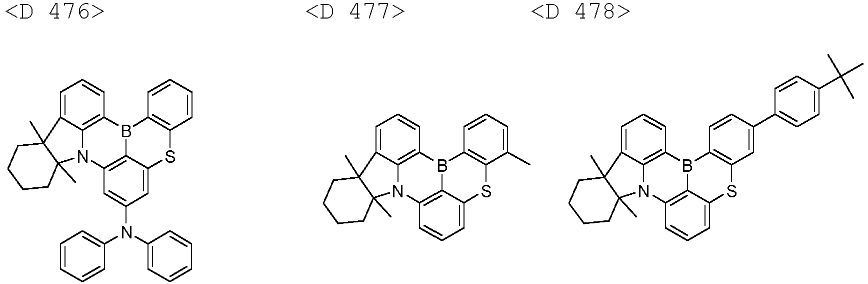

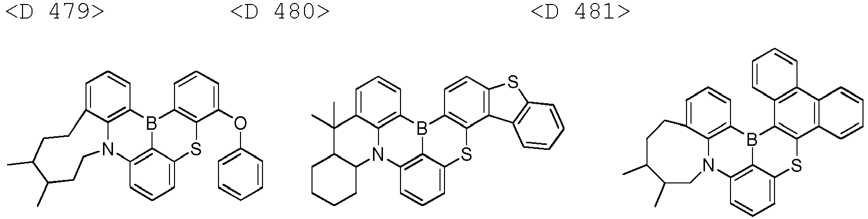

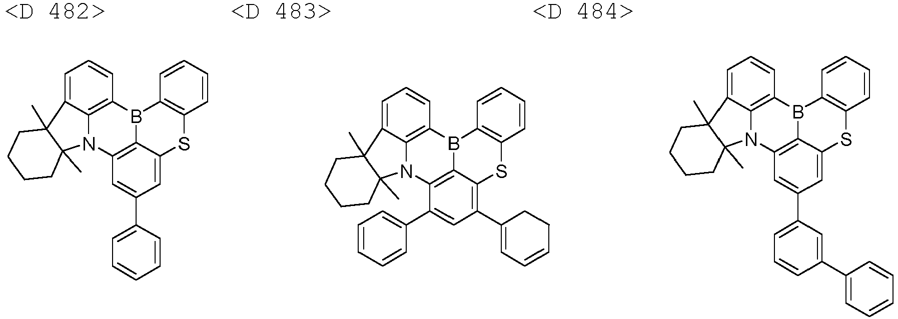

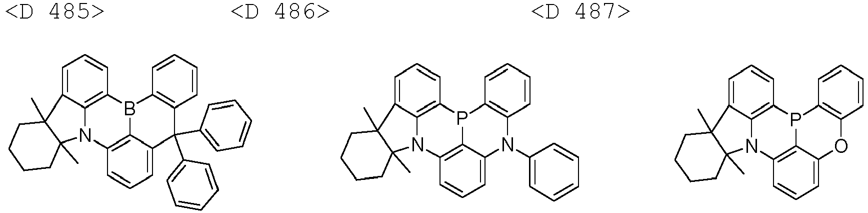

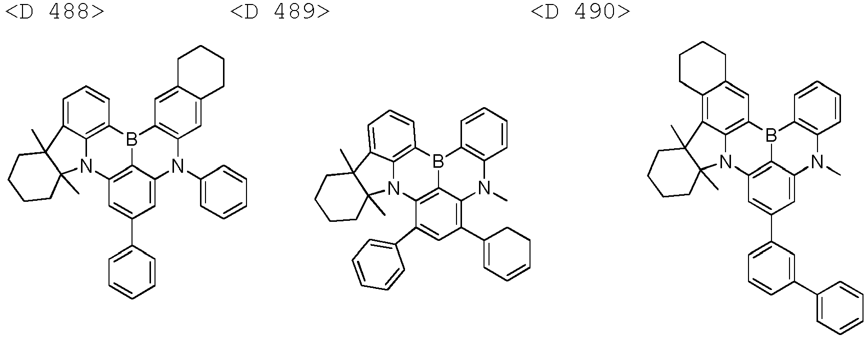

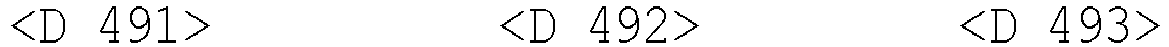

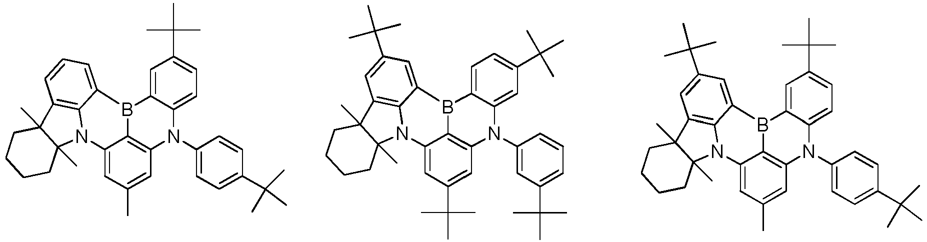

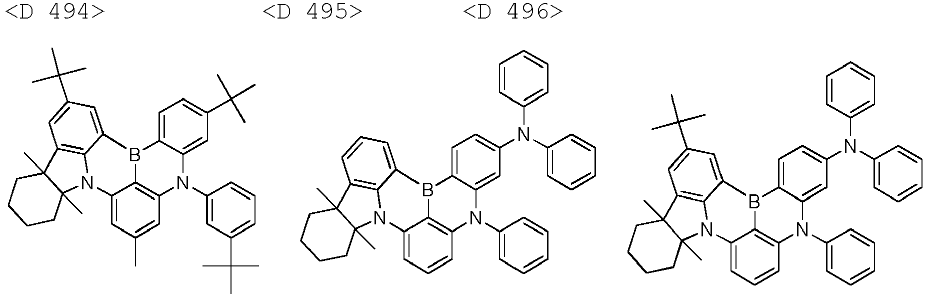

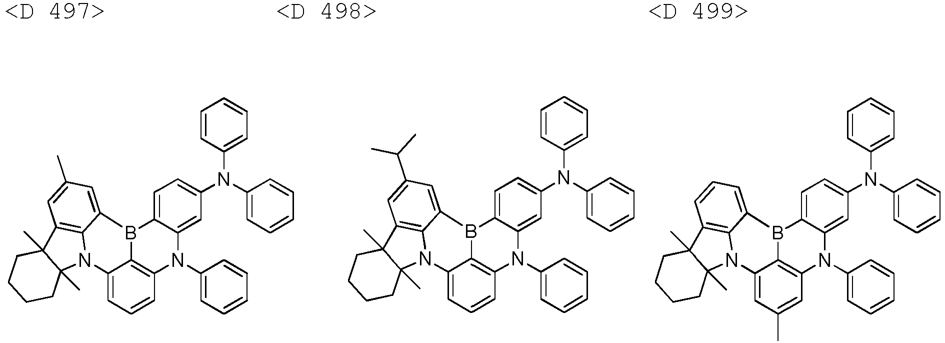

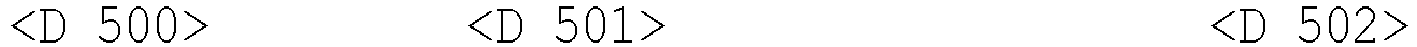

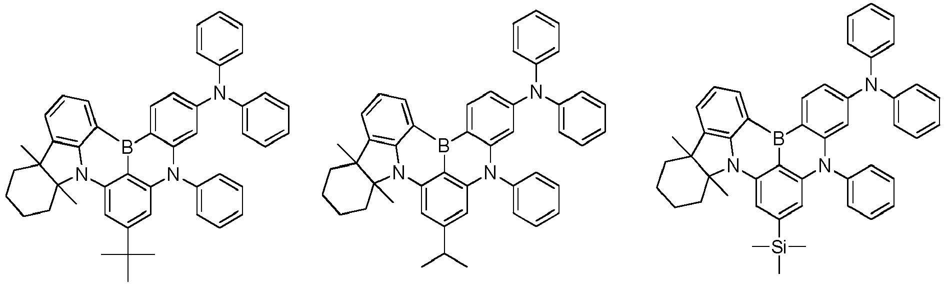

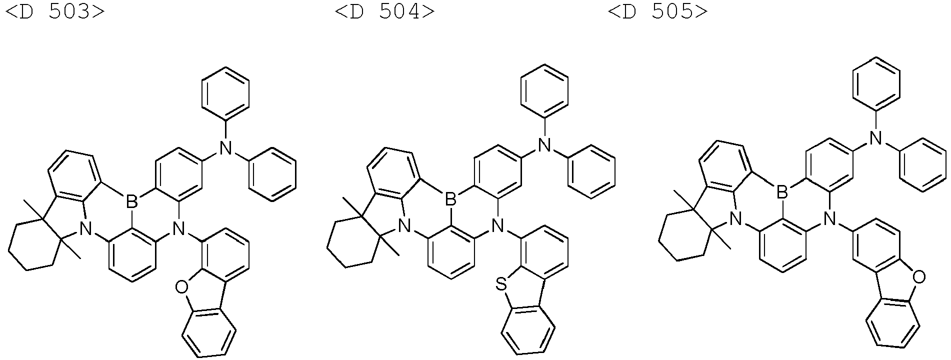

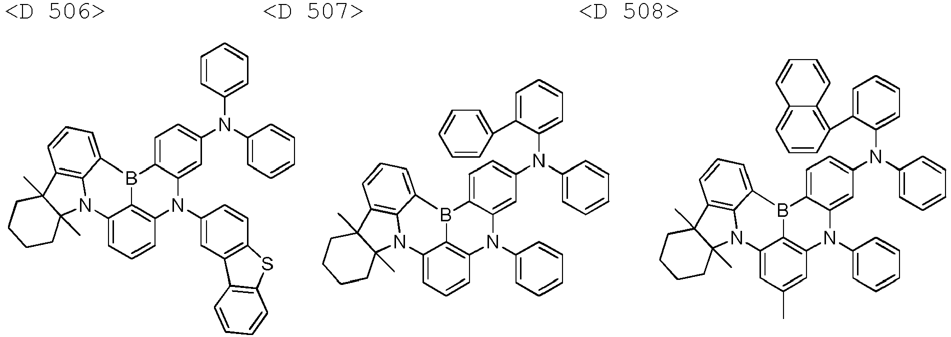

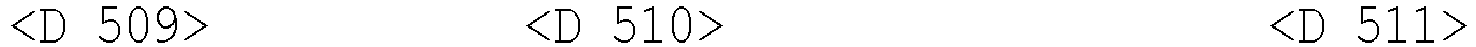

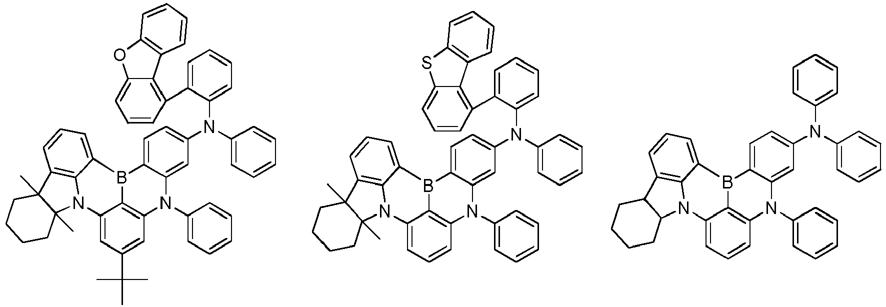

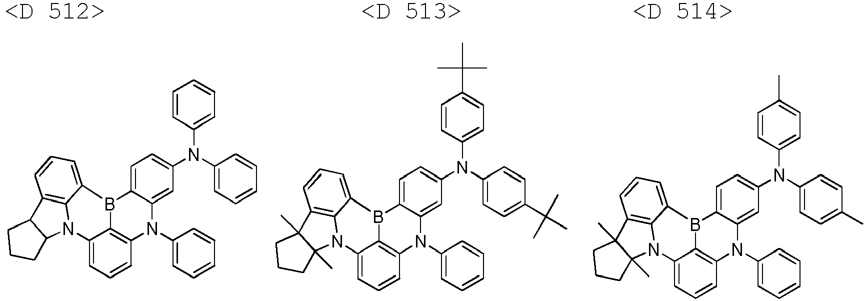

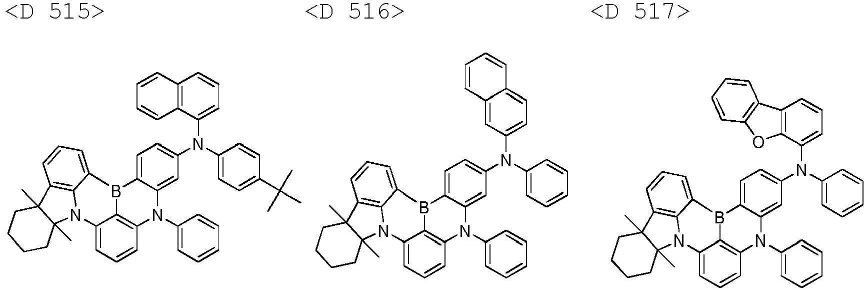

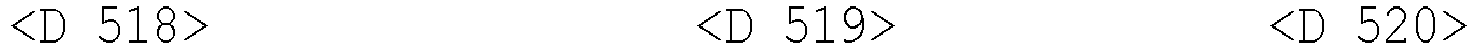

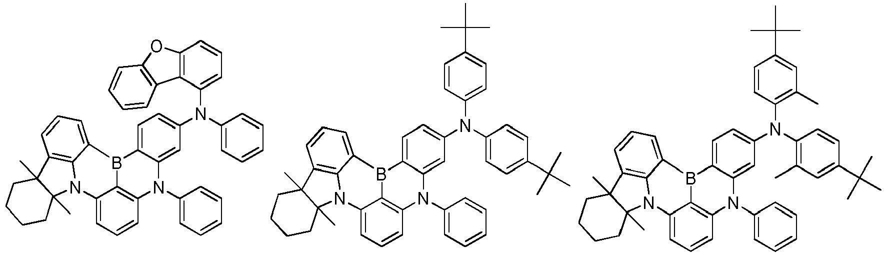

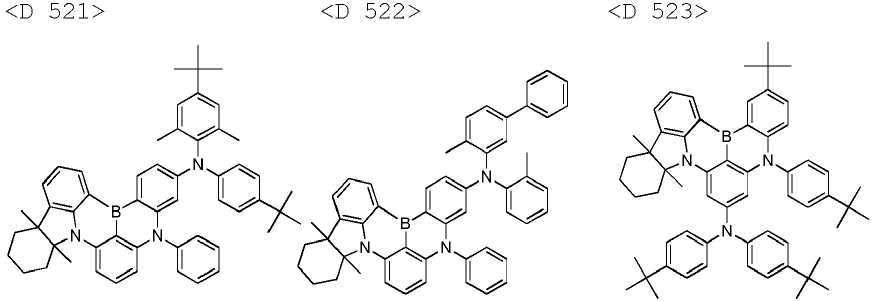

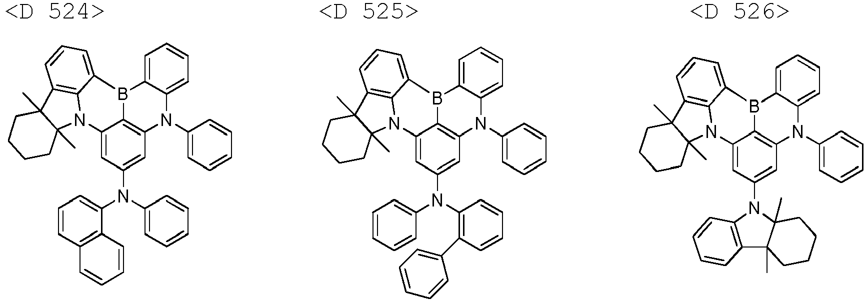

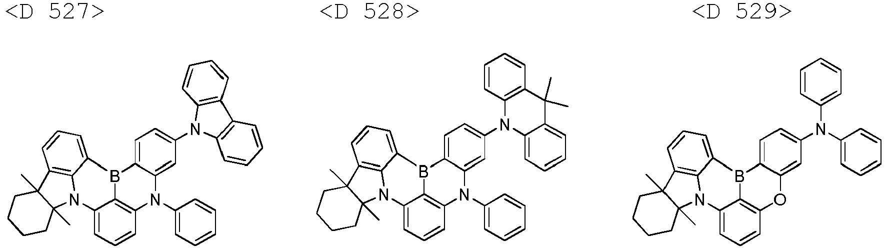

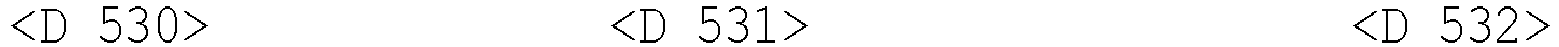

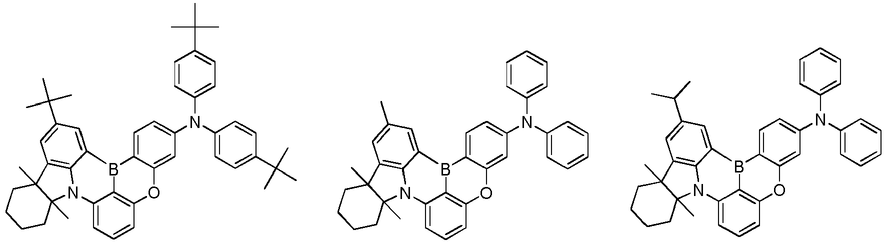

In a particular embodiment thereof, the present disclosure provides an organic light-emitting diode which includes: a first electrode; a second electrode facing the first electrode; and a first light-emitting layer containing a first host and a first dopant and a second light-emitting layer containing a second host and a second dopant sequentially between the first electrode and the second electrode, wherein at least one of the first host and the second host comprises at least one selected from among the compounds represented by Chemical Formula A or B. With such structural characteristics, the organic light-emitting diode according to the present disclosure can exhibit high luminous efficiency and longevity.

In this regard, the organic light-emitting diode may include at least one of a hole transport layer and a hole injection layer between the first electrode and the first light-emitting layer and at least one of an electron transport layer and an electron injection layer between the second light-emitting layer and the second electrode. In a particular embodiment, the organic light-emitting diode includes a hole transport layer and a hole injection layer between the first electrode and the first light-emitting electrode and an electron transport layer and an electron injection layer between the second light-emitting layer and the second electrode.

In the organic light-emitting diode according to some particular embodiments of the present disclosure, the first light-emitting layer may include at least one of the compounds represented by Chemical Formula A or B.

In this context, when the first light-emitting layer in the organic light-emitting diode according to the present disclosure includes any one of the compounds represented by Chemical Formula A or B, the second light-emitting layer may employ as a host an anthracene derivative represented by the following Chemical Formula E: 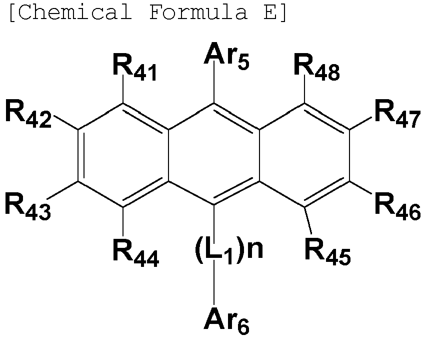 wherein,
R₄₁ to R₄₈, which may be same or different, are each independently any one selected from among a hydrogen atom, a deuterium atom, a substituted or unsubstituted alkyl of 1 to 30 carbon atoms, a substituted or unsubstituted aryl of 6 to 50 carbon atoms, a substituted or unsubstituted cycloalkyl of 3 to 30 carbon atoms, a substituted or unsubstituted heteroaryl of 2 to 50 carbon atoms, a substituted or unsubstituted alkylsilyl of 1 to 30 carbon atoms, a substituted or unsubstituted arylsilyl of 6 to 30 carbon atoms, a cyano, a nitro, and a halogen,
Ar₅ and Ar₆, which may be same or different, are each independently a substituted or unsubstituted aryl of 6 to 50 carbon atoms or a substituted or unsubstituted heteroaryl of 2 to 50 carbon atoms,
linker L1 is any one selected from among a single bond, a substituted or unsubstituted arylene of 6 to 20 carbon atoms, and a substituted or unsubstituted heteroarylene of 2 to 20 carbon atoms, and
n is an integer of 1 to 2, wherein when n is 2, the corresponding linkers L1's are same or different,
wherein, the term "substituted" in the expression "substituted or unsubstituted" used for compounds of Chemical Formula E means having at least one substituent selected from the group consisting of a deuterium atom, a cyano, a halogen, a hydroxy, a nitro, an alkyl of 1 to 24 carbon atoms, a halogenated alkyl of 1 to 24 carbon atoms, an alkenyl of 2 to 24 carbon atoms, an alkynyl of 2 to 24 carbon atoms, a heteroalkyl of 1 to 24 carbon atoms, an aryl of 6 to 24 carbon atoms, an arylalkyl of 7 to 24 carbon atoms, a heteroaryl of 2 to 24 carbon atoms or a heteroarylalkyl of 2 to 24 carbon atoms, an alkoxy of 1 to 24 carbon atoms, an alkylamino of 1 to 24 carbon atoms, an arylamino of 6 to 24 carbon atoms, a heteroarylamino of 1 to 24 carbon atoms, an alkylsilyl of 1 to 24 carbon atoms, an arylsilyl of 6 to 24 carbon atoms, and an aryloxy of 6 to 24 carbon atoms.

In a particular embodiment of the present disclosure, when the second light-emitting layer of the organic light-emitting diode includes an anthracene derivative represented by Chemical Formula E as a host, a preferred structure of the anthracene derivative of Chemical Formula may include an anthracene derivative represented by Chemical Formula E-1 or Chemical Formula E-2: 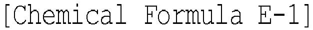 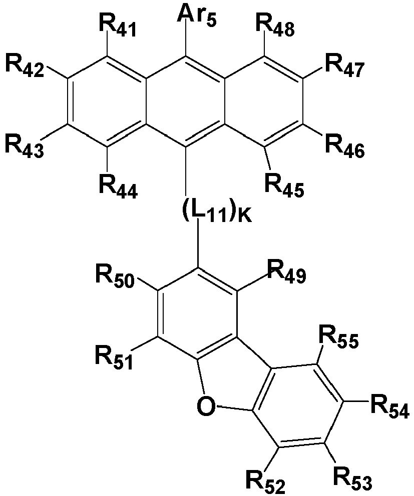 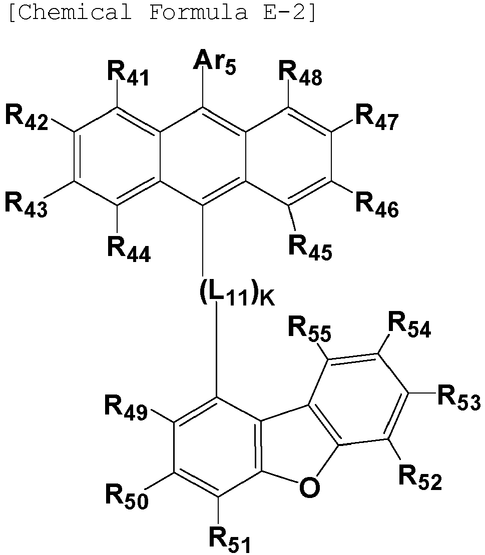 wherein,
R₄₁ to R₄₈ and R₄₉ to R₅₅, which may be same or different, are each independently any one selected from among a hydrogen atom, a deuterium atom, a substituted or unsubstituted alkyl of 1 to 30 carbon atoms, a substituted or unsubstituted aryl of 6 to 50 carbon atoms, a substituted or unsubstituted cycloalkyl of 3 to 30 carbon atoms, a substituted or unsubstituted heteroaryl of 2 to 50 carbon atoms, a substituted or unsubstituted alkylsilyl of 1 to 30 carbon atoms, a substituted or unsubstituted arylsilyl of 6 to 30 carbon atoms, a cyano, a nitro, and a halogen,
Ar₅ is a substituted or unsubstituted aryl of 6 to 50 carbon atoms or a substituted or unsubstituted heteroaryl of 2 to 50 carbon atoms,
linker L₁₁ is any one selected from among a single bond, a substituted or unsubstituted arylene of 6 to 20 carbon atoms, and a substituted or unsubstituted heteroarylene of 2 to 20 carbon atoms,
k is an integer of 1 to 2 wherein when k is 2, the corresponding L11's are same or different,
wherein, the term "substituted" in the expression "substituted or unsubstituted" used for compounds of Chemical Formula E-1 and E-2 means having at least one substituent selected from the group consisting of a deuterium atom, a cyano, a halogen, a hydroxy, a nitro, an alkyl of 1 to 24 carbon atoms, a halogenated alkyl of 1 to 24 carbon atoms, an alkenyl of 2 to 24 carbon atoms, an alkynyl of 2 to 24 carbon atoms, a heteroalkyl of 1 to 24 carbon atoms, an aryl of 6 to 24 carbon atoms, an arylalkyl of 7 to 24 carbon atoms, a heteroaryl of 2 to 24 carbon atoms or a heteroarylalkyl of 2 to 24 carbon atoms, an alkoxy of 1 to 24 carbon atoms, an alkylamino of 1 to 24 carbon atoms, an arylamino of 6 to 24 carbon atoms, a heteroarylamino of 1 to 24 carbon atoms, an alkylsilyl of 1 to 24 carbon atoms, an arylsilyl of 6 to 24 carbon atoms, and an aryloxy of 6 to 24 carbon atoms.

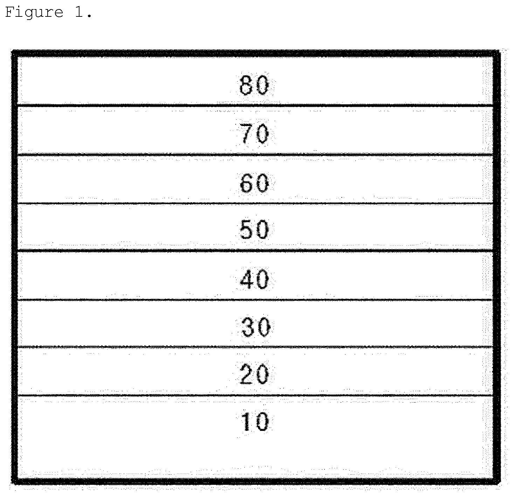

Here, the compound represented by Chemical Formula E-1 or E-2 is characterized by the structure in which position 1 or 2 of one phenyl ring or position 1' or 2' of the other phenyl ring in the dibenzofuran moiety shown in the following Diagram 1 may be connected to position 9 of the anthracenyl moiety or the linker L₁₁.

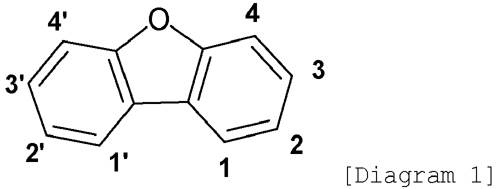

The substituent Ar₅ in the anthracene derivative represented by any one of Chemical Formulas E, E-1, and E-2 may be a substituent represented by the following Structural Formula C-1: 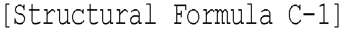 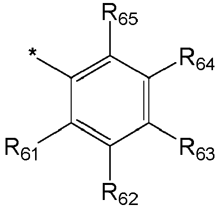
wherein, R₆₁ to R₆₅, which may be same or different, are each independently any one selected from among a hydrogen atom, a deuterium atom, a substituted or unsubstituted alkyl of 1 to 20 carbon atoms, a substituted or unsubstituted aryl of 6 to 50 carbon atoms, a substituted or unsubstituted arylalkyl of 7 to 50 carbon atoms, a substituted or unsubstituted cycloalkyl of 3 to 30 carbon atoms, a substituted or unsubstituted alkylsilyl of 1 to 30 carbon atoms, a substituted or unsubstituted arylsilyl of 6 to 30 carbon atoms, and a halogen, and
"-*" denotes a bonding site connecting to position 10 of the anthracenyl moidety in Chemical Formula E, E-1, or E-2.

In the organic light-emitting diode according to a preferred embodiment of the present disclosure, the linker L₁₁ in Chemical Formula E-1 or E-2 may be a single bond or a substituted or unsubstituted arylene of 6 to 14 carbon atoms and k is an integer of 1 to 2 wherein when k is 2, the corresponding L₁₃'s are same or different.

The anthracene derivative represented by Chemical Formula E in the organic light-emitting diode according to the present disclosure may be any one selected from among the following

### <Compound 101> to <Compound 187>:

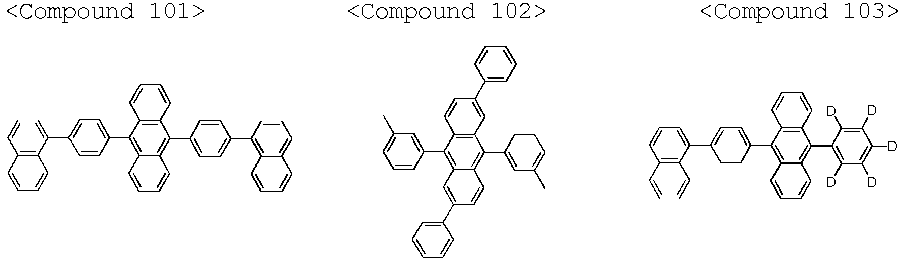

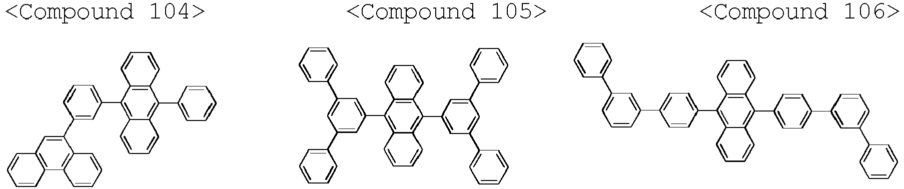

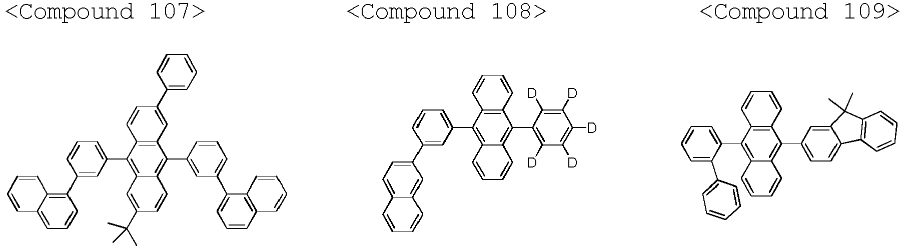

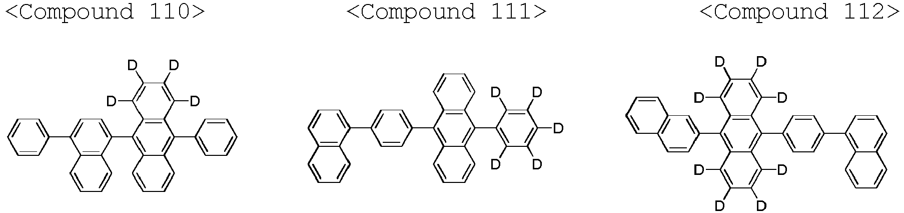

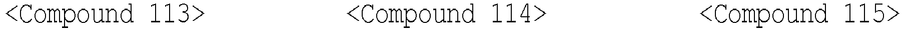

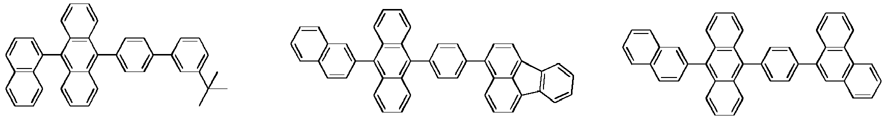

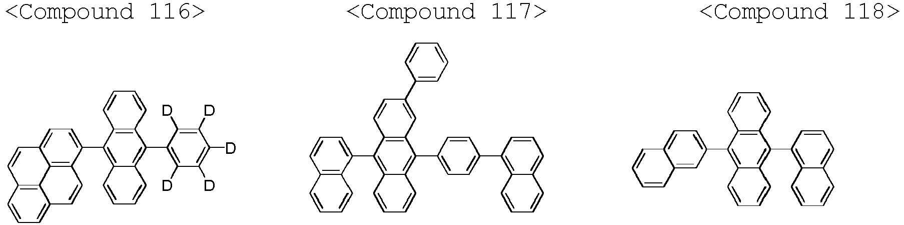

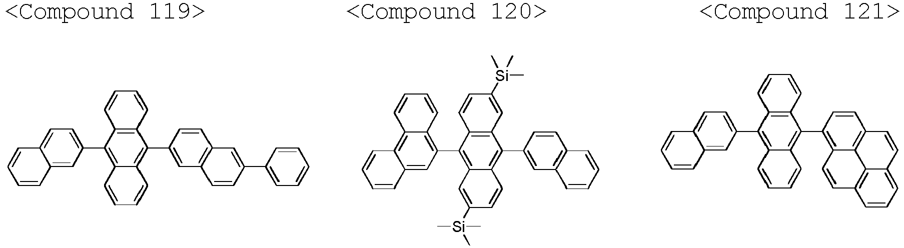

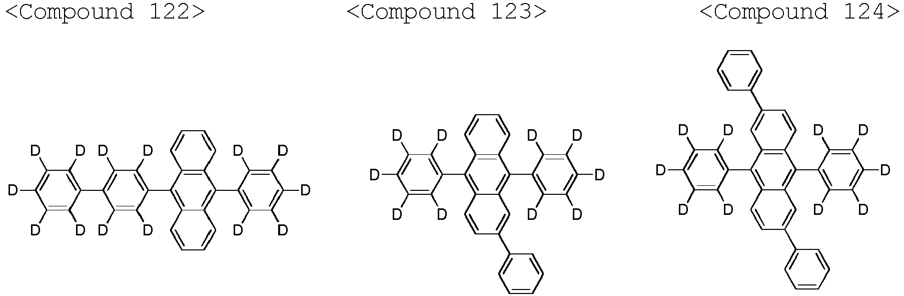

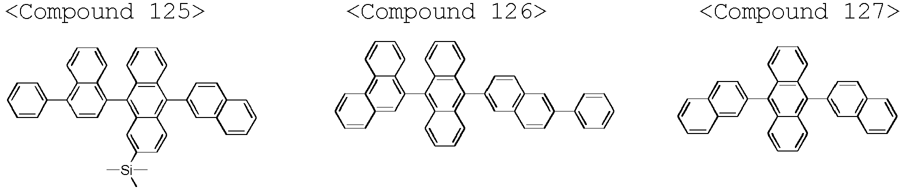

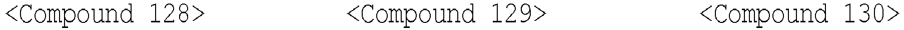

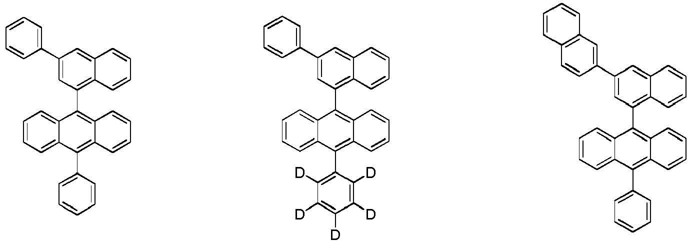

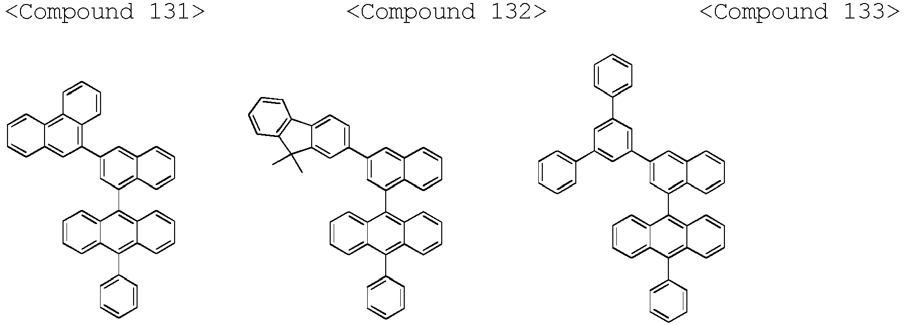

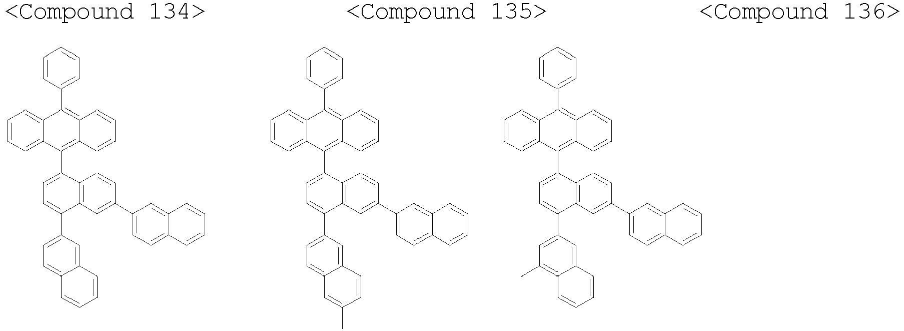

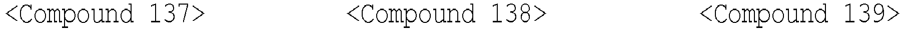

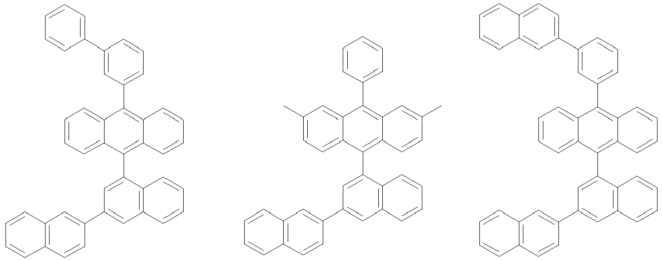

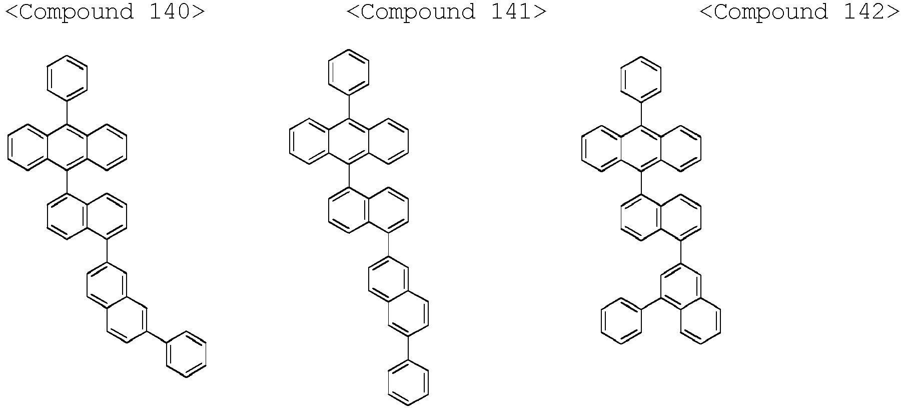

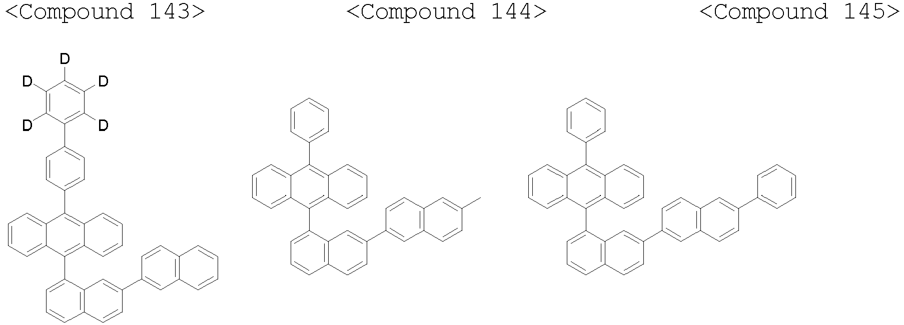

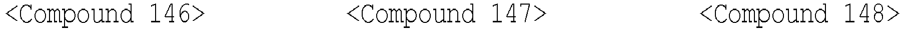

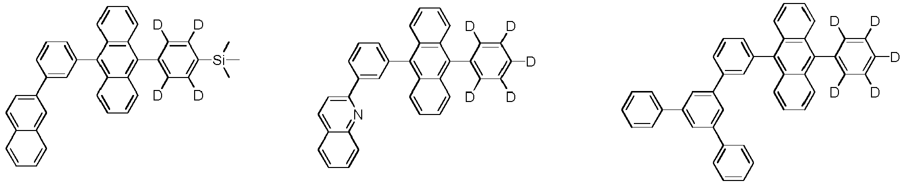

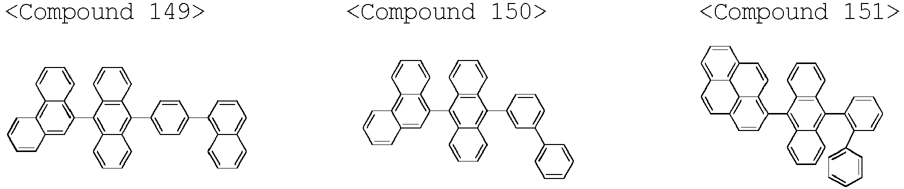

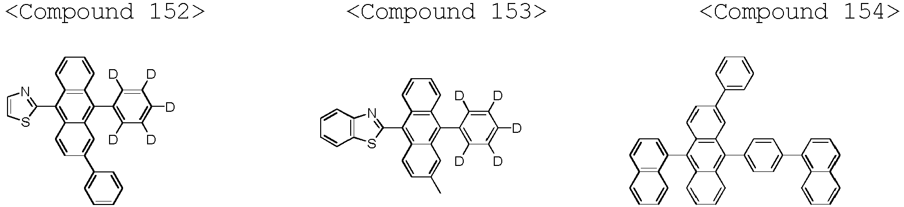

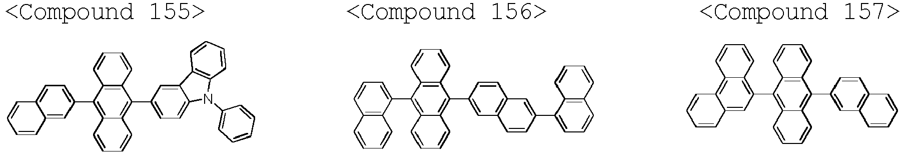

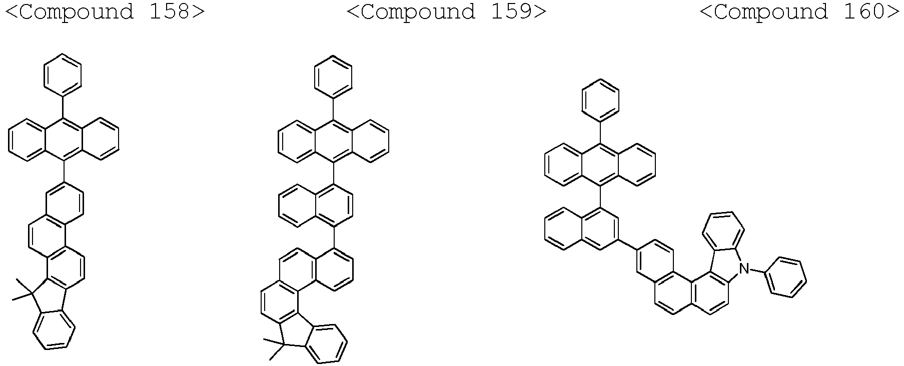

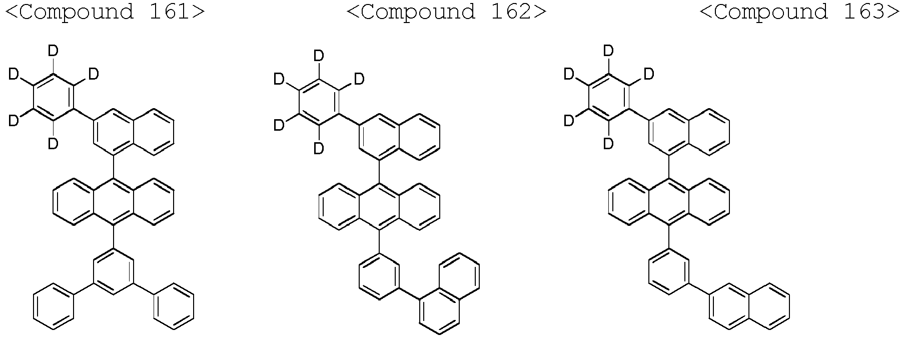

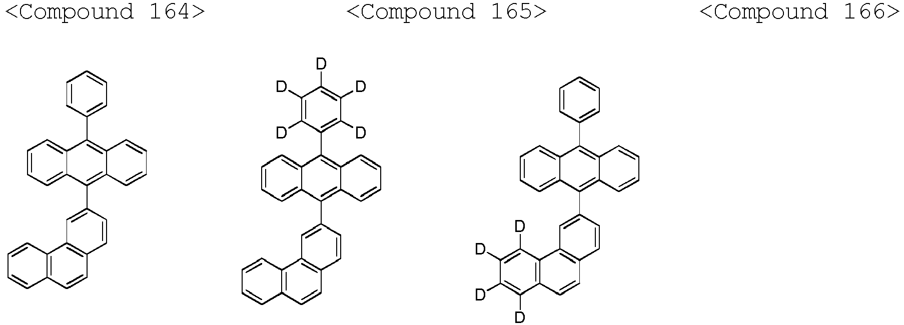

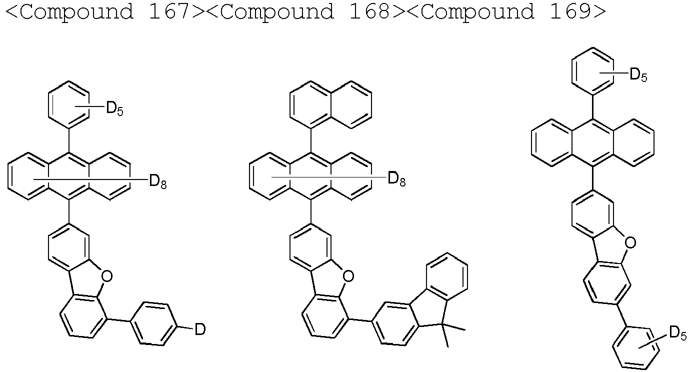

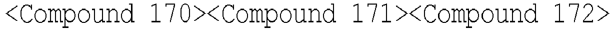

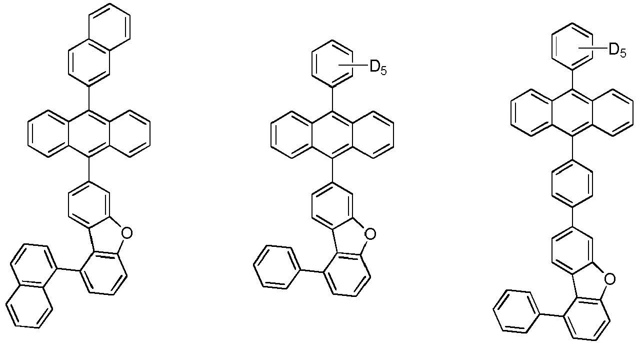

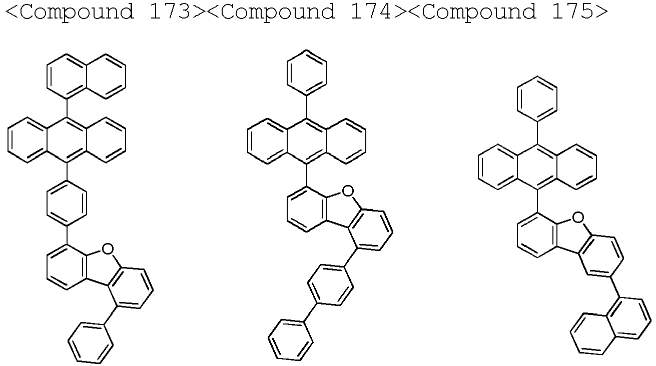

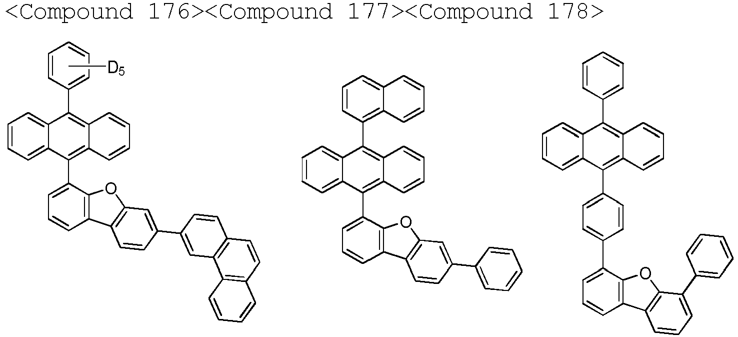

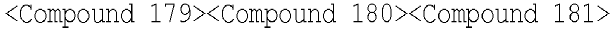

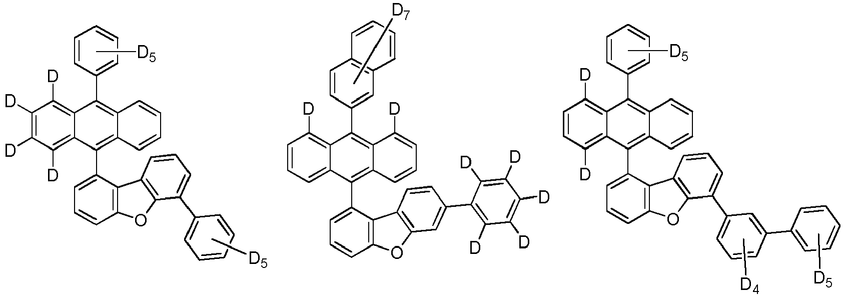

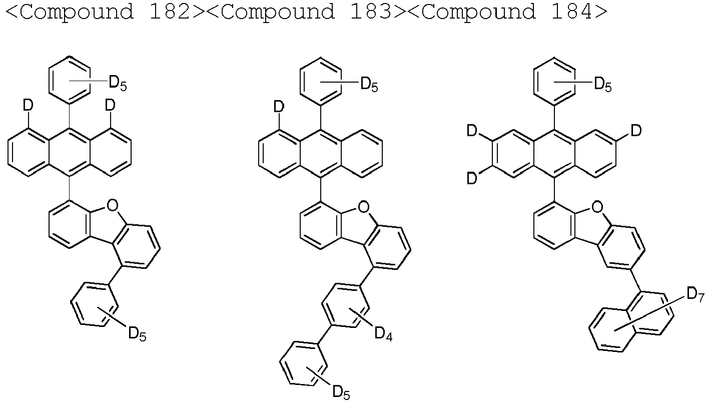

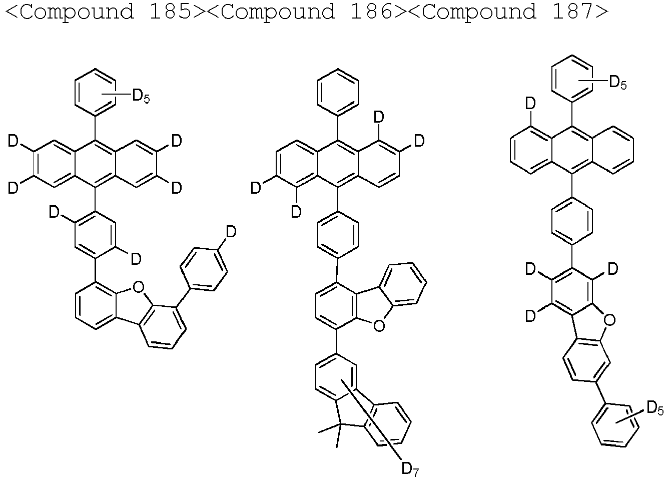

In another embodiment, the anthracene derivative represented by Chemical Formula E-1 or E-2 may be any one selected from among the following <Compound 201> to <Compound 260>:

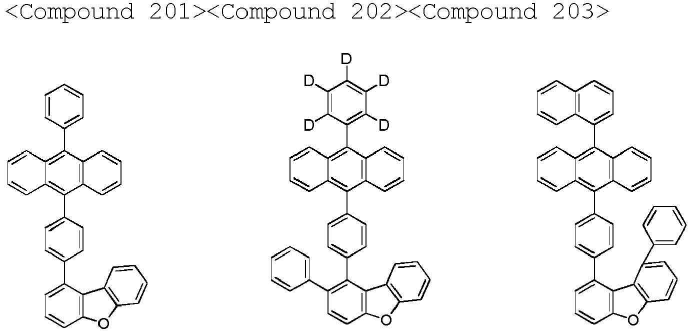

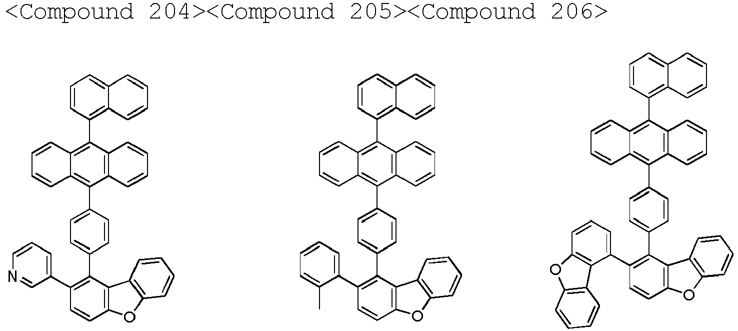

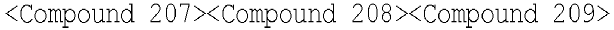

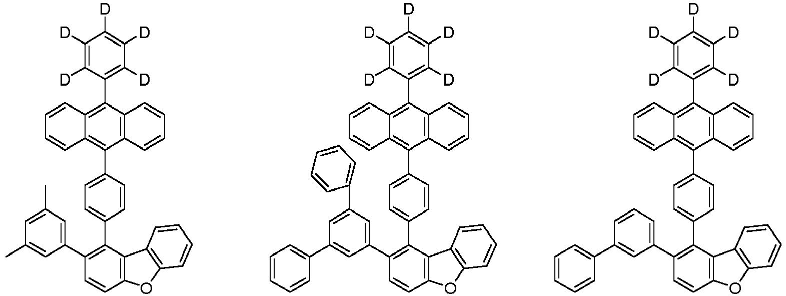

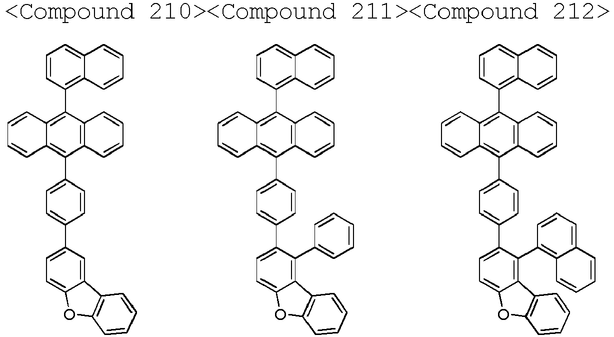

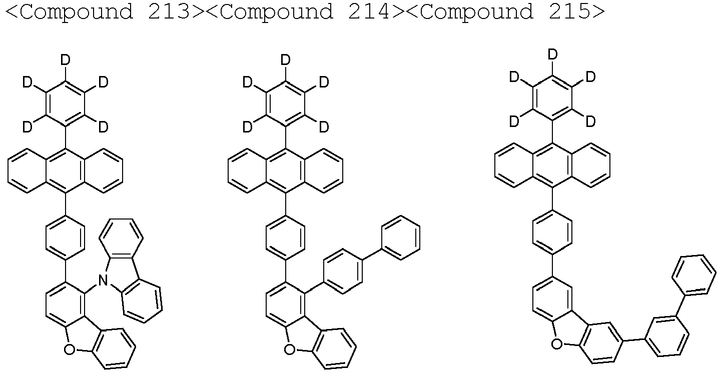

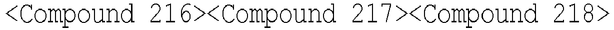

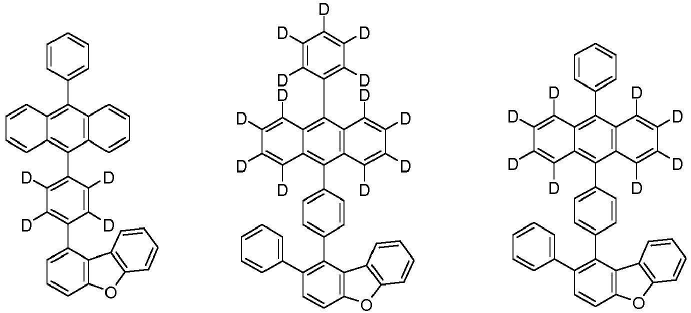

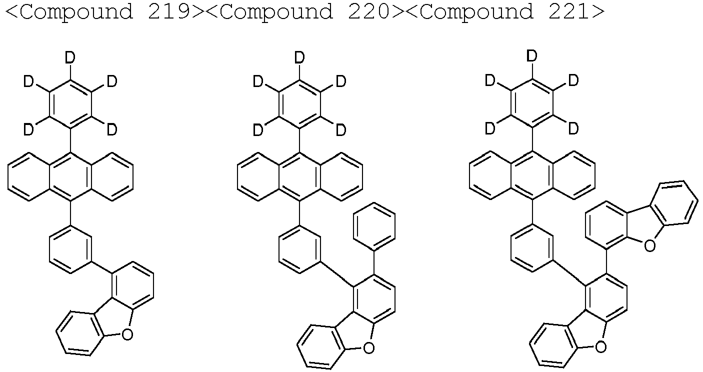

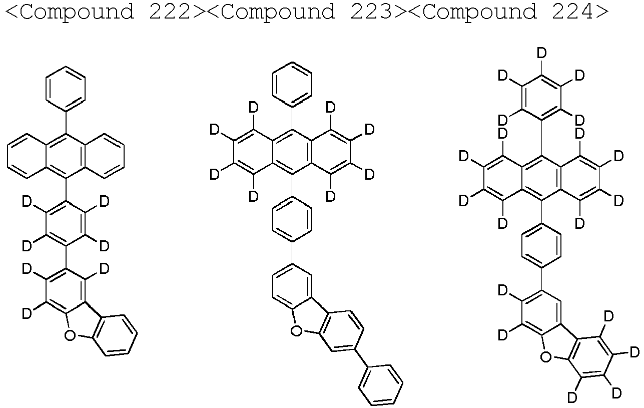

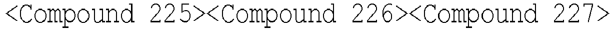

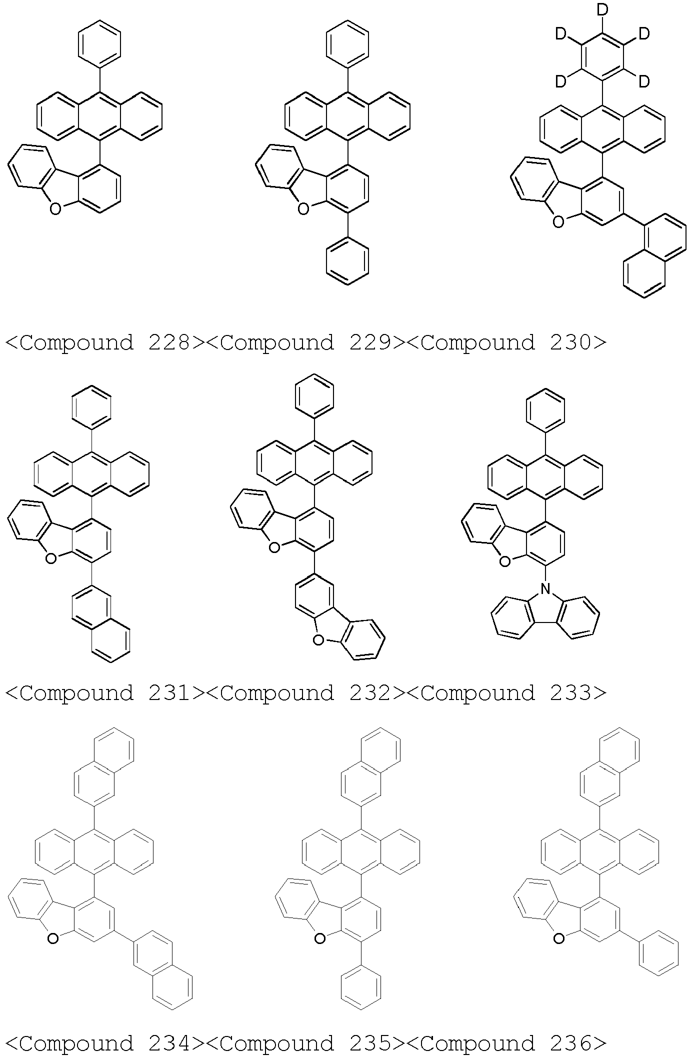

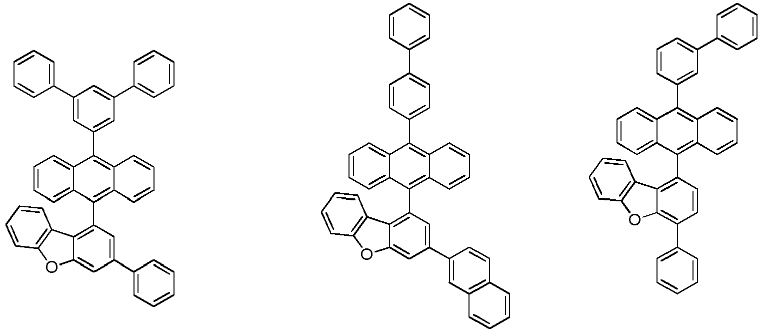

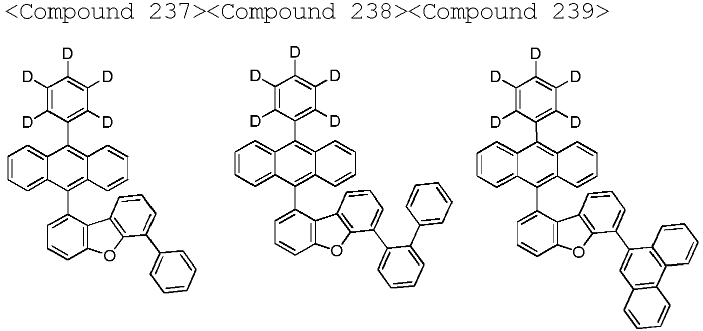

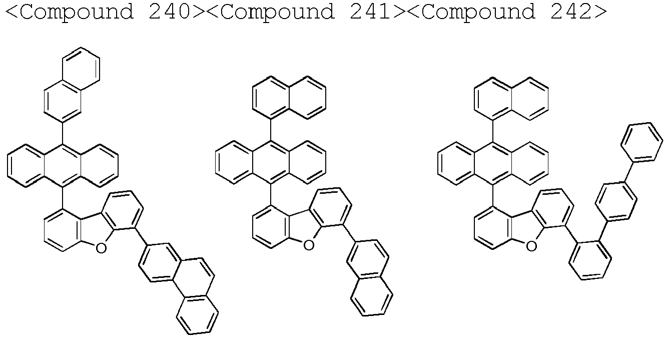

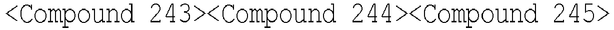

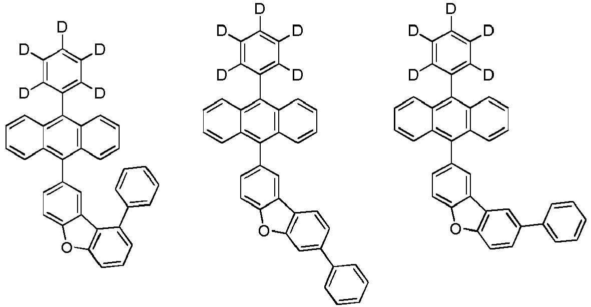

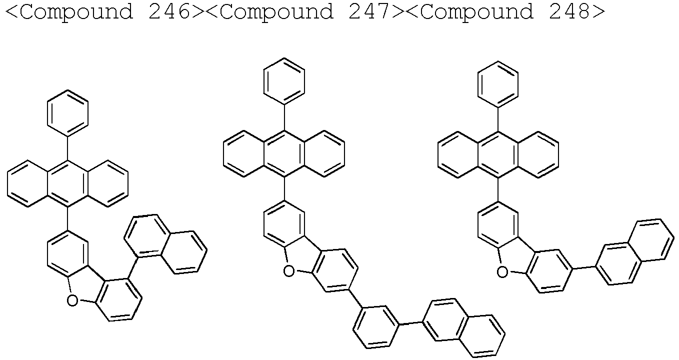

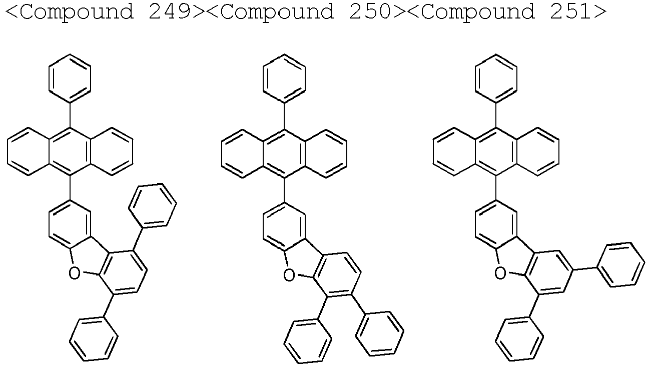

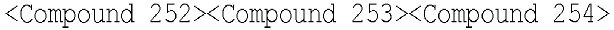

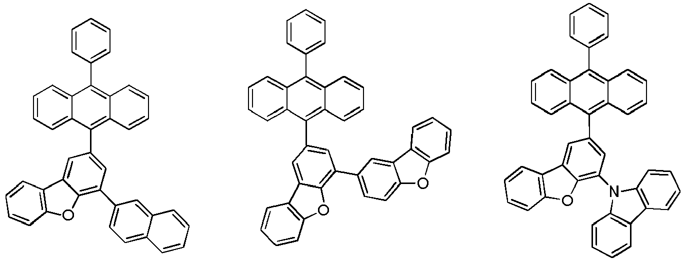

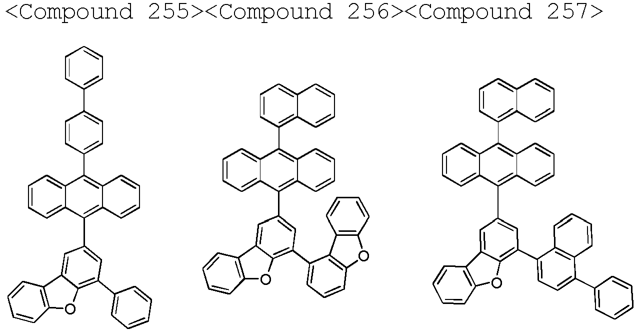

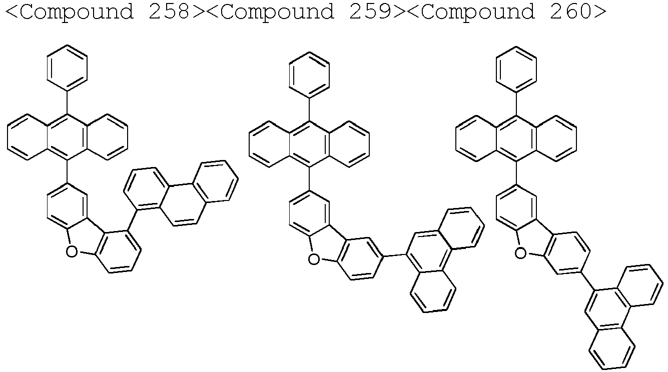

In some particular embodiment thereof, the present disclosure provides an organic light-emitting diode comprising: a first electrode; a second electrode facing the first electrode; and a first light-emitting layer including a first host and a first dopant and a second light-emitting layer including a second host and a second dopant sequentially between the first electrode and the second electrode, wherein the first host is a compound represented by Chemical Formula A or B in the first light-emitting layer and the second host is a compound represented by Chemical Formula E in the second light-emitting layer while the first light-emitting layer and the second light-emitting layer each independently employ as a dopant at least one selected from among compounds represented by Chemical Formulas D1 to D10:

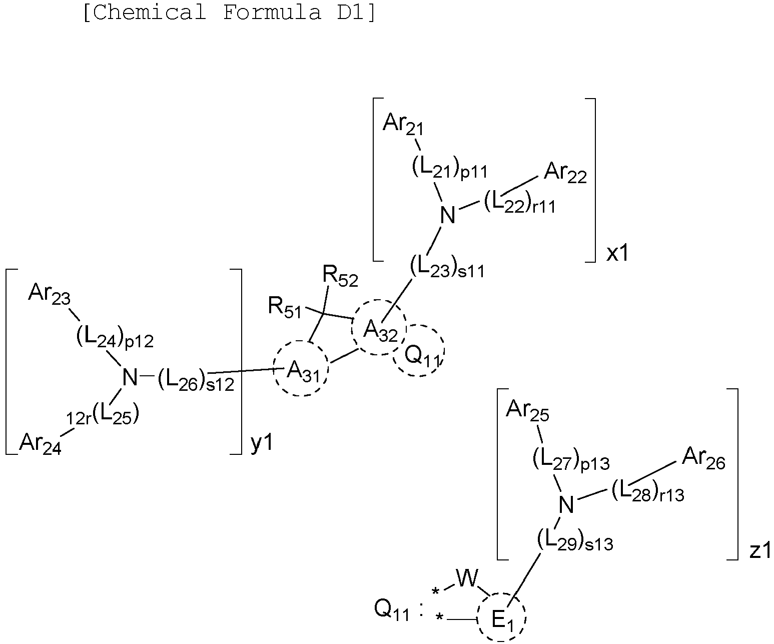

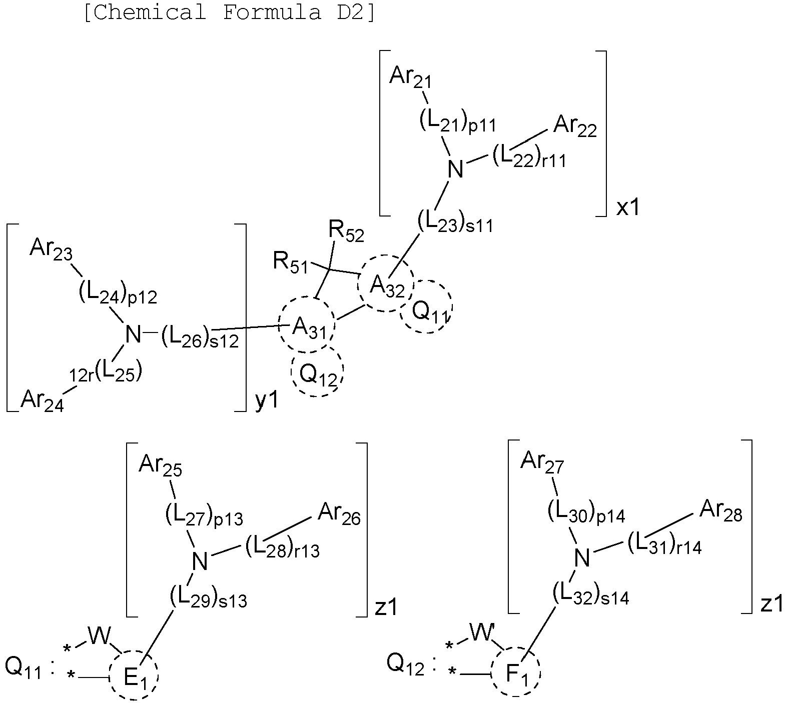

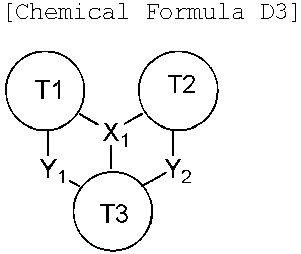

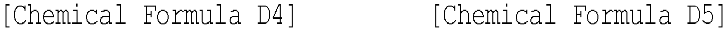

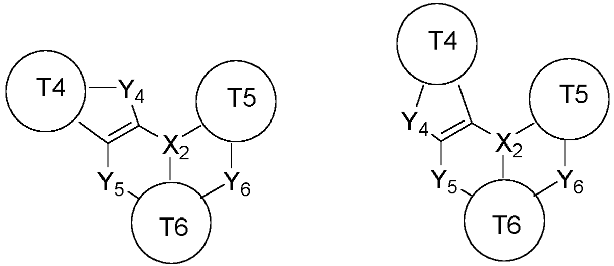

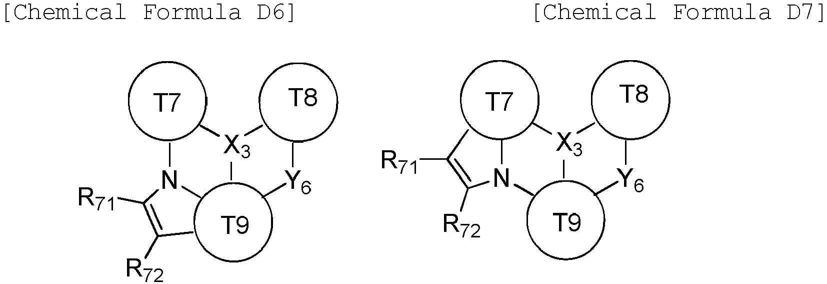

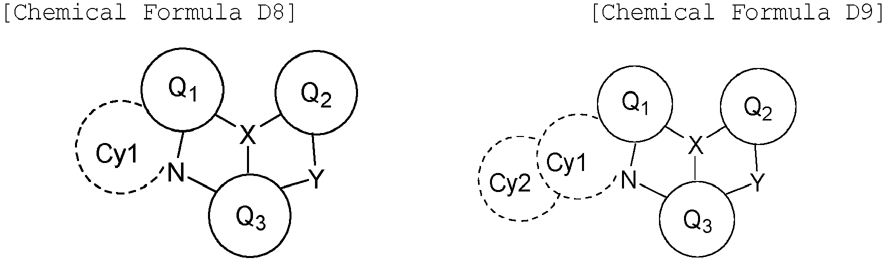

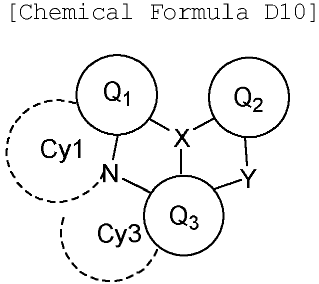

In a particular embodiment, the content of the dopant in the light-emitting layer may range from about 0.01 to 20 parts by weight, based on 100 parts by weight of the host, but is not limited thereto.

In addition to the above-mentioned dopants and hosts, the light-emitting layer may further include various hosts and dopant materials.

Below, the organic light-emitting diode of the present disclosure is explained with reference to the drawing.

Figure is a schematic cross-sectional view of the structure of an organic light-emitting diode according to an embodiment of the present disclosure.

As shown in Figure, the organic light-emitting diode according to an embodiment of the present disclosure comprises an anode 20, a hole transport layer 40, an organic light-emitting layer 50 containing a host and a dopant, an electron transport layer 60, and a cathode 80, wherein the anode and the cathode serve as a first electrode and a second electrode, respectively, with the interposition of the hole transport layer between the anode and the light-emitting layer, and the electron transport layer between the light-emitting layer and the cathode.

Furthermore, the organic light-emitting diode according to an embodiment of the present disclosure may comprise a hole injection layer 30 between the anode 20 and the hole transport layer 40, and an electron injection layer 70 between the electron transport layer 60 and the cathode 80.

Reference is made to Figure with regard to the organic light emitting diode of the present disclosure and the fabrication method therefor.

First, a substrate 10 is coated with an anode electrode material to form an anode 20. So long as it is used in a typical organic electroluminescence device, any substrate may be used as the substrate 10. Preferable is an organic substrate or transparent plastic substrate that exhibits excellent transparency, surface smoothness, ease of handling, and waterproofness. As the anode electrode material, indium tin oxide (ITO), indium zinc oxide (IZO), tin oxide (SnO₂), or zinc oxide (ZnO), which are transparent and superior in terms of conductivity, may be used.

A hole injection layer material is applied on the anode 20 by thermal deposition in a vacuum or by spin coating to form a hole injection layer 30. Subsequently, thermal deposition in a vacuum or by spin coating may also be conducted to form a hole transport layer 40 with a hole transport layer material on the hole injection layer 30.

So long as it is typically used in the art, any material may be selected for the hole injection layer without particular limitations thereto. Examples include, but are not limited to, 2-TNATA [4,4',4"-tris(2-naphthylphenyl-phenylamino)-triphenylamine], NPD[N,N'-di(1-naphthyl)-N,N'-diphenylbenzidine)], TPD[N,N'-diphenyl-N,N'-bis(3-methylphenyl)-1,1'-biphenyl-4,4'-diamine], and DNTPD [N,N'-diphenyl-N,N'-bis-[4-(phenyl-m-tolyl-amino)-phenyl]-biphenyl-4,4'-dia mine].

Any material that is typically used in the art may be selected for the hole transport layer without particular limitations thereto. Examples include, but are not limited to, N,N'-bis(3-methylphenyl)-N,N'-diphenyl-[1,1-biphenyl]-4,4'-diamine (TPD) and N,N'-di(naphthalen-1-yl)-N,N'-diphenylbenzidine (a-NPD).

In an embodiment of the present disclosure, an electron blocking layer may be additionally disposed on the hole transport layer. Functioning to prevent the electrons injected from the electron injection layer from entering the hole transport layer from the light-emitting layer, the electron blocking layer is adapted to increase the life span and luminous efficiency of the diode. The electron blocking layer may be formed at a suitable position between the light emitting layer and the hole injection layer. Particularly, the electron blocking layer may be formed between the light emitting layer and the hole transport layer.

Next, the light-emitting layer 50 may be deposited on the hole transport layer 40 or the electron blocking layer by deposition in a vacuum or by spin coating.

Herein, the light-emitting layer may contain a host and a dopant and the materials are as described above

In addition, the light-emitting layer is composed of a first light-emitting layer (not shown) and a second light-emitting layer (not shown) that may be formed of the same or different host and dopant materials through separate deposition or coating processes.

In more particular embodiments, the dopant may be at least one of the compounds represented by Chemical Formula A or B in the first light-emitting layer and at least one of the anthracene compounds represented by Chemical Formula E in the second light-emitting layer while the first and the second light-emitting layer may each independently contain as respective dopants any one selected from the compounds represented by Chemical Formula D1 to D10.

As for the host materials available for the first light-emitting layer and the second light-emitting layer in the present disclosure, the host material (BH1) used in the first light-emitting layer is lower in lowest unoccupied molecular orbital (LUMO) and higher in highest occupied molecular orbital (HOMO) than the host material (BH2) used in the second light-emitting layer, thereby forming a structure in which the injection of holes and/or electrons is easier in the host (BH1) used in the first light-emitting layer than the host (BH2) used in the second light-emitting layer.

In some embodiments of the present disclosure, the light-emitting layer particularly ranges in thickness from 50 to 2,000 Å.

Meanwhile, the electron transport layer 60 is applied on the light-emitting layer by deposition in a vacuum and spin coating.

A material for use in the electron transport layer functions to stably carry the electrons injected from the electron injection electrode (cathode), and may be an electron transport material known in the art. Examples of the electron transport material known in the art include quinoline derivatives, particularly, tris(8-quinolinolate)aluminum (Alq₃), Liq, TAZ, BAlq, beryllium bis(benzoquinolin-10-olate) (Bebq₂), Compound 201, Compound 202, BCP, and oxadiazole derivatives such as PBD, BMD, and BND, but are not limited thereto:

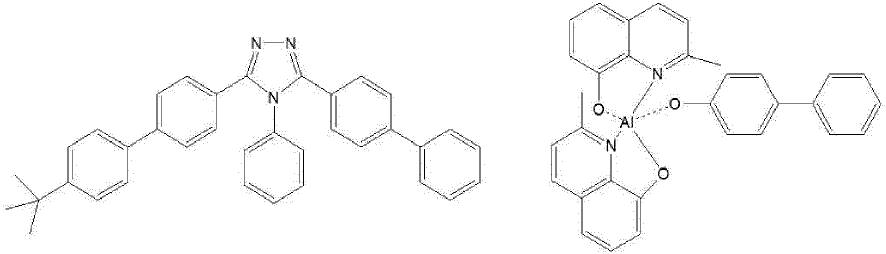

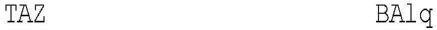

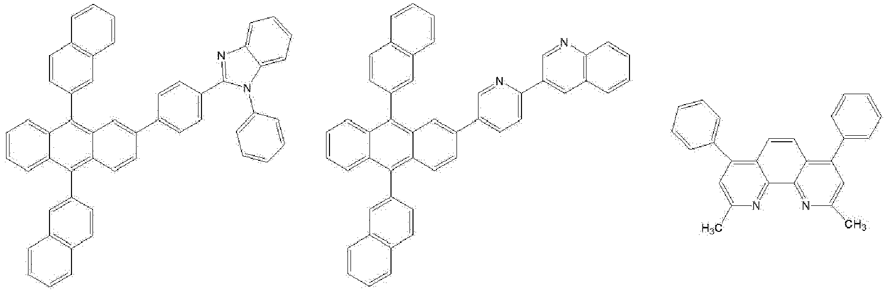

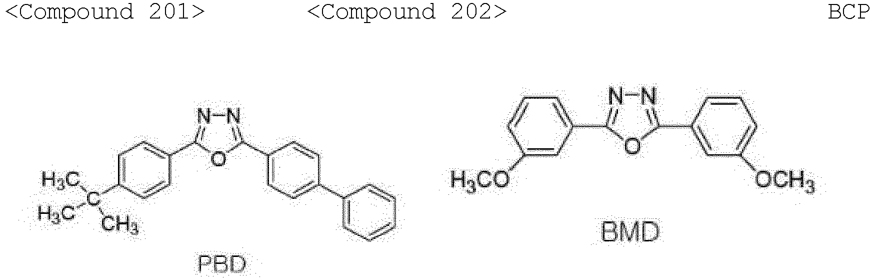

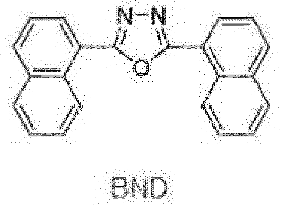

In the organic light emitting diode of the present disclosure, an electron injection layer (EIL) that functions to facilitate electron injection from the cathode may be deposited on the electron transport layer. The material for the EIL is not particularly limited.

Any material that is conventionally used in the art can be available for the electron injection layer without particular limitations. Examples include CsF, NaF, LiF, Li₂O, and BaO. Deposition conditions for the electron injection layer may vary, depending on compounds used, but may be generally selected from condition scopes that are almost the same as for the formation of hole injection layers.

The electron injection layer may range in thickness from about 1 Å to about 100 Å, and particularly from about 3 Å to about 90 Å. Given the thickness range for the electron injection layer, the diode can exhibit satisfactory electron injection properties without actually elevating a driving voltage.

In order to facilitate electron injection, the cathode may be made of a material having a small work function, such as metal or metal alloy such as lithium (Li), magnesium (Mg), calcium (Ca), an alloy aluminum (Al) thereof, aluminum-lithium (Al-Li), magnesium-indium (Mg-In), and magnesium-silver (Mg-Ag). Alternatively, ITO or IZO may be employed to form a transparent cathode for an organic light-emitting diode.

Moreover, the organic light-emitting diode of the present disclosure may further comprise a light-emitting layer containing a blue, green, or red luminescent material that emits radiations in the wavelength range of 380 nm to 800 nm. That is, the light-emitting layer in the present disclosure has a multi-layer structure wherein the blue, green, or red luminescent material may be a fluorescent material or a phosphorescent material.

Furthermore, at least one selected from among the layers may be deposited using a single-molecule deposition process or a solution process.

Here, the deposition process is a process by which a material is vaporized in a vacuum or at a low pressure and deposited to form a layer, and the solution process is a method in which a material is dissolved in a solvent and applied for the formation of a thin film by means of inkjet printing, roll-to-roll coating, screen printing, spray coating, dip coating, spin coating, etc.

Also, the organic light-emitting diode of the present disclosure may be applied to a device selected from among flat display devices, flexible display devices, monochrome or grayscale flat illumination devices, and monochrome or grayscale flexible illumination devices.

A better understanding of the present disclosure may be obtained through the following examples which are set forth to illustrate, but are not to be construed as limiting the present invention.

### (EXAMPLES)

### SYNTHESIS EXAMPLE 1. Synthesis of [H 1]

### Synthesis Example 1-(1): Synthesis of <1-a>

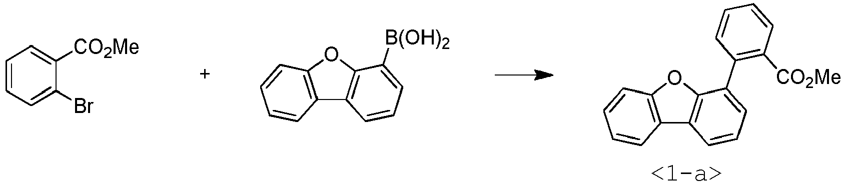

In a 500-mL round-bottom flask reactor, methyl 2-bromobenzoate (30.0 g, 0.140 mol), 4-dibenzofuran boronic acid (32.5 g, 0.153 mol), tetrakis(triphenylphosphine)palladium (3.2 g, 3 mmol), and potassium carbonate (38.6 g, 0.279 mol) were placed and mixed overnight together with toluene (210 mL), methanol (90 mL), and water (60 mL) under reflux. After completion of the reaction, the reactor was cooled to room temperature. Extraction was conducted with ethyl acetate, followed by separation of the organic layer thus formed. The organic layer was concentrated in a vacuum and purified by column chromatography to afford <1-a> (25.0 g, 59.1 %).

### Synthesis Example 1-(2): Synthesis of <1-b>

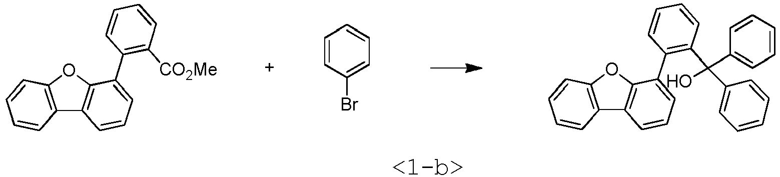

In a 500-ml round-bottom flask reactor, bromobenzene (28.6 g, 182 mmol) and tetrahydrofuran (220 ml) were chilled to -78°C under a nitrogen atmosphere. To the chilled reaction solution was dropwise added normal butyl lithium (104.6 ml, 167 mmol) at the same temperature. The reaction mixture was stirred for 2 hours and then again stirred at room temperature while <1-a> (22.0 g, 73 mmol) was added little by little thereto. After completion of the reaction, H₂O (50 ml0 was added to stop the progress of the reaction. Extraction was conducted with ethyl acetate and water. The organic layer thus formed was separated and concentrated in a vacuum to afford <1-b>. (28.0 g, 90 %)

### Synthesis Example 1-(3): Synthesis of <1-c>

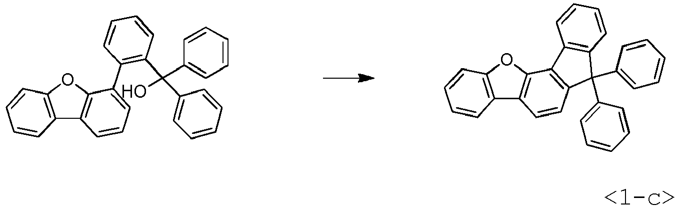

In a 500-ml round-bottom flask reactor, <1-b> (28.0 g, 66 mmol), acetic acid (310 ml) , and HCl (2 ml) were stirred together for 1 hour under reflux. When a solid was formed, the termination of the reaction was confirmed by thin layer chromatography. The reaction mixture was cooled to room temperature. The solid was filtered, washed with H₂O and methanol, and dried to afford <1-c>. (22.3 g, 83.2 %)

### Synthesis Example 1-(4): Synthesis of <1-d>

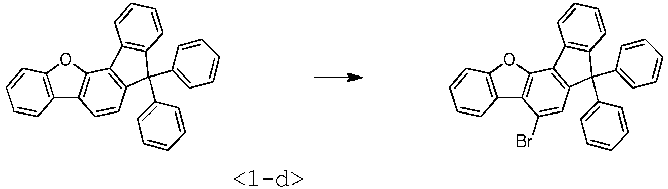

In a 2-L round-bottom flask reactor, <1-c> (22.3 g, 55 mmol) was dissolved in methylene chloride (500 ml). Drops of a mixture of bromine (8.72 g, 55 mmol) and methylene chloride (250 ml) were slowly added to the reactor, followed by stirring at room temperature for 3 hours. After completion of the reaction, the reaction mixture was washed with sodium hydrogen carbonate. The solid thus formed was filtered and recrystallized with toluene and acetone to afford <1-d>. (25.0 g, 94 %)

### Synthesis Example 1-(5): Synthesis of [H 1]

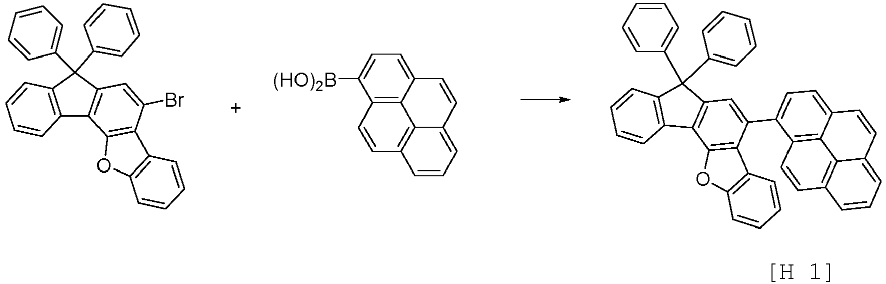

In a reaction reactor, <1-d> (10 g, 0.021 mol), pyrene-1-boronic acid (6.1 g, 0.025 mol), K₂CO₃ (5.7 g, 0.041 mol), Pd(PPh₃)₄ (0.5 g), toluene (40 mL), ethanol 30 mL, and distilled water (30 mL) were stirred together for 6 hours under reflux. Then, the reaction mixture was cooled and slurried with methanol before filtration. After hot filtration in toluene, recrystallization in acetone afforded [H 1] (4 g, 32 %).
MS (MALDI-TOF) : m/z 608.21 [M+]

### SYNTHESIS EXAMPLE 2. Synthesis of [H 25]

### Synthesis Example 2-(1): Synthesis of <2-a>

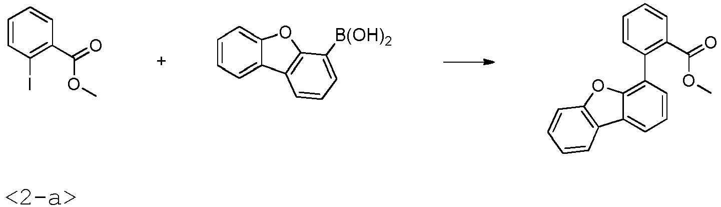

In a 500-mL round-bottom flask reactor, methyl 2-iodobenzoate (19.1 g, 73 mmol), 4-dibenzofuran boronic acid (18.7 g, 88 mmol), tetrakis(triphenylphosphine)palladium (1.7 g, 0.15 mmol), and potassium carbonate (20.2 g, 146.7 mmol) were added with toluene (125 mL), tetrahydrofuran (125 mL), and water (50 mL). The temperature of the reaction was elevated to 80°C and the mixture was stirred for 10 hours. After completion of the reaction, the reaction mixture was cooled to room temperature. After extraction with ethyl acetate, the organic layer thus formed was separated, concentrated in a vacuum, and purified by column chromatography to afford <2-a>. (9.5 g, 43 %)

### Synthesis Example 2-(2): Synthesis of <2-b>

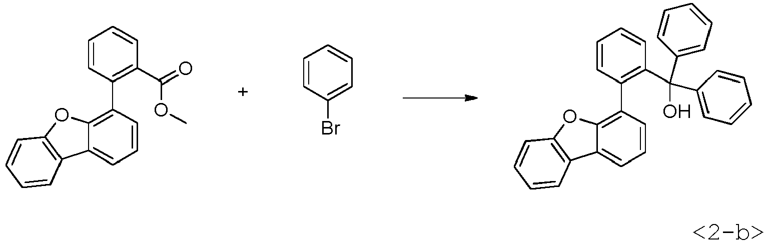

In a 2-L round-bottom flask reactor, bromobenzene (13.2 g, 83.97 mmol) and tetrahydrofuran (250 ml) was stirred together at a low temperature under a nitrogen condition. At -78°C, drops of n-butyl lithium (ca. 58 ml) were slowly added, followed by <2-a> (9.4 g 31.1 mmol). After completion of the reaction, water (100 ml) was added and stirred for 30 minutes.

### Extraction afforded <2-b>. (3.2 g, 24 %)

### Synthesis Example 2-(3): Synthesis of <2-c>

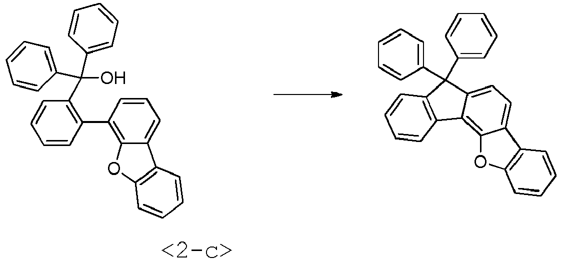

In a 2-L round-bottom flask reactor, <2-b> (55.0 g, 129 mmol), acetic acid (500 ml), and sulfuric acid (10 ml) were stirred together for 5 hours under reflux. After completion of the reaction, the reaction mixture was cooled to room temperature. The solid thus formed was filtered and washed with methanol to afford <2-c>. (50 g, 95 %)

### Synthesis Example 2-(4): Synthesis of <2-d>

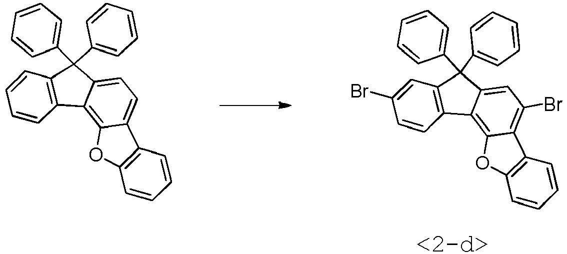

In a 2-L round-bottom flask reactor, <2-c> (50 g, 122 mmol) and dichloromethane (600 ml) were stirred together at room temperature. A dilution of bromine (13.7 ml, 85 mmol) in dichloromethane (50 ml) was added dropwise, followed by stirring for about 3 hours. Recrystallization in methanol accorded <2-d>. (45.6 g, 66 %)

### Synthesis Example 2-(5): Synthesis of [H 25]

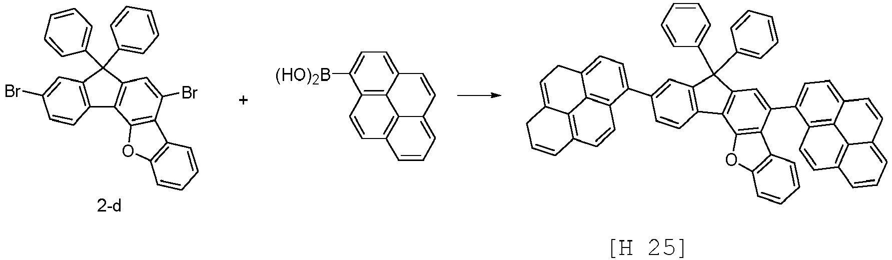

In a reaction reactor, <2-d> 12.0 g(0.021 mol), pyrene-1-boronic acid (11.5 g, 0.047 mol), K₂CO₃ (11.71 g, 0.085 mol), Pd(PPh₃)₄ (0.98 g), toluene (72 mL), ethanol (36 mL), and distilled water (36 mL) were stirred overnight together under reflux. Then, the reaction mixture was cooled and extracted with EA/distilled water. Hot filtration in toluene was followed by recrystallization in methanol. After column purification, recrystallization in toluene/acetone afforded [H 25] (7.8 g, 45 %).
MS (MALDI-TOF) : m/z 810.29 [M+]

### SYNTHESIS EXAMPLE 3. Synthesis of [H 39]

### Synthesis Example 3-(1): Synthesis of <3-a>

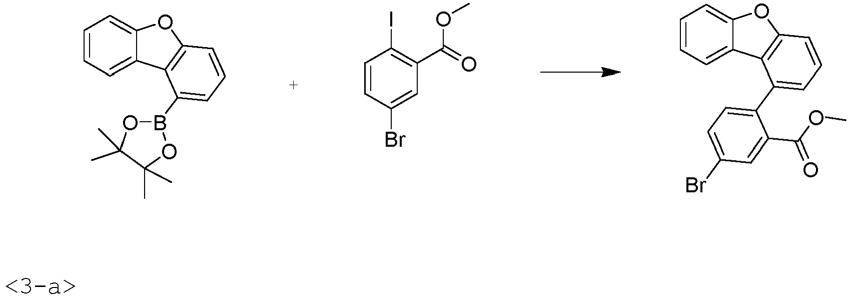

In a round-bottom flask, dibenzofuran-1-boronic acid pinacol ester (35 g, 118 mmol), methyl-5-bromo-2-iodobenzoate (40.5 g, 118 mmol), tetrakis(triphenylphosphine)palladium (2.7 g, 2.3 mmol), potassium carbonate (33 g, 237 mmol), toluene (200 ml), 1,4-dioxane (200 ml), and water (100 ml) were stirred together for 12 hours under reflux. After completion of the reaction, the reaction mixture was subjected to layer separation. The organic layer was concentrated in a vacuum, purified through column chromatography, and dried to <3-a> (33.5 g). (yield 74 %)

### Synthesis Example 3-(2): Synthesis of <3-b>

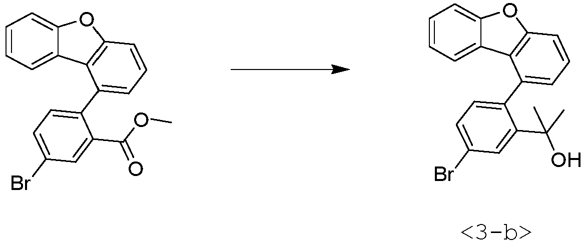

In a round-bottom flask, <3-a> 33.5 g (110 mmol) was added to tetrahydrofuran (150 ml) and chilled to -10°C, followed by slow addition of drops of 3M methyl magnesium bromide (85 ml, 254 mmol). Then, the mixture was heated to 40°C and stirred for 4 hours. Then, after the temperature was lowered to -10°C, drops of 2N HCl (70 ml) were slowly added, followed by an aqueous ammonium chloride solution (70 ml). Then, the temperature was elevated to room temperature. After completion of the reaction, the reaction mixture was washed with water and extracted with ethyl acetate. The organic layer thus formed was separated and concentrated in a vacuum. Purification by column chromatography was followed by drying to afford <3-b> (27 g). (yield 80 %)

### Synthesis Example 3-(3): Synthesis of <3-c>

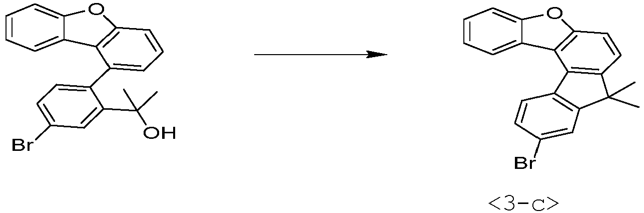

In a round-bottom flask, <3-b> (27 g, 89.2 mmol) and phosphoric acid (70 ml) were stirred together at room temperature for 12 hours under a nitrogen atmosphere. After completion of the reaction, extraction was conducted with ethyl acetate and water and the organic layer was concentrated. The concentrate was purified by column chromatography and dried to afford <3-c> (17.6 g). (yield 70 %)

### Synthesis Example 3-(4): Synthesis of <3-d>

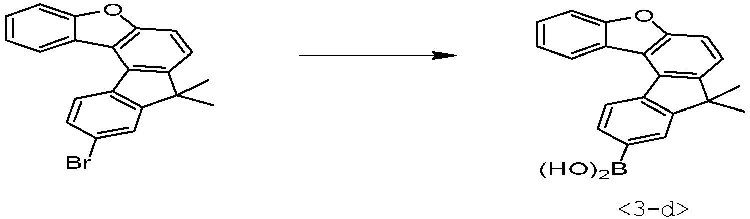

In a round-bottom flask, <3-c> (17.6 g, 48.4 mmol) was added with tetrahydrofuran (200 ml) and chilled to -78°C under a nitrogen atmosphere. Drops of 1.6 M n-buthyllithium (36.3 ml, 58.1 mmol) were slowly added. One hour later, trimethyl borate (7.0 ml, 62.9 mmol) was slowly introduced while the low temperature was maintained. Then, the mixture was stirred at room temperature. After completion of the reaction, the reaction mixture was subjected to layer separation, and the organic layer thus formed was concentrated in a vacuum, recrystallized in hexane, and dried to afford <3-d> (33 g). (yield 71 %).

### Synthesis Example 3-(5): Synthesis of <3-e>

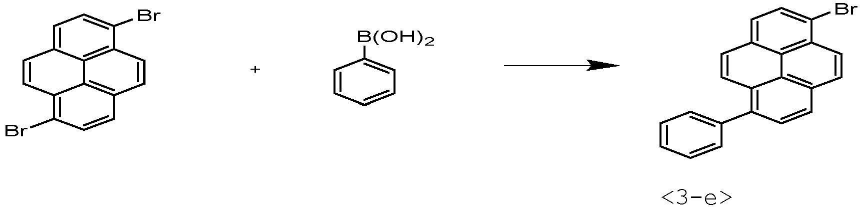

In a round-bottom flask purged with nitrogen, 1,6-dibromopyrene (100 g, 0.278 mol), phenyl boronic acid (33.9 g, 0.278 mol), tetrakis(triphenylphosphine)palladium (Pd[PPh₃]₄) (6.4 g, 0.006 mol), sodium carbonate (88.3 g, 0.833 mol), toluene (1400 ml), and water (420 ml) were stirred together for 9 hours under reflux. After completion of the reaction, the reaction mixture was cooled to room temperature and the solid thus formed was filtered off. The filtrate was subjected to extraction with ethyl acetate and water. The organic layer was dehydrated, concentrated in a vacuum, and purified by column chromatography to afford <3-e> (45.4 g). (yield 45.7 %).

### Synthesis Example 3-(6): Synthesis of [H 39]

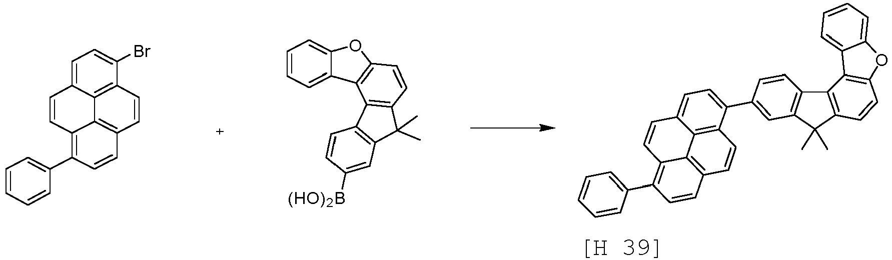

In a round-bottom flask, the intermediate <3-e> (10 g, 27.9 mmol), the intermediate <3-d> (9.2 g, 27.9 mmol), tetrakis(triphenylphosphine)palladium (0.6 g, 0.5 mmol), potassium carbonate (7.7 g, 55.9 mmol), toluene (35 ml), 1,4-dioxane (35 ml), and water (30 ml) were stirred together for 12 hour under reflux in a nitrogen atmosphere. After completion of the reaction, the reaction mixture was subjected to layer separation and the organic layer thus formed was concentrated in a vacuum, purified by column chromatography, and dried to afford [H 39] (9.8 g). (yield 63 %)
MS (MALDI-TOF) : m/z 560.21 [M+]

### SYNTHESIS EXAMPLE 4. Synthesis of [H 44]

### Synthesis Example 4-(1): Synthesis of <4-a>

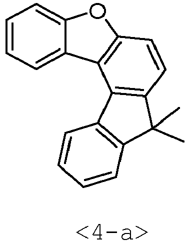

The same synthesis procedures were conducted as in Synthesis Examples 3-1 to 3-3, with the exception of using methyl-2-iodobenzoate instead of methyl-5-bromo-2-iodobenzoate in Synthesis Example 3-1, to afford <4-a>. (yield 60 %)

### Synthesis Example 4-(2): Synthesis of <4-b>

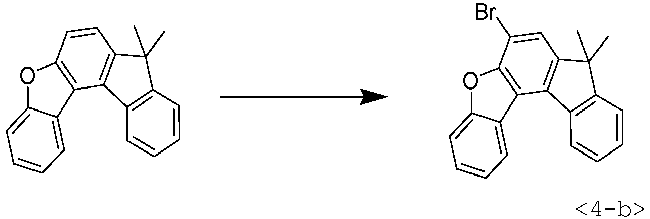

In a round-bottom flask, <4-a> (37.8 g, 133 mmol), N-bromosuccinimide (23.8 g, 133 mmol), and dimethylformamide (600 mL) were stirred together at 50°C for 12 hours under a nitrogen atmosphere. After completion of the reaction, the organic layer was concentrated in a vacuum and purified by column chromatography to afford <4-b> (33.8 g). (yield 70 %)

### Synthesis Example 4-(3): Synthesis of <4-c>

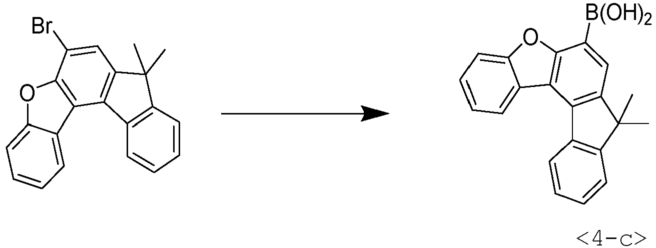

The same synthesis procedure was conducted as in Synthesis Example 3-4, with the exception of using <4-b> instead of <3-c>, to afford <4-c>. (yield 70 %)

### Synthesis Example 4-(4): Synthesis of [H 44]

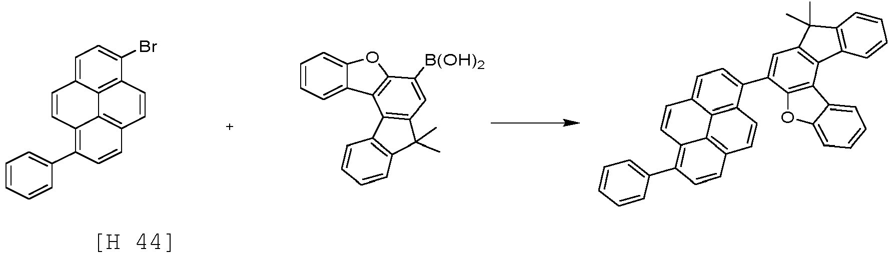

The same synthesis procedure was conducted as in Synthesis Example 3-6, with the exception of using <4-c> instead of <3-d>, to afford [H 44]. (yield 70 %)
MS(MALDI-TOF) : m/z 560.21 [M]+

### SYNTHESIS EXAMPLE 5. Synthesis of [H 58]

### Synthesis Example 5-(1): Synthesis of <5-a>

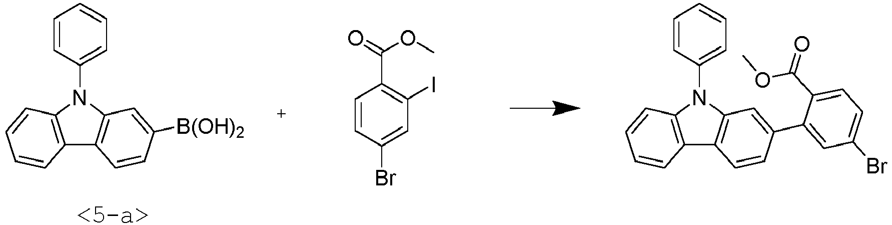

The same synthesis procedure was conducted as in Synthesis Example 3-1, with the exception of using 9-phenylcarbazole-2 boronic acid and methyl-4-bromo-2-iodobenzoic acid instead of dibenzofuran-1-boronic acid pinacol ester and methyl-5-bromo-2-iodobenzoate, respectively, to afford <5-a>. (yield 68 %)

### Synthesis Example 5-(2): Synthesis of <5-b>

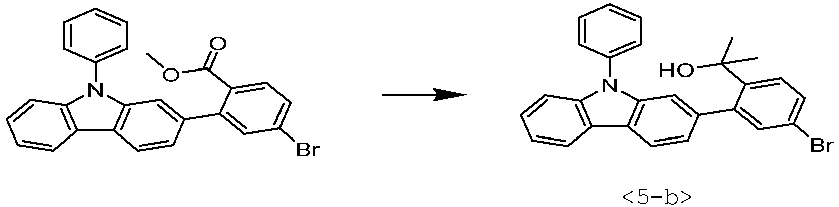

The same synthesis procedure was conducted as in Synthesis Example 3-2, with the exception of using <5-a> instead of <3-a>, to afford <5-b>. (yield 80 %)

### Synthesis Example 5-(3): Synthesis of <5-c>

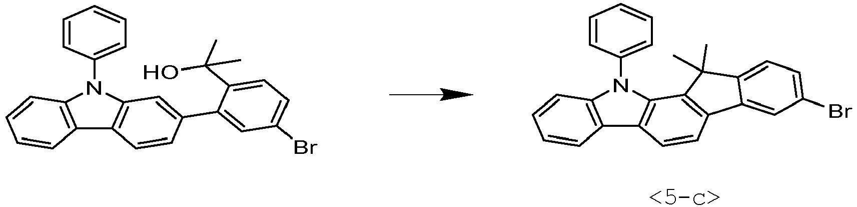

The same synthesis procedure was conducted as in Synthesis Example 3-3, with the exception of using <5-b> instead of <3-b>, to afford intermediate <5-c>. (yield 70 %)

### Synthesis Example 5-(4): Synthesis of <5-d>

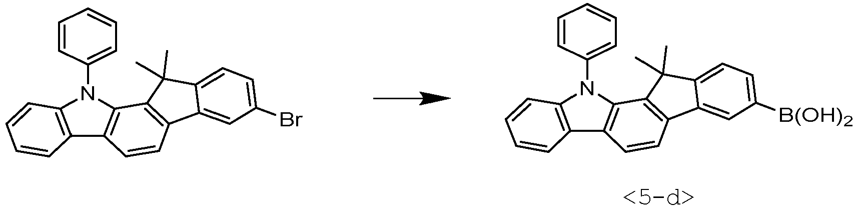

The same synthesis procedure was conducted as in Synthesis Example 3-4, with the exception of using <5-c> instead of <3-c>, to afford intermediate <5-d>. (yield 72 %)

### Synthesis Example 5-(5): Synthesis of [H 58]

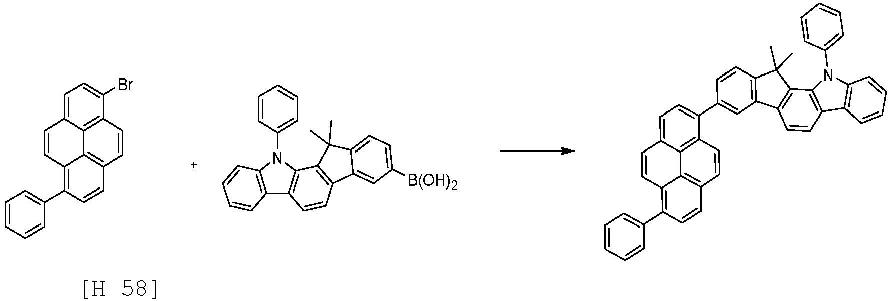

The same synthesis procedure was conducted as in Synthesis Example 3-6, with the exception of using <5-d> instead of <3-d>, to afford [H 58]. (yield 70 %)
MS(MALDI-TOF) : m/z 635.26 [M]+

### SYNTHESIS EXAMPLE 6. Synthesis of [H 85]

### Synthesis Example 6-(1): Synthesis of <6-a>

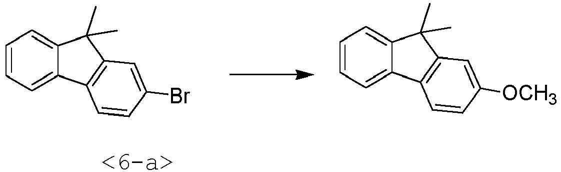

In a round-bottom flask, 2-bromo-9,9-dimethylfluorene (50 g, 183 mmol), a sodium methoxide solution (59.3 g (1098 mmol), copper iodide (10.4 g, 54.9 mmol), and methanol (200 ml) were stirred together for 12 hours under reflux in a nitrogen atmosphere. After completion of the reaction, the reaction mixture was subjected to layer separation. The organic layer was concentrated in a vacuum, purified by column chromatography, and dried to afford <6-a> (33.2 g). (yield 81 %)

### Synthesis Example 6-(2): Synthesis of <6-b>

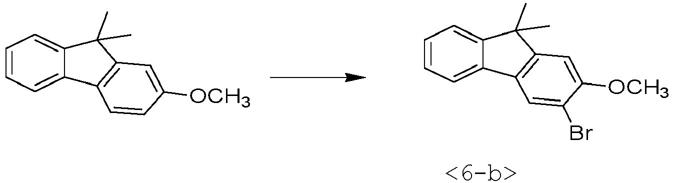

In a round-bottom flask, <6-a> (30 g, 133 mmol), N-bromosuccinimide (23.8 g, 133 mmol), and dimethylformamide (600 ml) were stirred together at 50°C for 12 hours under a nitrogen atmosphere. After completion of the reaction, the reaction mixture was concentrated in a vacuum, purified by column chromatography, and dried to afford <6-b> (28 g). (yield 70 %)

### Synthesis Example 6-(3): Synthesis of <6-c>

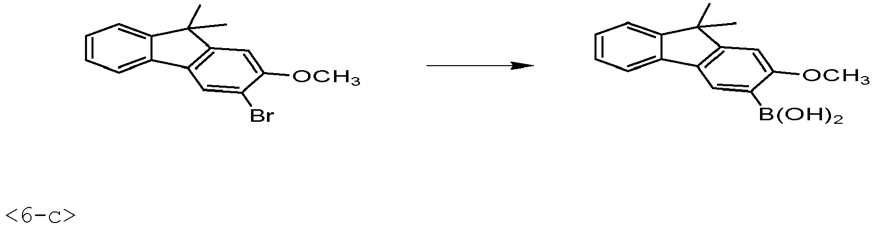

The same synthesis procedure was conducted as in Synthesis Example 3-4, with the exception of using <6-b> instead of <3-c>, to afford <6-c>. (yield 72 %)

### Synthesis Example 6-(4): Synthesis of <6-d>

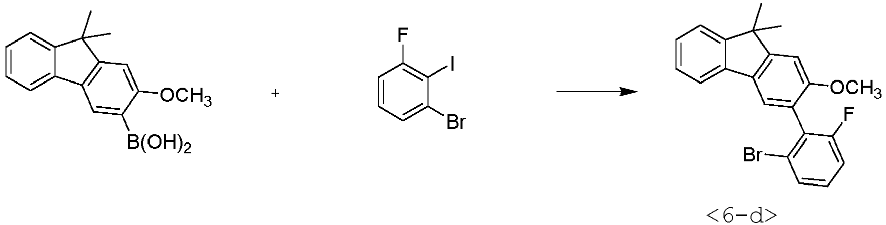

The same synthesis procedure was conducted as in Synthesis Example 3-1, with the exception of using <6-c> and 1-bromo-2-iodo-3-fluorobenzene instead of dibenzofuran-1-boronic acid pinacol ester and methyl-5-bromo-2-iodobenzoate, respectively, to afford <6-d>. (yield 70 %)

### Synthesis Example 6-(5): Synthesis of <6-e>

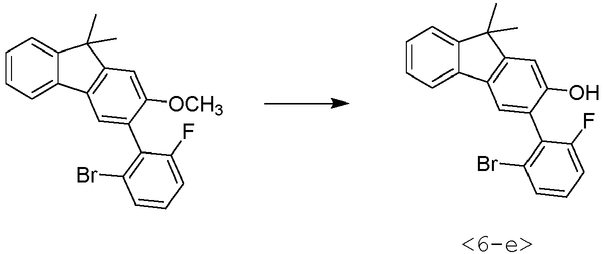

In a round-bottom flask, <6-d> (30 g, 85 mmol) and dichloromethane (300 ml) was introduced under a nitrogen atmosphere and cooled to 0°C. Then, a dilution of boron tribromide (63.9 g, 255 mmol) in dichloromethane (150 ml0 was slowly added in a dropwise manner. The mixture was stirred for 6 hours at room temperature. After completion of the reaction, the reaction mixture was subjected to layer separation and the organic solution was concentrated in a vacuum, purified by column chromatography, and dried to afford <6-e> (21.3 g). (yield 74 %)

### Synthesis Example 6-(6): Synthesis of <6-f>

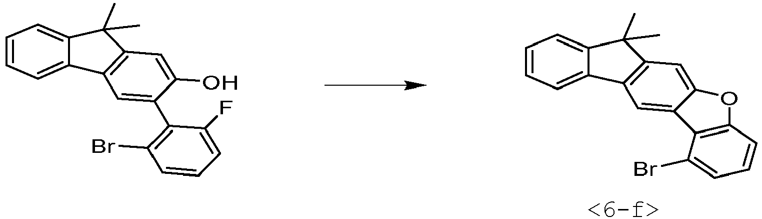

In a round-bottom flask, <6-e> (20 g, 59 mmol), potassium carbonate (13 g, 94.5 mmol), and 1-methyl-2-pyrrolidinone (200 ml) were together stirred at 150°C for 12 hours under a nitrogen atmosphere. After completion of the reaction, the reaction mixture was subjected to layer separation and the organic layer was concentrated in a vacuum, purified by column chromatography, and dried to afford <6-f> (13.5 g). (yield 72 %)

### Synthesis Example 6-(7): Synthesis of <6-g>

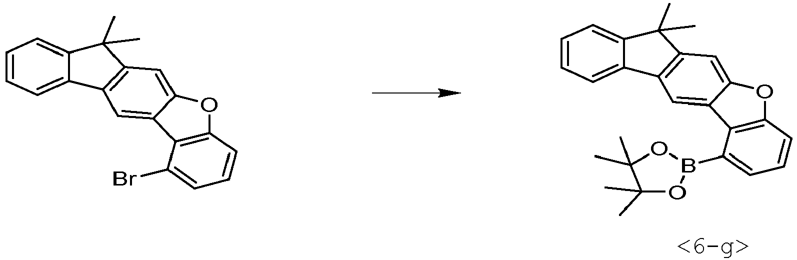

In a round-bottom flask, <6-f> (13 g, 40.8 mmol), bis(pinacolato)diborane (12.4 g, 48.9 mmol), tris(dibenzylideneacetone)palladium (2 g, 2.4 mmol), potassium acetate (11.6 g, 122 mmol), tricyclohexylphosphine (2.7 g, 9.8 mmol), and N-dimethylformamide (150 ml) were stirred together under reflux in a nitrogen atmosphere. After completion of the reaction, the reaction was subjected to layer separation and the organic layer was concentrated in a vacuum, purified by column chromatography, and dried to afford <6-g> (10.8 g). (yield 65 %)

### Synthesis Example 6-(8): Synthesis of [H 85]

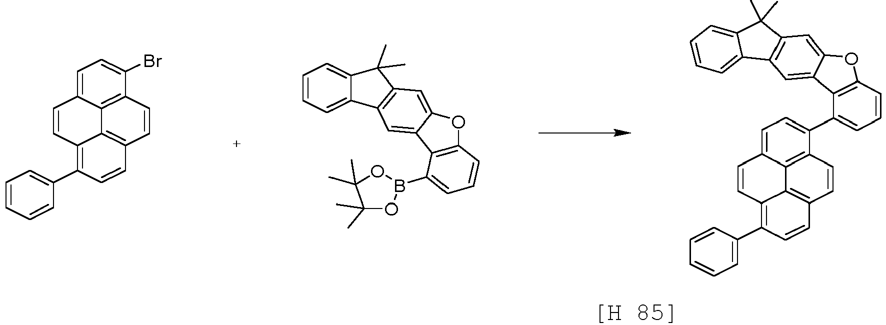

The same synthesis procedure was conducted as in Synthesis Example 3-6, with the exception of using <6-g> instead of <3-d>, to afford [H 85]. (yield 65 %)
MS(MALDI-TOF) : m/z 560.21 [M]+

### SYNTHESIS EXAMPLE 7. Synthesis of [H 102]

### Synthesis Example 7-(1): Synthesis of <7-a>

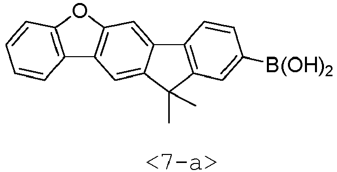

The same synthesis procedures were conducted as in Synthesis Examples 3-1 to 3-3 and 4-2 to 4-3, with the exception of using dibenzofuran-3-boronic acid and methyl-1-iodobenzoate instead of dibenzofuran-1-boronic acid pinacol ester and methyl-5-bromo-2-iodobenzoate in Synthesis Example 3-1, to afford <7-a>. (yield 70 %)

### Synthesis Example 7-(2): Synthesis of [H 102]

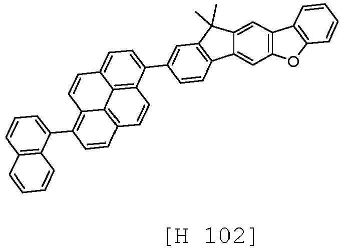

The same synthesis procedures were conducted as in Synthesis Examples 3-5 to 3-6, with the exception of using naphthalene boronic acid instead of phenyl boronic acid in Synthesis Example 3-5 and <7a> instead of <3-d> in Synthesis Example 3-6, to afford [H 102]. (yield 68 %)

MS(MALDI-TOF) : m/z 610.23 [M]+

### SYNTHESIS EXAMPLE 8. Synthesis of [H 54]

### Synthesis Example 8-(1): Synthesis of <8-a>

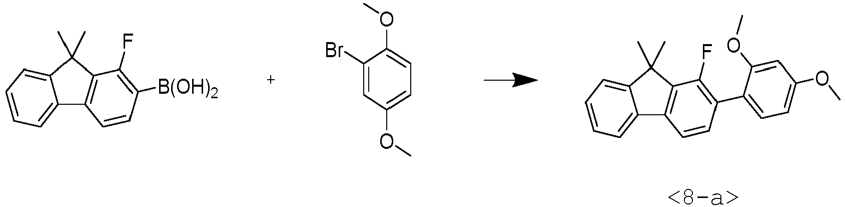

In a round-bottom flask, 1-fluoro-9,9'-dimethylfluoren-2-yl boronic acid (30 g, 0.117 mol), 2-bromo-1,4-dimethoxybenzene (30.5 g, 0.141 mol), tetrakis(triphenylphosphine)palladium (2.7 g, 0.002 mol), potassium carbonate (27.5 g, 0.199 mol), toluene (210 ml), ethanol (51 ml), and water (100 ml) were stirred together for 12 hours under reflux. After completion of the reaction, the reaction mixture was subjected to layer separation and the organic layer was concentrated in a vacuum and purified by column chromatography to afford <8-a> (28 g). (yield 72.2 %)

### Synthesis Example 8-(2): Synthesis of <8-b>

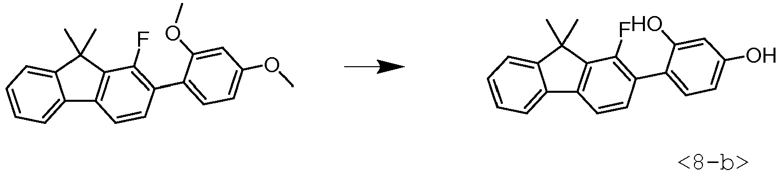

The same synthesis procedure was conducted as in Synthesis Example 6-5, with the exception of using <8-a> instead of <6-d>, to afford <8-b>. (yield 93.2 %)

### Synthesis Example 8-(3): Synthesis of <8-c>

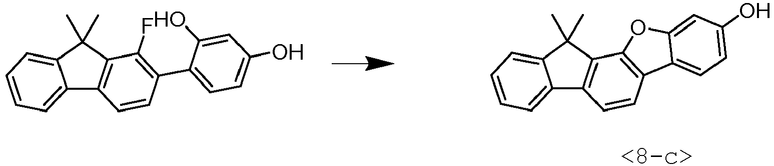

The same synthesis procedure was conducted as in Synthesis Example 6-6, with the exception of using <8-b> instead of <6-e>, to afford <8-c>. (yield 84.4 %)

### Synthesis Example 8-(4): Synthesis of <8-d>

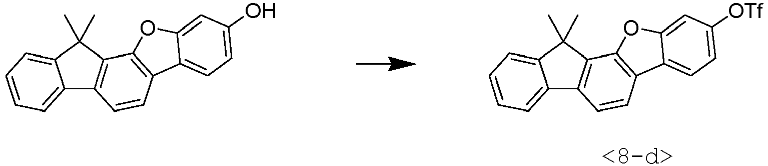

In a round-bottom flask, a mixture of <8-c> (19 g, 0.063 mol), pyridine (6.5 g, 0.082 mol), and dichloromethane (190 ml) was cooled to 0°C or less under a nitrogen atmosphere, followed by slowly adding drops of trifluoromethane sulfonic anhydride (19.6 g, 0.070 mol). Thereafter, the mixture was heated to room temperature and then stirred for 5 hours. After completion of the reaction, the reaction mixture was added with water and stirred to conduct layer separation. The organic layer was dehydrated and concentrated in a vacuum. Purification by column chromatography afforded <8-d> (23 g). (yield 84.1 %)

### Synthesis Example 8-(5): Synthesis of <8-e>

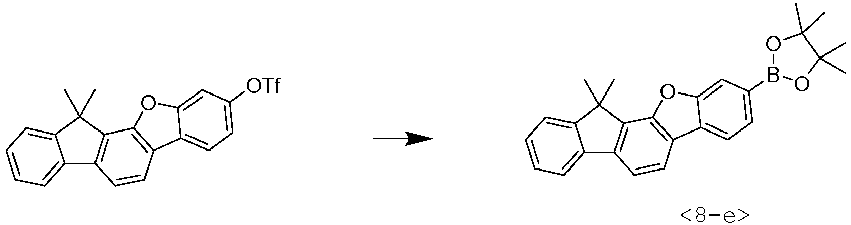

In a round-bottom flask, <8-d> (23 g, 0.053 mol), bis(pinacol)diboron (16.2 g, 0.064 mol), bis(diphenylphosphino)ferrocene dichloropalladium (0.9 g, 0.001 mol), calcium acetate (13.1 g, 0.133 mol), and 1,4-dioxane (230 ml) were stirred together for 12 hours. After completion of the reaction, the reaction mixture was cooled to room temperature and filtered through a silica gel pad topped with celite pad. The filtrate was concentrated and purified by column chromatography to afford <8-e> (17 g). (yield 77.9 %)

### Synthesis Example 8-(6): Synthesis of [H 54]

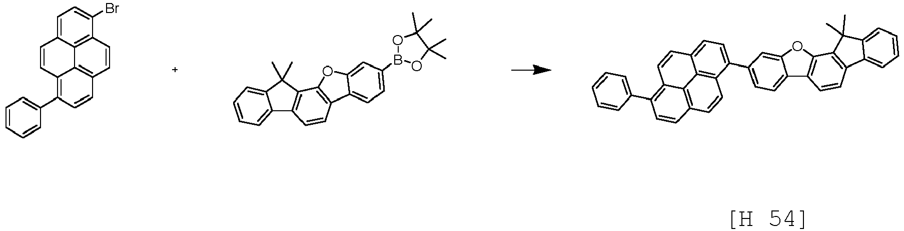

The same synthesis procedure was conducted as in Synthesis Example 3-6, with the exception of using <8-e> instead of <3-d>, to afford [H 54] (16 g). (yield 78.4 %)
MS(MALDI-TOF) : m/z 560.21 [M]+

### SYNTHESIS EXAMPLE 9. Synthesis of [H 7]

### Synthesis Example 9-(1): Synthesis of <9-a>

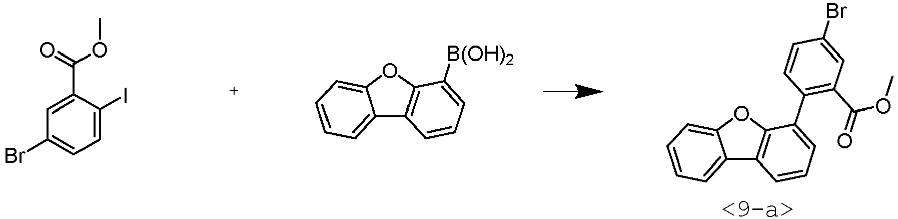

The same synthesis procedure was conducted as in Synthesis Example 3-1, with the exception of using dibenzofuran-4-boronic acid instead of dibenzofuran-1-boronic acid pinacol ester, to afford <9-a> (48 g). (yield 74 %)

### Synthesis Example 9-(2): Synthesis of <9-b>

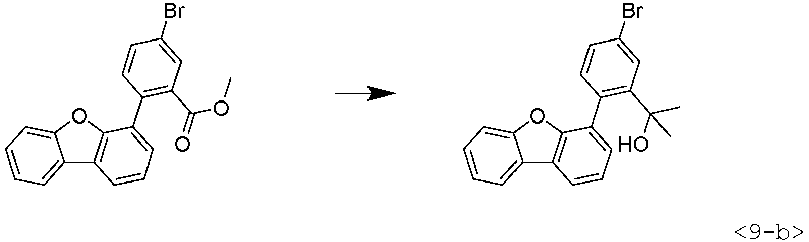

The same synthesis procedure was conducted as in Synthesis Example 3-2, with the exception of using <9-a> instead of <3-a>, to afford <9-b> (47 g). (yield 97.9 %)

### Synthesis Example 9-(3): Synthesis of <9-c>

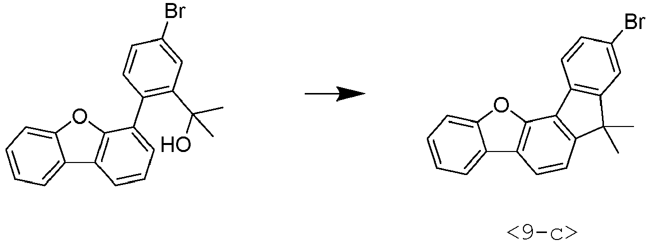

The same synthesis procedure was conducted as in Synthesis Example 3-3, with the exception of using <9-b> instead of <3-b>, to afford <9-c> (30 g). (yield 67.1 %)

### Synthesis Example 9-(4): Synthesis of <9-d>

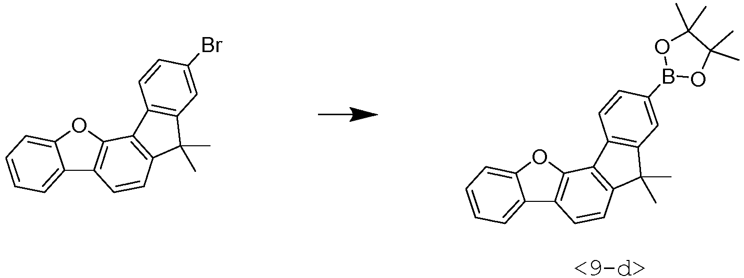

The same synthesis procedure was conducted as in Synthesis Example 8-5, with the exception of using <9-c> instead of <8-d>, to afford <9-d> (27 g). (yield 79.8 %)

### Synthesis Example 9-(5): Synthesis of [H 7]

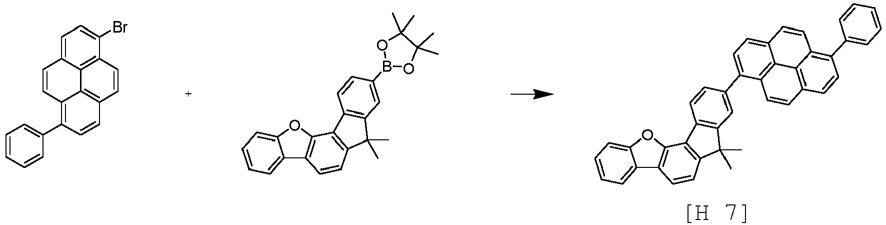

The same synthesis procedure was conducted as in Synthesis Example 3-6, with the exception of using <9-d> instead of <3-d>, to afford [H 7] (14 g). (yield 62.4 %)
MS(MALDI-TOF) : m/z 560.21 [M]+

### SYNTHESIS EXAMPLE 10. Synthesis of [H 14]

### Synthesis Example 10-(1): Synthesis of <10-a>

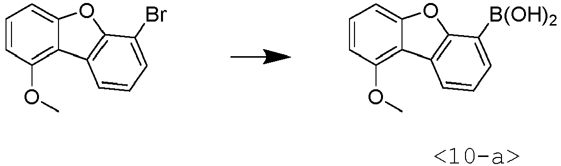

The same synthesis procedure was conducted as in Synthesis Example 3-4, with the exception of using 6-bromo-1-methoxydibenzofuran instead of <3-c>, to afford <10-a> (43 g). (yield 95.1 %)

### Synthesis Example 10-(2): Synthesis of <10-b>

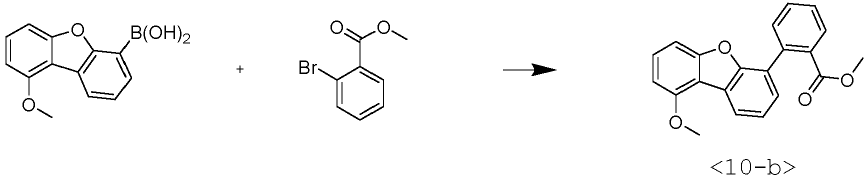

The same synthesis procedure was conducted as in Synthesis Example 3-1, with the exception of using <10-a> and methyl-2-bromobenzoate instead of dibenzofuran-1-boronic acid pinacol ester and methyl-5-bromo-2-iodobenzoate, respectively, to afford <10-b> (52 g). (yield 96.1 %)

### Synthesis Example 10-(3): Synthesis of <10-c>

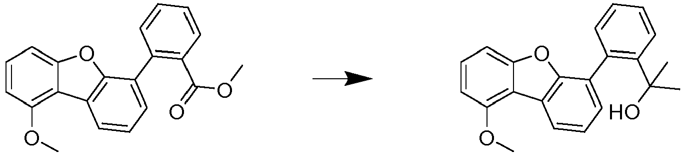

<10-c>

The same synthesis procedure was conducted as in Synthesis Example 3-2, with the exception of using <10-b> instead of <3-a>, to afford <10-c> (48 g). (yield 92.3 %)

### Synthesis Example 10-(4): Synthesis of <10-d>

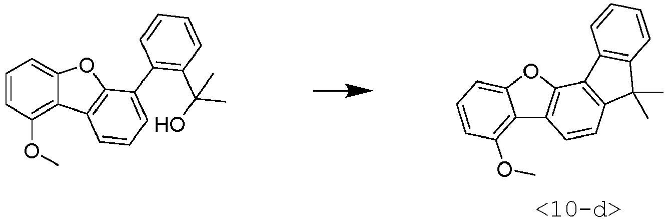

The same synthesis procedure was conducted as in Synthesis Example 3-3, with the exception of using <10-c> instead of <3-b>, to afford <10-d> (38 g). (yield 83.8 %)

### Synthesis Example 10-(5): Synthesis of <10-e>

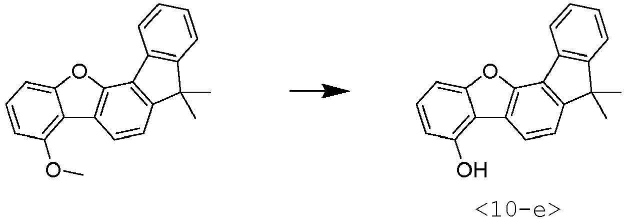

The same synthesis procedure was conducted as in Synthesis Example 6-5, with the exception of using <10-d> instead of <6-d>, to afford <10-e> (28 g). (yield 77.1 %)

### Synthesis Example 10-(6): Synthesis of <10-f>

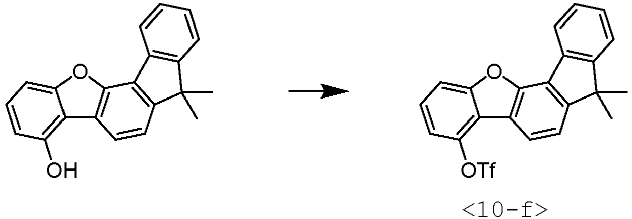

The same synthesis procedure was conducted as in Synthesis Example 8-4, with the exception of using <10-e> instead of <8-c>, to afford <10-f> (35 g). (yield 87.5 %)

### Synthesis Example 10-(7): Synthesis of <10-g>

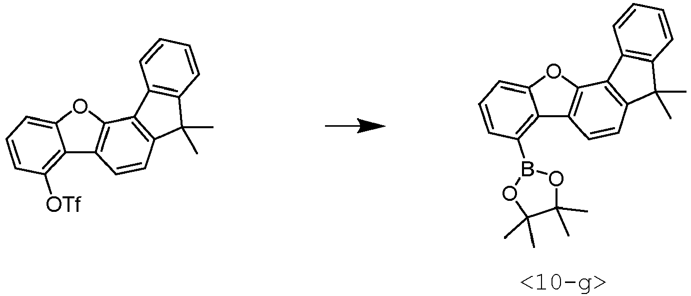

The same synthesis procedure was conducted as in Synthesis Example 8-5, with the exception of using <10-f> instead of <8-d>, to afford <10-g> (26 g). (yield 78.3 %)

### Synthesis Example 10-(8): Synthesis of [H 14]

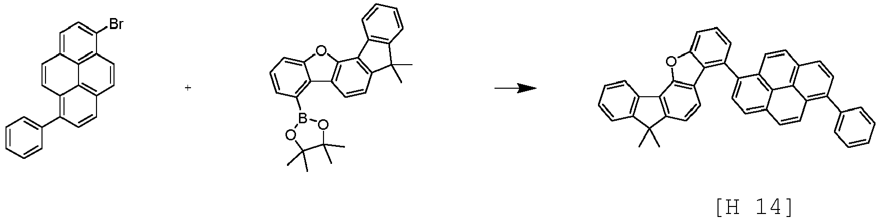

The same synthesis procedure was conducted as in Synthesis Example 3-6, with the exception of using <10-g> instead of <3-d>, to afford [H 14] (12 g). (yield 52.4 %)
MS(MALDI-TOF) : m/z 560.21 [M]+

### SYNTHESIS EXAMPLE 11. Synthesis of [H 17]

### Synthesis Example 11-(1): Synthesis of <11-a>

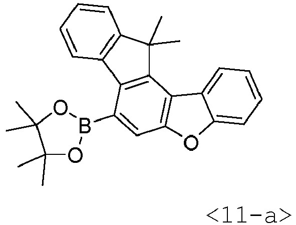

The same synthesis procedures were conducted as in Synthesis Examples 10-1 to 10-7, with the exception of using 2-bromo-3-methoxydibenzofuran instead of 6-bromo-1-methoxydibenzofuran in Synthesis Example 10-1, to afford <11-a> (23 g). (yield 54.3 %)

### Synthesis Example 11-(2): Synthesis of [H 17]

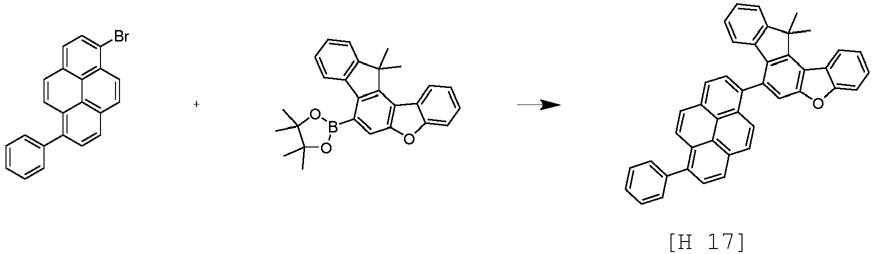

The same synthesis procedure was conducted as in Synthesis Example 3-6, with the exception of using <11-a> instead of <3-d>, to afford [H 17] (16.7 g). (yield 57.5 %)
MS(MALDI-TOF) : m/z 560.21 [M]+

### SYNTHESIS EXAMPLE 12. Synthesis of [H 90]

### Synthesis Example 12-(1): Synthesis of <12-a>

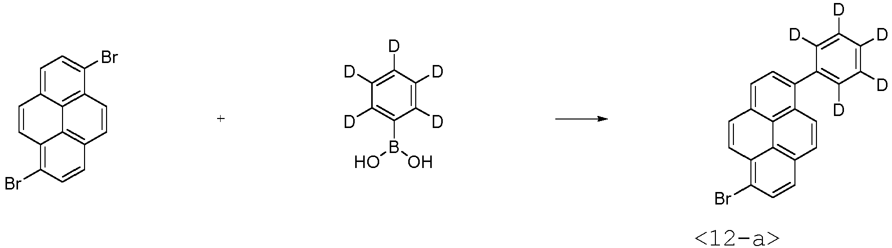

The same synthesis procedure was conducted as in Synthesis Example 3-5, with the exception of using phenyl boronic acid(D5) instead of phenyl boronic acid, to afford <12-a> (46 g). (yield 45.7 %)

### Synthesis Example 12-(2): Synthesis of [H 90]

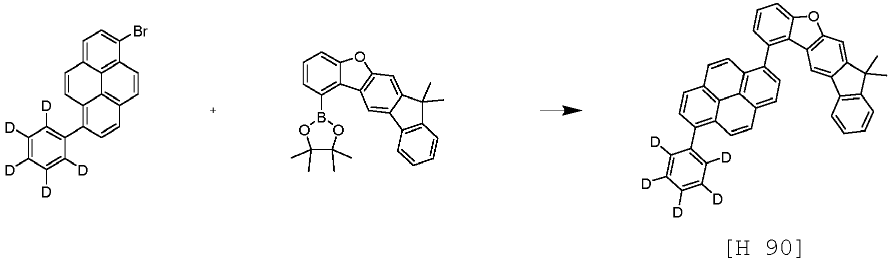

The same synthesis procedure was conducted as in Synthesis Example 6-8, with the exception of using <12-a> instead of <3-e>, to afford [H 90] (8.7 g). (yield 68.7 %)
MS(MALDI-TOF) : m/z 565.25[M]+

### SYNTHESIS EXAMPLE 13. Synthesis of [H 51]

### Synthesis Example 13-(1): Synthesis of [H 51]

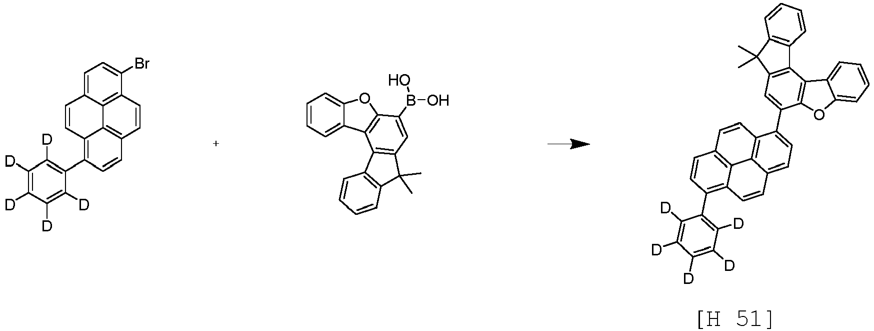

The same synthesis procedure was conducted as in Synthesis Example 4-4, with the exception of using <12-a> instead of <3-e>, to afford [H 51] (12.4 g). (yield 75 %)
MS(MALDI-TOF) : m/z 565.25[M]+

### EXAMPLES 1 to 14: Fabrication of Organic Light Emitting Diodes

An ITO glass substrate was patterned to have a translucent area of 2 mm × 2 mm and cleansed. The ITO glass was mounted in a vacuum chamber that was then set to have a base pressure of 1×10⁻⁷ torr. On the ITO glass substrate, films were sequentially formed of DNTPD (700 Å) and **α** -NPD (300 Å) in the order. Subsequently, light-emitting layers (200 Å) were formed of a combination of the host compounds according to the present disclosure and dopant compounds (1 wt%) listed, below. Then, [E-1] and [ET5] were deposited at a ratio of 1:1 to form an electron transport layer (300 Å), on which an electron injecting layer of [Chemical Formula E-1] (10 Å) was formed and then covered with an Al layer (1000 Å) to fabricate organic light-emitting diodes. The organic light-emitting diodes thus obtained were measured at 0.4 mA for luminescence properties and the measurements are summarized in Tables 1 and 2:

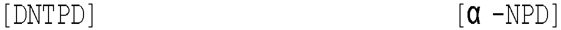

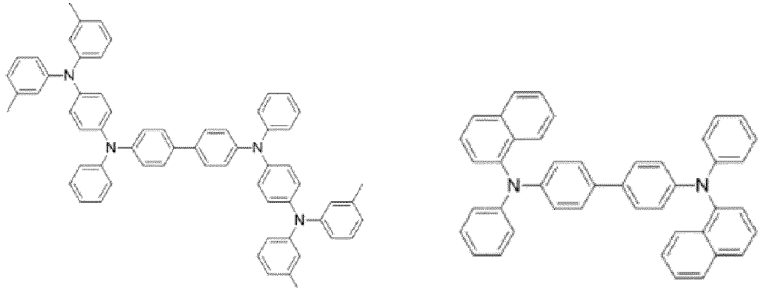

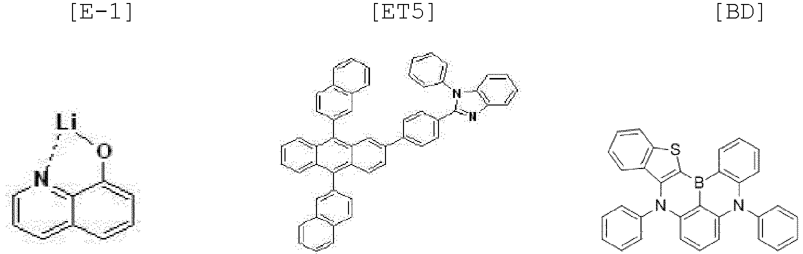

### COMPARATIVE EXAMPLES 1 AND 2

Organic light emitting diodes were fabricated in the same manner as in the above Examples, with the exception that the following compound 312 (compound 312 in Korean Patent No. 10-2018-0077887 A) or [BH 1] as a host, instead of the compounds according to the present disclosure. The luminescence of the organic light-emitting diodes thus obtained was measured at 0.4 mA and the measurements are summarized in Tables 1 and 2.

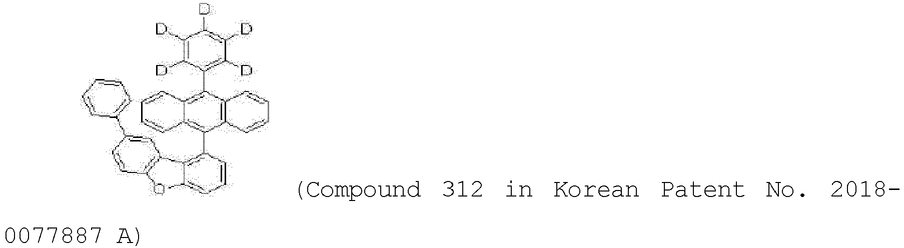

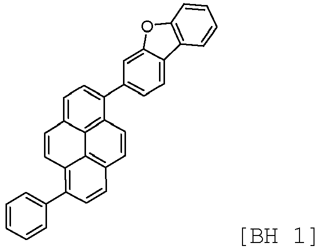

**TABLE 1 Luminous Efficiency**

| | Host | V | cd/A | Q.E | CIEx | CIEy |
|---|---|---|---|---|---|---|
| C. Ex. 1 | Compound 312 | 3.20 | 6.9 | 7.9 | 0.1311 | 0.1025 |
| C. Ex. 2 | BH 1 | 4.10 | 7.8 | 7.5 | 0.1330 | 0.1030 |
| Ex. 1 | H 1 | 4.28 | 8.91 | 10.1 | 0.1319 | 0.1033 |
| Ex. 2 | H 25 | 3.74 | 10.08 | 9 | 0.1354 | 0.1436 |
| Ex. 3 | H 27 | 3.69 | 11.8 | 8.6 | 0.1429 | 0.2192 |

As is understood from data of Table 1, the organic light emitting diodes according to the present disclosure exhibited excellent luminous efficiency, compared to that using the compound of Comparative Example 1, thus having the high plausibility of applying for organic light-emitting diodes.

**TABLE 1 Luminous Efficiency and Longevity**

| | Host | V | EQE | T97 |
|---|---|---|---|---|
| C. Ex. 2 | BH 1 | 4.1 | 7.5 | 138 |
| Ex. 4 | H 39 | 3.6 | 10.5 | 184 |
| Ex. 5 | H 44 | 3.3 | 10.6 | 180 |
| Ex. 6 | H 58 | 3.7 | 10.1 | 225 |
| Ex. 7 | H 85 | 3.6 | 10.3 | 214 |
| Ex. 8 | H 102 | 3.7 | 9.8 | 197 |
| Ex. 9 | H 54 | 3.7 | 10.0 | 208 |
| Ex. 10 | H 7 | 3.9 | 10.1 | 203 |
| Ex. 11 | H 14 | 3.8 | 10.2 | 200 |
| Ex. 12 | H 17 | 3.8 | 9.8 | 210 |
| Ex. 13 | H 90 | 3.5 | 10.0 | 250 |
| Ex. 14 | H 51 | 3.7 | 10.3 | 228 |

As is understood from data of Table 2, the organic light emitting diodes according to the present disclosure exhibited excellent luminous efficiency and longevity, compared to that using the compound of Comparative Example 2, thus having the high plausibility of applying for organic light-emitting diodes.

### EXAMPLES 15 TO 19: Fabrication of Organic Light-Emitting Diodes Including First and Second Light-Emitting Layers

An ITO glass substrate was patterned to have a translucent area of 2 mm × 2 mm and cleansed. The ITO glass was mounted in a vacuum chamber that was then set to have a base pressure of 1×10⁻⁷ torr. On the ITO glass substrate, films were sequentially formed of DNTPD (700 Å) and **α** -NPD (300 Å) in the order. A first light-emitting layer and a second light-emitting layer according to the present disclosure were formed sequentially. The first light-emitting layers (50 Å) were formed of a combination of the host compounds according to the present disclosure and the BD dopant compound (1 wt%). The second light-emitting layers (150 **Å**) were formed of a combination of the following Compound 313 (Compound 313 in Korean Patent No. 2018-0077887 A) and the BD dopant compound (1 wt%). Then, [E-1] and [ET5] were deposited at a ratio of 1:1 to form an electron transport layer (300 Å), on which an electron injecting layer of [E-1] (10 Å) was formed and then covered with an Al layer (1000 Å) to fabricate organic light-emitting diodes. The organic light-emitting diodes thus obtained were measured at 0.4 mA for luminescence properties and the measurements are summarized in Table 3, below.

### COMPARATIVE EXAMPLE 3

An organic light emitting diode was fabricated in the same manner as in the Examples 1 to 14, with the exception that the following compound 313 (Korean Patent No. 10-2018-0077887 A) or [BH 1] as a host, instead of the compounds according to the present disclosure. The luminescence of the organic light-emitting diode thus obtained was measured at 0.4 mA and the measurements are summarized in Table 3, below.

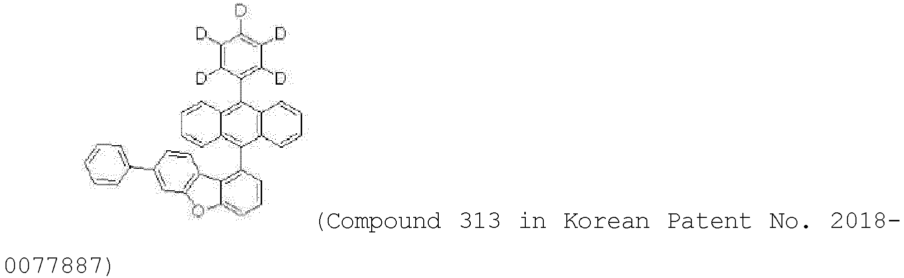

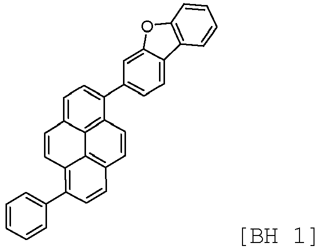

**TABLE 3**

| | 1^{ST} Light-Emitting Layer | 2^{nd} Light-Emitting Layer | V | cd/A | Q.E | CIEx | CIEy |
|---|---|---|---|---|---|---|---|
| Ex. 15 | H 1 | Compound 313 | 3.69 | 9.82 | 11.4 | 0.1318 | 0.0992 |
| Ex. 16 | H 25 | Compound 313 | 3.58 | 9.83 | 10.4 | 0.1306 | 0.1137 |
| Ex. 17 | H 27 | Compound 313 | 3.56 | 12.04 | 10.3 | 0.1353 | 0.1581 |
| Ex. 18 | H 90 | Compound 313 | 3.45 | 9.4 | 11.8 | 0.1330 | 0.0874 |
| Ex. 19 | H 44 | Compound | 3.1 | 9.0 | 11.4 | 0.1392 | 0.0738 |
| | | 313 | | | | | |
| C. Ex. 3 | Compound 313 | | 3.48 | 8.4 | 10 | 0.1322 | 0.0955 |

As is understood from the data of Table 3, the organic light-emitting diodes of the present disclosure that employ the host compounds according to the present disclosure and the conventional host compound in the first and second light-emitting layers, respectively, exhibited higher luminous efficiency than that using only the compound 313, thus having the high plausibility of applying for organic light-emitting diodes. In addition, the organic light-emitting diodes including two light-emitting layers (dual light-emitting layer) that contains the compound of Chemical Formula A or B as a host in Table 3 can be driven at lower voltages with higher luminous efficiency, than those including single light-emitting layers that contain the compound of Chemical Formula A or B as a host in Table A or B.

### Industrial Applicability

The organic light-emitting diodes fabricated using the compounds of the present disclosure exhibit improved luminous efficiency and longevity characteristics, compared to those using conventional compounds, thus finding advantageous applications in the organic light emitting diode field and related industrial fields.

## Claims

1. A compound, represented by the following [Chemical Formula A] or [Chemical Formula B]: 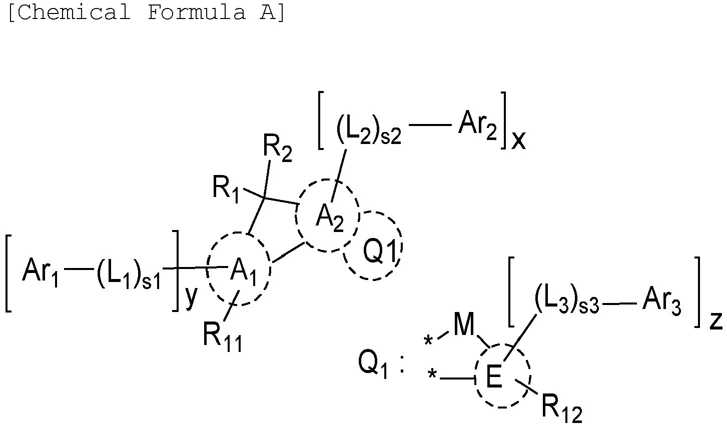 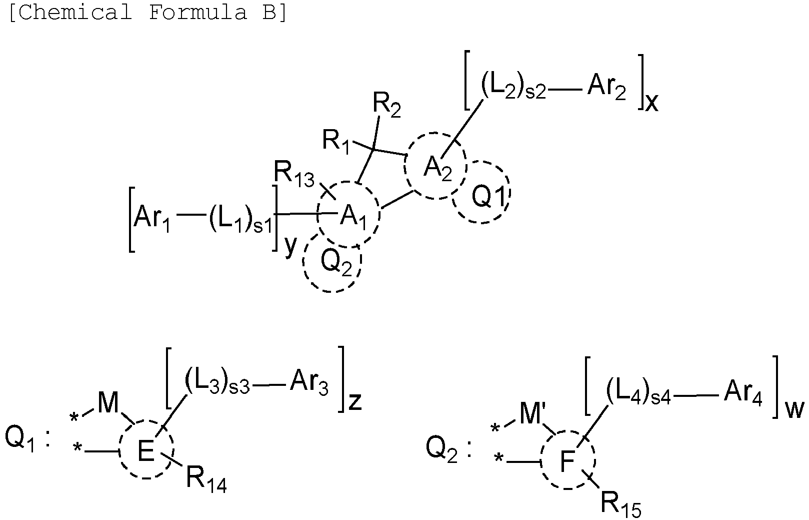
wherein, A₁, A₂, E, and F, which may be same or different, are each independently a substituted or unsubstituted aromatic hydrocarbon ring of 6 to 50 carbon atoms;
wherein two adjacent carbon atoms of the aromatic ring A₁ and two adjacent carbon atoms of the aromatic ring A₂ form a 5-membered fused ring together with the carbon atom having substituents R₁ and R₂ linked thereto;
linkers L₁ to L₄, which may be same or different, are each independently a single bond or a substituted or unsubstituted arylene of 6 to 20 carbon atoms;
M is one selected from N-R₃, CR₄R₅, O, and S,
M' is one selected from N-R₆, CR₇R₈, O, and S;
R1 to R8 and R11 to R15, which may be same or different, are each indepdently selected from a hydrogen atom, a deuterium atom, a substituted or unsubstituted alkyl of 1 to 30 carbon atoms, a substituted or unsubstituted aryl of 6 to 50 carbon atoms, a substituted or unsubstituted cycloalkyl of 3 to 30 carbon atoms, a substituted or unsubstituted heterocycloalkyl of 2 to 30 carbon atoms, a substituted or unsubstituted heteroaryl of 2 to 50 carbon atoms, a substituted or unsubstituted alkylsilyl of 1 to 30 carbon atoms, a substituted or unsubstituted arylsilyl of 6 to 30 carbon atoms, a cyano, a nitro, and a halogen,
with the proviso that R₁ and R₂ may be bonded to each other to form a mono- or polycyclic aliphatic or aromatic ring;
s1 to s4, which may be same or different, are each independently an integer of 1 to 3, with the proviso that when any of them is 2 or greater, the corresponding linkers L₁ to L₄ may be same or different,
x, y, z, and w, which may be same or different, are each independently an integer of 0 or 1, satisfying the following relationship:
x + y + z = 1 or x + y + z = 2 in Chemical Formula A,
x + y + z + w = 1 or x + y + z + w = 2 in Chemical Formula B,
two adjacent carbon atoms of the A₂ ring moiety of Chemical Formula A occupy respective positions * of Structural Formula Q₁ to form a fused ring,
two adjacent carbon atoms of the A₁ ring moiety of Chemical Formula B occupy respective positions * of structural Formula Q₂ to form a fused ring, and two adjacent carbon atoms of the A₂ ring moiety of Chemical Formula B occupy respective positions * of Structural Formula Q₁ to form a fused ring,
Ar₂ to Ar₄, which may be same or different, are each independently represented by the following Structural Formula C; 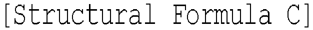 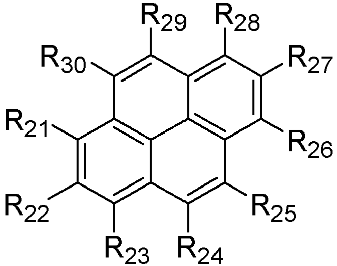
wherein R₂₁ to R₃₀, which may be same or different, are each independently any one selected from a hydrogen atom, a deuterium atom, a substituted or unsubstituted alkyl of 1 to 30 carbon atoms, a substituted or unsubstituted aryl of 6 to 50 carbon atoms, a substituted or unsubstituted cycloalkyl of 3 to 30 carbon atoms, a substituted or unsubstituted heteroaryl of 2 to 50 carbon atoms, a substituted or unsubstituted heterocycloalkyl of 2 to 30 carbon atoms, a substituted or unsubstituted alkylsilyl of 1 to 30 carbon atoms, a substituted or unsubstituted arylsilyl of 5 to 30 carbon atoms, a cyano, a nitro, and a halogen,
any one of R₂₁ to R₃₀ being a single bond to any one of the linkers L₁ to L₄;
wherein, the term "substituted" in the expression "a substituted or unsubstituted" used for the compound of Chemical Formulas A and B and Structural Formula C means having at least one substituent selected from the group consisting of a deuterium atom, a cyano, a halogen, a hydroxy, a nitro, an alkyl of 1 to 24 carbon atoms, a halogenated alkyl of 1 to 24 carbon atoms, an alkenyl of 1 to 24 carbon atoms, an alkynyl of 1 to 24 carbon atoms, a cycloalkyl of 3 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 2 to 24 carbon atoms, an alkoxy of 1 to 24 carbon atoms, an alkylamino of 1 to 24 carbon atoms, a diarylamino of 12 to 24 carbon atoms, a diheteroarylamino of 2 to 24 carbon atoms, an aryl(heteroaryl)amino of 7 to 24 carbon atoms, an alkylsilyl of 1 to 24 carbon atoms, an arylsilyl of 6 to 24 carbon atoms, an aryloxy of 6 to 24 carbon atoms, and an arylthionyl of 6 to 24 carbon atoms.

2. The compound of claim 1, wherein A₂, A₂, E, and F in Chemical Formula A or B are same or different and are each independently substituted or unsubstituted aromatic hydrocarbon rings of 6 to 18 carbon atoms.

3. The compound of claim 2, wherein the substituted or unsubstituted aromatic hydrocarbon rings of 6 to 18 carbon atoms are same or different and are each independently selected from among [Structural Formula 10] to [Structural Formula 21]: 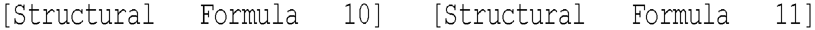 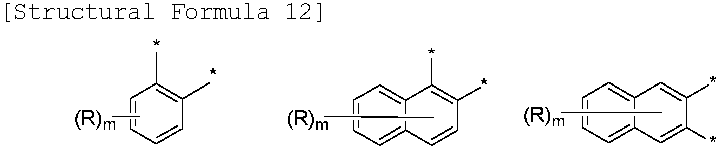 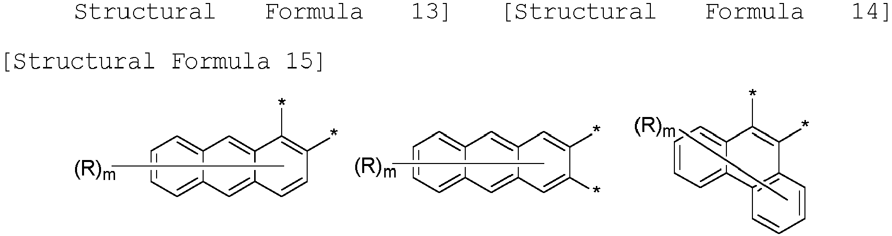 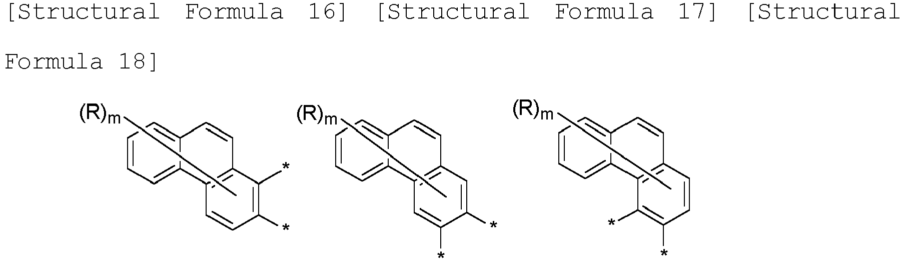 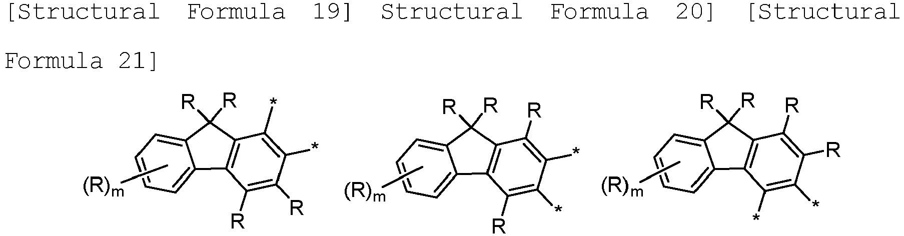
wherein, "-*" denotes a bonding site for forming a 5-membered ring bearing the carbon atom connected to the substituents R₁ and R₂ or a bonding site for forming a 5-membered ring bearing M of Structural Formula Q₁ and Q₂;
when one of the aromatic hydrocarbon rings of [Structural Formula 10] to [Structural Formula 21] for A₁ or A₂ is bonded to Structural Formula Q₁ or Structural Formula Q₂, two adjacent carbon atoms of the aromatic hydrocarbon ring occupy respective positions * of Structural Formula Q₁ or Q₂ to form a fused ring;
R is as defined for R₁ and R₂ in claim 1, and
m is an integer of 1 to 8, with a proviso that when m is 2 or greater or two or more R's exist, the corresponding R's are same or different.

4. The compound of claim 1, wherein the linkers L₁ to L₄ in Chemical Formula A or B may be a single bond or one selected from among compounds represented by the following Structural Formulas 1 to 5, and
s1 to s4 are each 1 or 2: 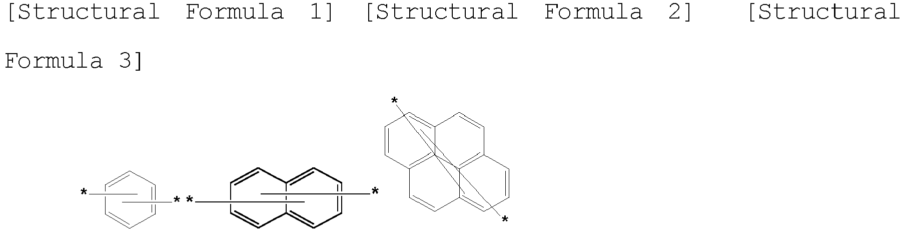 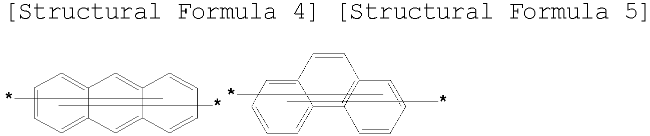 wherein each of the unsubstituted carbon atoms of the aromatic ring moiety in the linkers can be bound with a hydrogen atom or a deuterium atom.

5. The compound of claim 1, wherein,
x is 1 and y and z are each 0;
y is 1 and x and z are each 0; or
z is 1 and x and y are each 0 in Chemical Formula A, and
x is 1 and y, z, and w are each 0; or
x and y are each 1 and z and w are each 0 in Chemical Formula B.

6. The compound of claim 1, wherein the substituents R₁ and R₂ in Chemical Formula A or B, which are same or different, are each independently a substituted or unsubstituted alkyl of 1 to 10 carbon atoms and may be connected to each other to form a ring.

7. The compound of claim 1, wherein any one of R₂₁ to R₂₃ in Structural Formula C is a single bond to any one of the linkers L₁ to L₄ and R₁₁ to R₁₅, which are same or different, are each independently a substituent a hydrogen atom, a deuterium atom, a substituted or unsubstituted alkyl of 1 to 10 carbon atoms, a substituted or unsubstituted aryl of 6 to 18 carbon atoms, a substituted or unsubstituted alkylsilyl of 1 to 15 carbon atoms, a substituted or unsubstituted arylsilyl of 6 to 20 carbon atoms, a cyano, and a halogen.

8. The compound of claim 1, wherein R₂₁ to R₃₀ in Structural Formula C, which are same or different, are each independently any one selected from among a hydrogen atom, a deuterium atom, a substituted or unsubstituted alkyl of 1 to 6 carbon atoms, a substituted or unsubstituted aryl of 6 to 12 carbon atoms, and a substituted or unsubstituted heteroaryl of 2 to 12 carbon atoms, wherein at least one of R₂₁ to R₃₀ of Structural Formula C which are not connected to any of L₁ to L₄ is any one selected from among a substituted or unsubstituted alkyl of 1 to 6 carbon atoms, a substituted or unsubstituted aryl of 6 to 12 carbon atoms, and a substituted or unsubstituted heteroaryl of 2 to 12 carbon atoms.

9. The compound of claim 1, wherein the compound is any one selected from among [H 1] to [H 180]:

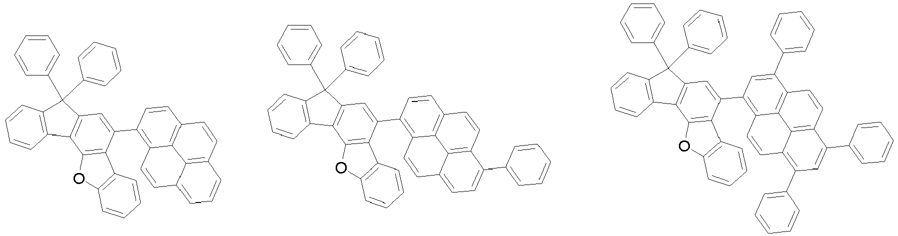

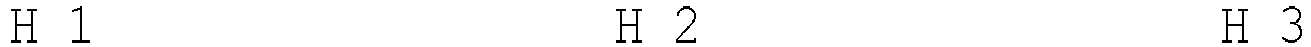

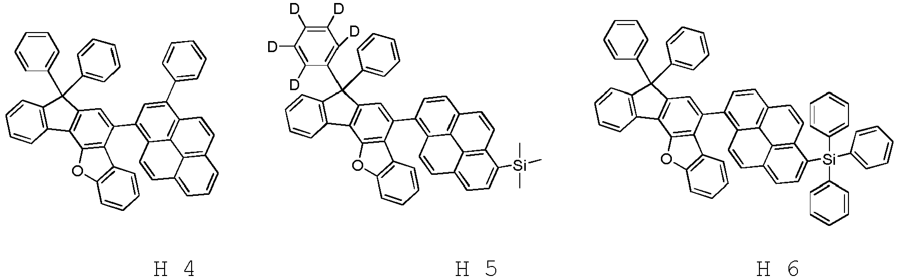

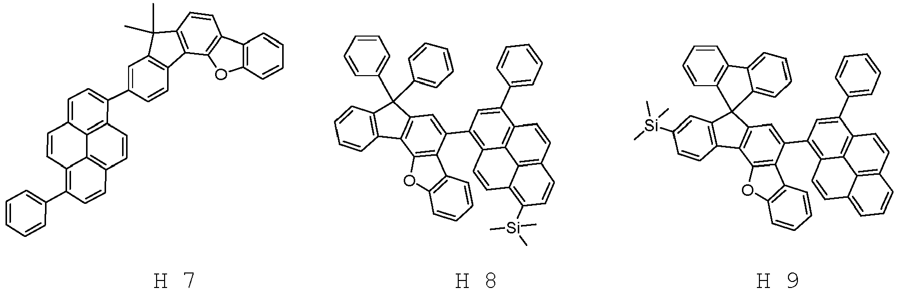

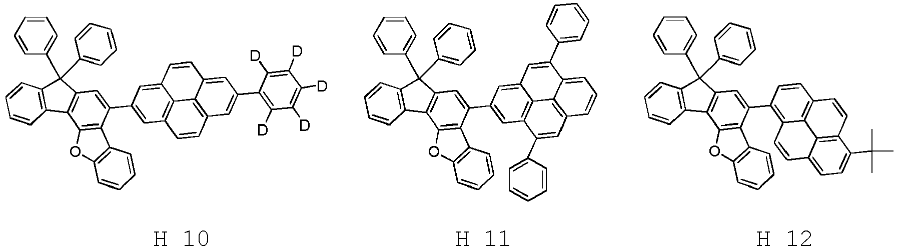

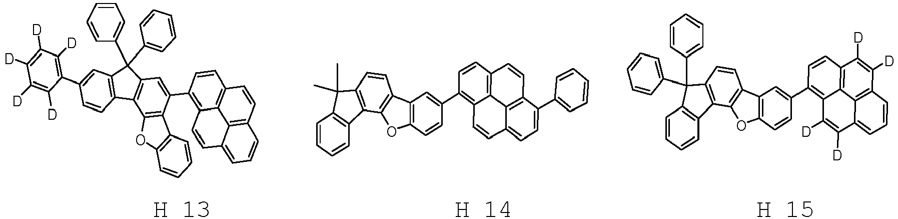

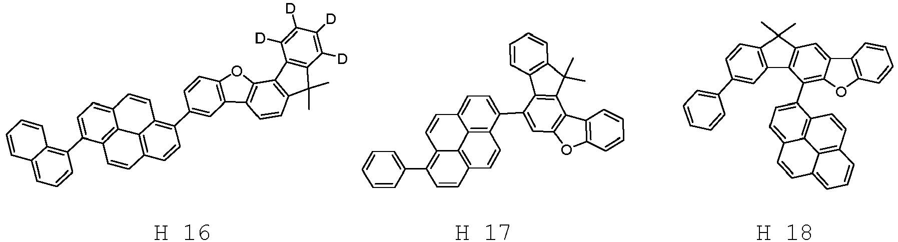

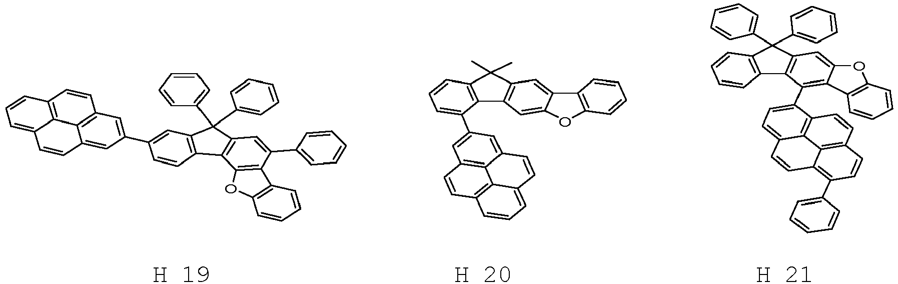

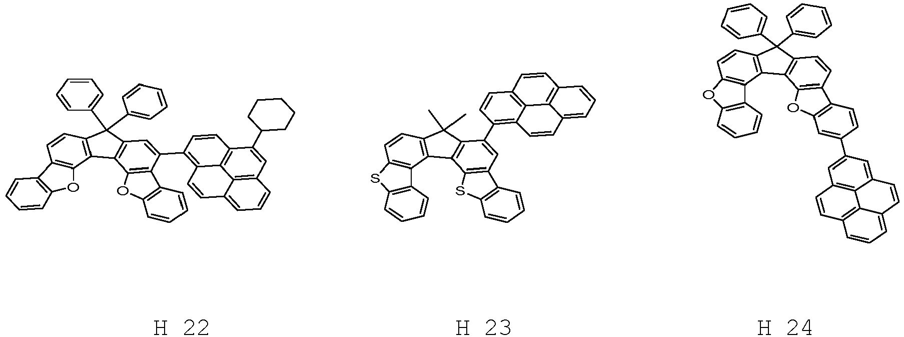

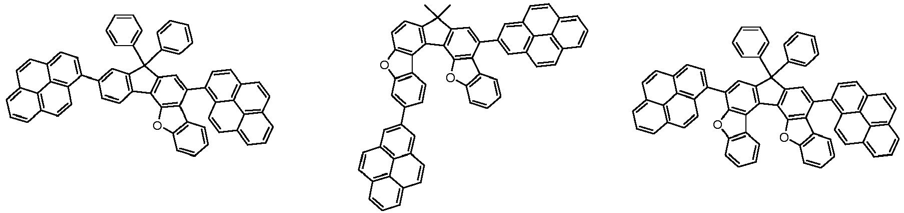

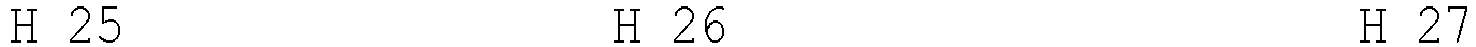

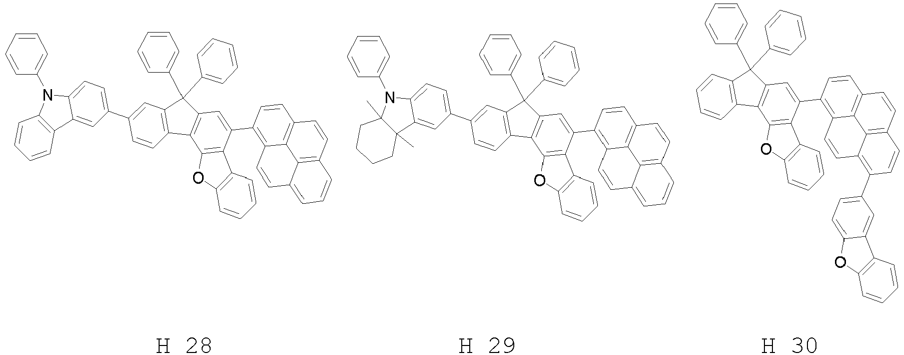

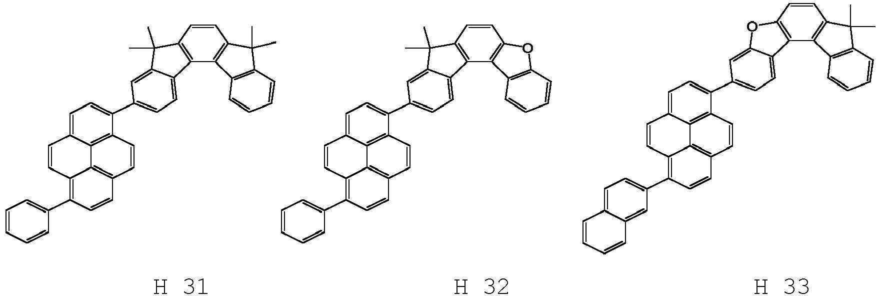

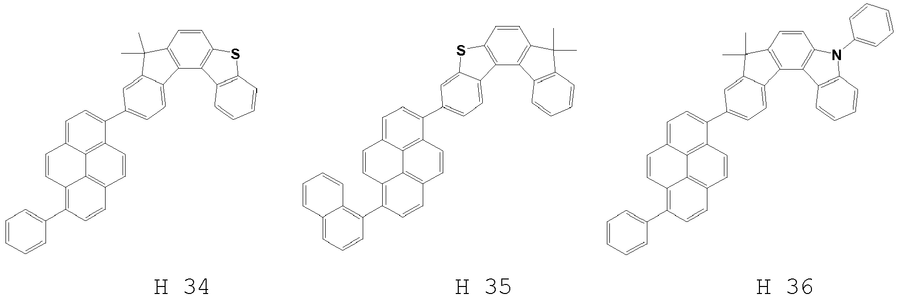

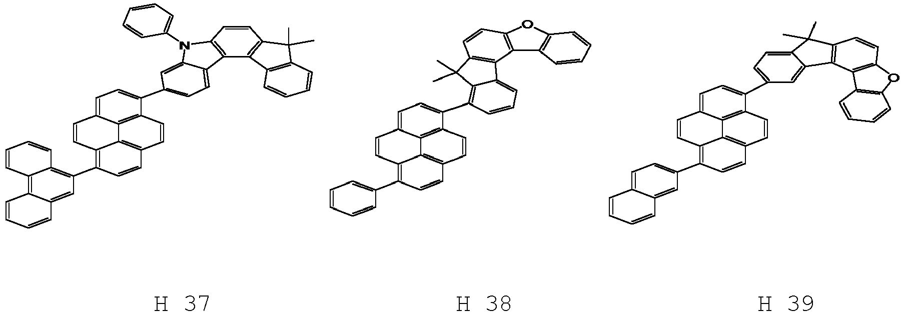

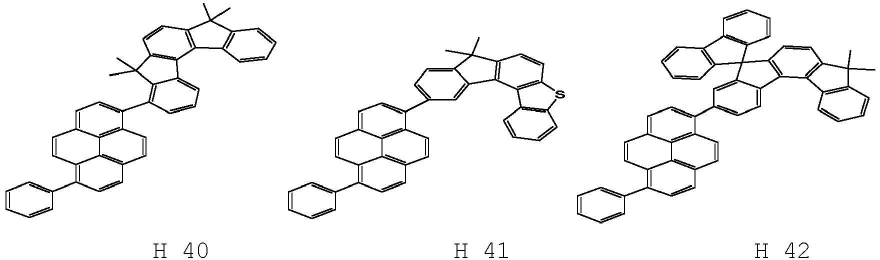

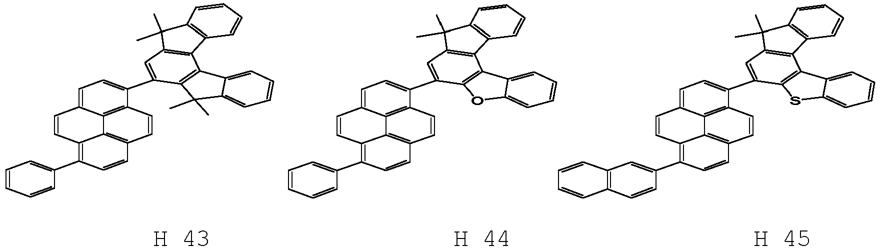

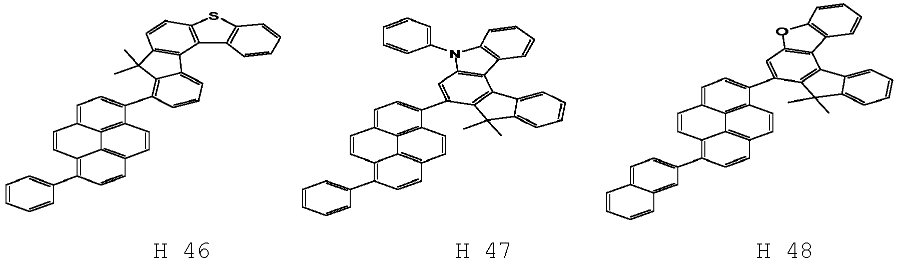

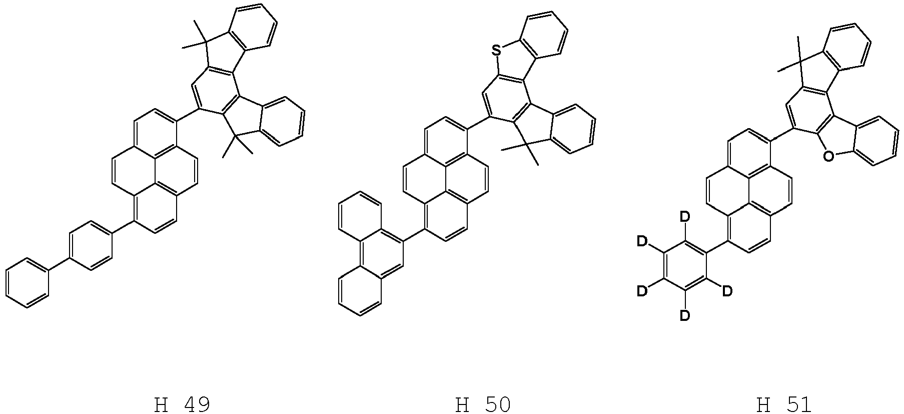

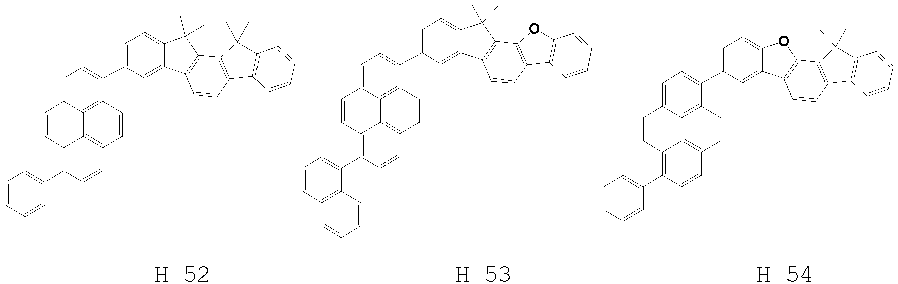

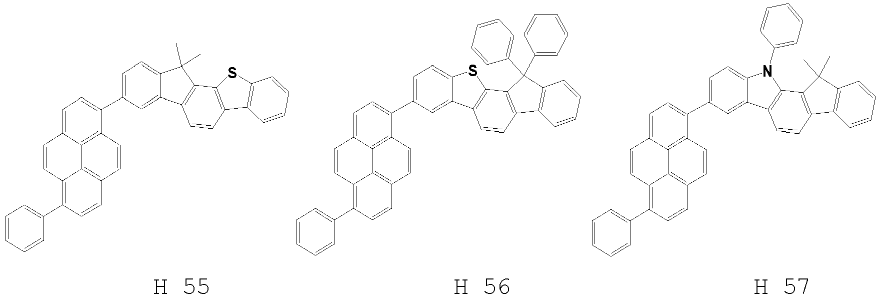

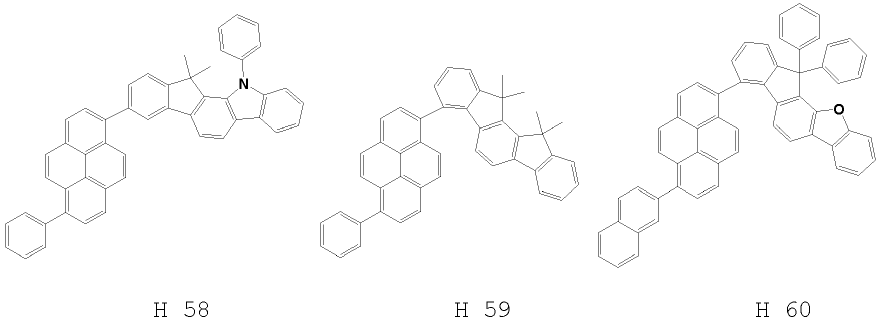

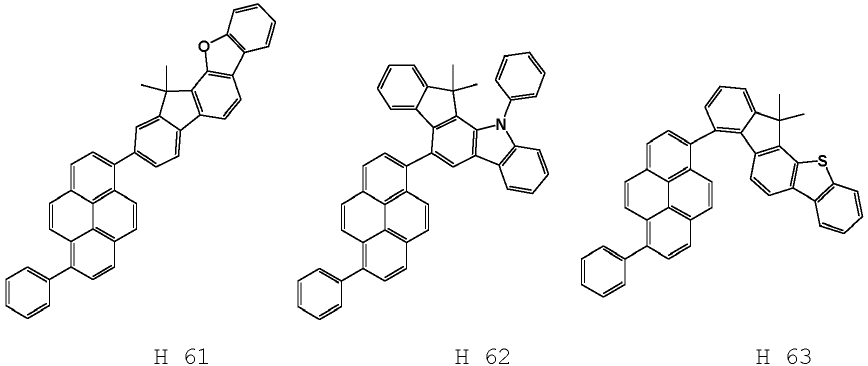

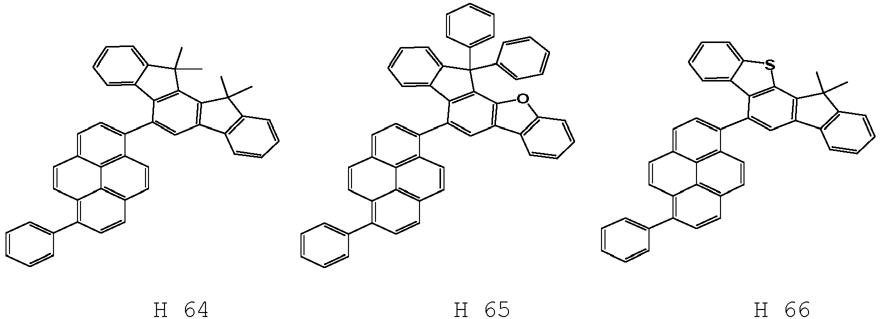

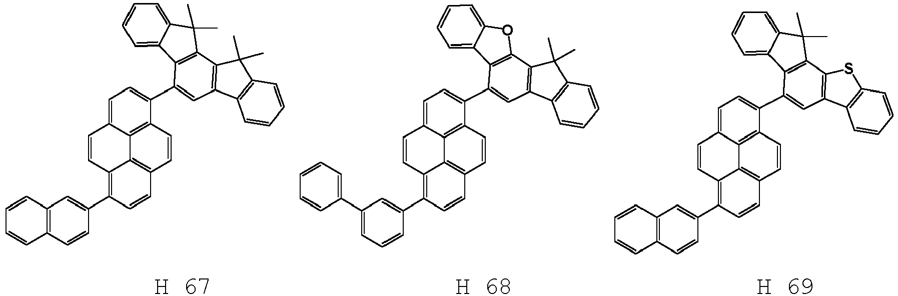

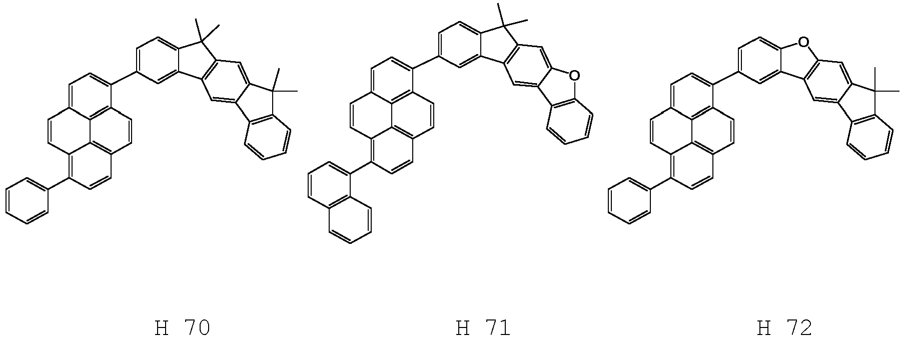

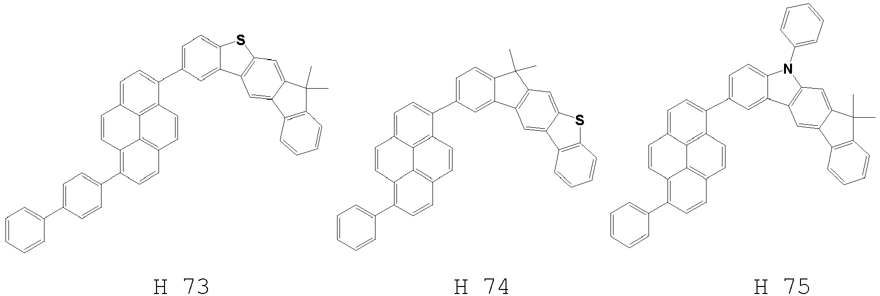

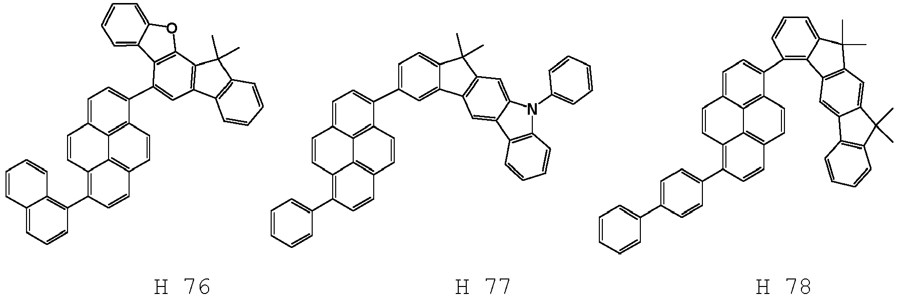

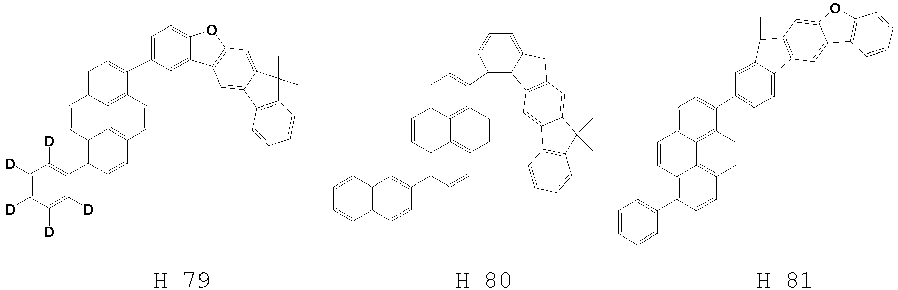

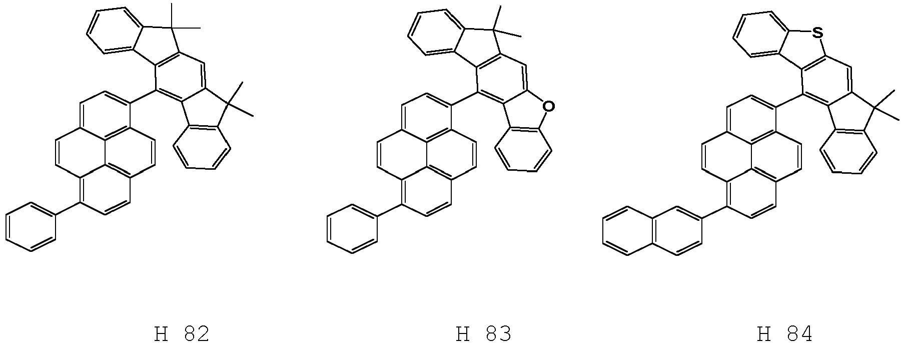

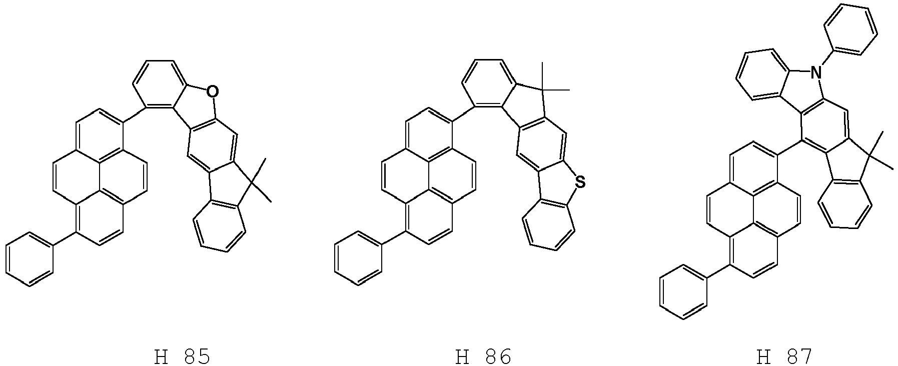

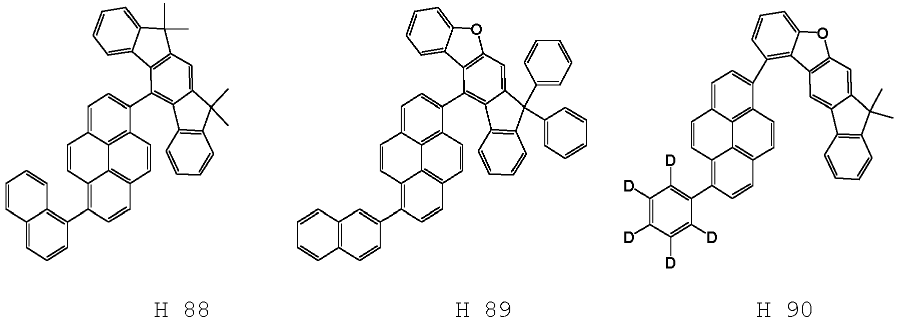

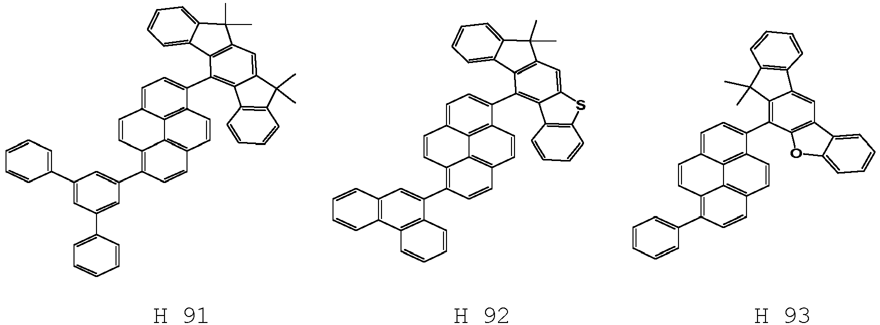

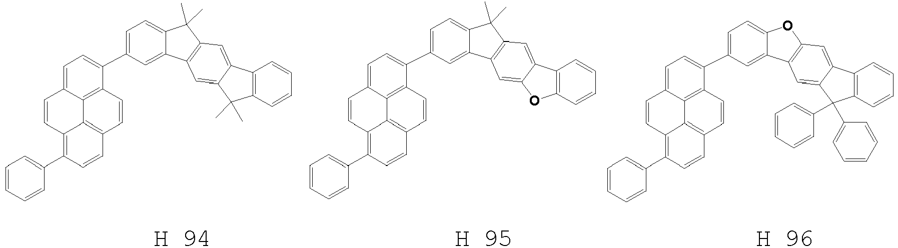

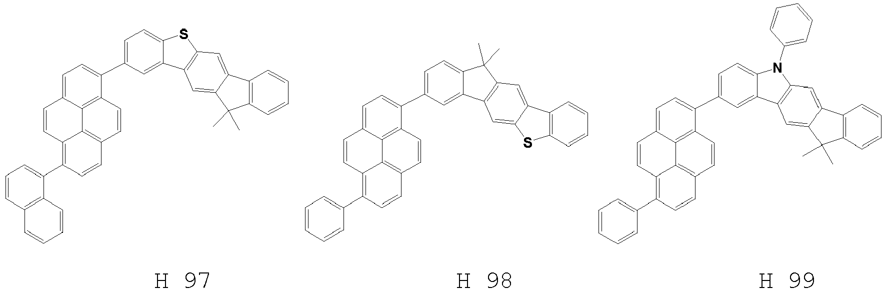

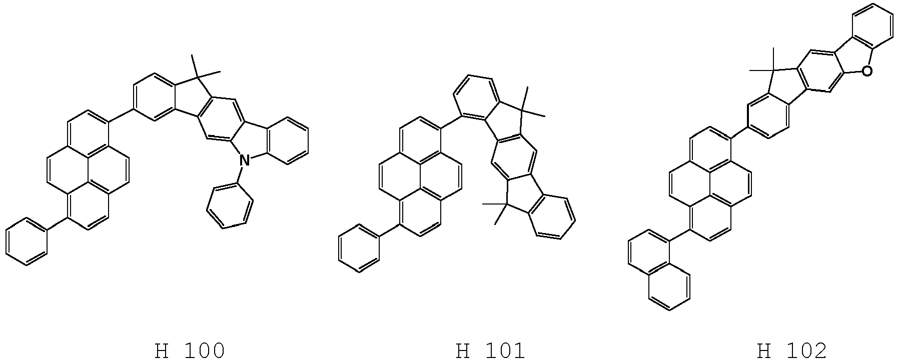

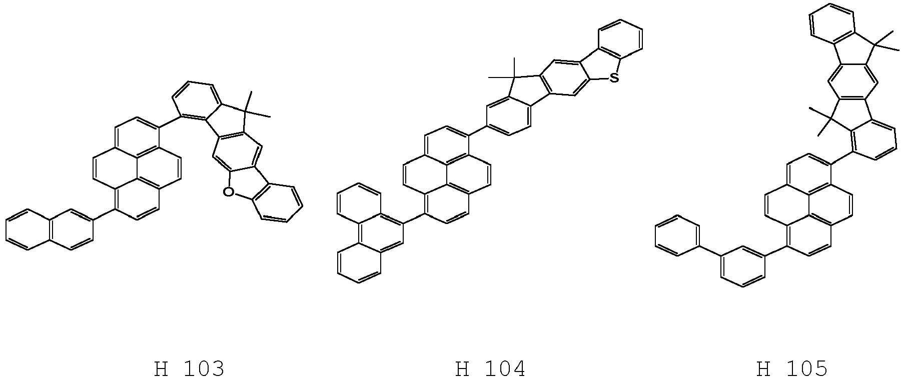

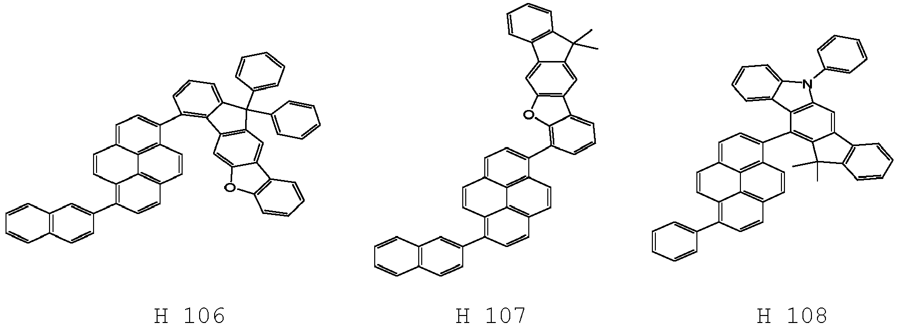

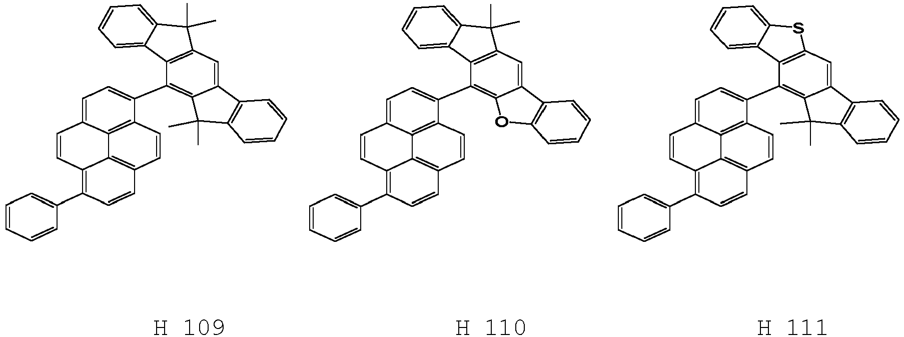

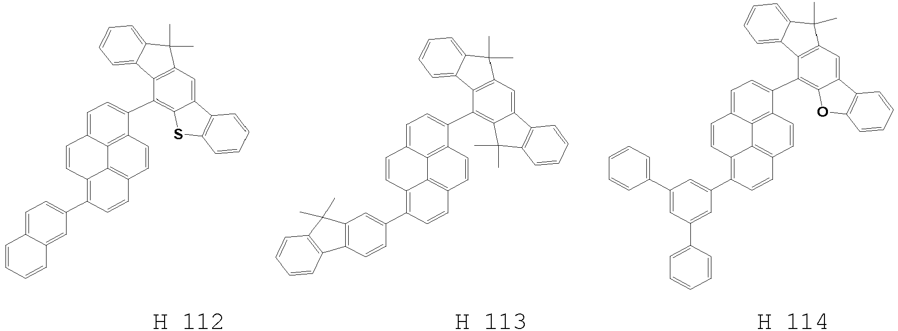

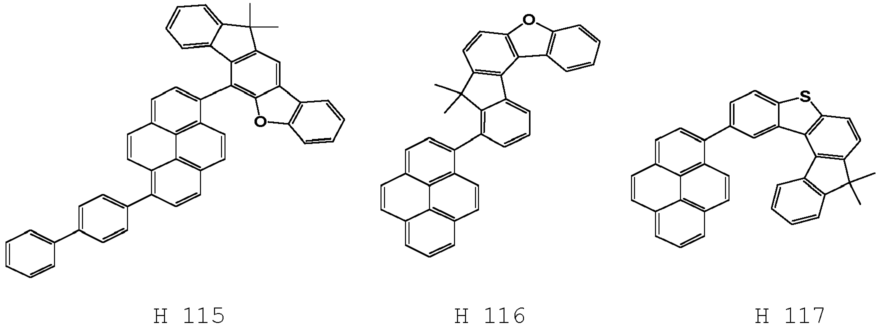

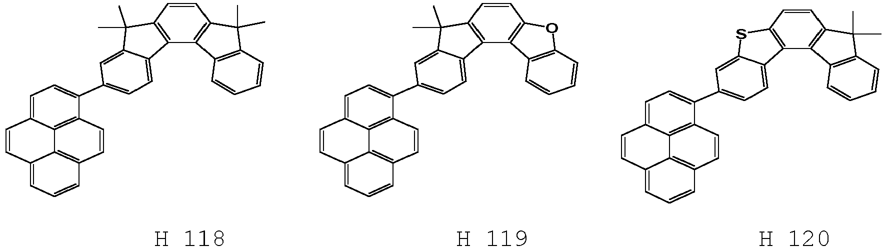

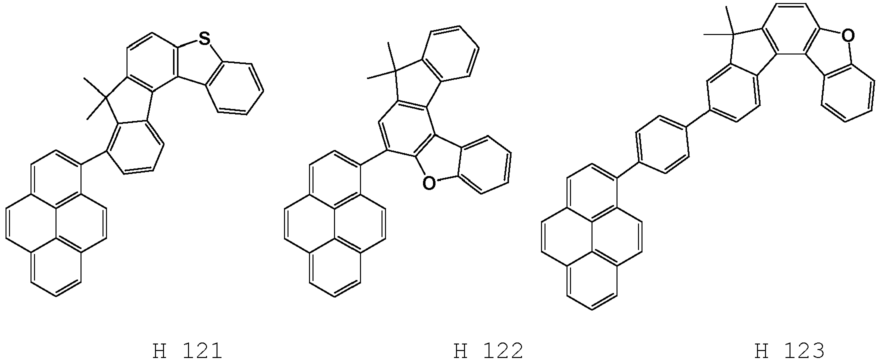

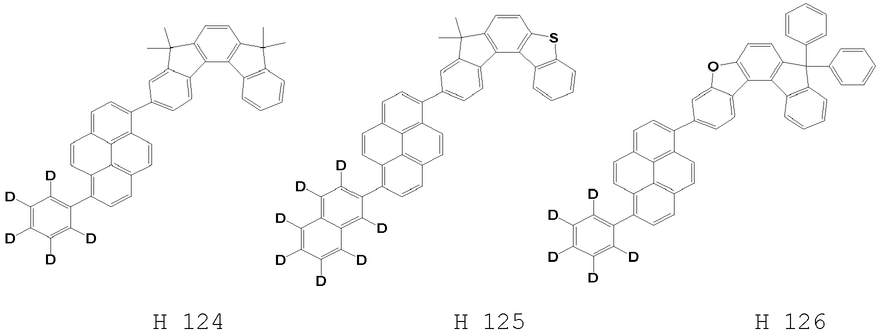

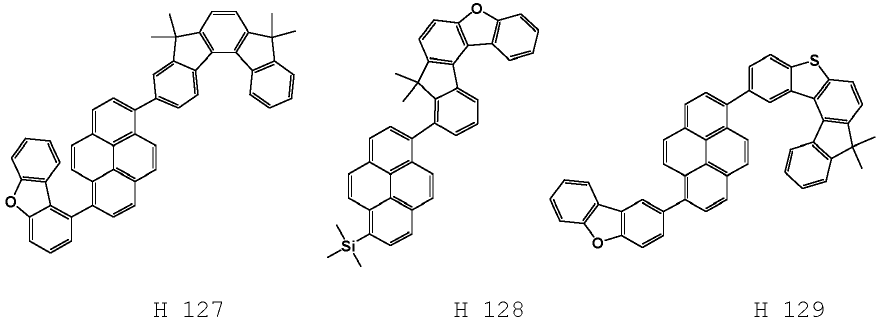

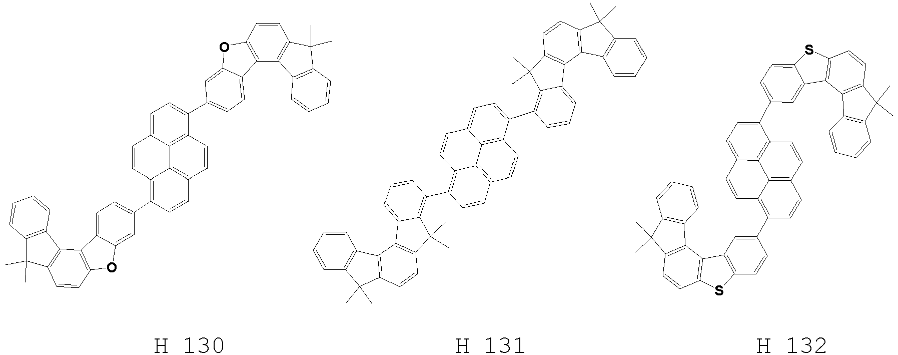

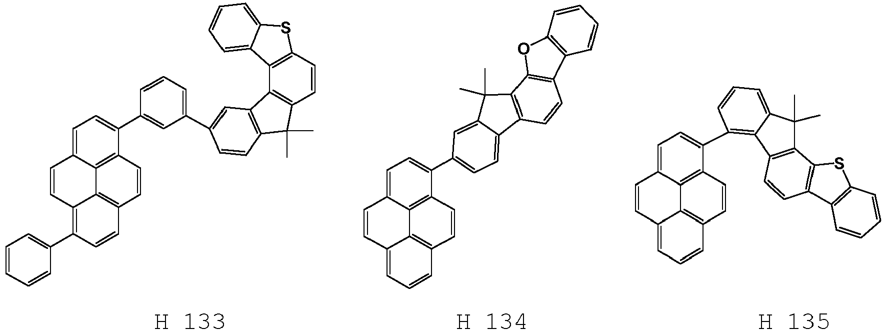

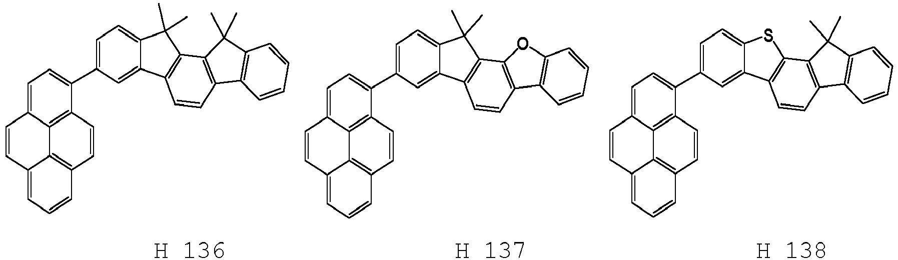

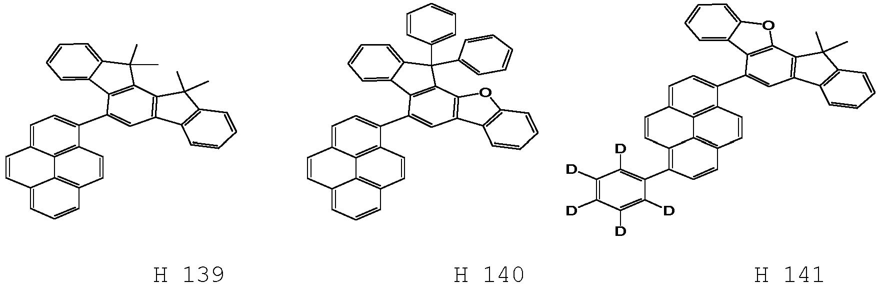

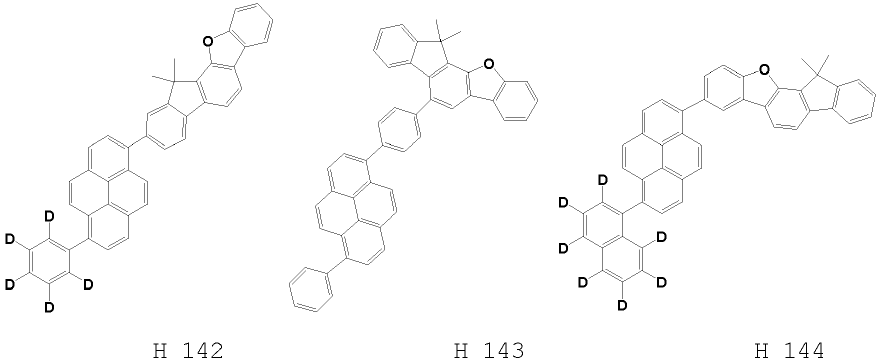

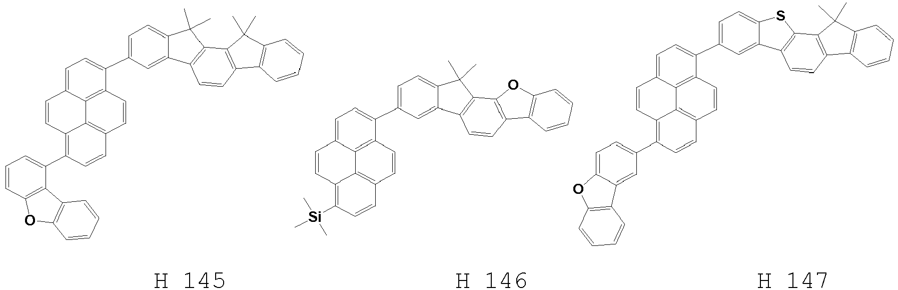

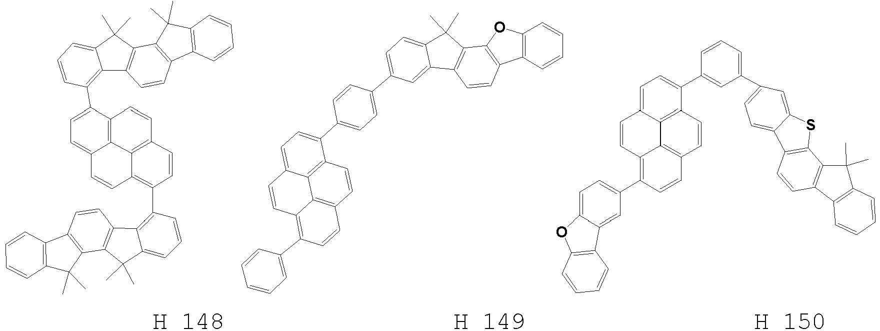

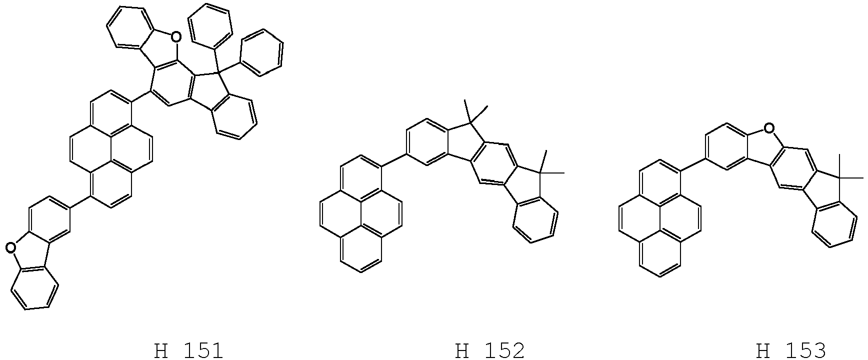

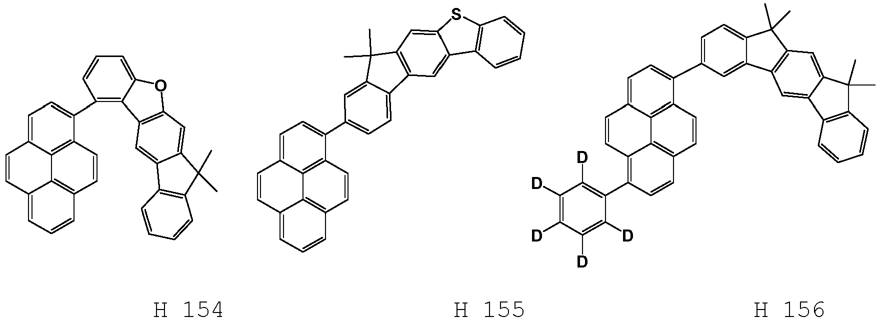

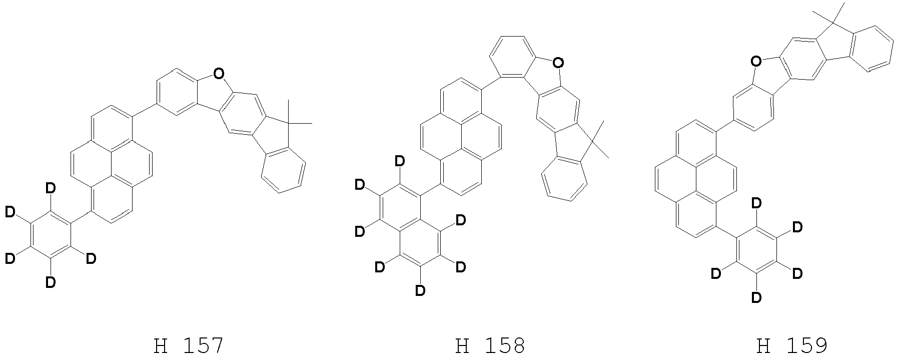

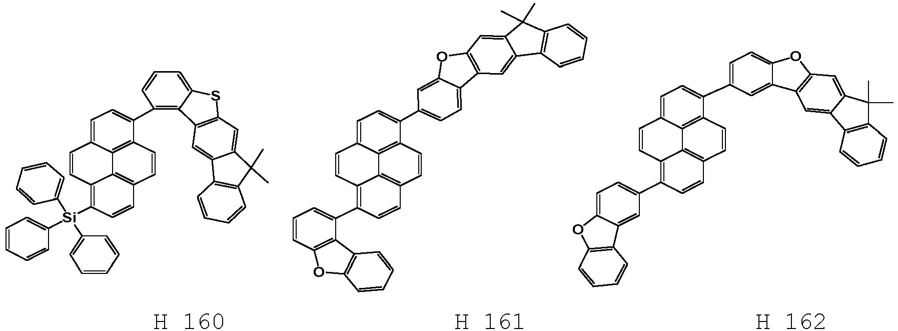

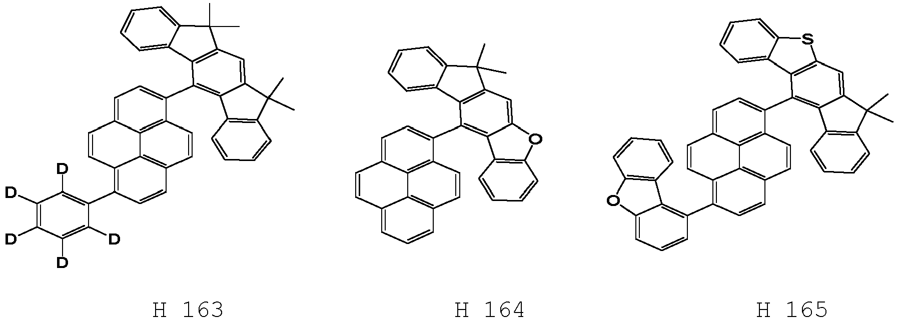

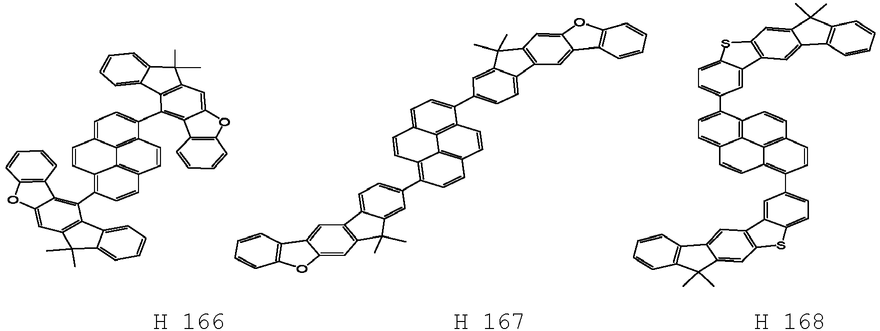

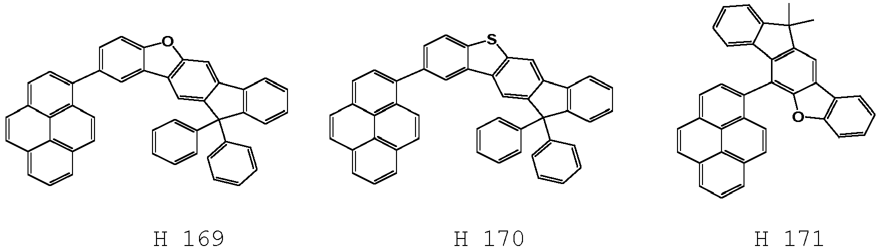

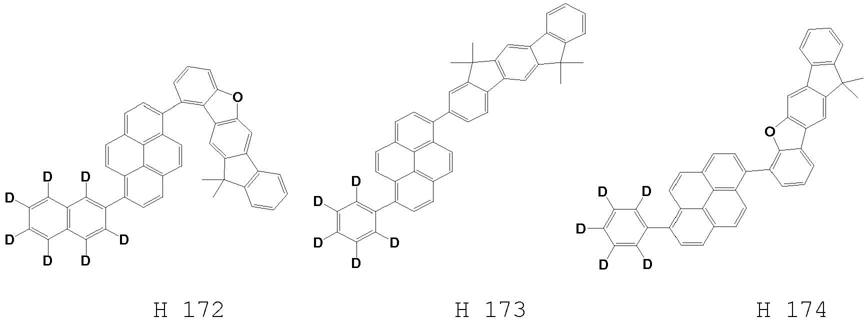

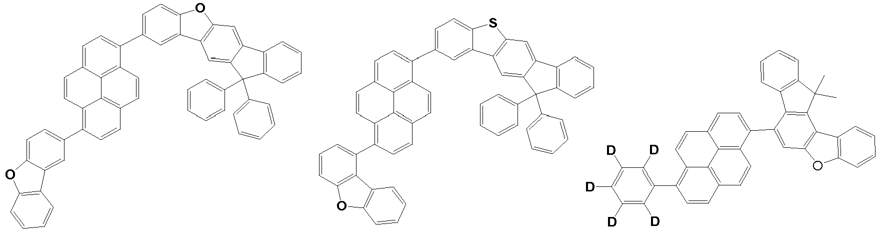

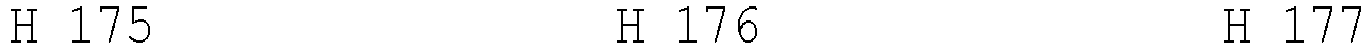

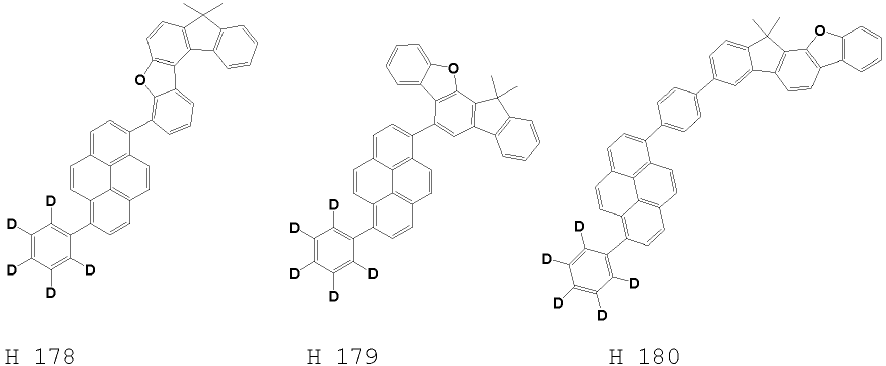

10. An organic light-emitting diode comprising:
a first electrode;
a second electrode facing the second electrode; and
an organic layer interposed between the first electrode and the second electrode,
wherein the organic layer comprises at least one of the compounds of any one of claims 1 to 9.

11. The organic light-emitting diode of claim 10, wherein the organic layer comprises at least one of a hole injection layer, a hole transport layer, a functional layer capable of both hole injection and hole transport, a light emitting layer, an electron transport layer, and an electron injection layer.

12. The organic light-emitting diode of claim 11, wherein the organic layer disposed between the first electrode and the second electrode comprises a light-emitting layer composed of a host and a dopant, wherein the compound represented by Chemical Formula A or B serves as the host.

13. The organic light-emitting diode of claim 12, wherein the dopant compound is at least one of the compounds represented by the following Chemical Formula D1 to Chemical Formula D10: 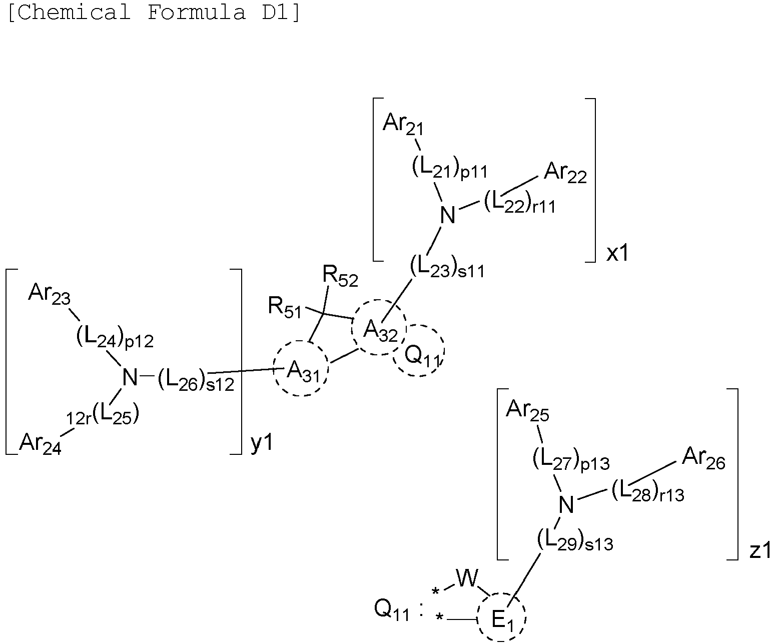 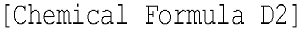 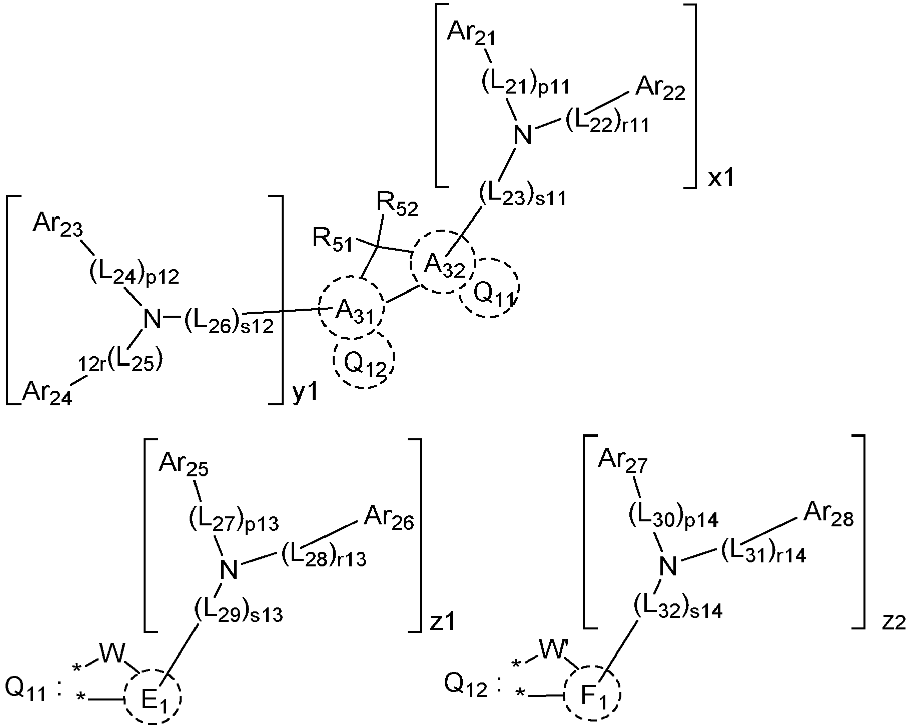 wherein,
A₃₁, A₃₂, E₁ and F₂, which may be same or different, are each independently a substituted or unsubstituted aromatic hydrocarbon ring of 6 to 50 carbon atoms, or a substituted or unsubstituted aromatic heteroring of 2 to 40 carbon atoms,
wherein two adjacent carbon atoms of the aromatic ring A₃₁ and two adjacent carbon atoms of the aromatic ring A₃₂ form a 5-membered fused ring together with a carbon atom to which substituents R₅₁ and R₅₂ are bonded;
linkers L₂₁ to L₃₂, which may be same or different, are each independently selected from a single bond, a substituted or unsubstituted alkylene of 1 to 60 carbon atoms, a substituted or unsubstituted alkenylene of 2 to 60 carbon atoms, a substituted or unsubstituted alkynylene of 2 to 60 carbon atoms, a substituted or unsubstituted cycloalkylene of 3 to 60 carbon atoms, a substituted or unsubstituted heterocycloalkylene of 2 to 60 carbon atoms, a substituted or unsubstituted arylene of 6 to 60 carbon atoms, and a substituted or unsubstituted heteroarylene of 2 to 60 carbon atoms;
W and W', which may be same or different, are each independently any one selected from among N-R₅₃, CR₅₄R₅₅, SiR₅₆R₅₇, GeR₅₈R₅₉, O, S, and Se;
R₅₁ to R₅₉, and Ar₂₁ to Ar₂₈, which may be the same or different, are each independently any one selected from among a hydrogen atom, a deuterium atom, a substituted or unsubstituted alkyl of 1 to 30 carbon atoms, a substituted or unsubstituted aryl of 6 to 50 carbon atoms, a substituted or unsubstituted alkenyl of 2 to 30 carbon atoms, a substituted or unsubstituted alkynyl of 2 to 30 carbon atoms, a substituted or unsubstituted cycloalkyl of 3 to 30 carbon atoms, a substituted or unsubstituted cycloalkenyl of 5 to 30 carbon atoms, a substituted or unsubstituted heteroaryl of 2 to 50 carbon atoms, a substituted or unsubstituted heterocycloalkyl of 2 to 30 carbon atoms, a substituted or unsubstituted alkoxy of 1 to 30 carbon atoms, a substituted or unsubstituted aryloxy of 6 to 30 carbon atoms, a substituted or unsubstituted alkylthioxy of 1 to 30 carbon atoms, a substituted or unsubstituted arylthioxy of 5 to 30 carbon atoms, a substituted or unsubstituted alkylamine of 1 to 30 carbon atoms, a substituted or unsubstituted arylamine of 5 to 30 carbon atoms, a substituted or unsubstituted alkylsilyl of 1 to 30 carbon atoms, a substituted or unsubstituted arylsilyl of 5 to 30 carbon atoms, a substituted or unsubstituted alkyl geraminum of 1 to 30 carbon atoms, a substituted or unsubstituted aryl geraminum of 1 to 30 carbon atoms a cyano, a nitro, and a halogen,
wherein R₅₁ and R₅₂ together may form a mono- or polycyclic aliphatic or aromatic ring that may be a heterocyclic ring bearing a heteroatom selected from N, O, P, Si, S, Ge, Se, and Te as a ring member;
p11 to p14, r11 to r14, and s11 to s14 are each independently an integer of 1 to 3, wherein when any of them is 2 or greater, the corresponding L₂₁ to L₃₂ may be same or different,
x1 is an integer of 1 or 2, and y1 and z1, which may be same or different, are each independently an integer of 0 to 3;
Ar₂₁ may form a ring with Ar₂₂, Ar₂₃ may form a ring with Ar₂₄, Ar₂₅ may form a ring with Ar₂₆, and Ar₂₇ may form a ring with Ar₂₈;
two adjacent carbon atoms of the A₃₂ ring moiety of Chemical Formula D1 may occupy respective positions * of Structural Formula Q₁₁ to form a fused ring; and
two adjacent carbon atoms of the A₃₁ ring moiety of Chemical Formula D2 may occupy respective positions * of structural Formula Q₁₂ to form a fused ring, and two adjacent carbon atoms of the A₃₂ ring moiety of Chemical Formula D2 may occupy respective positions * of Structural Formula Q₁₁ to form a fuse ring; 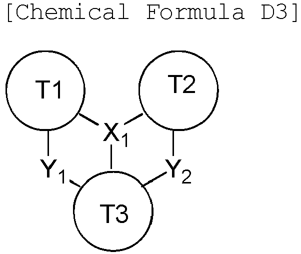
wherein,
X₂ is any one selected from among B, P, and P=O,
T₁ to T₃, which are same or different, are each independently a substituted or unsubstituted aromatic hydrocarbon ring of 6 to 50 carbon atoms, or a substituted or unsubstituted heteroaromatic ring of 2 to 40 carbon atoms;
Y₁ is any one selected from among N-R₆₁, CR₆₂R₆₃, O, S, and SiR₆₄R₆₅;
Y₂ is any one selected from among N-R₆₆, CR₆₆R₆₈, O, S, SiR₆₉R₇₀,
wherein R₆₁ to R₇₀, which may be same or different, are each independently any one selected from among a hydrogen atom, a deuterium atom, a substituted or unsubstituted alkyl of 1 to 30 carbon atoms, a substituted or unsubstituted aryl of 6 to 50 carbon atoms, a substituted or unsubstituted cycloalkyl of 3 to 30 carbon atoms, a substituted or unsubstituted heteroaryl of 2 to 50 carbon atoms, a substituted or unsubstituted alkoxy of 1 to 30 carbon atoms, a substituted or unsubstituted aryloxy of 6 to 30 carbon atoms, a substituted or unsubstituted alkylthioxy of 1 to 30 carbon atoms, a substituted or unsubstituted arylthioxy of 5 to 30 carbon atoms, a substituted or unsubstituted alkylamine of 1 to 30 carbon atoms, a substituted or unsubstituted arylamine of 5 to 30 carbon atoms, a substituted or unsubstituted alkylsilyl of 1 to 30 carbon atoms, a substituted or unsubstituted arylsilyl of 5 to 30 carbon atoms, a cyano, and a halogen, and wherein at least one of R₆₁ to R₃₀ may be connected to at least one of T₁ to T₃ to form an additional mono- or polycyclic aliphatic or aromatic ring; 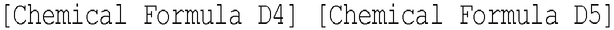 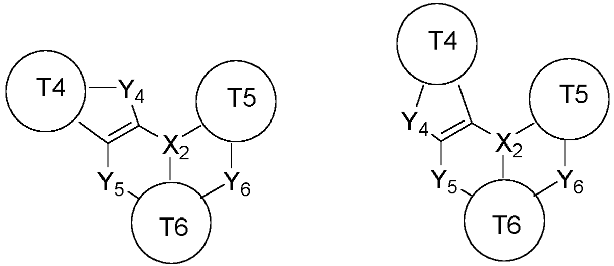
wherein,
X₂ is any one selected from among B, P, and P=O,
T₄ to T₆ are as defined for T₁ to T₃ in Chemical Formula D3,
Y₄ is any one selected from among N-R₆₁, CR₆₂R₆₃, O, S, and SiR₆₄R₆₅;
Y₅ is any one selected from among N-R₆₆, CR₆₆R₆₈, O, S, and SiR₆₉R₇₀, and
Y₆ is any one selected from among N-R₇₁, CR₇₃R₇₃, O, S, and SiR₇₄R₇₅,
R₆₁ to R₇₅ being as defined for R₆₁ to R₇₀ in [Chemical Formula D3]; 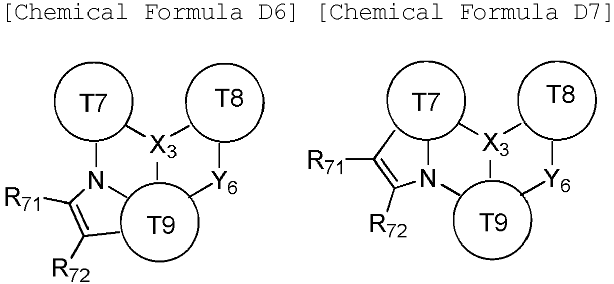
wherein, X₃ is any one selected from among B, P, and P=O, T₇ to T₉ are as defined for T₁ to T₃ in Chemical Formula D3,
Y₆ is any one seleced from among N-R₆₁, CR₆₂R₆₃, O, S, and SiR₆₄R₆₅,
R₆₁ to R₆₅, R₇₁ and R₇₂ are as defined for R₆₁ to R₇₀ in [Chemical Formula D3], respectively,
wherein R₇₁ and R₇₂ may be connected to each other to form an additional mono- or polycyclic aliphatic or aromatic ring or connected to the T7 or T9 ring moiety to form an additional mono- or polycyclic aliphatic or aromatic ring; and 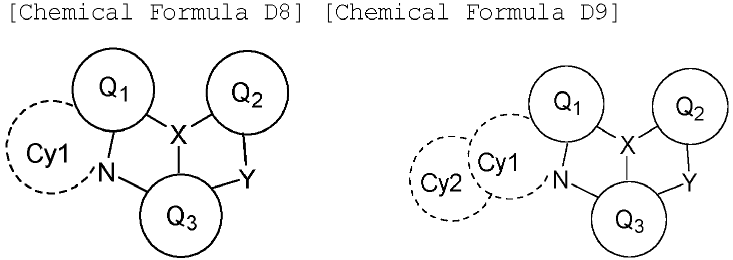 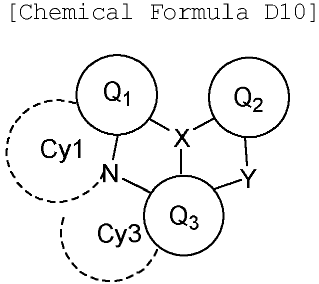
wherein,
X is any one selected from among B, P, and P=O,
Q₁ to Q₃ are as defined for T1 to T3 in Chemical Formula D3,
the linker Y is anly one selected from among N-R₃, CR₄R₅, O, S, and Se,
wherein R₃ to R₅ are as defined for R₆₁ to R₇₀ in Chemical Formula D3, respectively,
R₃ to R₅ may each be connected to the Q₂ or Q₃ ring moiety to form an additional mono- or polycyclic aliphatic or aromati ring,
R₄ and R₅ may be connected to each other to form an additional mono- or polycyclic aliphatic or aromatic ring,
the ring formed by Cy1 is a substituted or unsubstituted alkylene of 1 to 10 carbon atoms, except for the nitrogen (N) atom, the aromatic carbon atom of Q₁ to which the nitrogen (N) atom is connected, and the aromatic carbon atom of Q₁ to which Cy1 is to bond,
Cy2 in Chemical Formula D9 forms a saturated hydrocarbon ring added to Cy1 wherein the ring formed by Cy2 is a substituted or unsubstituted alkylene of 1 to 10 carbon atoms, except for the carbon atoms included in Cy1, and
the ring formed by Cy3 in Chemical Formula D10 is a substituted or unsubstituted alkylene of 1 to 10 carbon atoms, except for the aromatic carbon atom of Q₃ to which Cy3 is to bond, the aromatic carbon atom of Q₃ to which the nitrogen (N) atom is connected, the nitrogen (N) atom, and the carbon atom of Cy1 to which the nitrogen (N) atom is connected,
wherein the term "substituted" in the expression "substituted or unsubstituted" used for compounds of Chemical Formulas D1 to D10 means having at least one substituent selected from the group consisting of a deuterium atom, a cyano, a halogen, a hydroxy, a nitro, an alkyl of 1 to 24 carbon atoms, a halogenated alkyl of 1 to 24 carbon atoms, an alkenyl of 2 to 24 carbon atoms, an alkynyl of 2 to 24 carbon atoms, a heteroalkyl of 1 to 24 carbon atoms, an aryl of 6 to 24 carbon atoms, an arylalkyl of 7 to 24 carbon atoms, a heteroaryl of 2 to 24 carbon atoms or a heteroarylalkyl of 2 to 24 carbon atoms, an alkoxy of 1 to 24 carbon atoms, an alkylamino of 1 to 24 carbon atoms, an arylamino of 6 to 24 carbon atoms, a heteroarylamino of 1 to 24 carbon atoms, an alkylsilyl of 1 to 24 carbon atoms, an arylsilyl of 6 to 24 carbon atoms, and an aryloxy of 6 to 24 carbon atoms.

14. An organic light-emitting diode, comprising:
a first electrode;
a second electrode facing the first electrode; and
a first light-emitting layer containing a first host and a first dopant and a second light-emitting layer containing a second host and a second dopant sequentially between the first electrode and the second electrode,
wherein at least one of the first host and the second host comprises at least one selected from among the compounds of any one of claims 1 to 9.

15. The organic light-emitting diode of claim 14, wherein the organic light-emitting diode comprises at least one of a hole transport layer and a hole injection layer between the first electrode and the first light-emitting layer and at least one of an electron transport layer and an electron injection layer between the second light-emitting layer and the second electrode.

16. The organic light-emitting diode of claim 14, wherein the first light-emitting layer comprises at least one selected from the compounds of any one of claims 1 to 9.

17. The organic light-emitting diode of claim 16, wherein the second light-emitting layer comprises as a host an anthracene derivative represented by the following Chemical Formula E: 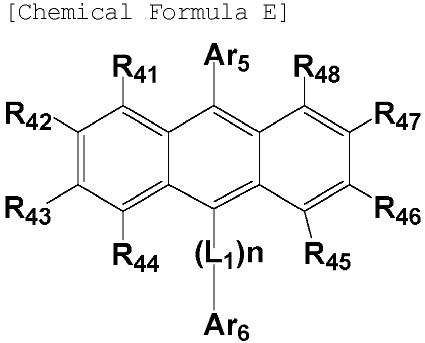 wherein,
R₄₁ to R₄₈, which may be same or different, are each independently any one selected from among a hydrogen atom, a deuterium atom, a substituted or unsubstituted alkyl of 1 to 30 carbon atoms, a substituted or unsubstituted aryl of 6 to 50 carbon atoms, a substituted or unsubstituted cycloalkyl of 3 to 30 carbon atoms, a substituted or unsubstituted heteroaryl of 2 to 50 carbon atoms, a substituted or unsubstituted alkylsilyl of 1 to 30 carbon atoms, a substituted or unsubstituted arylsilyl of 6 to 30 carbon atoms, a cyano, a nitro, and a halogen,
Ar₅ and Ar₆, which may be same or different, are each independently a substituted or unsubstituted aryl of 6 to 50 carbon atoms or a substituted or unsubstituted heteroaryl of 2 to 50 carbon atoms,
linker L1 is any one selected from among a single bond, a substituted or unsubstituted arylene of 6 to 20 carbon atoms, and a substituted or unsubstituted heteroarylene of 2 to 20 carbon atoms, and
n is an integer of 1 to 2, wherein when n is 2, the corresponding linkers L1's are same or different,
wherein, the term "substituted" in the expression "substituted or unsubstituted" used for compounds of Chemical Formula E means having at least one substituent selected from the group consisting of a deuterium atom, a cyano, a halogen, a hydroxy, a nitro, an alkyl of 1 to 24 carbon atoms, a halogenated alkyl of 1 to 24 carbon atoms, an alkenyl of 2 to 24 carbon atoms, an alkynyl of 2 to 24 carbon atoms, a heteroalkyl of 1 to 24 carbon atoms, an aryl of 6 to 24 carbon atoms, an arylalkyl of 7 to 24 carbon atoms, a heteroaryl of 2 to 24 carbon atoms or a heteroarylalkyl of 2 to 24 carbon atoms, an alkoxy of 1 to 24 carbon atoms, an alkylamino of 1 to 24 carbon atoms, an arylamino of 6 to 24 carbon atoms, a heteroarylamino of 1 to 24 carbon atoms, an alkylsilyl of 1 to 24 carbon atoms, an arylsilyl of 6 to 24 carbon atoms, and an aryloxy of 6 to 24 carbon atoms.

18. The organic light-emitting diode of claim 17, wherein the anthracene derivative represented by Chemical Formula is an anthracene derivative represented by Chemical Formula E-1 or Chemical Formula E-2: 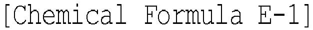 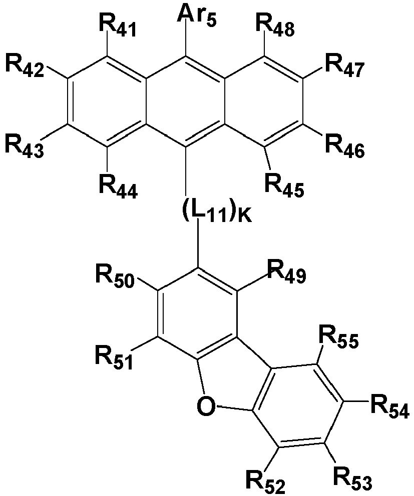 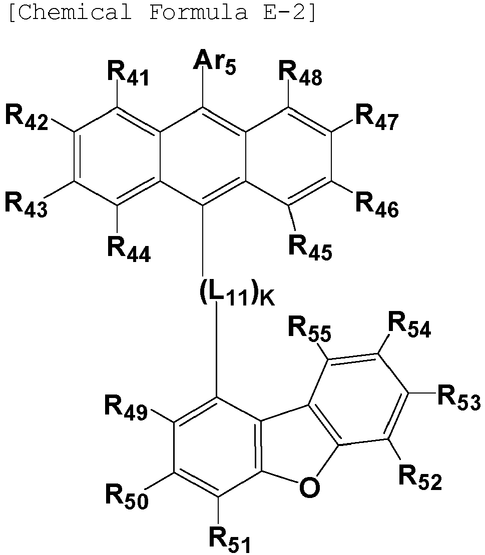 wherein,
R₄₁ to R₄₈ and R₄₉ to R₅₅, which may be same or different, are each independtly any one selected from among a hydrogen atom, a deuterium atom, a substituted or unsubstituted alkyl of 1 to 30 carbon atoms, a substituted or unsubstituted aryl of 6 to 50 carbon atoms, a substituted or unsubstituted cycloalkyl of 3 to 30 carbon atoms, a substituted or unsubstituted heteroaryl of 2 to 50 carbon atoms, a substituted or unsubstituted alkylsilyl of 1 to 30 carbon atoms, a substituted or unsubstituted arylsilyl of 6 to 30 carbon atoms, a cyano, a nitro, and a halogen,
Ar₅ is a substituted or unsubstituted aryl of 6 to 50 carbon atoms or a substituted or unsubstituted heteroaryl of 2 to 50 carbon atoms,
linker L₁₁ is any one selected from among a single bond, a substituted or unsubstituted arylene of 6 to 20 carbon atoms, and a substituted or unsubstituted heteroarylene of 2 to 20 carbon atoms,
k is an integer of 1 to 2 wherein when k is 2, the corresponding L11's are same or different,
wherein, the term "substituted" in the expression "substituted or unsubstituted" used for compounds of Chemical Formula E-1 and E-2 means having at least one substituent selected from the group consisting of a deuterium atom, a cyano, a halogen, a hydroxy, a nitro, an alkyl of 1 to 24 carbon atoms, a halogenated alkyl of 1 to 24 carbon atoms, an alkenyl of 2 to 24 carbon atoms, an alkynyl of 2 to 24 carbon atoms, a heteroalkyl of 1 to 24 carbon atoms, an aryl of 6 to 24 carbon atoms, an arylalkyl of 7 to 24 carbon atoms, a heteroaryl of 2 to 24 carbon atoms or a heteroarylalkyl of 2 to 24 carbon atoms, an alkoxy of 1 to 24 carbon atoms, an alkylamino of 1 to 24 carbon atoms, an arylamino of 6 to 24 carbon atoms, a heteroarylamino of 1 to 24 carbon atoms, an alkylsilyl of 1 to 24 carbon atoms, an arylsilyl of 6 to 24 carbon atoms, and an aryloxy of 6 to 24 carbon atoms.

19. The organic light-emitting diode of claim 17, wherein the first light-emitting layer and the second light-emitting layer each independently comprise as a dopant at least one selected from among compounds represented by Chemical Formulas D1 to D10: 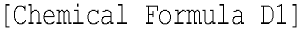 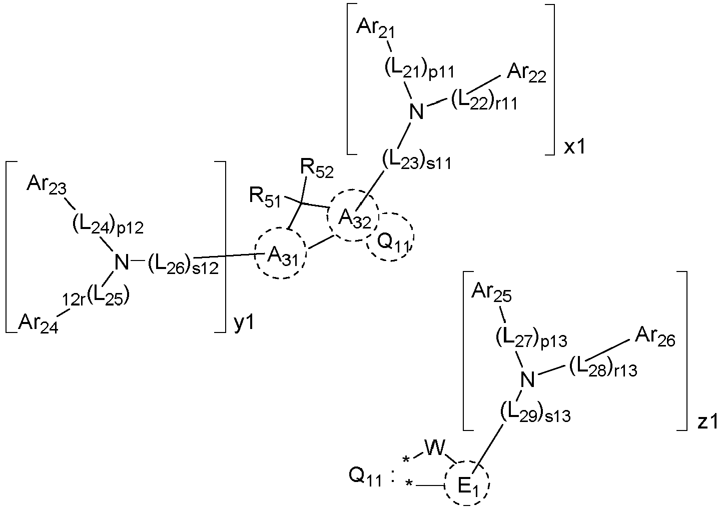 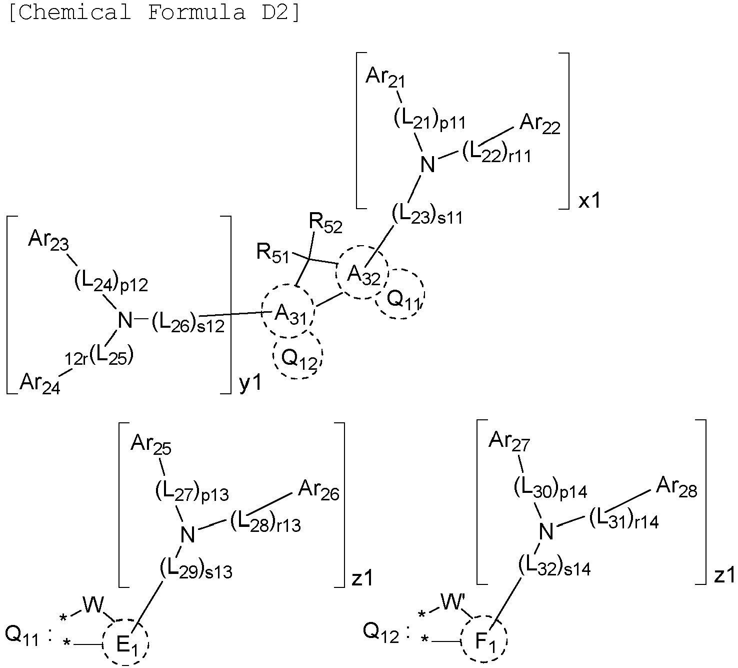 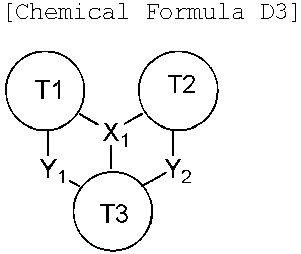 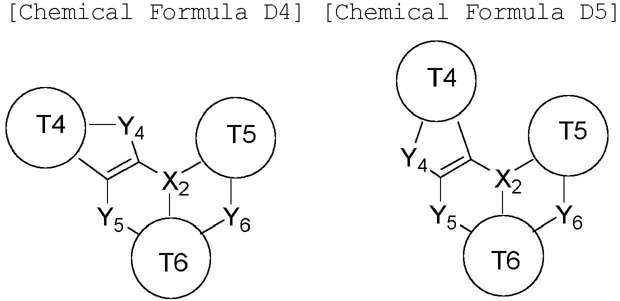 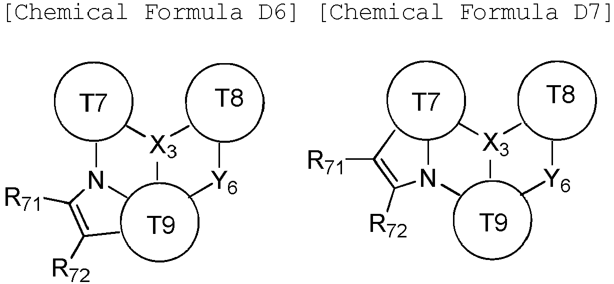 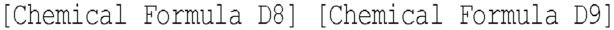 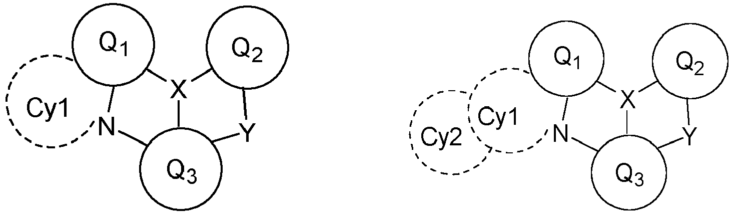 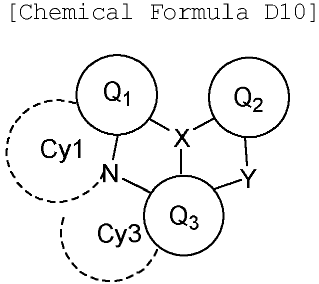 wherein Chemical Formulas D1 to D10 are same as defined claim 13.

20. The organic light-emitting diode of claim 11, wherein at least one selected from among the layers is formed using a deposition process or a solution process.

21. The organic light-emitting diode of claim 10, wherein the organic light-emitting diode is used for a device selected from among a flat display device; a flexible display device; a monochrome or grayscale flat illumination; and a monochrome or grayscale flexible illumination device.
